# EUROPEAN PATENT SPECIFICATION

(11) **EP 1 670 772 B1**
(45) Date of publication and mention of the grant of the patent: **17.08.2011**
(21) Application number: 04765514.7
(22) Date of filing: 21.09.2004
(51) Int. Cl.: C07D 257/04, C07D 271/10, C07D 277/24, C07D 271/06, C07D 213/50, C07D 285/12, C07C 317/08, C07D 249/08, C07D 275/06, A01N 43/713

(54) **DERIVATIVES OF 1,3-DIONES HAVING A HERBICIDAL ACTIVITY**
1,3-DIONDERIVATE MIT HERBIZIDER WIRKUNG
DERIVES DE 1,3-DIONES A ACTIVITE HERBICIDE

(30) Priority: 29.09.2003 IT MI20031855
(43) Date of publication of application: 21.06.2006
(73) Proprietor: Isagro Ricerca S.r.l., 20153 Milano (IT)
(72) Inventor: MEAZZA, Giovanni, I-21047 Saronno (Varese) (IT); PARAVIDINO, Piero, I-20018 Sedriano Milano (IT); BETTARINI, Franco, I-28100 Novara (IT); FORGIA, Daniele, I-28060 Casaleggio (Novara) (IT); FORNARA, Luca, I-20070 Cerro al Lambro, Milano (IT)
(74) Representative: Coletti, Raimondo
(86) International application number: PCT/EP2004/010653
(87) International publication number: WO 2005/030736

(56) References cited:
- PL-B- 171 529
- HARRIS R L N ET AL: "THE EFFECT OF C-2-CARBOXYPHENYL DERIVATIVES OF CERTAIN HETEROCYCLIC COMPOUNDS ON ROOT GEOTROPISM IN CRESS SEEDLINGS" PESTICIDE SCIENCE, ELSEVIER APPLIED SCIENCE PUBLISHER. BARKING, GB, vol. 11, no. 4, 1980, pages 439-444, XP009019048 ISSN: 0031-613X
- SAWICKI E ET AL: "REACTION OF THIAXANTHYDROL WITH COMPOUNDS CONTAINING ACTIVE HYDROGEN" JOURNAL OF ORGANIC CHEMISTRY, AMERICAN CHEMICAL SOCIETY. EASTON, US, vol. 21, February 1956 (1956-02), pages 183-189, XP002063988 ISSN: 0022-3263
- BAUMES R ET AL: "SUR UNE PRETENDUE FORME NH STABLE D'UNE PYRAZOLINE-3" JOURNAL OF HETEROCYCLIC CHEMISTRY, HETEROCORPORATION. PROVO, US, vol. 10, no. 5, October 1973 (1973-10), pages 763-767, XP002032956 ISSN: 0022-152X
- GHOSH ET AL: "Estrogenic diazenes: heterocyclic non-steroidal estrogens of unusual structure with selectivity for estrogen receptor subtypes" BIOORGANIC & MEDICINAL CHEMISTRY, ELSEVIER SCIENCE LTD, GB, vol. 11, no. 4, 10 December 2002 (2002-12-10), pages 629-657, XP002299673 ISSN: 0968-0896
- PENNING T D ET AL: "3,4- Diarylpyrazoles: potent and selective inhibitors of cyclooxygenase-2" BIOORGANIC & MEDICINAL CHEMISTRY LETTERS, OXFORD, GB, vol. 7, no. 16, 1997, pages 2121-2124, XP002235813 ISSN: 0960-894X
- STAUFFER, S.R. ET AL.: "Estrogen pyrazoles: defining the pyrazole core structure and the orientation of substituents in the ligand binding pocket of the estrogen receptor" BIOORG. MED. CHEM., vol. 9, 2001, pages 141-150, XP002311545
- PATENT ABSTRACTS OF JAPAN vol. 0031, no. 38 (C-064), 16 November 1979 (1979-11-16) -& JP 54 119027 A (UBE IND LTD; others: 01), 14 September 1979 (1979-09-14)
- LLORIS, M.E. ET AL.: "Alkylations of alpha-methyl substituted beta-diketones through their Cu(II) complexes. Preparation of sterically congested beta-diketones" TETRAHEDRON LETTERS, vol. 31, no. 51, 1990, pages 7489-7492, XP002311546
- SHOPPEE C W ET AL: "ACETYLENIC ALCOHOLS DERIVED FROM ALPHA-ALKYLDEOXYANISOINS, AND THE ALPHA-ALKYL-BETA-ETHYNYLSTILBENES" JOURNAL OF THE CHEMICAL SOCIETY, CHEMICAL SOCIETY. LETCHWORTH, GB, 1961, pages 1311-1321, XP009004205 ISSN: 0368-1769

## Description

The present invention relates to derivatives of 1,3-diones having a herbicidal activity.

The invention also relates to processes for the preparation of the above derivatives of 1,3-diones and their use as herbicides for the control of weeds in agricultural crops.

Various derivatives of 1,3-dines substituted in position 1 and 2 by aromatic and/or heteroaromatic groups are described in J. Indian.Chem.Soc. (1961), vol. 38, pages 343-345, J. Org. Chem. (1962), viol. 27, pages 1899-1901 and Tetrahedron (1963), vol. 19, pages 413-418.

A herbicidal activity has never been described for any of these compounds.

Reference is made only to PL-A-171259 disclosing herbicidal use of compounds of a general formula not allowing the R group to be a -C(R²)(R⁶)Ophenyl.

Also in the article "Pesticide Science 1980, 11, pages 439-444", the herbicidal use of compounds (I) is disclosed, said compounds not presenting the cyclic B group.

The Applicant has now surprisingly found that derivatives of 1,3-diones, in which the substituents in position 1 and 2 represent suitably substituted aryl, heteroaryl or heterocyclic groups, have a high herbicidal activity with respect to weeds in crops of agrarian interest.

An object of the present invention therefore relates to derivatives of 1,3-diones having general formula (I): 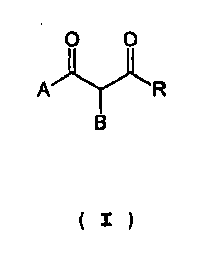 wherein:
- A represents:
   an aryl group optionally substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C6 haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl , C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, - S(O)ₘR₁, -Q, -ZQ₁,
   or it represents a heterocyclic group selected from pyridyl, pyrimidyl, quinolinyl, pyrazolyl, thiazolyl, oxazolyl, thienyl, furyl,
   4,4-dioxide-2,3-dihydro-1,4-benzoxathiin-7-yl,
   with said groups optionally substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁;
- B represents a D-(Rₓ)ₙ group;
- R represents a linear or branched C₁-C₆ haloalkyl group, a C₃-C₆ cycloalkyl or C₄-C₁₂ cycloalkylalkyl group optionally substituted with halogen atoms or C₁-C₆ alkyl;
- R₁ represents a C₁-C₆ alkyl group or a C₁-C₆ haloalkyl group;
- m is equal to 0, 1 or 2;
- t is equal to 1 or 2;
- R₂, R₃, R₆, R₇, R₈, R₉, the same or different, represent a hydrogen atom, a linear or branched C₁-C₆ alkyl group;
- R₄, R₅ represent a hydrogen atom, a linear or branched C₁-C₆ alkyl group in turn optionally substituted with halogen atoms;
- Q, Q₁, represent an aryl a C₃-C₆ cycloalkyl group, a heterocyclic group selected from pyrazolyl, tetrazolyl, tetrazolonyl, isoxazolyl, oxazolyl, thiazolyl, oxadiazolyl, thiadiazolyl, isothiazolyl, isoxazolinyl, 1,3-dioxolanyl, tetrahydropyranyl, oxethanyl, oxyranyl, thiazolidinyl, oxazolidinyl, said groups optionally substituted by one or more substituents selected from halogen, NO₂, OH, CN, CHO, linear or branched C₁-C₆ alkyl, linear or branched C₁-C₆ haloalkyl, linear or branched C₁-C₆ alkoxyl, linear or branched C₁-C₆ haloalkoxyl;
- Z = O, S(O)ᵣ;
- r is equal to 0, 1 or 2;
- D represents:
   a monocyclic heterocyclic group which can be connected to the rest of the structure either through one of its carbon atoms or, when possible, through one of its nitrogen atoms;
- Rₓ represents a substituent selected from hydrogen, halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, , -S(O)ₘR₁, -OS(O)ₜR₁, -SO₂NR₂R₃, -CO₂R₄, -COR₅, -CONR₆R₇, -CSNR₈R₉, -Q; if more than one Rₓ groups are present, they can be the same or different;
- n = 1-9;

A further object of the present invention relates to the use of derivatives of 1,3-diones having general formula (I') 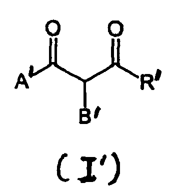 wherein:
- A' represents :
   an aryl group possibly substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl C₂-C₆ alkylthioalkoxyl, C₂-C₆ haloalkylthioalkoxyl, C₃-C₁₂ dialkoxyalkyl, C₃-C₁₂ dialkylthioakyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁, -OS(O)ₜR₁, SO₂NR₂R₃, -CO₂R₄, -COR₅, -CONR₆R₇, -CSNR₈R₉, -Q, -ZQ₁;
   or represents a heterocyclic group selected from pyridyl, pyrimidyl, quinolinyl, pyrazolyl, thiazolyl, oxazolyl, thienyl, furyl, 4,4-dioxide-2,3-dihydro-1,4-benzoxathiin-7-yl,
   with all these groups possibly substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl,
- B' represents a D' (Rₓ)ₙ group;
- R' represents a hydrogen atom, a linear or branched C₁-C₆ alkyl group, a linear or branched C₁-C₆ haloalkyl group, a C₃-C₆ cycloalkyl group or a C₄-C₁₂ cycloalkylalkyl group possibly substituted with halogen atoms or C₁-C₆ alkyl;
- R₁ represents a C₁-C₆ alkyl or C₁-C₆ haloalkyl group;
- m is equal to 0, 1 or 2;
- t is equal to 1 or 2; .
- R₂, R₃, R₆, R₇, R₈, R₉, the same or different, represent a hydrogen atom, a linear or branched C₁-C₆ alkyl group;
- R₄, R₅ represent a hydrogen atom, a linear or branched C₁-C₆ alkyl group in turn possibly substituted with halogen atoms;
- Q, Q₁, represent an aryl group, a C₃-C₆ cycloalkyl group, a heterocyclic group selected from, pyrazolyl, tetrazolyl, tetrazolonyl, isoxazolyl, oxazolyl, thiazolyl, oxadiazolyl, thiadiazolyl, isothiazolyl, isoxazolinyl, 1,3-dioxolanyl, tetrahydropyranyl, oxethanyl, oxyranyl, thiazolidinyl, oxazolidinyl, said groups optionally substituted by one or more substituents selected from halogen, NO₂, CN, CHO, linear or branched C₁-C₆ alkyl, linear or branched C₁-C₆ haloalkyl, linear or branched C₁-C₆ alkoxyl, linear or branched C₁-C₆ haloalkoxyl;
- Z = O, S(O)r;
- r is equal to 0, 1 or 2;
- D' represents:
   a heterocyclic group which can be mono or polycyclic and can be connected to the rest of the structure either through one of its carbon atoms or, when possible, through one of its nitrogen atoms;
- Rₓ represents a substituent selected from hydrogen, halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ alkylthioalkoxyl, C₂-C₆ haloalkylthioalkoxyl, C₃-C₁₂ dialkoxyalkyl, C₃-C₁₂ dialkyl thioalkyl, C₃-Cₗ₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁, -OS(O)ₜR₁, -SO₂NR₂R₃, -CO₂R₄, -COR₅, -CONR₆R₇, -CSNR₈R₉, -Q; if more than one Rₓ groups are present, they can be the same or different;
- n = 1-9;
and of their agronomically compatible salts , as herbicides.

Examples of D' groups include: pyrrolyl, pyrrolidinonyl, thienyl, furyl, pyrazolyl, imidazolyl, imidazolidinonyl, triazolyl, triazolonyl, tetrazolyl, tetrazolonyl, thiazolyl, isothiazolyl, dithiol, oxathiol, isoxazolyl, isoxazolinyl, oxazolyl, oxadiazolyl, thiadiazolyl, oxatriazolyl, dioxazolyl, oxathiazolyl, pyridyl, N-oxidopyridyl, pyrimidyl, pyrimidinonyl, pyridazinyl, pyrazinyl, triazinyl, tetrazinyl, piperazinyl, oxazinyl, oxathiazinyl, morfolinyl, benzofuranyl, isobenzofuranyl, benzothienyl, isobenzothienyl, indolyl, isoindolyl, benzoxazolyl, benzothiazolyl, benzimidazolyl, benzopyrazolyl, benzotriazolyl, benzoxadiazolyl, benzothiadiazolyl, quinolinyl, quinazolinyl, quinoxalinyl, pyridopyrimidinyl, oxazolepyridinyl, chromenyl, thiochromenyl, purine.

A C₁-C₆ alkyl group means a linear or branched C₁-C₆ alkyl group.

Examples of these groups are: methyl, ethyl, propyl, isopropyl, butyl, isobutyl, tert-butyl.

A C₁-C₆ haloalkyl group means a linear or branched C₁-C₆ alkyl group, substituted with one or more halogen atoms, the same or different.

Examples of this group are: fluoromethyl, chlorodifluoromethyl, difluoromethyl, trifluoromethyl, dichloromethyl, trichloromethyl, 2,2,2-trifluoroethyl, 2,2,2-trichloroethyl, 1,1,2,2,2-pentafluoroethyl, 1,1,2,2-tetrafluoroethyl, 1,2,2,2-tetrafluoroethyl, 2,2,3,3-tetrafluoropropyl, 2,2,3,3,3-pentafluoropropyl.

A C₂-C₆ alkenyl group means a linear or branched C₂-C₆ alkenyl group.
Examples of this group are: ethenyl, propenyl, butenyl.

A C₂-C₆ haloalkenyl group means a linear or branched C₂-C₆ alkenyl group, substituted by one or more halogen atoms, the same or different.

Example of this group are: 3,3-dichloroprop-2-enyl, 3,3-difluoroprop-2-enyl, 3,3,3-trifluoropropenyl.

Halogen atom means a halogen atom selected from fluorine, chlorine, bromine or iodine.

A C₃-C₆ cycloalkyl group means a cycloalkyl group consisting of 3 to 6 carbon atoms, possibly substituted by one or more substituents the same or different.

Examples of this group are: cyclopropyl, cyclopentyl.

Examples of alkoxy groups are: methoxy, ethoxy.

Examples of haloalkoxyl groups are: difluoromethoxy, trifluoromethoxy, 1,1,2,2-tetrafluoroethoxy, 1,1,2,3,3,3-hexafluoropropoxy.

A mono or polycyclic heterocyclic group, means a ring which can be unsaturated, partially saturated or completely saturated, and can consist of from three to eighteen units containing at least one heteroatom selected from nitrogen, oxygen and sulphur; this group can be condensed with other rings of the heterocyclic or carbocyclic type, which, in turn, can be of the aromatic type, partially saturated or completely saturated.

The compounds having general formula (I') can exist in different tautomeric forms, as shown hereinafter:

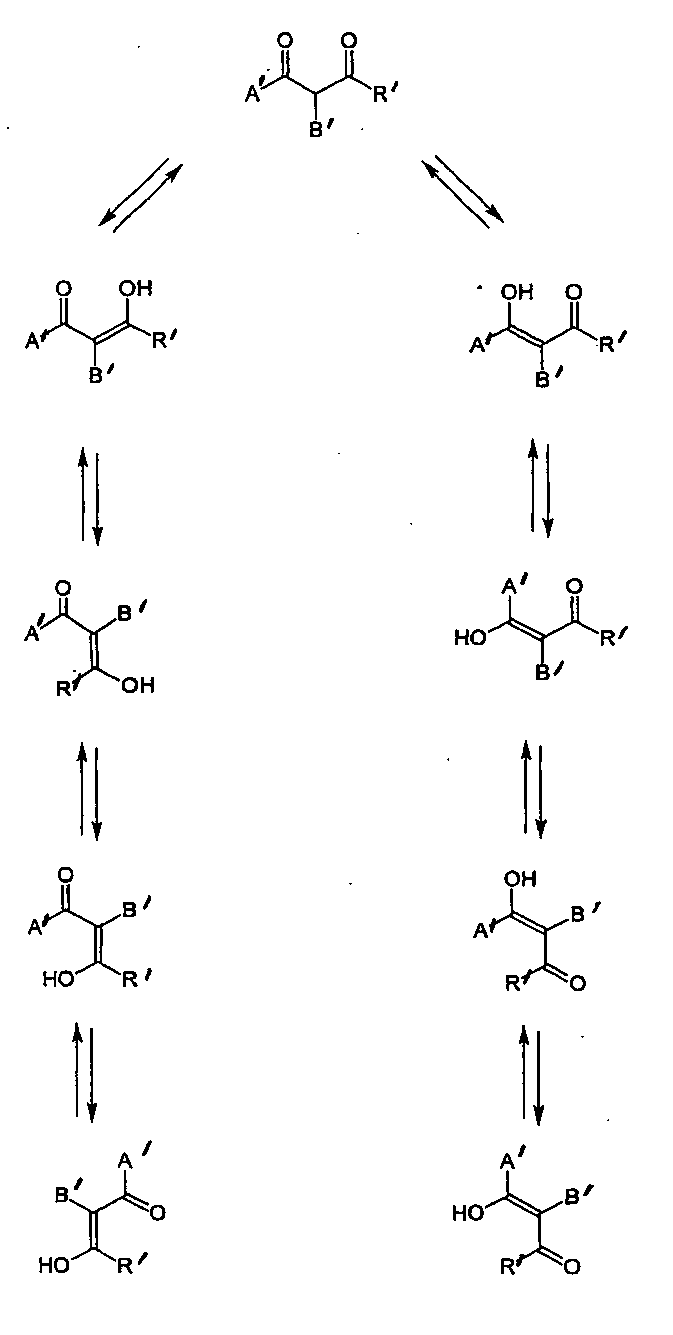

The tautomeric and/or isomeric forms of compounds (I') and the mixtures of the same in any possible proportions, are considered included in the present patent application.

If the particular groups A', B' and R' allow the existence of other tautomeric forms, these forms are definitely included within the scope of the present invention.

The salts of compounds (I') which have agronomical compatibility are also considered within the spirit of this patent.

As stated before, the derivatives of 1,3-diones having general formula (I') have a high herbicidal activity.

Specific examples of compounds having general formula (I') of interest for their activity are shown in table 1:

| | | |
|---|---|---|
| 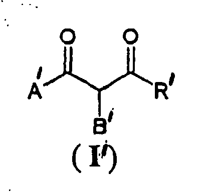 | | |

| **A'** | **B'** | **R'** |
|---|---|---|
| 2-NO₂-4-SO₂MePh | 1,2,4-oxadiazol-5-yl | H |
| 2-NO₂-4-SO₂MePh | 1,2,4-oxadiazol-5-yl | methyl |
| 2-NO₂-4-SO₂MePh | 1,2,4-oxadiazol-5-yl | i-propyl |
| 2-NO₂-4-SO₂MePh | 1,2,4-oxadiazol-5-yl | cyclopropyl |
| 2-NO₂-4-SO₂MePh | 1,2,4-oxadiazol-5-yl | CF₃ |
| 2-NO₂-4-SO₂MePh | 3-methyl-1,2,4-oxadiazol-5-yl | H |
| 2-NO₂-4-SO₂MePh | 3-methyl-1,2,4-oxadiazol-5-yl | methyl |
| 2-NO₂-4-SO₂MePh | 3-methyl-1,2,4-oxadiazol-5-yl | i-propyl |
| 2-NO₂-4-SO₂MePh | 3-methyl-1,2,4-oxadiazol-5-yl | cyclopropyl |
| 2-NO₂-4-SO₂MePh | 3-methyl-1,2,4-oxadiazol-5-yl | CF₃ |
| 2-NO₂-4-SO₂MePh | 3-trifluoromethyl-1,2,4-oxadiazol-5-yl | H |
| 2-NO₂-4-SO₂MePh | 3-trifluoromethyl-1,2,4-oxadiazol-5-yl | methyl |
| 2-NO₂-4-SO₂MePh | 3-trifluoromethyl-1,2,4-oxadiazol-5-yl | i-propyl |
| 2-NO₂4-SO₂MePh | 3-trifluoromethyl-1,2,4-oxadiazol-5-yl | cyclopropyl |
| 2-NO₂-4-SO₂MePh | 3-trifluoromethyl-1,2,4-oxadiazol-5-yl | CF₃ |
| 2-NO₂-4-SO₂MePh | 1,2,4-oxadiazol-3-yl | H |
| 2-NO₂-4-SO₂MePh | 1,2,4-oxadiazol-3-yl | methyl |
| 2-NO₂-4-SO₂MePh | 1,2,4-oxadiazol-3-yl | i-propyl |
| 2-NO₂-4-SO₂MePh | 1,2,4-oxadiazol-3-yl | cyclopropyl |
| 2-NO₂-4-SO₂MePh | 1,2,4-oxadiazol-3-yl | CF₃ |
| 2-NO₂-4-SO₂MePh | 5-methyl-1,2,4-oxadiazol-3-yl | H |
| 2-NO₂-4-SO₂MePh | 5-methyl-1,2,4-oxadiazol-3-yl | methyl |
| 2-NO₂-4-SO₂MePh | 5-methyl-1,2,4-oxadiazol-3-yl | i-propyl |
| 2-NO₂-4-SO₂MePh | 5-methyl-1,2,4-oxadiazol-3-yl | cyclopropyl |
| 2-NO₂-4-SO₂MePh | 5-methyl-1,2,4-oxadiazol-3-yl | CF₃ |
| 2-NO₂-4-SO₂MePh | 5-trifluoromethyl-1,2,4-oxadiazol-3-yl | H |
| 2-NO₂-4-SO₂MePh | 5-trifluoromethyl-1,2,4-oxadiazol-3-yl | methyl |
| 2-NO₂-4-SO₂MePh | 5-trifluoromethyl-1,2,4-oxadiazol-3-yl | i-propyl |
| 2-NO₂-4-SO₂MePh | 5-trifluoromethyl-1,2,4-oxadiazol-3-yl | cyclopropyl |
| 2-NO₂-4-SO₂MePh | 5-trifluoromethyl-1,2,4-oxadiazol-3-yl | CF₃ |
| 2-NO₂-4-SO₂MePh | 5-chloro-1,2,4-oxadiazol-3-yl | H |
| 2-NO₂-4-SO₂MePh | 5-chloro-1,2,4-oxadiazol-3-yl | methyl |
| 2-NO₂-4-SO₂MePh | 5-chloro-1,2,4-oxadiazol-3-yl | i-propyl |
| 2-NO₂-4-SO₂MePh | 5-chloro-1,2,4-oxadiazol-3-yl | cyclopropyl |
| 2-NO₂-4-SO₂MePh | 5-chloro-1,2,4-oxadiazol-3-yl | CF₃ |
| 2-NO₂-4-SO₂MePh | 1,3,4-oxadiazol-2-yl | H |
| 2-NO₂-4-SO₂MePh | 1,3,4-oxadiazol-2-yl | methyl |
| 2-NO₂-4-SO₂MePh | 1,3,4-oxadiazol-2-yl | i-propyl |
| 2-NO₂-4-SO₂MePh | 1, 3,4-oxadiazol-2-yl | cyclopropyl |
| 2-NO₂-4-SO₂MePh | 1,3,4-oxadiazol-2-yl | CF₃ |
| 2-NO₂-4-SO₂MePh | 5-methylsulfonyl-1,3,4-oxadiazol-2-yl | H |
| 2-NO₂-4-SO₂MePh | 5-methylsulfonyl-1,3,4-oxadiazol-2-yl | methyl |
| 2-NO₂-4-SO₂MePh | 5-methylsulfonyl-1,3,4-oxadiazol-2-yl | i-propyl |
| 2-NO₂-4-SO₂MePh | 5-methylsulfonyl-1,3,4-oxadiazol-2-yl | cyclopropyl |
| 2-NO₂-4-SO₂MePh | 5-methylsulfonyl-1,3,4-oxadiazol-2-yl | CF₃ |
| 2-NO₂-4-SO₂MePh | 5-methyl-1,3,4-oxadiazol-2-yl | H |
| 2-NO₂-4-SO₂MePh | 5-methyl-1,3,4-oxadiazol-2-yl | methyl |
| 2-NO₂-4-SO₂MePh | 5-methyl-1,3,4-oxadiazol-2-yl | i-propyl |
| 2-NO₂-4-SO₂MePh | 5-methyl-1,3,4-oxadiazol-2-yl | cyclopropyl |
| 2-NO₂-4-SO₂MePh | 5-methyl-1,3,4-oxadiazol-2-yl | CF₃ |
| 2-NO₂-4-SO₂MePh | 5-trihuoromethyl-1,3,4-oxadiazol-2-yl | H |
| 2-NO₂-4-SO₂MePh | 5-trifluoromethyl-1,3,4-oxadiazol-2-yl | methyl |
| 2-NO₂-4-SO₂MePh | 5-trifluoromethyl-1,3,4-oxadiazol-2-yl | i-propyl |
| 2-NO₂-4-SO₂MePh | 5-trifluoromethyl-1,3,4-oxadiazol-2-yl | cyclopropyl |
| 2-NO₂-4-SO₂MePh | 5-trifluoromethyl-1,3,4-oxadiazol-2-yl | CF₃ |
| 2-NO₂-4-SO₂MePh | 1,2,3-triazol-4-yl | H |
| 2-NO₂-4-SO₂MePh | 1,2,3 triazol-4-yl | methyl |
| 2-NO₂-4-SO₂MePh | 1,2,3-triazol-4-yl | i-propyl |
| 2-NO₂-4-SO₂MePh | 1,2,3 triazol-4-yl | cyclopropyl |
| 2-NO₂-4-SO₂MePh | 1,2,3-tdazol-4yl | CF₃ |
| 2-NO₂-4-SO₂MePh | 1-methyl-1,2,3 triazol-4-yl | H |
| 2-NO₂-4-SO₂MePh | 1-methyl-1,2,3-triazol-4-yl | methyl |
| 2-NO₂-4-SO₂MePh | 1-methyl-1,2,3-triazol-4-yl | i-propyl |
| 2-NO₂-4-SO₂MePh | 1-methyl-1,2,3-triazol-4-yl | cyclopropyl |
| 2-NO₂-4-SO₂MePh | 1-methyl-1,2,3 triazol-4-yl | CF₃ |
| 2-NO₂-4-SO₂MePh | 2-methyl-1,2,3-triazol-4-yl | H |
| 2-NO₂-4-SO₂MePh | 2-methyl-1,2,3-triazol-4-yl | methyl |
| 2-NO₂-4-SO₂MePh | 2-methyl-1,2,3-triazol-4-yl | i-propyl |
| 2-NO₂-4-SO₂MePh | 2-methyl-1,2,3-triazol-4-yl | cyclopropyl |
| 2-NO₂-4-SO₂MePh | 2-methyl-1,2,3-triazol-4-yl | CF₃ |
| 2-NO₂-4-SO₂MePh | 1,2,3-triazol-1-yl | H |
| 2-NO₂-4-SO₂MePh | 1,2,3-triazol-1-yl | methyl |
| 2-NO₂-4-SO₂MePh | 1,2,3-triazol-1-yl | i-propyl |
| 2-NO₂-4-SO₂MePh | 1,2,3-triazol-1-yl | cyclopropyl |
| 2-NO₂-4-SO₂MePh | 1,2,3-triazol-1-yl | CF₃ |
| 2-NO₂-4-SO₂MePh | 1,2,3-triazol-2-yl | H |
| 2-NO₂-4-SO₂MePh | 1,2,3-triazol-2-yl | methyl |
| 2-NO₂-4-SO₂MePh | 1,2,3-triazol-2-yl | i-propyl |
| 2-NO₂-4-SO₂MePh | 1,2,3-triazol-2-yl | cyclopropyl |
| 2-NO₂-4-SO₂MePh | 1,2,3-triazol-2-yl | CF₃ |
| 2-NO₂-4-SO₂MePh | 1,2,4-triazol-1-yl | H |
| 2-NO₂-4-SO₂MePh | 1,2,4-triazol-1-yl | methyl |
| 2-NO₂-4-SO₂MePh | 1,2,4-triazol-1-yl | i-propyl |
| 2-NO₂-4-SO₂MePh | 1,2,4-triazol-1-yl | cyclopropyl |
| 2-NO₂-4-SO₂MePh | 1,2,4-triazol-1-yl | CF₃ |
| 2-NO₂-4-SO₂MePh | imidazol-2-yl | H |
| 2-NO₂-4-SO₂MePh | imidazol-2-yl | methyl |
| 2-NO₂-4-SO₂MePh | imidazol-2-yl | i-propyl |
| 2-NO₂-4-SO₂MePh | imidazol-2-yl | cyclopropyl |
| 2-NO₂-4-SO₂MePh | imidazol-2-yl | CF₃ |
| 2-NO₂-4-SO₂MePh | imidazol-1-yl | H |
| 2-NO₂-4-SO₂MePh | imidazol-1-yl | methyl |
| 2-NO₂-4-SO₂MePh | imidazol-1-yl | i-propyl |
| 2-NO₂-4-SO₂MePh | imidazol-1-yl | cyclopropyl |
| 2-NO₂-4-SO₂MePh | imidazol-1-yl | CF₃ |
| 2-NO₂-4-SO₂MePh | imidazol-4-yl | H |
| 2-NO₂-4-SO₂MePh | imidazol-4-yl | methyl |
| 2-NO₂-4-SO₂MePh | imidazol-4-yl | i-propyl |
| 2-NO₂-4-SO₂MePh | imidazol-4-yl | cyclopropyl |
| 2-NO₂-4-SO₂MePh | imidaaol-4-yl | CF₃ |
| 2-NO₂-4-SO₂MePh | thiazol-2-yl | H |
| 2-NO₂-4-SO₂MePh | thiazol-2-yl | methyl |
| 2-NO₂-4-SO₂MePh | thiazol-2-yl | i-propyl |
| 2-NO₂-4-SO₂MePh | thiazol-2-yl | cyclopropyl |
| 2-NO₂-4-SO₂MePh | thiazol-2-yl | CF₃ |
| 2-NO₂-4-SO₂MePh | 4-methylthiazol-2-yl | H |
| 2-NO₂-4-SO₂MePh | 4-methylthiazol-2-yl | methyl |
| 2-NO₂-4-SO₂MePh | 4-methylthiazol-2-yl | i-propyl |
| 2-NO₂-4-SO₂MePh | 4-methylthiazol-2-yl | cyclopropyl |
| 2-NO₂-4-SO₂MePh | 4-methylthiazol-2-yl | CF₃ |
| 2-NO₂-4-SO₂MePh | oxazol-2-yl | H |
| 2-NO₂-4-SO₂MePh | oxazol-2-yl | methyl |
| 2-NO₂-4-SO₂MePh | oxazol-2-yl | i-propyl |
| 2-NO₂-4-SO₂MePh | oxazol-2-yl | cyclopropyl |
| 2-NO₂-4-SO₂MePh | oxazol-2-yl | CF₃ |
| 2-NO₂-4-SO₂MePh | 4,5-dimethyloxazol-2-yl | H |
| 2-NO₂-4-SO₂MePh | 4,5-dimethyloxazol-2-yl | methyl |
| 2-NO₂-4-SO₂MePh | 4,5-dimethyloxazol-2-yl | i-propyl |
| 2-NO₂-4-SO₂MePh | 4,5-dimethyloxazol-2-yl | cyclopropyl |
| 2-NO₂-4-SO₂MePh | 4,5-dimethyloxazol-2-yl | CF₃ |
| 2-NO₂-4-SO₂MePh | 2-oxazolin-2-yl | H |
| 2-NO₂-4-SO₂MePh | 2-oxazolin-2-yl | methyl |
| 2-NO₂-4-SO₂MePh | 2-oxazolin-2-yl | i-propyl |
| 2-NO₂-4-SO₂MePh | 2-oxazolin-2-yl | cyclopropyl |
| 2-NO₂-4-SO₂MePh | 2-oxazolin-2-yl | CF₃ |
| 2-NO₂-4-SO₂MePh | 4,4-dimethyl-2-oxazolin-2-yl | H |
| 2-NO₂-4-SO₂MePh | 4,4-dimethyl-2-oxazolin-2-yl | methyl |
| 2-NO₂-4-SO₂MePh | 4,4-dimethyl-2-oxazolin-2-yl | i-propyl |
| 2-NO₂-4-SO₂MePh | 4,4-dimethyl-2-oxazolin-2-yl | cyclopropyl |
| 2-NO₂-4-SO₂MePh | 4,4-dimethyl-2-oxazolin-2-yl | CF₃ |
| 2-NO₂-4-SO₂MePh | 1,2,4-thiadiazol-5-yl | H |
| 2-NO₂-4-SO₂MePh | 1,2,4-thiadiazol-5-yl | methyl |
| 2-NO₂-4-SO₂MePh | 1,2,4-thiadiazol-5-yl | i-propyl |
| 2-NO₂-4-SO₂MePh | 1,2,4-thiadiazol-5-yl | cyclopropyl |
| 2-NO₂-4-SO₂MePh | 1,2,4-thiadiazol-5-yl | CF₃ |
| 2-NO₂-4-SO₂MePh | 3-methyl-1,2,4-thiadiazol-5-yl | H |
| 2-NO₂-4-SO₂MePh | 3-methyl-1,2,4-thiadiazol-5-yl | methyl |
| 2-NO₂-4-SO₂MePh | 3-methyl-1,2,4-thiadiazol-5-yl | i-propyl |
| 2-NO₂-4-SO₂MePh | 3-methyl-1,2,4-thiadiazol-5-yl | cyclopropyl |
| 2-NO₂-4-SO₂MePh | 3-methyl-1,2,4-thiadiazol-5-yl | CF₃ |
| 2-NO₂-4-SO₂MePh | 3-trifluoromethyl-1,2,4-tMadiazol-5-yl | H |
| 2-NO₂-4-SO₂MePh | 3-trifluoromethyl-1,2,4-thiadiazol-5-yl | methyl |
| 2-NO₂-4-SO₂MePh | 3-trifluoromethyl-1,2,4-thiadiazol-5-yl | i-propyl |
| 2-NO₂-4-SO₂MePh | 3-trifluoromethyl-1,2,4-thiadiazol-5-yl | cyclopropyl |
| 2-NO₂-4-SO₂MePh | 3-trifluoromethyl-1,2,4-thiadiazol-5-yl | CF₃ |
| 2-NO₂-4-SO₂MePh | 1,2,4-thiadiazol-3-yl | H |
| 2-NO₂-4-SO₂MePh | 1,2,4-thiadiazol-3-yl | methyl |
| 2-NO₂-4-SO₂MePh | 1,2,4-thiadiazol-3-yl | i-propy, |
| 2-NO₂-4-SO₂MePh | 1,2,4-thiadiaol-3-yl | cyclopropyl |
| 2-NO₂-4-SO₂MePh | 1,2,4-tbiadiazol-3-yl | CF₃ |
| 2-NO₂-4-SO₂MePh | 5-methyl-1,2,4-thiadiazol-3-yl | H |
| 2-NO₂-4-SO₂MePh | 5-methyl-1,2,4-thiadiazol-3-yl | methyl |
| 2-NO₂-4-SO₂MePh | 5-methyl-1,2,4-thiadiazol-3-yl | i-propyl |
| 2-NO₂-4-SO₂MePh | 5-methyl-1,2,4-thiadiazol-3-yl | cyclopropyl |
| 2-NO₂-4-SO₂MePh | 5-methyl-1,2,4-thiadiazol-3-yl | CF₃ |
| 2-NO₂-4-SO₂MePh | 5-trifluoromethyl-1,2,4-thiadiazol-3-yl | H |
| 2-NO₂-4-SO₂MePh | 5-trifluoromethyl-1,2,4-thiadiazol-3-yl | methyl |
| 2-NO₂-4-SO₂MePh | 5-trifluoromethyl-1,2,4-thiadiazol-3-yl | i-propyl |
| 2-NO₂-4-SO₂MePh | 5-trifluoromethyl-1,2,4-thiadiazol-3-yl | cyclopropyl |
| 2-NO₂-4-SO₂MePh | 5-trifluoromethyl-1,2,4-thiadiazol-3-yl | CF₃ |
| 2-NO₂-4-SO₂MePh | 1,3,4-thiadiazol-2-yl | H |
| 2-NO₂-4-SO₂MePh | 1,3,4-thiadiazol-2-yl | methyl |
| 2-NO₂-4-SO₂MePh | 1,3,4-thiadiazol-2-yl | i-propyl |
| 2-NO₂-4-SO₂MePh | 1,3,4-thiadiazol-2-yl | cyclopropyl |
| 2-NO₂-4-SO₂MePh | 1,3,4-thiadiazol-2-yl | CF₃ |
| 2-NO₂-4-SO₂MePh | 5-methylsulfonyl-1,3,4-thiadiazol-2-yl | H |
| 2-NO₂-4-SO₂MePh | S-methylsulfonyl-1,3,4-thiadiazol-2-yl | methyl |
| 2-NO₂-4-SO₂MePh | 5-methylsulfonyl-1,3,4-thiadiazol-2-yl | i-propyl |
| 2-NO₂-4-SO₂MePh | 5-methylsulfonyl-1,3,4-thiadiazol-2-yl | cyclopropyl |
| 2-NO₂-4-SO₂MePh | 5-methylsulfonyl-1,3,4-thiadiazol-2-yl | CF₃ |
| 2-NO₂-4-SO₂MePh | 5-methyl-1,3,4-thiadiazol-2-yl | H |
| 2-NO₂-4-SO₂MePh | 5-methyl-1,3,4-thiadiazol-2-yl | methyl |
| 2-NO₂-4-SO₂MePh | 5-methyl-1,3,4-thiadiazol-2-yl | i-propyl |
| 2-NO₂-4-SO₂MePh | 5-methyl-1,3,4-thiadiazol-2-yl | cyclopropyl |
| 2-NO₂-4-SO₂MePh | 5-methyl-1,3,4-thiadiazol-2-yl | CF₃ |
| 2-NO₂-4-SO₂MePh | benzoxazol-2-yl | H |
| 2-NO₂-4-SO₂MePh | benzoxazol-2-yl | methyl |
| 2-NO₂-4-SO₂MePh | benzoxazol-2-yl | i-propyl |
| 2-NO₂-4-SO₂MePh | benzoxazol-2-yl | cyclopropyl |
| 2-NO₂-4-SO₂MePh | benzoxazol-2-yl | CF₃ |
| 2-NO₂-4-SO₂MePh | 6-methylbenzoxazol-2-yl | H |
| 2-NO₂-4-SO₂MePh | 6-methylbenzoxazol-2-yl | methyl |
| 2-NO₂-4-SO₂MePh | 6-methylbenzoxazol-2-yl | i-propyl |
| 2-NO₂-4-SO₂MePh | 6-methylbenzoxazol-2-yl | cyclopropyl |
| 2-NO₂-4-SO₂MePh | 6-methylbenzoxazol-2-yl | CF₃ |
| 2-NO₂-4-SO₂MePh | benzothiazol-2-yl | H |
| 2-NO₂-4-SO₂MePh | benzothiazol-2-yl | methyl |
| 2-NO₂-4-SO₂MePh | benzothiazol-2-yl | i-propyl |
| 2-NO₂-4-SO₂MePh | benzothiazol-2-yl | cyclopropyl |
| 2-NO₂-4-SO₂MePh | benzothiazol-2-yl | CF₃ |
| 2-NO₂-4-SO₂MePh | pyrazol-1-yl | H |
| 2-NO₂-4-SO₂MePh | pyrazol-1-yl | methyl |
| 2-NO₂-4-SO₂MePh | pyrazol-1-yl | i-propyl |
| 2-NO₂-4-SO₂MePh | pyrazol-1-yl | cyclopropyl |
| 2-NO₂-4-SO₂MePh | pyrazol-1-y | CF₃ |
| 2-NO₂-4-SO₂MePh | pyrazol-3-yl | H |
| 2-NO₂-4-SO₂MePh | pyrazol-3-yl | methyl |
| 2-NO₂-4-SO₂MePh | pyrazol-3-yl | i-propyl |
| 2-NO₂-4-SO₂MePh | pyrazol-3-yl | cyclopropyl |
| 2-NO₂-4-SO₂MePh | pyrazol-3-yl | CF₃ |
| 2-NO₂-4-SO₂MePh | 1-methylpyrazol-3-yl | H |
| 2-NO₂-4-SO₂MePh | 1-methylpyrazol-3-yl | methyl |
| 2-NO₂-4-SO₂MePh | 1-methylpyrazol-3-yl | i-propyl |
| 2-NO₂-4-SO₂MePh | 1-methylpyrazol-3-yl | cyclopropyl |
| 2-NO₂-4-SO₂MePh | 1-methylpyrazol-3-yl | CF₃ |
| 2-NO₂-4-SO₂MePh | tetrazol-1-yl | H |
| 2-NO₂-4-SO₂MePh | tetrazol-1-yl | methyl |
| 2-NO₂-4-SO₂MePh | tetraz,ol-1-yl | i-propyl |
| 2-NO₂-4-SO₂MePh | tetrazol-1-yl | cyclopropyl |
| 2-NO₂-4-SO₂MePh | tetrazol-1-yl | CF₃ |
| 2-NO₂-4-SO₂MePh | 5-methyltetrazol-1-yl | H |
| 2-NO₂-4-SO₂MePh | 5-methyltetrazol-1-yl | methyl |
| 2-NO₂-4-SO₂MePh | 5-methyltetrazol-1-yl | i-propyl |
| 2-NO₂-4-SO₂MePh | 5-methyltetrazol-1-yl | cyclopropyl |
| 2-NO₂-4-SO₂MePh | 5-methyltetrazol-1-yl | CF₃ |
| 2-NO₂-4-SO₂MePh | tetrazol-2-yl | H |
| 2-NO₂-4-SO₂MePh | tetrazol-2-yl | methyl |
| 2-NO₂-4-SO₂MePh | tetrazol-2-yl | i-propyl |
| 2-NO₂-4-SO₂MePh | tetrazol-2-yl | cyclopropyl |
| 2-NO₂-4-SO₂MePh | tetrazol-2-yl | CF₃ |
| 2-NO₂-4-SO₂MePh | 5-methyltetrazol-2-yl | H |
| 2-NO₂-4-SO₂MePh | 5-methyltetrazol-2-yl | methyl |
| 2-NO₂-4-SO₂MePh | 5-methyltetrazol-2-yl | i-propyl |
| 2-NO₂-4-SO₂MePh | 5-methyltetrazol-2-yl | cyclopropyl |
| 2-NO₂-4-SO₂MePh | 5-methyltetrazol-2-yl | CF₃ |
| 2-NO₂-4-SO₂MePh | 1-methyltetrazol-5-yl | H |
| 2-NO₂-4-SO₂MePh | 1-methyltetrazol-5-yl | methyl |
| 2-NO₂-4-SO₂MePh | 1-methyltetrazol-5-yl | i-propyl |
| 2-NO₂4-SO₂MePh | 1-methyttetrazol-5-yl | cyclopropyl |
| 2-NO₂-4-SO₂MePh | 1-methyltetrazol-5-yl | CF₃ |
| 2-NO₂-4-SO₂MePh | 2-methyltetrazol-5-yl | H |
| 2-NO₂-4-SO₂MePh | 2-methyltetrazol-5-yl | methyl |
| 2-NO₂4-SO₂MePh | 2-methyltetrazol-5-yl | i-propyl |
| 2-NO₂4-SO₂MePh | 2-methyltetrazol-5-yl | cyclopropyl |
| 2-NO₂4-SO₂MePh | 2-methyltetrazol-5-yl | CF₃ |
| 2-NO₂-4-SO₂MePh | pyridin-2-yl | H |
| 2-NO₂-4-SO₂MePh | pyridin-2-yl | methyl |
| 2-NO₂-4-SO₂MePh | pyridin-2-yl | i-propyl |
| 2-NO₂-4-SO₂MePh | pyridin-2-yl | cyclopropyl |
| 2-NO₂-4-SO₂MePh | pyridin-2-yl | CF₃ |
| 2-NO₂-4-SO₂MePh | pyridin-4-yl | H |
| 2-NO₂-4-SO₂MePh | pyridin-4-yl | methyl |
| 2-NO₂-4-SO₂MePh | pyridin-4-yl | i-propyl |
| 2-NO₂-4-SO₂MePh | pyndin-4-yl | cyclopropyl |
| 2-NO₂-4-SO₂MePh | pyridin-4-yl | CF₃ |
| 2-NO₂-4-SO₂MePh | pyridin-3-yl | H |
| 2-NO₂-4-SO₂MePh | pyridin-3-yl | methyl |
| 2-NO₂-4-SO₂MePh | pyridin-3-yl | i-propyl |
| 2-NO₂-4-SO₂MePh | pyridin-3-yl | cyclopropyl |
| 2-NO₂-4-SO₂MePh | pyridin-3-yl | CF₃ |
| 2-NO₂-4-SO₂MePh | 3-nitropyridin-4-yl | H |
| 2-NO₂-4-SO₂MePh | 3-nitropyridin-4-yl | methyl |
| 2-NO₂-4-SO₂MePh | 3-nitropyridin-4-yl | i-propyl |
| 2-NO₂-4-SO₂MePh | 3-nitropyridin-4-yl | cyclopropyl |
| 2-NO₂-4-SO₂MePh | 3-nitropyridin-4-yl | CF₃ |
| 2-NO₂-4-SO₂MePh | 5-cyanopyridin-2-yl | H |
| 2-NO₂-4-SO₂MePh | 5-cyanopyridin-2-yl | methyl |
| 2-NO₂-4-SO₂MePh | 5-cyanopyridin-2-yl | i-propyl |
| 2-NO₂-4-SO₂MePh | 5-cyanopyridin-2-yl | cyclopropyl |
| 2-NO₂-4-SO₂MePh | 5-cyanopyridin-2-yl | CF₃ |
| 2-NO₂-4-SO₂MePh | 5-trifluoromethylpyridin-2-yl | H |
| 2-NO₂-4-SO₂MePh | 5-trifluoromethylpyridin-2-yl | methyl |
| 2-NO₂-4-SO₂MePh | 5-trifluoromethylpyridin-2-yl | i-propyl |
| 2-NO₂-4-SO₂MePh | 5-trifluoromethylpyridin-2-yl | cyclopropyl |
| 2-NO₂-4-SO₂MePh | 5-trifluoromethylpyridin-2-yl | CF₃ |
| 2-NO₂-4-SO₂MePh | pyrimidin-2-yl | H |
| 2-NO₂-4-SO₂MePh | pyrimidin-2-yl | methyl |
| 2-NO₂-4-SO₂MePh | pyrimidin-2-yl | i-propyl |
| 2-NO₂-4-SO₂MePh | pyrimidin-2-yl | cyclopropyl |
| 2-NO₂-4-SO₂MePh | pyrimidin-2-yl | CF₃ |
| 2-NO₂-4-SO₂MePh | pyrimidin-4-yl | H |
| 2-NO₂-4-SO₂MePh | pyrimidin-4-yl | methyl |
| 2-NO₂-4-SO₂MePh | pyrimidin-4-yl | i-propyl |
| 2-NO₂-4-SO₂MePh | pyrimidin-4-yl | cyclopropyl |
| 2-NO₂-4-SO₂MePh | pyrimidin-4-yl | CF₃ |
| 2-NO₂-4-SO₂MePh | 6-chloropyrimidin-4-yl | methyl |
| 2-NO₂-4-SO₂MePh | 6-chloropyrimidin-4-yl | i-propyl |
| 2-NO₂-4-SO₂MePh | 6-chloropyrimidin-4-yl | cyclopropyl |
| 2-NO₂-4-SO₂MePh | 6-chloropyrimidin-4-yl | CF₃ |
| 2-NO₂-4-SO₂MePh | pyndazin-3-yl | H |
| 2-NO₂-4-SO₂MePh | pyridazin 3-yl | methyl |
| 2-NO₂-4-SO₂MePh | pyridazin-3-yl | i-propyl |
| 2-NO₂-4-SO₂MePh | pyridain-3-yl | cyclopropyl |
| 2-NO₂-4-SO₂MePh | pyridazin-3-yl | CF₃ |
| 2-NO₂-4-SO₂MePh | 6-chloropyridazin-3-yl | methyl |
| 2-NO₂-4-SO₂MePh | 6-chloropyridazin-3-yl | i-propyl |
| 2-NO₂-4-SO₂MePh | 6-chlompyridazin-3-yl | cyclopropyl |
| 2-NO₂-4-SO₂MePh | 6-chloropyridazin-3-yl | CF₃ |
| 2-NO₂-4-SO₂MePh | pyrazin-2-yl | methyl |
| 2-NO₂-4-SO₂MePh | pyrazin-2-yl | i-propyl |
| 2-NO₂-4-SO₂MePh | pyrazin-2-yl | cyclopropyl |
| 2-NO₂-4-SO₂MePh | pyrazin-2-yl | CF₃ |
| 2-NO₂-4-SO₂MePh | triazin-2-yl | methyl |
| 2-NO₂-4-SO₂MePh | triazin-2-yl | i-propyl |
| 2-NO₂-4-SO₂MePh | triazin-2-yl | cyclopropyl |
| 2-NO₂-4-SO₂MePh | triazin-2-yl | CF₃ |
| 2-NO₂-4-SO₂MePh | quinolin-2-yl | methyl |
| 2-NO₂-4-SO₂MePh | quinolin-2-yl | i-propyl |
| 2-NO₂-4-SO₂MePh | quinolin-2-yl | cyclopropyl |
| 2-NO₂-4-SO₂MePh | quinolin-2-yl | CF₃ |
| 2-NO₂-4-SO₂MePh | 4,4,trimethyl-5,6-dihydro-1,3(4H)-oxazin-2-yl | H |
| 2-NO₂-4-SO₂MePh | 4,4,trimethyl-5,6-dihydro-1,3(4H)-oxazin-2-yl | methyl |
| 2-NO₂-4-SO₂MePh | 4,4,trimethyl-5,6-dihydro-1,3(4H)-oxazin-2-yl | i-propyl |
| 2-NO₂-4-SO₂MePh | 4,4,trimethyl-5,6-dihydro-1,3(4H)-oxazin-2-yl | cyclopropyl |
| 2-NO₂-4-SO₂MePh | 4,4,trimethyl-5,6-dihydro-1,3(4H)-oxazin-2-yl | CF₃ |
| 2-NO₂-4-SO₂MePh | 2-oxazolidinon-3-yl | H |
| 2-NO₂-4-SO₂MePh | 2-oxazolidinon-3-yl | methyl |
| 2-NO₂-4-SO₂MePh | 2-oxazolidinon-3-yl | i-propyl |
| 2-NO₂-4-SO₂MePh | 2-oxazolidinon-3-yl | cyclopropyl |
| 2-NO₂-4-SO₂MePh | 2-oxazolidinon-3-yl | CF₃ |
| 2-NO₂-4-SO₂MePh | 2-pyrrolidinon-1-yl | methyl |
| 2-NO₂-4-SO₂MePh | 2-pyrrolidinon-1-yl | i-propyl |
| 2-NO₂-4-SO₂MePh | 2-pyrrolidinon-1-yl | cyclopropyl |
| 2-NO₂-4-SO₂MePh | 2-pyrrolidinon-1-yl | CF₃ |
| 2-NO₂-4-SO₂MePh | 3-methylisoxazol-5-yl | methyl |
| 2-NO₂-4-SO₂MePh | 3-methylisoxazol-5-yl | i-propyl |
| 2-NO₂-4-SO₂MePh | 3-methylisoxazol-5-yl | cyclopropyl |
| 2-NO₂-4-SO₂MePh | 3-methylisoxazol-5-yl | CF₃ |
| 2-Cl-4-SO₂MePh | 1,2,4-oxadiazol-5-yl | H |
| 2-Cl-4-SO₂MePh | 1,2,4-oxadiazol-5-yl | methyl |
| 2-Cl-4-SO₂MePh | 1,2,4-oxadiazol-5-yl | i-propyl |
| 2-Cl-4-SO₂MePh | 1,2,4-oxadiazol-5-yl | cyclopropyl |
| 2-Cl-4-SO₂MePh | 1,2,4-oxadiazol-5-yl | CF₃ |
| 2-Cl-4-SO₂MePh | 3-methyl-1,2,4-oxadiazol-5-yl | H |
| 2-Cl-4-SO₂MePh | 3-methyl-1,2,4-oxadiazol-5-yl | methyl |
| 2-Cl-4-SO₂MePh | 3-methyl-1,2,4-oxadiazol-5-yl | i-propyl |
| 2-Cl-4-SO₂MePh | 3-methyl-1,2,4-oxadiazol-5-yl | cyclopropyl |
| 2-Cl-4-SO₂MePh | 3-methyl-1,2,4-oxadiazol-5-yl | CF₃ |
| 2-Cl-4-SO₂MePh | 3-trifluoromethyl-1,2,4-oxadiazol-5-yl | H |
| 2-Cl-4-SO₂MePh | 3-trifluoromethyl-1,2,4-oxadiazol-5-yl | methyl |
| 2-Cl-4-SO₂MePh | 3-trifluoromethyl-1,2,4-oxadiazol-5-yl | i-propyl |
| 2-Cl-4-SO₂MePh | 3-trifluoromethyl-1,2,4-oxadiazol-5-yl | cyclopropyl |
| 2-Cl-4-SO₂MePh | 3-trifluoromethyl-1,2,4-oxadiazol-5-yl | CF₃ |
| 2-Cl-4-SO₂MePh | 1,2,4-oxadiazol-3-yl | H |
| 2-Cl-4-SO₂MePh | 1,2,4-oxadiazol-3-yl | methyl |
| 2-Cl-4-SO₂MePh | 1,2,4-oxadiazol-3-yl | i-propyl |
| 2-Cl-4-SO₂MePh | 1,2,4-oxadiazol-3-yl | cyclopropyl |
| 2-Cl-4-SO₂MePh | 1,2,4-oxadiazol-3-yl | CF₃ |
| 2-Cl-4-SO₂MePh | 5-methyl-1,2,4-oxadiazol-3-yl | H |
| 2-Cl-4-SO₂MePh | 5-methyl-1,2,4-oxadiazol-3-yl | methyl |
| 2-Cl-4-SO₂MePh | 5-methyl-1,2,4-oxadiazol-3-yl | i-propyl |
| 2-Cl-4-SO₂MePh | 5-methyl-1,2,4-oxadiazol-3-yl | cyclopropyl |
| 2-Cl-4-SO₂MePh | 5-methyl-1,2,4-oxadiazol-3-yl | CF₃ |
| 2-Cl-4-SO₂MePh | 5-trifluoromethyl-1,2,4-oxadiazol-3-yl | H |
| 2-Cl-4-SO₂MePh | 5-trifluoromethyl-1,2,4-oxadiazol-3-yl | methyl |
| 2-Cl-4-SO₂MePh | 5-trifluoromethyl-1,2,4-oxadiazol-3-yl | i-propyl |
| 2-Cl-4-SO₂MePh | 5-trifluoromethyl-1,2,4-oxadiazol-3-yl | cyclopropyl |
| 2-Cl-4-SO₂MePh | 5-trifluoromethyl-1,2,4-oxadiazol-3-yl | CF₃ |
| 2-Cl-4-SO₂MePh | 5-chloro-1,2,4-oxadiazol-3-yl | H |
| 2-Cl-4-SO₂MePh | 5-chloro-1,2,4-oxadiazol-3-yl | methyl |
| 2-Cl-4-SO₂MePh | 5-chloro-1,2,4-oxadiazol-3-yl | i-propyl |
| 2-Cl-4-SO₂MePh | 5-chloro-1,2,4-oxadiazol-3-yl | cyclopropyl |
| 2-Cl-4-SO₂MePh | 5-chloro-1,2,4-oxadiazol-3-yl | CF₃ |
| 2-Cl-4-SO₂MePh | 1,3,4-oxadiazol-2-yl | H |
| 2-Cl-4-SO₂MePh | 1,3,4-oxadiazol-2-yl | methyl |
| 2-Cl-4-SO₂MePh | 1,3,4-oxadiazol-2-yl | i-propyl |
| 2-Cl-4-SO₂MePh | 1,3,4-oxadiazol-2-yl | cyclopropyl |
| 2-Cl-4-SO₂MePh | 1,3,4-oxadiazol-2-yl | CF₃ |
| 2-Cl-4-SO₂MePh | 5-methylsulfonyl-1,3,4-oxadiazol-2-yl | H |
| 2-Cl-4-SO₂MePh | 5-methylsulfonyl-1,3,4-oxadiazol-2-yl | methyl |
| 2-Cl-4-SO₂MePh | 5-methylsulfonyl-1,3,4-oxadiazol-2-yl | i-propyl |
| 2-Cl-4-SO₂MePh | 5-methylsulfonyl-1,3,4-oxadiazol-2-yl | cyclopropyl |
| 2-Cl-4-SO₂MePh | 5-methylsulfonyl-1,3,4-oxadiazol-2-yi | CF₃ |
| 2-Cl-4-SO₂MePh | 5-methyl-1,3,4-oxadiazol-2-yl | H |
| 2-Cl-4-SO₂MePh | 5-methyl-1,3,4-oxadiazol-2-yl | methyl |
| 2-Cl-4-SO₂MePh | 5-methyl-1,3,4-oxadiazol-2-yl | i-propyl |
| 2-Cl-4-SO₂MePh | 5-methyl-1,3,4-oxadiazol-2-yl | cyclopropyl |
| 2-Cl-4-SO₂MePh | 5-methyl-1,3,4-oxadiazol-2-yl | CF₃ |
| 2-Cl-4-SO₂MePh | 5-trifluoromethyl-1,3,4-oxadiazol-2-yl | H |
| 2-Cl-4-SO₂MePh | 5-trifluoromethyl-1,3,4-oxadiazol-2-yl | methyl |
| 2-Cl-4-SO₂MePh | 5-trifluoromethyl-1,3,4-oxadiazol-2-yl | i-propyl |
| 2-Cl-4-SO₂MePh | 5-trifluoromethyl-1,3,4-oxadiazol-2-yl | cyclopropyl |
| 2-Cl-4-SO₂MePh | 5-trifluoromethyl-1,3,4-oxadiazol-2-yl | CF₃ |
| 2-Cl-4-SO₂MePh | 1,2,3-triazol4-yl | H |
| 2-Cl-4-SO₂MePh | 1,2,3-triazol-4-yl | methyl |
| 2-Cl-4-SO₂MePh | 1,2,3-triazol-4-yl | i-propyl |
| 2-Cl-4-SO₂MePh | 1,2,3-triazol-4-yl | cyclopropyl |
| 2-Cl-4-SO₂MePh | 1,2,3-triazol-4-yl | CF₃ |
| 2-Cl-4-SO₂MePh | 1-methyl-1,2,3-triazol-4-yl | H |
| 2-Cl-4-SO₂MePh | 1-methyl-1,2,3-triazol-4-yl | methyl |
| 2-Cl-4-SO₂MePh | 1-methyl-1,2,3-triazol-4-yl | i-propyl |
| 2-Cl-4-SO₂MePh | 1-methyl-1,2,3-triazol-4-yl | cyclopropyl |
| 2-Cl-4-SO₂MePh | 1-methyl-1,2,3-triazol-4-yl | CF₃ |
| 2-Cl-4-SO₂MePh | 2-methyl-1,2,3-triazol-4-yl | H |
| 2-Cl-4-SO₂MePh | 2-methyl-1,2,3-triazol-4-yl | methyl |
| 2-Cl-4-SO₂MePh | 2-methyl-1,2,3-triazol-4-yl | i-propyl |
| 2-Cl-4-SO₂MePh | 2-methyl-1,2,3-triazol-4-yl | cyclopropyl |
| 2-Cl-4-SO₂MePh | 2-methyl-1,2,3-triazol-4-yl | CF₃ |
| 2-Cl-4-SO₂MePh | 1,2,3-triazol-1-yl | H |
| 2-Cl-4-SO₂MePh | 1,2,3-triazol-1-yl | methyl |
| 2-Cl-4-SO₂MePh | 1,2,3-triazol-1-yl | i-propyl |
| 2-Cl-4-SO₂MePh | 1,2,3-triazol-1-yl | cyclopropyl |
| 2-Cl-4-SO₂MePh | 1,2,3-triazol-1-yl | CF₃ |
| 2-Cl-4-SO₂MePh | 1,2,3-triazol-2-yl | H |
| 2-Cl-4-SO₂MePh | 1,2,3-triazol-2-yl | methyl |
| 2-Cl-4-SO₂MePh | 1,2,3-triazol-2-yl | i-propyl |
| 2-Cl-4-SO₂MePh | 1,2,3-triazol-2-yl | cyclopropyl |
| 2-Cl-4-SO₂MePh | 1,2,3-triazol-2-yl | CF₃ |
| 2-Cl-4-SO₂MePh | 1,2,4-triazol-1-yl | H |
| 2-Cl-4-SO₂MePh | 1,2,4-triazol-1-yl | methyl |
| 2-Cl-4-SO₂MePh | 1,2,4-triazol-1-yl | i-propyl |
| 2-Cl-4-SO₂MePh | 1,2,4-triazol-1-yl | cyclopropyl |
| 2-Cl-4-SO₂MePh | 1,2,4-triazol-1-yl | CF₃ |
| 2-Cl-4-SO₂MePh | imidazol-2-yl | H |
| 2-Cl-4-SO₂MePh | imidazol-2-yl | methyl |
| 2-Cl-4-SO₂MePh | imidazol-2-yl | i-propyl |
| 2-Cl-4-SO₂MePh | imidazol-2-yl | cyclopropyl |
| 2-Cl-4-SO₂MePh | imidazol-2-yl | CF₃ |
| 2-Cl-4-SO₂MePh | imidazol-1-yl | H |
| 2-Cl-4-SO₂MePh | imidazol-1-yl | methyl |
| 2-Cl-4-SO₂MePh | imidazol-1-yl | i-propyl |
| 2-Cl-4-SO₂MePh | imidazol-1-yl | cyclopropyl |
| 2-Cl-4-SO₂MePh | imidazol-1-yl | CF₃ |
| 2-Cl-4-SO₂MePh | imidazol-4-yl | H |
| 2-Cl-4-SO₂MePh | imidazol-4-yl | methyl |
| 2-Cl-4-SO₂MePh | imidazol-4-yl | i-propyl |
| 2-Cl-4-SO₂MePh | imidazol-4-yl | cyclopropyl |
| 2-Cl-4-SO₂MePh | imidazol-4-yl | CF₃ |
| 2-Cl-4-SO₂MePh | thiazol-2-yl | H |
| 2-Cl-4-SO₂MePh | thiazol-2-yl | methyl |
| 2-Cl-4-SO₂MePh | thiazol-2-yl | i-propyl |
| 2-Cl-4-SO₂MePh | thiazol-2-yl | cyclopropyl |
| 2-Cl-4-SO₂MePh | thiazol-2-yl | CF₃ |
| 2-Cl-4-SO₂MePh | 4-methylthiazol-2-yl | H |
| 2-Cl-4-SO₂MePh | 4-methylthiazol-2-yl | methyl |
| 2-Cl-4-SO₂MePh | 4-methylthiazol-2-yl | i-propyl |
| 2-Cl-4-SO₂MePh | 4-methylthiazol-2-yl | cyclopropyl |
| 2-Cl-4-SO₂MePh | 4-methylthiazol-2-yl | CF₃ |
| 2-Cl-4-SO₂MePh | oxazol-2-yl | H |
| 2-Cl-4-SO₂MePh | oxazol-2-yl | methyl |
| 2-Cl-4-SO₂MePh | oxazol-2-yl | i-propyl |
| 2-Cl-4-SO₂MePh | oxazol-2-yl | cyclopropyl |
| 2-Cl-4-SO₂MePh | oxazol-2-yl | CF₃ |
| 2-Cl-4-SO₂MePh | 4,5-dimethyloxazol-2-yl | H |
| 2-Cl-4-SO₂MePh | 4,5-dimethyloxazol-2-yl | methyl |
| 2-Cl-4-SO₂MePh | 4,5-dimethyloxazol-2-yl | i-propyl |
| 2-Cl-4-SO₂MePh | 4,5-dimethyloxazol-2-yl | cyclopropyl |
| 2-Cl-4-SO₂MePh | 4,5-dimethyloxazol-2-yl | CF₃ |
| 2-Cl-4-SO₂MePh | 2-oxazolin-2-yl | H |
| 2-Cl-4-SO₂MePh | 2-oxazolin-2-yl | methyl |
| 2-Cl-4-SO₂MePh | 2-oxazolin-2-yl | i-propyl |
| 2-Cl-4-SO₂MePh | 2-oxazolin-2-yl | cyclopropyl |
| 2-Cl-4-SO₂MePh | 2-oxazolin-2-yl | CF₃ |
| 2-Cl-4-SO₂MePh | 4,4-dimethyl-2-oxazolin-2-yl | H |
| 2-Cl-4-SO₂MePh | 4,4-dimethyl-2-oxazolin-2-yl | methyl |
| 2-Cl-4-SO₂MePh | 4,4-dimethyl-2-oxazolin-2-yl | i-propyl |
| 2-Cl-4-SO₂MePh | 4,4-dimethyl-2-oxazolin-2-yl | cyclopropyl |
| 2-Cl-4-SO₂MePh | 4,4-dimethyl-2-oxazolin-2-yl | CF₃ |
| 2-Cl-4-SO₂MePh | 1,2,4-thiadiazol-5-yl | H |
| 2-Cl-4-SO₂MePh | 1,2,4-thiadiazol-5-yl | methyl |
| 2-Cl-4-SO₂MePh | 1,2,4-thiadiazol-5-yl | i-propyl |
| 2-Cl-4-SO₂MePh | 1,2,4-thiadiazol-5-yl | cyclopropyl |
| 2-Cl-4-SO₂MePh | 1,2,4-thiadiazol-5-yl | CF₃ |
| 2-Cl-4-SO₂MePh | 3-methyl-1,2,4-thiadiazol-5-yl | H |
| 2-Cl-4-SO₂MePh | 3-methyl-l,2,4-thiadiazol-5-yl | methyl |
| 2-Cl-4-SO₂MePh | 3-methyl-1,2,4-thiadiazol-5-yl | i-propyl |
| 2-Cl-4-SO₂MePh | 3-methyl-1,2,4-thiadiazol-5-yl | cyclopropyl |
| 2-Cl-4-SO₂MePh | 3-mdhyl-1,2,4-thiadiazol-5-yl | CF₃ |
| 2-Cl-4-SO₂MePh | 3-trifluoromethyl-1,2,4-thiadiazol-5-yl | H |
| 2-Cl-4-SO₂MePh | 3-trifluoromethyl-1,2,4-thiadiazol-5-yl | methyl |
| 2-Cl-4-SO₂MePh | 3-trifluoromethyl-1,2,4-thiadiazol-5-yl | i-propyl |
| 2-Cl-4-SO₂MePh | 3-trifluoromethyl-1,2,4-thiadiazol-5-yl | cyclopropyl |
| 2-Cl-4-SO₂MePh | 3-trifluoromethyl-1,2,4-thiadiazol-5-yl | CF₃ |
| 2-Cl-4-SO₂MePh | 1,2,4-thiadiazol-3-yl | H |
| 2-Cl-4-SO₂MePh | 1,2,4-thiadiazol-3-yl | methyl |
| 2-Cl-4-SO₂MePh | 1,2,4-thiadiazol-3-yl | i-propyl |
| 2-Cl-4-SO₂MePh | 1,2,4-thiadiazol-3-yl | cyclopropyl |
| 2-Cl-4-SO₂MePh | 1,2,4-thiadiazol-3-yl | CF₃ |
| 2-Cl-4-SO₂MePh | 5-methyl-1,2,4-thiadiazol-3-yl | H |
| 2-Cl-4-SO₂MePh | 5-methyl-1,2,4-thiadiazol-3-yl | methyl |
| 2-Cl-4-SO₂MePh | 5-methyl-1,2,4-thiadiazol-3-yl | i-propyl |
| 2-Cl-4-SO₂MePh | 5-methyl-1,2,4-thiadiazol-3-yl | cyclopropyl |
| 2-Cl-4-SO₂MePh | 5-methyl-1,2,4-thiadiazol-3-yl | CF₃ |
| 2-Cl-4-SO₂MePh | 5-trifluoromethyl-1,2,4-thiadiazol-3-yl | H |
| 2-Cl-4-SO₂MePh | 5-bifluoromethyl-1,2,4-thiadiazol-3-yl | methyl |
| 2-Cl-4-SO₂MePh | 5-trifluoromethyl-1,2,4-thiadiazol-3-yl | i-propyl |
| 2-Cl-4-SO₂MePh | 5-trifluoromethyl-1,2,4-thiadiazol-3-yl | cyclopropyl |
| 2-Cl-4-SO₂MePh | 5-trifluoromethyl-1,2,4-thiadiazol-3-yl | CF₃ |
| 2-Cl-4-SO₂MePh | 1,3,4-thiadiazol-2-yl | H |
| 2-Cl-4-SO₂MePh | 1,3,4-thiadiazol-2-yl | methyl |
| 2-Cl-4-SO₂MePh | 1,3,4-thiadiazol-2-yl | i-propyl |
| 2-Cl-4-SO₂MePh | 1,3,4-thiadiazol-2-yl | cyclopropyl |
| 2-Cl-4-SO₂MePh | 1,3,4-thiadiazol-2-yl | CF₃ |
| 2-Cl-4-SO₂MePh | 5-methylsulfonyl-1,3,4-thiadiazol-2-yl | H |
| 2-Cl-4-SO₂MePh | 5-methylsulfonyl-1,3,4-thiadiazol-2-yl | methyl |
| 2-Cl-4-SO₂MePh | 5-methylsulfonyl-1,3,4-thiadiazol-2-yl | i-propyl |
| 2-Cl-4-SO₂MePh | 5-methylsulfonyl-1,3,4-thiadiazol-2-yl | cyclopropyl |
| 2-Cl-4-SO₂MePh | 5-methylsulfonyl-1,3,4-thiadiazol-2-yl | CF₃ |
| 2-Cl-4-SO₂MePh | 5-methyl-1,3,4-thiadiazol-2-yl | H |
| 2-Cl-4-SO₂MePh | 5-methyl-1,3,4-thiadiazol-2-yl | methyl |
| 2-Cl-4-SO₂MePh | 5-methyl-1,3,4-thiadiazol-2-yl | i-propyl |
| 2-Cl-4-SO₂MePh | 5-methyl-1,3,4-thiadiazol-2-yl | cyclopropyl |
| 2-Cl-4-SO₂MePh | 5-methyl-1,3,4-thiadiazol-2-yl | CF₃ |
| 2-Cl-4-SO₂MePh | benzoxazol-2-yl | H |
| 2-Cl-4-SO₂MePh | benzoxazol-2-yl | methyl |
| 2-Cl-4-SO₂MePh | benzoxazol-2-yl | i-propyl |
| 2-Cl-4-SO₂MePh | benzoxazol-2-yl | cyclopropyl |
| 2-Cl-4-SO₂MePh | benzoxazol-2-yl | CF₃ |
| 2-Cl-4-SO₂MePh | 6-methylbenzoxazol-2-yl | H |
| 2-Cl-4-SO₂MePh | 6-methylbenzoxazol-2-yl | methyl |
| 2-Cl-4-SO₂MePh | 6-methylbenzoxazol-2-yl | i-propyl |
| 2-Cl-4-SO₂MePh | 6-methylbenzoxazol-2-yl | cyclopropyl |
| 2-Cl-4-SO₂MePh | 6-methylbenzoxazol-2-yl | CF₃ |
| 2-Cl-4-SO₂MePh | benzothiazol-2-yl | H |
| 2-Cl-4-SO₂MePh | benzothiazol-2-yl | methyl |
| 2-Cl-4-SO₂MePh | benzothiazol-2-yl | i-propyl |
| 2-Cl-4-SO₂MePh | benzothiazol-2-yl | cyclopropyl |
| 2-Cl-4-SO₂MePh | benzothiazol-2-yl | CF₃ |
| 2-Cl-4-SO₂MePh | pyrazol-1-yl | H |
| 2-Cl-4-SO₂MePh | pyrazol-1-yl | methyl |
| 2-Cl-4-SO₂MePh | pyrazol-1-yl | i-propyl |
| 2-Cl-4-SO₂MePh | pyrazol-1-yl | cyclopropyl |
| 2-Cl-4-SO₂MePh | pyrazol-1-yl | CF₃ |
| 2-Cl-4-SO₂MePh | pyrazol-3-yl | H |
| 2-Cl-4-SO₂MePh | pyrazol-3-yl | methyl |
| 2-Cl-4-SO₂MePh | pyrazol-3-yl | i-propyl |
| 2-Cl-4-SO₂MePh | pyrazol-3-yl | cyclopropyl |
| 2-Cl-4-SO₂MePh | pyrazol-3-yl | CF₃ |
| 2-Cl-4-SO₂MePh | 1-methylpyrazol-3-yl | H |
| 2-Cl-4-SO₂MePh | 1-methylpyrazol-3-yl | methyl |
| 2-Cl-4-SO₂MePh | 1-methylpyrazol-3-yl | i-propyl |
| 2-Cl-4-SO₂MePh | 1-methylpyrazol-3-yl | cyclopropyl |
| 2-Cl-4-SO₂MePh | 1-methylpyrazol-3-yl | CF₃ |
| 2-Cl-4-SO₂MePh | tetrazol-1-yl | H |
| 2-Cl-4-SO₂MePh | tetrazol-1-yl | methyl |
| 2-Cl-4-SO₂MePh | tetrazol-1-yl | i-propyl |
| 2-Cl-4-SO₂MePh | tetrazol-1-yl | cyclopropyl |
| 2-Cl-4-SO₂MePh | tetrazol-1-yl | CF₃ |
| 2-Cl-4-SO₂MePh | 5-methyltetrazol-1-yl | H |
| 2-Cl-4-SO₂MePh | 5-methyltetrazol-1-yl | methyl |
| 2-Cl-4-SO₂MePh | 5-methyltetrazol-1-yl | i-propyl |
| 2-Cl-4-SO₂MePh | 5-methyltetrazol-1-yl | cyclopropyl |
| 2-Cl-4-SO₂MePh | 5-methyltetrazol-1-yl | CF₃ |
| 2-Cl-4-SO₂MePh | tetrazol-2-yl | H |
| 2-Cl-4-SO₂MePh | tetrazol-2-yl | methyl |
| 2-Cl-4-SO₂MePh | tetiazol-2-yl | i-propyl |
| 2-Cl-4-SO₂MePh | tetrazol-2-yl | cyclopropyl |
| 2-Cl-4-SO₂MePh | tetrazol-2-yl | CF₃ |
| 2-Cl-4-SO₂MePh | 5-methyltetrazol-2-yl | H |
| 2-Cl-4-SO₂MePh | 5-methyltetrazol-2-yl | methyl |
| 2-Cl-4-SO₂MePh | 5-methyltetrazol-2-yl | i-propyl |
| 2-Cl-4-SO₂MePh | 5-methyltetrazol-2-yl | cyclopropyl |
| 2-Cl-4-SO₂MePh | 5-methyltetrazol-2-yl | CF₃ |
| 2-Cl-4-SO₂MePh | 1-methyltetrazol-5-yl | H |
| 2-Cl-4-SO₂MePh | 1-methyltetrazol-5-yl | methyl |
| 2-Cl-4-SO₂MePh | 1-methyketrazol-5-yl | i-propyl |
| 2-Cl-4-SO₂MePh | 1-methyltetrazol-5-yl | cyclopropyl |
| 2-Cl-4-SO₂MePh | 1-methyltetrazol-5-yl | CF₃ |
| 2-Cl-4-SO₂MePh | 2-methyltetrazol-5-yl | t-butile |
| 2-Cl-4-SO₂MePh | 2-methyltetrazol-5-yl | methyl |
| 2-Cl-4-SO₂MePh | 2-methyltetrazol-5-yl | i-propyl |
| 2-Cl-4-SO₂MePh | 2-methyltetrazol-5-yl | cyclopropyl |
| 2-Cl-4-SO₂MePh | 2-methyltetrazol-5-yl | CF₃ |
| 2-Cl-4-SO₂MePh | pyridin-2-yl | H |
| 2-Cl-4-SO₂MePh | pyridin-2-yl | methyl |
| 2-Cl-4-SO₂MePh | pyridin-2-yl | i-propyl |
| 2-Cl-4-SO₂MePh | pyridin-2-yl | cyclopropyl |
| 2-Cl-4-SO₂MePh | pyridin-2-yl | CF₃ |
| 2-Cl-4-SO₂MePh | pyridin-4-yl | H |
| 2-Cl-4-SO₂MePh | pyridin-4-yl | methyl |
| 2-Cl-4-SO₂MePh | pyridin-4-yl | i-propyl |
| 2-Cl-4-SO₂MePh | pyridin-4-yl | cyclopropyl |
| 2-Cl-4-SO₂MePh | pyridin-4-yl | CF₃ |
| 2-Cl-4-SO₂MePh | pyridin-3-yl | H |
| 2-Cl-4-SO₂MePh | pyridin-3-yl | methyl |
| 2-Cl-4-SO₂MePh | pyridin-3-yl | i-propyl |
| 2-Cl-4-SO₂MePh | pyridin-3-yl | cyclopropyl |
| 2-Cl-4-SO₂MePh | pyridin-3 yl | CF₃ |
| 2-Cl-4-SO₂MePh | 3-nitropyridin-4-yl | H |
| 2-Cl-4-SO₂MePh | 3-nitropyridin-4-yl | methyl |
| 2-Cl-4-SO₂MePh | 3-nitropyridin-4-yl | i-propyl |
| 2-Cl-4-SO₂MePh | 3-nitropyridin-4-yl | cyclopropyl |
| 2-Cl-4-SO₂MePh | 3-nitropyridin-4-yl | CF₃ |
| 2-Cl-4-SO₂MePh | 5-cyanopyridin-2-yl | H |
| 2-Cl-4-SO₂MePh | 5-cyanopyridin-2-yl | methyl |
| 2-Cl-4-SO₂MePh | 5-cyanopyridin-2-yl | i-propyl |
| 2-Cl-4-SO₂MePh | 5-cyanopyridin-2-yl | cyclopropyl |
| 2-Cl-4-SO₂MePh | 5-cyanopyridin-2-yl | CF₃ |
| 2-Cl-4-SO₂MePh | 5-trifluoromethylpyridin-2-yl | H |
| 2-Cl-4-SO₂MePh | 5-trifluoromethylpyridin-2-yl | methyl |
| 2-Cl-4-SO₂MePh | 5-trifluoromethylpyridin-2-yl | i-propyl |
| 2-Cl-4-SO₂MePh | 5-trifluoromethylpyridin-2-yl | cyclopropyl |
| 2-Cl-4-SO₂MePh | 5-trifluoromethylpyridin-2-yl | CF₃ |
| 2-Cl-4-SO₂MePh | pyrimidin-2-yl | H |
| 2-Cl-4-SO₂MePh | pyrimidin-2-yl | methyl |
| 2-Cl-4-SO₂MePh | pyrimidin-2-yl | i-propyl |
| 2-Cl-4-SO₂MePh | pyrimidin-2-yl | cyclopropyl |
| 2-Cl-4-SO₂MePh | pyrimidin-2-yl | CF₃ |
| 2-Cl-4-SO₂MePh | pyrimidin-4-yl | H |
| 2-Cl-4-SO₂MePh | pyrimidin-4-yl | methyl |
| 2-Cl-4-SO₂MePh | pyrimidin-4-yl | i-propyl |
| 2-Cl-4-SO₂MePh | pyrimidin-4-yl | cyclopropyl |
| 2-Cl-4-SO₂MePh | pyrimidin-4-yl | CF₃ |
| 2-Cl-4-SO₂MePh | 6-chloropyrimidin-4-yl | methyl |
| 2-Cl-4-SO₂MePh | 6-chloropyrimidin-4-yl | i-propyl |
| 2-Cl-4-SO₂MePh | 6-chloropyrimidin-4-yl | cyclopropyl |
| 2-Cl-4-SO₂MePh | 6-chloropyrimidin-4-yl | CF₃ |
| 2-Cl-4-SO₂MePh | pyridazin-3-yl | H |
| 2-Cl-4-SO₂MePh | pyridazin-3-yl | methyl |
| 2-Cl-4-SO₂MePh | pyridazin-3-yl | i-propyl |
| 2-Cl-4-SO₂MePh | pyridazin-3-yl | cyclopropyl |
| 2-Cl-4-SO₂MePh | pyridazin-3-yl | CF₃ |
| 2-Cl-4-SO₂MePh | 6-chloropyridazin-3-yl | methyl |
| 2-Cl-4-SO₂MePh | 6-chloropyridazin-3-yl | i-propyl |
| 2-Cl-4-SO₂MePh | 6-chloropyridazin-3-yl | cyclopropyl |
| 2-Cl-4-SO₂MePh | 6-chloropyridazin-3-yl | CF₃ |
| 2-Cl-4-SO₂MePh | pyrazin-2-yl | methyl |
| 2-Cl-4-SO₂MePh | pyrazin-2-yl | i-propyl |
| 2-Cl-4-SO₂MePh | pyrazin-2-yl | cyclopropyl |
| 2-Cl-4-SO₂MePh | pyrazin-2-yl | CF₃ |
| 2-Cl-4-SO₂MePh | triazin-2-yl | methyl |
| 2-Cl-4-SO₂MePh | triazin-2-yl | i-propyl |
| 2-Cl-4-SOMePh | triazin-2-yl | cyclopropyl |
| 2-Cl-4-SO₂MePh | triazin-2-yl | CF₃ |
| 2-Cl-4-SO₂MePh | quinolin-2-yl | methyl |
| 2-Cl-4-SO₂MePh | quinolin-2-yl | i-propyl |
| 2-Cl4-SO₂MePh | quinolin-2-yl | cyclopropyl |
| 2-Cl-4-SO₂MePh | quinolin-2-yl | CF₃ |
| 2-Cl-4-SO₂MePh | 4,4,6-trimethyl-5,6-dihydro-1,3(4H)oxazin-2-yl | H |
| 2-Cl-4-SO₂MePh | 4,4,6-trimethyl-5,6-dihydro-1,3(4H)oxazin-2-yl | methyl |
| 2-Cl-4-SO₂MePh | 4,4,6-trimethyl-5,6-dihydro-1,3(4H)-oxazin-2-yl | i-propyl |
| 2-Cl-4-SO₂MePh | 4,4,6-trimethyl-5,6-dihydro-1,3(4H)-oxazin-2-yl | cyclopropyl |
| 2-Cl-4-SO₂MePh | 4,4,6-trimethyl-5,6-dihydro-1,3(4H)-oxazin-2-yl | CF₃ |
| 2-Cl-4-SO₂MePh | 2-oxazolidinon-3-yl | H |
| 2-Cl-4-SO₂MePh | 2-oxazolidinon-3-yl | methyl |
| 2-Cl-4-SO₂MePh | 2-oxazolidinon-3-yl | i-propyl |
| 2-Cl-4-SO₂MePh | 2-oxazolidinon-3-yl | cyclopropyl |
| 2-Cl-4-SO₂MePh | 2-oxazolidinon-3-yl | CF₃ |
| 2-Cl-4-SO₂MePh | 2-pyrrolidinon-1-yl | methyl |
| 2-Cl-4-SO₂MePh | 2-pyrrolidinon-1-yl | i-propyl |
| 2-Cl-4-SO₂MePh | 2-pyrrolidinon-1-yl | cyclopropyl |
| 2-Cl-4-SO₂MePh | 2-pyrrolidinon-1-yl | CF₃ |
| 2-Cl-4-SO₂MePh | 3-methylisoxazol-5-yl | methyl |
| 2-Cl-4-SO₂MePh | 3-methylisoxazol-5-yl | i-propyl |
| 2-Cl-4-SO₂MePh | 3-methylisoxazol-5-yl | cyclopropyl |
| 2-Cl-4-SO₂MePh | 3-methylisoxazol-5-yl | CF₃ |
| 4-Cl-2 NO₂Ph | 1,2,4-oxadiazol-5-yl | H |
| 4-Cl-2-NO₂Ph | 1,2,4-oxadiazol-5-yl | methyl |
| 4-Cl-2-NO₂Ph | 1,2,4-oxadiazol-5-yl | i-propyl |
| 4-Cl-2-NO₂Ph | 1,2,4-oxadiazol-5-yl | cyclopropyl |
| 4-Cl-2-NO₂Ph | 1,2,4-oxadiazol-5-yl | CF₃ |
| 4-Cl-2-NO₂Ph | 3-methyl-1,2,4-oxadiazol-5-yl | H |
| 4-Cl-2-NO₂Ph | 3-methyl-1,2,4-oxadiazol-5-yl | methyl |
| 4-Cl-2-NO₂Ph | 3-methyl-1,2,4-oxadiazol-5-yl | i-propyl |
| 4-Cl-2-NO₂Ph | 3-methyl-1,2,4-oxadiazol-5-yl | cyclopropyl |
| 4-Cl-2-NO₂Ph | 3-methyl-1,2,4-oxadiazol-5-yl | CF₃ |
| 4-Cl-2-NO2Ph | 3-trifluoromethyl-1,2,4-oxadiazol-5-yl | H |
| 4-Cl-2-NO₂Ph | 3-trifluoromethyl-1,2,4-oxadiazol-5-yl | methyl |
| 4-Cl-2-NO₂Ph | 3-trifluoromethyl-1,2,4-oxadiazol-5-yl | i-propyl |
| 4-Cl-2-NO₂Ph | 3-trifluoromethyl-1,2,4-oxadiazol-5-yl | cyclopropyl |
| 4-Cl-2-NO₂Ph | 3-trifluoromethyl-1,2,4-oxadiazol-5-yl | CF₃ |
| 4-Cl-2-NO₂Ph | 1,2,4-oxadiazol-3-yl | H |
| 4-Cl-2-NO₂Ph | 1,2,4-oxadiazol-3-yl | methyl |
| 4-Cl-2-NO₂Ph | 1,2,4-oxadiazol-3-yl | i-propyl |
| 4-Cl-2-NO₂Ph | 1,2,4-oxadiazol-3-yl | cyclopropyl |
| 4-Cl-2-NO₂Ph | 1,2,4-oxadiazol-3-yl | CF₃ |
| 4-Cl-2-NO₂Ph | 5-methyl-1,2,4-oxadiazol-3-yl | H |
| 4-Cl-2-NO₂Ph | 5-methyl-1,2,4-oxadiazol-3-yl | methyl |
| 4-Cl-2-NO₂Ph | 5-methyl-1,2,4-oxadiazol-3-yl | i-propyl |
| 4-Cl-2-NO₂Ph | 5-methyl-1,2,4-oxadiazol-3-yl | cyclopropyl |
| 4-Cl-2-NO₂Ph | 5-methyl-1,2,4-oxadiazol-3-yl | CF₃ |
| 4-Cl-2-NO₂Ph | 5-trifluoromethyl-1,2,4-oxadiazol-3-yl | H |
| 4-Cl-2-NO₂Ph | S-trifluoromethyl-1,2,4-oxadiazol-3-yl | methyl |
| 4-Cl-2-NO₂Ph | 5-trifluoromethyl-1,2,4-oxadiazol-3-yl | i-propyl |
| 4-Cl-2-NO₂Ph | 5-trifluoromethyl-1,2,4-oxadiazol-3-yl | cyclopropyl |
| 4-Cl-2-NO₂Ph | 5-trifluoromethyl-1,2,4-oxadiazol-3-yl | CF₃ |
| 4-Cl-2-NO₂Ph | 5-chloro-1,2,4-oxadiazol-3-yl | H |
| 4-Cl-2-NO₂Ph | 5-chloro-1,2,4-oxadiazol-3-yl | methyl |
| 4-Cl-2-NO₂Ph | 5-chloro-1,2,4-oxadiazol-3-yl | i-propyl |
| 4-Cl-2-NO₂Ph | 5-chloro-1,2,4-oxadiazol-3-yl | cyclopropyl |
| 4-Cl-2-NO₂Ph | 5-chloro-1,2,4-oxadiazol-3-yl | CF₃ |
| 4-Cl-2-NO₂Ph | 1,3,4-oxadiazol-2-yl | H |
| 4-Cl-2-NO₂Ph | 1,3,4-oxadiazol-2-yl | methyl |
| 4-Cl-2-NO₂Ph | 1,3,4-oxadiazol-2-yl | i-propyl |
| 4-Cl-2-NO₂Ph | 1,3,4-oxadiazol-2-yl | cyclopropyl |
| 4-Cl-2-NO₂Ph | 1,3,4-oxadiazol-2-yl | CF₃ |
| 4-Cl-2-NO₂Ph | 5-methylsulfonyl-1,3,4-oxadiazol-2-yl | H |
| 4-Cl-2-NO₂Ph | 5-methylsulfonyl-1,3,4-oxadiazol-2-yl | methyl |
| 4-Cl-2-NO₂Ph | 5-methylsulfonyl-1,3,4-oxadiazol-2-yl | i-propyl |
| 4-Cl-2-NO₂Ph | 5-methylsulfonyl-1,3,4-oxadiazol-2-yl | cyclopropyl |
| 4-Cl-2-NO₂Ph | 5-methylsulfonyl-1,3,4-oxadiazol-2-yl | CF₃ |
| 4-Cl-2-NO₂Ph | 5-methyl-1,3,4-oxadiazol-2-yl | H |
| 4-Cl-2-NO₂Ph | 5-methyl-1,3,4-oxadiazol-2-yl | methyl |
| 4-Cl-2-NO₂Ph | 5-methyl-1,3,4-oxadiazol-2-yl | i-propyl |
| 4-Cl-2-NO₂Ph | 5-methyl-1,3,4-oxadiazol-2-yl | cyclopropyl |
| 4-Cl-2-NO₂Ph | 5-methyl-1,3,4-oxadiazol-2-yl | CF₃ |
| 4-Cl-2-NO₂Ph | 5-trifluoromethyl-1,3,4-oxadiazol-2-yl | H |
| 4-Cl-2-NO₂Ph | 5-trifluoromethyl-1,3,4-oxadiazol-2-yl | methyl |
| 4-Cl-2-NO₂Ph | 5-trifluoromethyl-1,3,4-oxadiazol-2-yl | i-propyl |
| 4-Cl-2-NO₂Ph | 5-trifluoromethyl-1,3,4-oxadiazol-2-yl | cyclopropyl |
| 4-Cl-2-NO₂Ph | 5-trifluoromethyl-1,3,4-oxadiazol-2-yl | CF₃ |
| 4-Cl-2-NO₂Ph | 1,2,3-triazol-4-yl | H |
| 4-Cl-2-NO₂Ph | 1,2,3-triazol-4-yl | methyl |
| 4-Cl-2-NO₂Ph | 1,2,3-triazol-4-yl | i-propyl |
| 4-Cl-2-NO₂Ph | 1,2,3-triazol-4-yl | cyclopropyl |
| 4-Cl-2-NO₂Ph | 1,2,3-triazol-4-yl | CF₃ |
| 4-Cl-2-NO₂Ph | 1-methyl-1,2,3-triazol-4-yl | H |
| 4-Cl-2-NO₂Ph | 1-methyl-1,2,3-triazol-4-yl | methyl |
| 4-Cl-2-NO₂Ph | 1-methyl-1,2,3-triazol-4-yl | i-propyl |
| 4-Cl-2-NO₂Ph | 1-methyl-1,2,3-triazol-4-yl | cyclopropyl |
| 4-Cl-2-NO₂Ph | 1-methyl-1,2,3-triazol-4-yl | CF₃ |
| 4-Cl-2-NO₂Ph | 2-methyl-1,2,3-triazol-4-yl | H |
| 4-Cl-2-NO₂Ph | 2-methyl-1,2,3-triazol-4-yl | methyl |
| 4-Cl-2-NO₂Ph | 2-methyl-1,2,3-triazol-4-yl | i-propyl |
| 4-Cl-2-NO₂Ph | 2-methyl-1,2,3-triazol-4-yl | cyclopropyl |
| 4-Cl-2-NO₂Ph | 2-methyl-1,2,3-triazol-4-yl | CF₃ |
| 4-Cl-2-NO₂Ph | 1,2,3-triazol-1-yl | H |
| 4-Cl-2-NO₂Ph | 1,2,3-triazol-1-yl | methyl |
| 4-Cl-2-NO₂Ph | 1,2,3-triazol-1-yl | i-propyl |
| 4-Cl-2-NO₂Ph | 1,2,3-triazol-1-yl | cyclopropyl |
| 4-Cl-2-NO₂Ph | 1,2,3-triazol-1-yl | CF₃ |
| 4-Cl-2-NO₂Ph | 1,2,3-triazol-2-yl | H |
| 4-Cl-2-NO₂Ph | 1,2,3-triazol-2-yl | methyl |
| 4-Cl-2-NO₂Ph | 1,2,3-triazol-2-yl | i-propyl |
| 4-Cl-2-NO₂Ph | 1,2,3-triazol-2-yl | cyclopropyl |
| 4-Cl-2-NO₂Ph | 1,2,3-triazol-2-yl | CF₃ |
| 4-Cl-2-NO₂Ph | 1,2,4-triazol-1-yl | H |
| 4-Cl-2-NO₂Ph | 1,2,4-triazol-1-yl | methyl |
| 4-Cl-2-NO₂Ph | 1,2,4-triazol-1-yl | i-propyl |
| 4-Cl-2-NO₂Ph | 1,2,4-triazol-1-yl | cyclopropyl |
| 4-Cl-2-NO₂Ph | 1,2,4-triazol-1-yl | CF₃ |
| 4-Cl-2-NO₂Ph | imidazol-2-yl | H |
| 4-Cl-2-NO₂Ph | imidazol-2-yl | methyl |
| 4-Cl-2-NO₂Ph | imidazol-2-yl | i-propyl |
| 4-Cl-2-NO₂Ph | imidazol-2-yl | cyclopropyl |
| 4-Cl-2-NO₂Ph | imidazol-2-yl | CF₃ |
| 4-Cl-2-NO₂Ph | imidazol-1-yl | H |
| 4-Cl-2-NO₂Ph | imidazol-1-yl | methyl |
| 4-Cl-2-NO₂Ph | imidazol-1-yl | i-propyl |
| 4-Cl-2-NO₂Ph | imidazol-1-yl | cyclopropyl |
| 4-Cl-2-NO₂Ph | imidazol-1-yl | CF₃ |
| 4-Cl-2-NO₂Ph | imidazol-4-yl | H |
| 4-Cl-2-NO₂Ph | imidazol-4-yl | methyl |
| 4-Cl-2-NO₂Ph | imidazol-4-yl | i-propyl |
| 4-Cl-2-NO₂Ph | imidazol-4-yl | cyclopropyl |
| 4-Cl-2-NO₂Ph | imidazol-4-yl | CF₃ |
| 4-Cl-2-NO₂Ph | thiazol-2-yl | H |
| 4-Cl-2-NO₂Ph | thiazol-2-yl | methyl |
| 4-Cl-2-NO₂Ph | thiazol-2-yl | i-propyl |
| 4-Cl-2-NO₂Ph | thiazol-2-yl | cyclopropyl |
| 4-Cl-2-NO₂Ph | thiazol-2-yl | CF₃ |
| 4-Cl-2-NO₂Ph | 4-methylthiazol-2-yl | H |
| 4-Cl-2-NO₂Ph | 4-methylthiazol-2-yl | methyl |
| 4-Cl-2-NO₂Ph | 4-methylthiazol-2-yl | i-propyl |
| 4-Cl-2-NO₂Ph | 4-methylthiazol-2-yl | cyclopropyl |
| 4-Cl-2-NO₂Ph | 4-methylthiazol-2-yl | CF₃ |
| 4-Cl-2-NO₂Ph | oxazol-2-yl | H |
| 4-Cl-2-NO₂Ph | oxazol-2-yl | methyl |
| 4-Cl-2-NO₂Ph | oxazol-2-yl | i-propyl |
| 4-Cl-2-NO₂Ph | oxazol-2-yl | cyclopropyl |
| 4-Cl-2-NO₂Ph | oxazol-2-yl | CF₃ |
| 4-Cl-2-NO₂Ph | 4,5-dimethyloxazol-2-yl | H |
| 4-Cl-2-NO₂Ph | 4,5-dimethyloxazol-2-yl | methyl |
| 4-Cl-2-NO₂Ph | 4,5-dimethyloxazol-2-yl | i-propyl |
| 4-Cl-2-NO₂Ph | 4,5-dimethyloxazol-2-yl | cyclopropyl |
| 4-Cl-2-NO₂Ph | 4,5-dimethyloxazol-2-yl | CF₃ |
| 4-Cl-2-NO₂Ph | 2-oxazolin-2-yl | H |
| 4-Cl-2-NO₂Ph | 2-oxazolin-2-yl | methyl |
| 4-Cl-2-NO₂Ph | 2-oxazolin-2-yl | i-propyl |
| 4-Cl-2-NO₂Ph | 2-oxazolin-2-yl | cyclopropyl |
| 4-Cl-2-NO₂Ph | 2-oxazolin-2-yl | CF₃ |
| 4-Cl-2-NO₂Ph | 4,4-dimethyl-2-oxazolin-2-yl | H |
| 4-Cl-2-NO₂Ph | 4,4-dimethyl-2-oxazolin-2-yl | methyl |
| 4-Cl-2-NO₂Ph | 4,4-dimethyl-2-oxazolin-2-yl | i-propyl |
| 4-Cl-2-NO₂Ph | 4,4-dimethyl-2-oxazolin-2-yl | cyclopropyl |
| 4-Cl-2-NO₂Ph | 4,4-dimethyl-2-oxazolin-2-yl | CF₃ |
| 4-Cl-2-NO₂Ph | 1,2,4-thiadiazol-5-yl | H |
| 4-Cl-2-NO₂Ph | 1,2,4-thiadiazol-5-yl | methyl |
| 4-Cl-2-NO₂Ph | 1,2,4-thiadiazol-5-yl | i-propyl |
| 4-Cl-2-NO₂Ph | 1,2,4-thiadiazol-5-yl | cyclopropyl |
| 4-Cl-2-NO₂Ph | 1,2,4-thiadiazol-5-yl | CF₃ |
| 4-Cl-2-NO₂Ph | 3-methyl-1,2,4-thiadiazol-5-yl | H |
| 4-Cl-2-NO₂Ph | 3-methyl-1,2,4-thiadiazol-5-yl | methyl |
| 4-Cl-2-NO₂Ph | 3-methyl-1,2,4-thiadiazol-5-yl | i-propyl |
| 4-Cl-2-NO₂Ph | 3-methyl-1,2,4-thiadiazol-5-yl | cyclopropyl |
| 4-Cl-2-NO₂Ph | 3-methyl-1,2,4-thiadiazol-5-yl | CF₃ |
| 4-Cl-2-NO₂Ph | 3-trifluoromethyl-1,2,4-thiadiazol-5-yl | H |
| 4-Cl-2-NO₂Ph | 3-trifluoromethyl-1,2,4-thiadiazol-5-yl | methyl |
| 4-Cl-2-NO₂Ph | 3-trifluoromethyl-1,2,4-thiadiazol-5-yl | i-propyl |
| 4-Cl-2-NO₂Ph | 3-trifluoromethyl-1,2,4-thiadiazol-5-yl | cyclopropyl |
| 4-Cl-2-NO₂Ph | 3-trifluoromethyl-1,2,4-thiadiazol-5-yl | CF₃ |
| 4-Cl-2-NO₂Ph | 1,2,4-thiadiazol-3-yl | H |
| 4-Cl-2-NO₂Ph | 1,2,4-thiadiazol-3-yl | methyl |
| 4-Cl-2-NO₂Ph | 1,2,4-thiadiazol-3-yl | i-propyl |
| 4-Cl-2-NO₂Ph | 1,2,4-thiadiazol-3-yl | cyclopropyl |
| 4-Cl-2-NO₂Ph | 1,2,4-thiadiazol-3-yl | CF₃ |
| 4-Cl-2-NO₂Ph | 5-methyl-1,2,4-thiadiazol-3-yl | H |
| 4-Cl-2-NO₂Ph | 5-methyl-1,2,4-thiadiazol-3-yl | methyl |
| 4-Cl-2-NO₂Ph | 5-methyl-1,2,4-thiadiazol-3-yl | i-propyl |
| 4-Cl-2-NO₂Ph | 5-methyl-1,2,4-thiadiazol-3-yl | cyclopropyl |
| 4-Cl-2-NO₂Ph | 5-methyl-1,2,4-thiadiazol-3-yl | CF₃ |
| 4-Cl-2-NO₂Ph | 5-trifluoromethyl-1,2,4-thiadiazol-3-yl | H |
| 4-Cl-2-NO₂Ph | 5-trifluoromethyl-1,2,4-thiadiazol-3-yl | methyl |
| 4-Cl-2-NO₂Ph | 5-trifluoromethyl-l,2,4-thiadiazol-3-yl | i-propyl |
| 4-Cl-2-NO₂Ph | 5-trifluoromethyl-1,2,4-thiadiazol-3-yl | cyclopropyl |
| 4-Cl-2-NO₂Ph | 5-trifluoromethyl-1,2,4-thiadiazol-3-yl | CF₃ |
| 4-Cl-2-NO₂Ph | 1,3,4-thiadiazol-2-yl | H |
| 4-Cl-2-NO₂Ph | 1,3,4-thiadiazol-2-yl | methyl |
| 4-ClI-2-NO₂Ph | 1,3,4-thiadiazol-2-yl | i-propyl |
| 4-Cl-2-NO₂Ph | 1,3,4-thiadiazol-2-yl | cyclopropyl |
| 4-Cl-2-NO₂Ph | 1,3,4-thiadiazol-2-yl | CF₃ |
| 4-Cl-2-NO₂Ph | 5-methylsulfonyl-1,3,4-thiadiazol-2-yl | H |
| 4-Cl-2-NO₂Ph | 5-methylsulfonyl-1,3,4-thiadiazol-2-yl | methyl |
| 4-Cl-2-NO₂Ph | 5-methylsulfonyl-1,3,4-thiadiazol-2-yl | i-propyl |
| 4-Cl-2-NO₂Ph | 5-methylsulfonyl-1,3,4-thiadiazol-2-yl | cyclopropyl |
| 4-Cl-2-NO₂Ph | 5-methylsulfonyl-1,3,4-thiadiazol-2-yl | CF₃ |
| 4-Cl-2-NO₂Ph | 5-methyl-1,3,4-thiadiazol-2-yl | H |
| 4-Cl-2-NO₂Ph | 5-methyl-1,3,4-thiadiazol-2-yl | methyl |
| 4-Cl-2-NO₂Ph | 5-methyl-1,3,4-thiadiazol-2-yl | i-propyl |
| 4-Cl-2-NO₂Ph | 5-methyl-1,3,4-thiadiazol-2-yl | cyclopropyl |
| 4-Cl-2-NO₂Ph | 5-methyl-1,3,4-thiadiazol-2-yl | CF₃ |
| 4-Cl-2-NO₂Ph | benzoxazol-2-yl | H |
| 4-Cl-2-NO₂Ph | benzoxazol-2-yl | methyl |
| 4-Cl-2-NO₂Ph | benzoxazol-2-yl | i-propyl |
| 4-Cl-2-NO₂Ph | benzoxazol-2-yl | cyclopropyl |
| 4-Cl-2-NO₂Ph | benzoxazol-2-yl | CF₃ |
| 4-Cl-2-NO₂Ph | 6-methylbenzoxazol-2-yl | H |
| 4-Cl-2-NO₂Ph | 6-methylbenzoxazol-2-yl | methyl |
| 4-Cl-2-NO₂Ph | 6-methylbenzoxazol-2-yl | i-propyl |
| 4-Cl-2-NO₂Ph | 6-methylbenzoxazol-2-yl | cyclopropyl |
| 4-Cl-2-NO₂Ph | 6-methylbenzoxazol-2-yl | CF₃ |
| 4-Cl-2-NO₂Ph | benzothiazol-2-yl | H |
| 4-Cl-2-NO₂Ph | benzothiazol-2-yl | methyl |
| 4-Cl-2-NO₂Ph | benzothiazol-2-yl | i-propyl |
| 4-Cl-2-NO₂Ph | benzothiazol-2-yl | cyclopropyl |
| 4-Cl-2-NO₂Ph | benzothiazol-2-yl | CF₃ |
| 4-Cl-2-NO₂Ph | pyrazol-1-yl | H |
| 4-Cl-2-NO₂Ph | pyrazol-1-yl | methyl |
| 4-Cl-2-NO₂Ph | pyrazol-1-yl | i-propyl |
| 4-Cl-2-NO₂Ph | pyrazol-1-yl | cyclopropyl |
| 4-Cl-2-NO₂Ph | pyrazol-1-yl | CF₃ |
| 4-Cl-2-NO₂Ph | pyrazol-3-yl | H |
| 4-Cl-2-NO₂Ph | pyrazol-3-yl | methyl |
| 4-Cl-2-NO₂Ph | pyrazol-3-yl | i-propyl |
| 4-Cl-2-NO₂Ph | pyrazol-3-yl | cyclopropyl |
| 4-Cl-2-NO₂Ph | pyrazol-3-yl | CF₃ |
| 4-Cl-2-NO₂Ph | 1-methylpyrazol-3-yl | H |
| 4-Cl-2-NO₂Ph | 1-methylpyrazol-3-yl | methyl |
| 4-Cl-2-NO₂Ph | 1-methylpyrazol-3-yl | i-propyl |
| 4-Cl-2-NO₂Ph | 1-methylpyrazol-3-yl | cyclopropyl |
| 4-Cl-2-NO₂Ph | 1-methylpyrazol-3-yl | CF₃ |
| 4-Cl-2-NO₂Ph | tetrazol-1-yl | H |
| 4-Cl-2-NO₂Ph | te2razol-1-yl | methyl |
| 4-Cl-2-NO₂Ph | tetrazol-1-yl | i-propyl |
| 4-Cl-2-NO₂Ph | tetrazol-1-yl | cyclopropyl |
| 4-Cl-2-NO₂Ph | tetrazol-1-yl | CF₃ |
| 4-Cl-2-NO₂Ph | 5-methyltetrazol-1-yl | H |
| 4-Cl-2-NO₂Ph | 5-methyltetxazol-1-yl | methyl |
| 4-Cl-2-NO₂Ph | 5-methyltetrazol-1-yl | i-propyl |
| 4-Cl-2-NO₂Ph | 5-methyltetrazol-1-yl | cyclopropyl |
| 4-Cl-2-NO₂Ph | 5-methyltetrazol-1-yl | CF₃ |
| 4-Cl-2-NO₂Ph | tetrazol-2-yl | H |
| 4-Cl-2-NO₂Ph | tetrazol-2-yl | methyl |
| 4-Cl-2-NO₂Ph | tetrazol-2-yl | i-propyl |
| 4-Cl-2-NO₂Ph | tetrazol-2-yl | cyclopropyl |
| 4-Cl-2-NO₂Ph | tetrazol-2-yl | CF₃ |
| 4-Cl-2-NO₂Ph | 5-methyltetrazol-2-yl | H |
| 4-Cl-2-NO₂Ph | 5-methyltetrazol-2-yl | methyl |
| 4-Cl-2-NO₂Ph | 5-methyltetrazol-2-yl | i-propyl |
| 4-Cl-2-NO₂Ph | 5-methyltetrazol-2-yl | cyclopropyl |
| 4-Cl-2-NO₂Ph | 5-methyltetrazol-2-yl | CF₃ |
| 4-Cl-2-NO₂Ph | 1-methyltetrazol-5-yl | H |
| 4-Cl-2-NO₂Ph | 1-methyltetrazol-5-yl | methyl |
| 4-Cl-2-NO₂Ph | 1-methyltetrazol-5-yl | i-propyl |
| 4-Cl-2-NO₂Ph | 1-methyltetrazol-5-yl | cyclopropyl |
| 4-Cl-2-NO₂Ph | 1-methyltetrazol-5-yl | CF₃ |
| 2-Cl-4-NO₂Ph | 2-methyltetrazol-5-yl | cyclopropyl |
| 4-Cl-2-NO₂Ph | 2-methyltetrazol-5-yl | methyl |
| 4-Cl-2-NO₂Ph | 2-methyltetrazol-5-yl | i-propyl |
| 4-Cl-2-NO₂Ph | 2-methyltetrazol-5-yl | cyclopropyl |
| 4-Cl-2-NO₂Ph | 2-methyltetrazol-5-yl | CF₃ |
| 2,4-(NO₂)₂Ph | 2-methyltetrazol-5-yl | cyclopropyl |
| 4-Cl-2-NO₂Ph | pyridin-2-yl | methyl |
| 4-Cl-2-NO₂Ph | pyridin-2-yl | i-propyl |
| 4-Cl-2-NO₂Ph | pyridin-2-yl | cyclopropyl |
| 4-Cl-2-NO₂Ph | pyridin-2-yl | CF₃ |
| 4-Cl-2-NO₂Ph | pyridin-4-yl | H |
| 4-Cl-2-NO₂Ph | pyridin-4-yl | methyl |
| 4-Cl-2-NO₂Ph | pyridin-4-yl | i-propyl |
| 4-Cl-2-NO₂Ph | pyridin-4-yl | cyclopropyl |
| 4-Cl-2-NO₂Ph | pyridin-4-yl | CF₃ |
| 4-Cl-2-NO₂Ph | pyridin-3-yl | H |
| 4-Cl-2-NO₂Ph | pyridin-3-yl | methyl |
| 4-Cl-2-NO₂Ph | pyridin-3-yl | i-propyl |
| 4-Cl-2-NO₂Ph | pyridin-3-yl | cyclopropyl |
| 4-Cl-2-NO₂Ph | pyridin-3-yl | CF₃ |
| 4-Cl-2-NO₂Ph | 3-nitropyridin-4-yl | H |
| 4-Cl-2-NO₂Ph | 3-nitropyridin-4-yl | methyl |
| 4-Cl-2-NO₂Ph | 3-nitropyridin-4-yl | i-propyl |
| 4-Cl-2-NO₂Ph | 3-nitropyridin-4-yl | cyclopropyl |
| 4-Cl-2-NO₂Ph | 3-nitropyridin-4-yl | CF₃ |
| 4-Cl-2-NO₂Ph | 5-cyanopyridin-2-yl | H |
| 4-Cl-2-NO₂Ph | 5-cyanopyridin-2-yl | methyl |
| 4-Cl-2-NO₂Ph | 5-cyanopyridin-2-yl | i-propyl |
| 4-Cl-2-NO₂Ph | 5-cyanopyridin-2-yl | cyclopropyl |
| 4-Cl-2-NO₂Ph | 5-cyanopyridin-2-yl | CF₃ |
| 4-Cl-2-NO₂Ph | 5-trifluoromethylpyridin-2-yl | H |
| 4-Cl-2-NO₂Ph | 5-trifluoromethylpyridin-2-yl | methyl |
| 4-Cl-2-NO₂Ph | 5-trifluoromethylpyridin-2-yl | i-propyl |
| 4-Cl-2-NO₂Ph | 5-trifluoromethylpyridin-2-yl | cyclopropyl |
| 4-Cl-2-NO₂Ph | 5-trifluoromethylpyridin-2-yl | CF₃ |
| 4-Cl-2-NO₂Ph | pyrimidin-2-yl | H |
| 4-Cl-2-NO₂Ph | pyrimidin-2-yl | methyl |
| 4-Cl-2-NO₂Ph | pyrimidin-2-yl | i-propyl |
| 4-Cl-2-NO₂Ph | pyrimidin-2-yl | cyclopropyl |
| 4-Cl-2-NO₂Ph | pyrimidin-2-yl | CF₃ |
| 4-Cl-2-NO₂Ph | pyrimidin-4-yl | H |
| 4-Cl-2-NO₂Ph | pyrimidin-4-yl | methyl |
| 4-Cl-2-NO₂Ph | pyrimidin-4-yl | i-propyl |
| 4-Cl-2-NO₂Ph | pyrimidin-4-yl | cyclopropyl |
| 4-Cl-2-NO₂Ph | pyrimidin-4-yl | CF₃ |
| 4-Cl-2-NO₂Ph | 6-chloropyrimidin-4-yl | methyl |
| 4-Cl-2-NO₂Ph | 6-chloropyrimidin-4-yl | i-propyl |
| 4-Cl-2-NO₂Ph | 6-chloropyrimidin-4-yl | cyclopropyl |
| 4-Cl-2-NO₂Ph | 6-chloropyrimidin-4-yl | CF₃ |
| 2,4-(Cl)₂Ph | 1-methyltetrazol-5-yl | t-butil |
| 4-Cl-2-NO₂Ph | pyridazin-3-yl | methyl |
| 4-Cl-2-NO₂Ph | pyridazin-3-yl | i-propyl |
| 4-Cl-2-NO₂Ph | pyridazin-3-yl | cyclopropyl |
| 4-Cl-2-NO₂Ph | pyridazin-3-yl | CF₃ |
| 4-Cl-2-NO₂Ph | 6-chloropyridazin-3-yl | methyl |
| 4-Cl-2-NO₂Ph | 6-chloropyridazin-3-yl | i-propyl |
| 4-Cl-2-NO₂Ph | 6-chloropyridazin-3-yl | cyclopropyl |
| 4-Cl-2-NO₂Ph | 6-chloropyridazin-3-yl | CF₃ |
| 4-Cl-2-NO₂Ph | pyrazin-2-yl | methyl |
| 4-Cl-2-NO₂Ph | pyrazin-2-yl | i-propyl |
| 4-Cl-2-NO₂Ph | pyrazin-2-yl | cyclopropyl |
| 4-Cl-2-NO₂Ph | pyrazin-2-yl | CF₃ |
| 4-Cl-2-NO₂Ph | triazin-2-yl | methyl |
| 4-Cl-2-NO₂Ph | triazin-2-yl | i-propyl |
| 4-Cl-2-NO₂Ph | triazin-2-yl | cyclopropyl |
| 4-Cl-2-NO₂Ph | triazin-2-yl | CF₃ |
| 4-Cl-2-NO₂Ph | quinolin-2-yl | methyl |
| 4-Cl-2-NO₂Ph | quinolin-2-yl | i-propyl |
| 4-Cl-2-NO₂Ph | quinolin-2-yl | cyclopropyl |
| 4-Cl-2-NO₂Ph | quinolin-2-yl | CF₃ |
| 4-Cl-2-NO₂Ph | 4,4,6-trimethyl-5,6-dihydro-1,3(4H)-oxazin-2-yl | H |
| 4-Cl-2-NO₂Ph | 4,4,6-trimethyl-5,6-dihydro-1,3(4H)-oxazin-2-yl | methyl |
| 4-Cl-2-NO₂Ph | 4,4,6-trimethyl-5,6-dihydro-1,3(4H)-oxazin-2-yl | i-propyl |
| 4-Cl-2-NO₂Ph | 4,4,6-trimethyl-5,6-dihydro-1,3(4H)-oxazin-2-yl | cyclopropyl |
| 4-Cl-2-NO₂Ph | 4,4,6-trimethyl-5,6-dihydro-1,3(4H)-oxazin-2-yl | CF₃ |
| 4-Cl-2-NO₂Ph | 2-oxazolidinon-3-yl | H |
| 4-Cl-2-NO₂Ph | 2-oxazolidinon-3-yl | methyl |
| 4-Cl-2-NO₂Ph | 2-oxazolidinon-3-yl | i-propyl |
| 4-Cl-2-NO₂Ph | 2-oxazolidinon-3-yl | cyclopropyl |
| 4-Cl-2-NO₂Ph | 2-oxazolidinon-3-yl | CF₃ |
| 4-Cl-2-NO₂Ph | 2-pyrrolidinon-1-yl | methyl |
| 4-Cl-2-NO₂Ph | 2-pyrrolidinon-1-yl | i-propyl |
| 4-Cl-2-NO₂Ph | 2-pyrrolidinon-1-yl | cyclopropyl |
| 4-Cl-2-NO₂Ph | 2-pyrrolidinon-1-yl | CF₃ |
| 4-Cl-2-NO₂Ph | 3-methylisoxazol-5-yl | methyl |
| 4-Cl-2-NO₂Ph | 3-methylisoxazol-5-yl | i-propyl |
| 4-Cl-2-NO₂Ph | 3-methylisoxazol-5-yl | cyclopropyl |
| 4-Cl-2-NO₂Ph | 3-methylisoxazol-5-yl | CF₃ |
| 2-SO₂Me-4-CF₃Ph | 1,2,4-oxadiazol-5-yl | H |
| 2-SO₂Me-4-CF₃Ph | 1,2,4-oxadiazol-5-yl | methyl |
| 2-SO₂Me-4-CF₃Ph | 1,2,4-oxadiazol-5-yl | i-propyl |
| 2-SO₂Me-4-CF₃Ph | 1,2,4-oxadiazol-5-yl | cyclopropyl |
| 2-SO₂Me-4-CF₃Ph | 1,2,4-oxadiazol-5-yl | CF₃ |
| 2-SO₂Me-4-CF₃Ph | 3-methyl-1,2,4-oxadiazol-5-yl | H |
| 2-SO₂Me-4CF₃Ph | 3-methyl-1,2,4-oxadiazol-5-yl | methyl |
| 2-SO₂Me-4-CF₃Ph | 3-methyl-1,2,4-oxadiazol-5-yl | i-propyl |
| 2-SO₂Me-4-CF₃Ph | 3-methyl-1,2,4-oxadiazol-5-yl | cyclopropyl |
| 2-SO₂Me-4-CF₃Ph | 3-methyl-1,2,4-oxadiazol-5-yl | CF₃ |
| 2-SO₂Me-4-CF₃Ph | 3-trifluoromethyl-1,2,4-oxadiazol-5-yl | H |
| 2-SO₂Me-4-CF₃Ph | 3-trifluoromethyl-1,2,4-oxadiazol-5-yl | methyl |
| 2-SO₂Me-4-CF₃Ph | 3-trifluoromethyl-1,2,4-oxadiazol-5-yl | i-propyl |
| 2-SO₂Me-4-CF₃Ph | 3-trifluoromethyl-1,2,4-oxadiazol-5-yl | cyclopropyl |
| 2-SO₂Me-4-CF₃Ph | 3-trifluoromethyl-1,2,4-oxadiazol-5-yl | CF₃ |
| 2-SO₂Me-4-CF₃Ph | 1,2,4-oxadiazol-3-yl | H |
| 2-SO₂Me-4-CF₃Ph | 1,2,4-oxadiazol-3-yl | methyl |
| 2-SO₂Me-4-CF₃Ph | 1,2,4-oxadiazol-3-yl | i-propyl |
| 2-SO₂Me-4-CF₃Ph | 1,2,4-oxadiazol-3-yl | cyclopropyl |
| 2-SO₂Me-4-CF₃Ph | 1,2,4-oxadiazol-3-yl | CF₃ |
| 2-SO₂Me-4-CF₃Ph | 5-methyl-l,2,4-oxadiazol-3-yl | H |
| 2-SO₂Me-4-CF₃Ph | 5-methyl-1,2,4-oXadiazol-3-yl | methyl |
| 2-SO₂Me-4-CF₃Ph | 5-methyl-1,2,4-oxadiazol-3-yl | i-propyl |
| 2-SO₂Me-4-CF₃Ph | 5-methyl-1,2,4-oxadiazol-3-yl | cyclopropyl |
| 2-SO₂Me-4-CF₃Ph | 5-methyl-1,2,4-oxadiazol-3-yl | CF₃ |
| 2-SO₂Me-4-CF₃Ph | 5-trifluoromethyl-1,2,4-oxadiazol-3-yl | H |
| 2-SO₂Me-4-CF₃Ph | 5-trifluoromethyl-1,2,4-oxadiazol-3-yl | methyl |
| 2-SO₂Me-4-CF₃Ph | 5-trifluoromethyl-1,2,4-oxadiazol-3-yl | i-propyl |
| 2-SO₂Me-4-CF₃Ph | 5-trifluoromethyl-1,2,4-oxadiazol-3-yl | cyclopropyl |
| 2-SO₂Me-4-CF₃Ph | 5-trifluoromethyl-1,2,4-oxadiazol-3-yl | CF₃ |
| 2-SO₂Me-4-CF₃Ph | 5-chloro-1,2,4-oxadiazol-3-yl | H |
| 2-SO₂Me-4-CF₃Ph | 5-chloro-1,2,4-oxadiazol-3-yl | methyl |
| 2-SO₂Me-4-CF₃Ph | 5-chloro-1,2,4-oxadiazol-3-yl | i-propyl |
| 2-SO₂Me-4-CF₃Ph | 5-chloro-1,2,4-oxadiazol-3-yl | cyclopropyl |
| 2-SO₂Me-4-CF₃Ph | 5-chloro-1,2,4-oxadiazol-3-yl | CF₃ |
| 2-SO₂Me-4-CF₃Ph | 1,3,4-oxadiazol-2-yl | H |
| 2-SO₂Me-4-CF₃Ph | 1,3,4-oxadiazol-2-yl | methyl |
| 2-SO₂Me-4-CF₃Ph | 1,3,4-oxadiazol-2-yl | i-propyl |
| 2-SO₂Me-4-CF₃Ph | 1,3,4-oxadiazol-2-yl | cyclopropyl |
| 2-SO₂Me-4-CF₃Ph | 1,3,4-oxadiazol-2-yl | CF₃ |
| 2-SO₂Me-4-CF₃Ph | 5-methylsulfonyl-1,3,4-oxadiazol-2-yl | H |
| 2-SO₂Me-4-CF₃Ph | 5-methylsulfonyl-1,3,4-oxadiazol-2-yl | methyl |
| 2-SO₂Me-4-CF₃Ph | 5-methylsulfonyl-1,3,4-oxadiazol-2-yl | i-propyl |
| 2-SO₂Me-4-CF₃Ph | 5-methylsulfonyl-1,3,4-oxadiazol-2-yl | cyclopropyl |
| 2-SO₂Me-4-CF₃Ph | 5-methylsulfonyl-1,3,4-oxadiazol-2-yl | CF₃ |
| 2-SO₂Me-4-CF₃Ph | 5-methyl-1,3,4-oxadiazol-2-yl | H |
| 2-SO₂Me-4-CF₃Ph | 5-methyl-1,3,4-oxadiazol-2-yl | methyl |
| 2-SO₂Me-4-CF₃Ph | 5-methyl-1,3,4-oxadiazol-2-yl | i-propyl |
| 2-SO₂Me-4-CF₃Ph | 5-methyl-1,3,4-oxadiazol-2-yl | cyclopropyl |
| 2-SO₂Me-4-CF₃Ph | 5-methyl-1,3,4-oxadiazol-2-yl | CF₃ |
| 2-SO₂Me-4-CF₃Ph | 5-trifluoromethyl-1,3,4-oxadiazol-2-yl | H |
| 2-SO₂Me-4-CF₃Ph | 5-TRifluoromethyl-1,3,4-oxadiazol-2-yl | methyl |
| 2-SO₂Me-4-CF₃Ph | 5-trifluoromethyl-1,3,4-oxadiazol-2-yl | i-propyl |
| 2-SO₂Me-4-CF₃Ph | 5-trifluoromethyl-1,3,4-oxadiazol-2-yl | cyclopropyl |
| 2-SO₂Me-4-CF₃Ph | 5-trifluoromethyl-1,3,4-oxadiazol-2-yl | CF₃ |
| 2-SO₂Me-4-CF₃Ph | 1,2,3-triazol-4-yl | H |
| 2-SO₂Me-4-CF₃Ph | 1,2,3-triazol-4-yl | methyl |
| 2-SO₂Me-4-CF₃Ph | 1,2,3-triazol-4-yl | i-propyl |
| 2-SO₂Me-4-CF₃Ph | 1,2,3-triazol-4-yl | cyclopropyl |
| 2-SO₂Me-4-CF₃Ph | 1,2,3-triazol-4-yl | CF₃ |
| 2-SO₂Me-4-CF₃Ph | 1-methyl-1,2,3-triazol-4-yl | H |
| 2-SO₂Me-4-CF₃Ph | 1-methyl-1,2,3-triazol-4-yl | methyl |
| 2-SO₂Me-4-CF₃Ph | 1-methyl-1,2,3-triazol-4-yl | i-propyl |
| 2-SO₂Me-4-CF₃Ph | 1-methyl-1,2,3-triazol-4-yl | cyclopropyl |
| 2-SO₂Me-4-CF₃Ph | 1-methyl-1,2,3-triazol-4-yl | CF₃ |
| 2-SO₂Me-4-CF₃Ph | 1-methyl-1,2,3-triazol-4-yl | H |
| 2-SO₂Me-4-CF₃Ph | 1-methyl-1,2,3-triazol-4-yl | methyl |
| 2-SO₂Me-4-CF₃Ph | 1-methyl-1,2,3-triazol-4-yl | i-propyl |
| 2-SO₂Me-4-CF₃Ph | 1-methyl-1,2,3-triazol-4-yl | cyclopropyl |
| 2-SO₂Me-4-CF₃Ph | 1-methyl-1,2,3-triazol-4-yl | CF₃ |
| 2-SO₂Me-4-CF₃Ph | 1,2,3-triazol-1-yl | H |
| 2-SO₂Me-4-CF₃Ph | 1,2,3-triazol-1-yl | methyl |
| 2-SO₂Me-4-CF₃Ph | 1,2,3-triazol-1-yl | i-propyl |
| 2-SO₂Me-4-CF₃Ph | 1,2,3-triazol-1-yl | cyclopropyl |
| 2-SO₂Me-4-CF₃Ph | 1,2,3-triazol-1-yl | CF₃ |
| 2-SO₂Me-4-CF₃Ph | 1,2,3-triazol-1-yl | H |
| 2-SO₂Me-4-CF₃Ph | 1,2,3-triazol-1-yl | methyl |
| 2-SO₂Me-4-CF₃Ph | 1,2,3-triazol-1-yl | i-propyl |
| 2-SO₂Me-4-CF₃Ph | 1,2,3-triazol-1-yl | cyclopropyl |
| 2-SO₂Me-4-CF₃Ph | 1,2,3-triazol-1-yl | CF₃ |
| 2-SO₂Me-4-CF₃Ph | 1,2,3-triazol-1-yl | H |
| 2-SO₂Me-4-CF₃Ph | 1,2,3-triazol-1-yl | methyl |
| 2-SO₂Me-4-CF₃Ph | 1,2,3-triazol-1-yl | i-propyl |
| 2-SO₂Me-4-CF₃Ph | 1,2,3-triazol-1-yl | cyclopropyl |
| 2-SO₂Me-4-CF₃Ph | 1,2,3-triazol-1-yl | CF₃ |
| 2-SO₂Me-4-CF₃Ph | imidazol-2-yl | H |
| 2-SO₂Me-4-CF₃Ph | imidazol-2-yl | methyl |
| 2-SO₂Me-4-CF₃Ph | imidazol-2-yl | i-propyl |
| 2-SO₂Me-4-CF₃Ph | imidazol-2-yl | cyclopropyl |
| 2-SO₂Me-4-CF₃Ph | imidazol-2-yl | CF₃ |
| 2-SO₂Me-4-CF₃Ph | imidazol-1-yl | H |
| 2-SO₂Me-4-CF₃Ph | imidazol-1-yl | methyl |
| 2-SO₂Me-4-CF₃Ph | imidazol-1-yl | i-propyl |
| 2-SO₂Me-4-CF₃Ph | imidazol-1-yl | cyclopropyl |
| 2-SO₂Me-4-CF₃Ph | imidazol-1-yl | CF₃ |
| 2-SO₂Me-4-CF₃Ph | imidazol-4-yl | H |
| 2-SO₂Me-4-CF₃Ph | imidazol-4-yl | methyl |
| 2-SO₂Me-4-CF₃Ph | imidazol-4-yl | i-propyl |
| 2-SO₂Me-4-CF₃Ph | imidazol-4-yl | cyclopropyl |
| 2-SO₂Me-4-CF₃Ph | imidazol-4-yl | CF₃ |
| 2-SO₂Me-4-CF₃Ph | thiazol-2-yl | H |
| 2-SO₂Me-4-CF₃Ph | thiazol-2-yl | methyl |
| 2-SO₂Me-4-CF₃Ph | thiazol-2-yl | i-propyl |
| 2-SO₂Me-4-CF₃Ph | thiazol-2-yl | cyclopropyl |
| 2-SO₂Me-4-CF₃Ph | thiazol-2-yl | CF₃ |
| 2-SO₂Me-4-CF₃Ph | 4-methylthiazol-2-yl | H |
| 2-SO₂Me-4-CF₃Ph | 4-methylthiazol-2-yl | methyl |
| 2-SO₂Me-4-CF₃Ph | 4-methylthiazol-2-yl | i-propyl |
| 2-SO₂Me-4-CF₃Ph | 4-methylthiazol-2-yl | cyclopropyl |
| 2-SO₂Me-4-CF₃Ph | 4-methylthiazol-2-yl | CF₃ |
| 2-SO₂Me-4-CF₃Ph | oxazol-2-yl | H |
| 2-SO₂Me-4-CF₃Ph | oxazol-2-yl | methyl |
| 2-SO₂Me-4-CF₃Ph | oxazol-2-yl | i-propyl |
| 2-SO₂Me-4-CF₃Ph | oxazol-2-yl | cyclopropyl |
| 2-SO₂Me-4-CF₃Ph | oxazol-2-yl | CF₃ |
| 2-SO₂Me-4-CF₃Ph | 4,5-dimethyloxazol-2-yl | H |
| 2-SO₂Me-4-CF₃Ph | 4,5-dimethyloxazol-2-yl | methyl |
| 2-SO₂Me-4-CF₃Ph | 4,5-dimethyloxazol-2-yl | i-propyl |
| 2-SO₂Me-4-CF₃Ph | 4,5-dimethyloxazol-2-yl | cyclopropyl |
| 2-SO₂Me-4-CF₃Ph | 4,5-dimethyloxazol-2-yl | CF₃ |
| 2-SO₂Me-4-CF₃Ph | 2-oxazolin-2-yl | H |
| 2-SO₂Me-4-CF₃Ph | 2-oxazolin-2 yl | methyl |
| 2-SO₂Me-4-CF₃Ph | 2-oxazolin-2-yl | i-propyl |
| 2-SO₂Me-4-CF₃Ph | 2-oxazolin-2-yl | cyclopropyl |
| 2-SO₂Me-4-CF₃Ph | 2-oxazolin-2-yl | CF₃ |
| 2-SO₂Me-4-CF₃Ph | 4,4-dimethyl-2-oxazolin Z-yl | H |
| 2-SO₂Me-4-CF₃Ph | 4,4-dimethyl-2-oxazolin-2-yl | methyl |
| 2-SO₂Me-4-CF₃Ph | 4,4-dimethyl-2-oxazolin-2-yl | i-propyl |
| 2-SO₂Me-4-CF₃Ph | 4,4-dimethyl-2-oxazolin-2-yl | cyclopropyl |
| 2-SO₂Me-4-CF₃Ph | 4,4-dimethyl-2-oxazolin-2-yl | CF₃ |
| 2-SO₂Me-4-CF₃Ph | 1,2,4-thiadiazol-5-yl | H |
| 2-SO₂Me-4-CF₃Ph | 1,2,4-thiadiazol-5-yl | methyl |
| 2-SO₂Me-4-CF₃Ph | 1,2,4-thiadiazol-5-yl | i-propyl |
| 2-SO₂Me-4-CF₃Ph | 1,2,4-thiadiazol-5-yl | cyclopropyl |
| 2-SO₂Me-4-CF₃Ph | 1,2,4-thiadiazol-5-yl | CF₃ |
| 2-SO₂Me-4-CF₃Ph | 3-methyl-1,2,4-thiadiazol-5-yl | H |
| 2-SO₂Me-4-CF₃Ph | 3-methyl-1,2,4-thiadiazol-5-yl | methyl |
| 2-SO₂Me-4-CF₃Ph | 3-methyl-1,2,4-thiadiazol-5-yl | i-propyl |
| 2-SO₂Me-4-CF₃Ph | 3-methyl-1,2,4-thiadiazol-5-yl | cyclopropyl |
| 2-SO₂Me-4-CF₃Ph | 3-methyl-1,2,4-thiadiazol-5-yl | CF₃ |
| 2-SO₂Me-4-CF₃Ph | 3-trifluoromethyl-1,2,4-thiadiazol-5-yl | H |
| 2-SO₂Me-4-CF₃Ph | 3-trifluoromethyl-1,2,4 thiadiazol-5-yl | methyl |
| 2-SO₂Me-4-CF₃Ph | 3-trifluoromethyl-1,2,4-thiadiazol-5-yl | i-propyl |
| 2-SO₂Me-4-CF₃Ph | 3-trifluoromethyl-1,2,4-thiadiazol-5-yl | cyclopropyl |
| 2-SO₂Me-4-CF₃Ph | 3-trifluoromethyl-1,2,4-thiadiazol-5-yl | CF₃ |
| 2-SO₂Me-4-CF₃Ph | 1,2,4-thiadiazol-3-yl | H |
| 2-SO₂Me-4-CF₃Ph | 1,2,4-thiadiazol-3-yl | methyl |
| 2-SO₂Me-4-CF₃Ph | 1,2,4-thiadiazol-3-yl | i-propyl |
| 2-SO₂Me-4-CF₃Ph | 1,2,4-thiadiazol-3-yl | cyclopropyl |
| 2-SO₂Me-4-CF₃Ph | 1,2,4-thiadiazol-3-yl | CF₃ |
| 2-SO₂Me-4-CF₃Ph | 5-methyl-1,2,4-thiadiazol-3-yl | H |
| 2-SO₂Me-4-CF₃Ph | 5-methyl-1,2,4-thiadiazol-3-yl | methyl |
| 2-SO₂Me-4-CF₃Ph | 5-methyl-1,2,4-thiadiazol-3-yl | i-propyl |
| 2-SO₂Me-4-CF₃Ph | 5-methyl-1,2,4-thiadiazol-3-yl | cyclopropyl |
| 2-SO₂Me-4-CF₃Ph | 5-methyl-1,2,4-thiadiazol-3-yl | CF₃ |
| 2-SO₂Me-4-CF₃Ph | 5-trifluoromethyl-1,2,4-thiadiazol-3-yl | H |
| 2-SO₂Me-4-CF₃Ph | 5-trifluoromethyl-1,2,4-thiadiazol-3-yl | methyl |
| 2-SO₂Me-4-CF₃Ph | 5-trifluoromethyl-1,2,4-thiadiazol-3-yl | i-propyl |
| 2-SO₂Me-4-CF₃Ph | 5-trifluoromethyl-1,2,4-thiadiazol-3-yl | cyclopropyl |
| 2-SO₂Me-4-CF₃Ph | 5-trifluoromethyl-1,2,4-thiadiazol-3-yl | CF₃ |
| 2-SO₂Me-4-CF₃Ph | 1,3,4 thiadiazol-2-yl | H |
| 2-SO₂Me-4-CF₃Ph | 1,3,4 thiadiazol-2-yl | methyl |
| 2-SO₂Me-4-CF₃Ph | 1,3,4 thiadiazol-2-yl | i-propyl |
| 2-SO₂Me-4-CF₃Ph | 1,3,4 thiadiazol-2-yl | cyclopropyl |
| 2-SO₂Me-4-CF₃Ph | 1,3,4 thiadiazol-2-yl | CF₃ |
| 2-SO₂Me-4-CF₃Ph | 5-methylsulfonyl-1,3,4-thiadiazol-2-yl | H |
| 2-SO₂Me-4-CF₃Ph | 5-methylsulfonyl-1,3,4-thiadiazol-2-yl | methyl |
| 2-SO₂Me-4-CF₃Ph | 5-methylsulfonyl-1,3,4-thiadiazol-2-yl | i-propyl |
| 2-SO₂Me-4-CF₃Ph | 5-methylsulfonyl-1,3,4-thiadiazol-2-yl | cyclopropyl |
| 2-SO₂Me-4-CF₃Ph | 5-methylsulfonyl-1,3,4-thiadiazol-2-yl | CF₃ |
| 2-SO₂Me-4-CF₃Ph | 5-methyl-1,3,4-thiadiazol-2-yl | H |
| 2-SO₂Me-4-CF₃Ph | 5-methyl-1,3,4-thiadiazol-2-yl | methyl |
| 2-SO₂Me-4-CF₃Ph | 5-methyl-1,3,4-thiadiazol-2-yl | i-propyl |
| 2-SO₂Me-4-CF₃Ph | 5-methyl-1,3,4-thiadiazol-2-yl | cyclopropyl |
| 2-SO₂Me-4-CF₃Ph | 5-methyl-1,3,4-thiadiazol-2-yl | CF₃ |
| 2-SO₂Me-4-CF₃Ph | benzoxazol-2-yl | H |
| 2-SO₂Me-4-CF₃Ph | benzoxazol-2-yl | methyl |
| 2-SO₂Me-4-CF₃Ph | benzoxazol-2-yl | i-propyl |
| 2-SO₂Me-4-CF₃Ph | benzoxazol-2-yl | cyclopropyl |
| 2-SO₂Me-4-CF₃Ph | benzoxazol-2-yl | CF₃ |
| 2-SO₂Me-4-CF₃Ph | 6-methylbenzoxazol-2-yl | H |
| 2-SO₂Me-4-CF₃Ph | 6-methylbenzoxazol-2-yl | methyl |
| 2-SO₂Me-4-CF₃Ph | 6-methylbenzoxazol-2-yl | i-propyl |
| 2-SO₂Me-4-CF₃Ph | 6-methylbenzoxazol-2-yl | cyclopropyl |
| 2-S0₂Me-4-CF₃Ph | 6-methylbenzoxazol-2-yl | CF₃ |
| 2-SO₂Me-4-CF₃Ph | benzothiazol-2-yl | H |
| 2-SO₂Me-4-CF₃Ph | benzothiazol-2-yl | methyl |
| 2-SO₂Me-4-CF₃Ph | benzothiazol-2-yl | i-propyl |
| 2-SO₂Me-4-CF₃Ph | benzothiazol-2-yl | cyclopropyl |
| 2-SO₂Me-4-CF₃Ph | benzothiazol-2-yl | CF₃ |
| 2-SO₂Me-4-CF₃Ph | pyrazol-1-yl | H |
| 2-SO₂Me-4-CF₃Ph | pyrazol-1-yl | methyl |
| 2-SO₂Me-4-CF₃Ph | pyrazol-1-yl | i-propyl |
| 2-SO₂Me-4-CF₃Ph | pyrazol-1-yl | cyclopropyl |
| 2-SO₂Me-4-CF₃Ph | pyrazol-1-yl | CF₃ |
| 2-SO₂Me-4-CF₃Ph | pyrazol-3-yl | H |
| 2-SO₂Me-4-CF₃Ph | pyrazol-3-yl | methyl |
| 2-SO₂Me-4-CF₃Ph | pyrazol-3-yl | i-propyl |
| 2-SO₂Me-4-CF₃Ph | pyrazol-3-yl | cyclopropyl |
| 2-SO₂Me-4-CF₃Ph | pyrazol-3-yl | CF₃ |
| 2-SO₂Me-4-CF₃Ph | 1-methylpyrazol-3-yl | H |
| 2-SO₂Me-4-CF₃Ph | 1-methylpyrazol-3-yl | methyl |
| 2-SO₂Me-4-CF₃Ph | 1-methylpyrazol-3-yl | i-propyl |
| 2-SO₂Me-4-CF₃Ph | 1-methylpyrazol-3-yl | cyclopropyl |
| 2-SO₂Me-4-CF₃Ph | 1-methylpyrazol-3-yl | CF₃ |
| 2-SO₂Me-4-CF₃Ph | tetrazol-1-yl | H |
| 2-SO₂Me-4-CF₃Ph | tetrazol-1-yl | methyl |
| 2-SO₂Me-4-CF₃Ph | tetrazol-1-yl | i-propyl |
| 2-SO₂Me-4-CF₃Ph | tetrazol-1-yl | cyclopropyl |
| 2-SO₂Me-4-CF₃Ph | tetrazol-1-yl | CF₃ |
| 2-SO₂Me-4-CF₃Ph | 5-methyltetrazol-1-yl | H |
| 2-SO₂Me-4-CF₃Ph | 5-methyltetrazol-1-yl | methyl |
| 2-SO₂Me-4-CF₃Ph | 5-methyltetrazol-1-yl | i-propyl |
| 2-SO₂Me-4-CF₃Ph | 5-methyltetrazol-1-yl | cyclopropyl |
| 2-SO₂Me-4-CF₃Ph | 5-methyltetrazol-1-yl | CF₃ |
| 2-SO₂Me-4-CF₃Ph | tetrazol-2-yl | H |
| 2-SO₂Me-4-CF₃Ph | tetrazol-2-yl | methyl |
| 2-SO₂Me-4-CF₃Ph | tetrazol-2-yl | i-propyl |
| 2-SO₂Me-4-CF₃Ph | tetrazol-2-yl | cyclopropyl |
| 2-SO₂Me-4-CF₃Ph | tetrazol-2-yl | CF₃ |
| 2-SO₂Me-4-CF₃Ph | 5-methyltetrazol-2-yl | H |
| 2-SO₂Me-4-CF₃Ph | 5-methyltetrazol-2-yl | methyl |
| 2-SO₂Me-4-CF₃Ph | 5-methyltetrazol-2-yl | i-propyl |
| 2-SO₂Me-4-CF₃Ph | 5-methyltetrazol-2-yl | cyclopropyl |
| 2-SO₂Me-4-CF₃Ph | 5-methyltetrazol-2-yl | CF₃ |
| 2-SO₂Me-4-CF₃Ph | 1-methyltetrazol-5-yl | H |
| 2-SO₂Me-4-CF₃Ph | 1-methyltetrazol-5-yl | methyl |
| 2-SO₂Me-4-CF₃Ph | 1-methyltetrazol-5-yl | i-propyl |
| 2-SO₂Me-4-CF₃Ph | 1-methyltetrazol-5-yl | cyclopropyl |
| 2-SO₂Me-4-CF₃Ph | 1-methyltetrazol-5-yl | CF₃ |
| 2-SO₂Me-4-CF₃Ph | 2-methyltetrazol-5-yl | H |
| 2-SO₂Me-4-CF₃Ph | 2-methyltetrazol-5-yl | methyl |
| 2-SO₂Me-4-CF₃Ph | 2-methyltetrazol-5-yl | i-propyl |
| 2-SO₂Me-4-CF₃Ph | 2-methyltetrazol-5-yl | cyclopropyl |
| 2-SO₂Me-4-CF₃Ph | 2-methyltetrazol-5-yl | CF₃ |
| 2-SO₂Me-4-CF₃Ph | pyridin-2-yl | H |
| 2-SO₂Me-4-CF₃Ph | pyridin-2-yl | methyl |
| 2-SO₂Me-4-CF₃Ph | pyridin-2-yl | i-propyl |
| 2-SO₂Me-4-CF₃Ph | pyridin-2-yl | cyclopropyl |
| 2-SO₂Me-4-CF₃Ph | pyridin-2-yl | CF₃ |
| 2-SO₂Me-4-CF₃Ph | pyridin-4-yl | H |
| 2-SO₂Me-4-CF₃Ph | pyridin-4-yl | methyl |
| 2-SO₂Me-4-CF₃Ph | pyridin-4-yl | i-propyl |
| 2-SO₂Me-4-CF₃Ph | pyridin-4-yl | cyclopropyl |
| 2-SO₂Me-4-CF₃Ph | pyridin-4-yl | CF₃ |
| 2-SO₂Me-4-CF₃Ph | pyridin-3-yl | H |
| 2-SO₂Me-4-CF₃Ph | pyridin-3-yl | methyl |
| 2-SO₂Me-4-CF₃Ph | pyridin-3-yl | i-propyl |
| 2-SO₂Me-4-CF₃Ph | pyridin-3-yl | cyclopropyl |
| 2-SO₂Me-4-CF₃Ph | pyridin-3-yl | CF₃ |
| 2-SO₂Me-4-CF₃Ph | 3-nitropyridin-4-yl | H |
| 2-SO₂Me-4-CF₃Ph | 3-nitropyridin-4-yl | methyl |
| 2-SO₂Me-4-CF₃Ph | 3-nitropyridm-4-yl | i-propyl |
| 2-SO₂Me-4-CF₃Ph | 3-nitropyridin-4-yl | cyclopropyl |
| 2-SO₂Me-4-CF₃Ph | 3-nitropyridin-4-yl | CF₃ |
| 2-SO₂Me-4-CF₃Ph | 5-cyanopyridin-2-yl | H |
| 2-SO₂Me-4-CF₃Ph | 5-cyanopyridin-2-yl | methyl |
| 2-SO₂Me-4-CF₃Ph | 5-cyanopyridin-2-yl | i-propyl |
| 2-SO₂Me-4-CF₃Ph | 5-cyanopyridin-2-yl | cyclopropyl |
| 2-SO₂Me-4-CF₃Ph | 5-cyanopyridin-2-yl | CF₃ |
| 2-SO₂Me-4-CF₃Ph | 5-trifluoromethylpyridin-2-yl | H |
| 2-SO₂Me-4-CF₃Ph | 5-trifluoromethylpyridin-2-yl | methyl |
| 2-SO₂Me-4-CF₃Ph | 5-trifluoromethylpyridin-2-yl | i-propyl |
| 2-SO₂Me-4-CF₃Ph | 5-trifluoromethylpyridin-2-yl | cyclopropyl |
| 2-SO₂Me-4-CF₃Ph | 5-trifluoromethylpyridin-2-yl | CF₃ |
| 2-SO₂Me-4-CF₃Ph | pyrimidin-2-yl | H |
| 2-SO₂Me-4-CF₃Ph | pyrimidin-2-yl | methyl |
| 2-SO₂Me-4-CF₃Ph | pyrimidin-2-yl | i-propyl |
| 2-SO₂Me-4-CF₃Ph | pyrimidin-2-yl | cyclopropyl |
| 2-SO₂Me-4-CF₃Ph | pyrimidin-2-yl | CF₃ |
| 2-SO₂Me-4-CF₃Ph | pyrimidin-4-yl | H |
| 2-SO₂Me-4-CF₃Ph | pyrimidin-4-yl | methyl |
| 2-SO₂Me-4-CF₃Ph | pyrimidin-4-yl | i-propyl |
| 2-SO₂Me-4-CF₃Ph | pyrimidin-4-yl | cyclopropyl |
| 2-SO₂Me-4-CF₃Ph | pyrimidin-4-yl | CF₃ |
| 2-SO₂Me-4-CF₃Ph | 6-chloropyrimidin-4-yl | methyl |
| 2-SO₂Me-4-CF₃Ph | 6-chloropyrimidin-4-yl | i-propyl |
| 2-SO₂Me-4-CF₃Ph | 6-chloropyrimidin-4-yl | cyclopropyl |
| 2-SO₂Me-4-CF₃Ph | 6-chloropyrimidin-4-yl | CF₃ |
| 2-SO₂Me-4-CF₃Ph | pyridazin-3-yl | H |
| 2-SO₂Me-4-CF₃Ph | pyridazin-3-yl | methyl |
| 2-SO₂Me-4-CF₃Ph | pyridazin-3-yl | i-propyl |
| 2-SO₂Me-4-CF₃Ph | pyridazin-3-yl | cyclopropyl |
| 2-SO₂Me-4-CF₃Ph | pyridazin-3-yl | CF₃ |
| 2-SO₂Me-4-CF₃Ph | 6-chloropyridazin-3-yl | methyl |
| 2-SO₂Me-4-CF₃Ph | 6-chloropyridazin-3-yl | i-propyl |
| 2-SO₂Me-4-CF₃Ph | 6-chloropyridazin-3-yl | cyclopropyl |
| 2-SO₂Me-4-CF₃Ph | 6-chloropyridazin-3-yl | CF₃ |
| 2-SO₂Me-4-CF₃Ph | pyrazin-2-yl | methyl |
| 2-SO₂Me-4-CF₃Ph | pyrazin-2-yl | i-propyl |
| 2-SO₂Me-4-CF₃Ph | pyrazin-2-yl | cyclopropyl |
| 2-SO₂Me-4-CF₃Ph | pyrazin-2-yl | CF₃ |
| 2-SO₂Me-4-CF₃Ph | triazin-2-yl | methyl |
| 2-SO₂Me-4-CF₃Ph | triazin-2-yl | i-propyl |
| 2-SO₂Me-4-CF₃Ph | triazin-2-yl | cyclopropyl |
| 2-SO₂Me-4-CF₃Ph | triazin-2-yl | CF₃ |
| 2-SO₂Me-4-CF₃Ph | quinolin-2-yl | methyl |
| 2-SO₂Me-4-CF₃Ph | quinolin-2-yl | i-propyl |
| 2-SO₂Me-4-CF₃Ph | quinoli.n-2-yl | cyclopropyl |
| 2-SO₂Me-4-CF₃Ph | quinolin-2-yl | CF₃ |
| 2-SO₂Me-4-CF₃Ph | 4,4,6-trimethyl-5,6-dihydro-1,3(4)-oxazin-2-yl | H |
| 2-SO₂Me-4-CF₃Ph | 4,4,6-trimethyl-5,6-dihydro-1,3(4)-oxazin-2-yl | methyl |
| 2-SO₂Me-4-CF₃Ph | 4,4,6-trimethyl-5,6-dihydro-1,3(4)-oxazin-2-yl | i-propyl |
| 2-SO₂Me-4-CF₃Ph | 4,4,6-trimethyl-5,6-dihydro-1,3(4)-oxazin-2-yl | cyclopropyl |
| 2-SO₂Me-4-CF₃Ph | 4,4,6-trimethyl-5,6-dihydro-1,3(4)-oxazin-2-yl | CF₃ |
| 2-SO₂Me-4-CF₃Ph | 2-oxazolidinon-3-yl | H |
| 2-SO₂Me-4-CF₃Ph | 2-oxazolidinon-3-yl | methyl |
| 2-SO₂Me-4-CF₃Ph | 2-oxazolidinon-3-yl | i-propyl |
| 2-SO₂Me-4-CF₃Ph | 2-oxazolidinon-3-yl | cyclopropyl |
| 2-SO₂Me-4-CF₃Ph | 2-oxazolidinon-3-yl | CF₃ |
| 2-SO₂Me-4-CF₃Ph | 2-pyrrolidinon-1-yl | methyl |
| 2-SO₂Me-4-CF₃Ph | 2-pyrrolidinon-1-yl | i-propyl |
| 2-SO₂Me-4-CF₃Ph | 2-pyrrolidinon-1-yl | cyclopropyl |
| 2-SO₂Me-4-CF₃Ph | 2-pyrrolidinon-1-yl | CF₃ |
| 2-SO₂Me-4-CF₃Ph | 3-methylisoxazol-5-yl | methyl |
| 2-SO₂Me-4-CF₃Ph | 3-methylisoxazol-5-yl | i-propyl |
| 2-SO₂Me-4-CF₃Ph | 3-methylisoxazol-5-yl | cyclopropyl |
| 2-SO₂Me-4-CF₃Ph | 3-methylisoxazol-5-yl | CF₃ |
| 3-Cl-5-CF₃Pyridin-2-yl | 1,2,4-oxadiazol-5-yl | H |
| 3-Cl-5-CF₃Pyridin-2-yl | 1,2,4-oxadiazol-5-yl | methyl |
| 3-Cl-5-CF₃Pyridin-2-yl | 1,2,4-oxadiazol-5-yl | i-propyl |
| 3-Cl-5-CF₃Pyridin-2-yl | 1,2,4-oxadiazol-5-yl | cyclopropyl |
| 3-Cl-5-CF₃Pyridin-2-yl | 1,2,4-oxadiazol-5-yl | CF₃ |
| 3-Cl-5-CF₃Pyridin-2-yl | 3-methyl-1,2,4-oxadiazol-5-yl | H |
| 3-Cl-5-CF₃Pyridin-2-yl | 3-methyl-1,2,4-oxadiazol-5-yl | methyl |
| 3-Cl-5-CF₃Pyridin-2-yl | 3-methyl-1,2,4-oxadiazol-5-yl | i-propyl |
| 3-Cl-5-CF₃Pyridin-2-yl | 3-methyl-1,2,4-oxadiazol-5-yl | cyclopropyl |
| 3-Cl-5-CF₃Pyridin 2-yl | 3-methyl-1,2,4-oxadiazol-5-yl | CF₃ |
| 3-Cl-5-CF₃Pyridin-2-yl | 3-trifluoromethyl-1,2,4-oxadiazol-5-yl | H |
| 3-Cl-5-CF₃Pyridin-2-yl | 3 trifluoromethyl-1,2,4-oxadiazol-5-yl | methyl |
| 3-Cl-5-CF₃Pyridin-2-yl | 3-trifluoromethyl-1,2,4-oxadiazol-5-yl | i-propyl |
| 3-Cl-5-CF₃Pyridin-2-yl | 3-trifluoromethyl-1,2,4-oxadiazol-5-yl | cyclopropyl |
| 3-Cl-5-CF₃Pyridin-2-yl | 3-trifluoromethyl-1,2,4-oxadiazol-5-yl | CF₃ |
| 3-Cl-5-CF₃Pyridin-2-yl | 1,2,4-oxadiazol-3-yl | H |
| 3-Cl-5-CF₃Pyridin-2-yl | 1,2,4-oxadiaaol-3-yl | methyl |
| 3-Cl-5-CF₃Pyridin-2-yl | 1,2,4-oxadiazol-3-yl | i-propyl |
| 3-Cl-5-CF₃Pyridin-2-yl | 1,2,4-oxadiazol-3-yl | cyclopropyl |
| 3-Cl-5-CF₃Pyridin-2-yl | 1,2,4-oxadiazol-3-yl | CF₃ |
| 3-Cl-5-CF₃Pyridin-2-yl | 5-methyl-1,2,4-oxadiazol-3-yi | H |
| 3-Cl-5-CF₃Pyridin-2-yl | 5-methyl-1,2,4-oxadiazol-3-yl | methyl |
| 3-Cl-5-CF₃Pyridin-2-yl | 5-methyl-1,2,4-oxadiazol-3-yl | i-propyl |
| 3-Cl-5-CF₃Pyridin-2-yl | 5-methyl-1,2,4-oxadiazol-3-yl | cyclopropyl |
| 3-Cl-5-CF₃Pyridin-2-yl | 5-methyl-1,2,4-oxadiazol-3-yl | CF₃ |
| 3-Cl-5-CF₃Pyridin-2-yl | 5-trifluoromethyl-1,2,4-oxadiazol-3-yl | H |
| 3-Cl-5-CF₃Pyridin-2-yl | 5-trifluoromethyl-1,2,4-oxadiazol-3-yl | methyl |
| 3-Cl-5-CF₃Pyridin-2-yl | 5-trifluoromethyl-1,2,4-oxadiazol-3-yl | i-propyl |
| 3-Cl-5-CF₃Pyridin-2-yl | 5-trifluoromethyl-1,2,4-oxadiazol-3-yl | cyclopropyl |
| 3-Cl-5-CF₃Pyridin-2-yl | 5-trifluoromethyl-1,2,4-oxadiazol-3-yl | CF₃ |
| 3-Cl-5-CF₃Pyridin-2-yl | 5-chloro-1,2,4-oxadiazol-3-yl | H |
| 3-Cl-5-CF₃Pyridin-2-yl | 5-chloro- 1,2,4-oxadiazol-3-yl | methyl |
| 3-Cl-5-CF₃Pyridin-2-yl | 5-chloro-1,2,4-oxadiazol-3-yl | i-propyl |
| 3-Cl-5-CF₃Pyridin-2-yl | 5-chloro-1,2,4-oxadiazol-3-yl | cyclopropyl |
| 3-Cl-5-CF₃Pyridin-2-yl | 5-chloro-1,2,4-oxadiazol-3-yl | CF₃ |
| 3-Cl-5-CF₃Pyridin-2-yl | 1,3,4-oxadiazol-2-yl | H |
| 3-Cl-5-CF₃Pyridin-2-yl | 1,3,4-oxadiazol-2-yl | methyl |
| 3-Cl-5-CF₃Pyridin-2-yl | 1,3,4-oxadiazol-2-yl | i-propyl |
| 3-Cl-5-CF₃Pyridin-2-yl | 1,3,4-oxadiazol-2-yl | cyclopropyl |
| 3-Cl-5-CF₃Pyridin-2-yl | 1,3,4-oxadiazol-2-yl | CF₃ |
| 3-Cl-5-CF₃Pyridin-2-yl | 5-methylsulfonyl-1,3,4-oxadiazol-2-yl | H |
| 3-Cl-5-CF₃Pyridin-2-yl | 5-methyl-1,2,4-oxadiazol-5-yl | methyl |
| 3-Cl-5-CF₃Pyridin-2-yl | 5-methylsulfonyl-1,3,4-oxadiazol-2-yl | i-propyl |
| 3-Cl-5-CF₃Pyridin-2-yl | 5-methylsulfonyl-1,3,4-oxadiazol-2-yl | cyclopropyl |
| 3-Cl-5-CF₃Pyridin-2-yl | 5-methylsulfonyl-1,3,4-oxadiazol-2-yl | CF₃ |
| 3-Cl-5-CF₃Pyridin-2-yl | 5-methyl-1,3,4-oxadiazol-2-yl | H |
| 3-Cl-5-CF₃Pyridin-2-yl | 5-methyl-1,3,4-oxadiazol-2-yl | methyl |
| 3-Cl-5-CF₃Pyridin-2-yl | 5-methyl-1,3,4-oxadiazol-2-yl | i-propyl |
| 3-Cl-5-CF₃Pyridin-2-yl | 5-methyl-1,3,4-oxadiazol-2-yl | cyclopropyl |
| 3-Cl-5-CF₃Pyridin-2-yl | 5-methyl-1,3,4-oxadiazol-2-yl | CF₃ |
| 3-Cl-5-CF₃Pyridin-2-yl | 5-trifluoromethyl-1,3,4-oxadiazol-2-yl | H |
| 3-Cl-5-CF₃Pyridin-2-yl | 5-trifluoromethyl-1,3,4-oxadiazol-2-yl | methyl |
| 3-Cl-5-CF₃Pyridin-2-yl | 5-trifluoromethyl-1,3,4-oxadiazol-2-yl | i-propyl |
| 3-Cl-5-CF₃Pyridin-2-yl | 5-trifluorometuyl-1,3,4-oxadiazol-2-yl | cyclopropyl |
| 3-Cl-5-CF₃Pyridin-2-yl | 5-trifluoromethyl-1,3,4-oxadiazol-2-yl | CF₃ |
| 3-Cl-5-CF₃Pyridin-2-yl | 1,2,3-triazol-4-yl | H |
| 3-Cl-5-CF₃Pyridin-2-yl | 1,2,3-triazol-4-yl | methyl |
| 3-Cl-5-CF₃Pyridin-2-yl | 1,2,3-triazol-4-yl | i-propyl |
| 3-Cl-5-CF₃Pyridin-2-yl | 1,2,3-triazol-4-yl | cyclopropyl |
| 3-Cl-5-CF₃Pyridin-2-yl | 1,2,3 triazol-4-yl | CF₃ |
| 3-Cl-5-CF₃Pyridin-2-yl | 1-methyl-1,2,3-triazol-4-yl | H |
| 3-Cl-5-CF₃Pyridin-2-yl | 1-methyl-1,2,3-triazol-4-yl | methyl |
| 3-Cl-5-CF₃Pyridin-2-yl | 1-methyl-1,2,3-triazol-4-yl | i-propyl |
| 3-Cl-5-CF₃Pyridin-2-yl | 1-methyl-1,2,3-triazol-4-yl | cyclopropyl |
| 3-Cl-5-CF₃Pyridin-2-yl | 1-methyl-1,2,3-triazol-4-yl | CF₃ |
| 3-Cl-5-CF₃Pyridin-2-yl | 2-methyl-1,2,3-triazol-4-yl | H |
| 3-Cl-5-CF₃Pyridin-2-yl | 2-methyl-1,2,3-triazol-4-yl | methyl |
| 3-Cl-5-CF₃Pyridin-2-yl | 2-methyl-1,2,3-triazol-4-yl | i-propyl |
| 3-Cl-5-CF₃Pyridin-2-yl | 2-methyl-1,2,3 triazol-4-yl | cyclopropyl |
| 3-Cl-5-CF₃Pyridin-2-yl | 2-methyl-1,2,3-triazol-4-yl | CF₃ |
| 3-Cl-5-CF₃Pyridin-2-yl | 1,2,3-triazol-1-yl | H |
| 3-Cl-5-CF₃Pyridin-2-yl | 1,2,3-triazol-1-yl | methyl |
| 3-Cl-5-CF₃Pyridin-2-yl | 1,2,3-triazol-1-yl | i-propyl |
| 3-Cl-5-CF₃Pyridin-2-yl | 1,2,3-triazol-1-yl | cyclopropyl |
| 3-Cl-5-CF₃Pyridin-2-yl | 1,2,3-triazol-1-yl | CF₃ |
| 3-Cl-5-CF₃Pyridin-2-yl | 1,2,3-triazol-2-yl | H |
| 3-Cl-5-CF₃Pyridin-2-yl | 1,2,3-triazol-1-yl | methyl |
| 3-Cl-5-CF₃Pyridin-2-yl | 1,2,3-triazol-1-yl | i-propyl |
| 3-Cl-5-CF₃Pyridin-2-yl | 1,2,3-triazol-1-yl | cyclopropyl |
| 3-Cl-5-CF₃Pyridin-2-yl | 1,2,3-triazol-2-yl | CF₃ |
| 3-Cl-5-CF₃Pyridin-2-yl | 1,2,3-triazol-1-yl | H |
| 3-Cl-5-CF₃Pyridin-2-yl | 1,2,3-triazol-1-yl | methyl |
| 3-Cl-5-CF₃Pyridin-2-yl | 1,2,3-triazol-1-yl | i-propyl |
| 3-Cl-5-CF₃Pyridin-2-yl | 1,2,3-triazol-1-yl | cyclopropyl |
| 3-Cl-5-CF₃Pyridin-2-yl | 1,2,3-triazol-1-yl | CF₃ |
| 3-Cl-5-CF₃Pyridin-2-yl | imidazol-2-yl | H |
| 3-Cl-5-CF₃Pyridin-2-yl | imidazol-2-yl | methyl |
| 3-Cl-5-CF₃Pyridin-2-yl | imidazol-2-yl | i-propyl |
| 3-Cl-5-CF₃Pyridin-2-yl | imidazol-2-yl | cyclopropyl |
| 3-Cl-5-CF₃Pyridin-2-yl | imidazol-2-yl | CF₃ |
| 3-Cl-5-CF₃Pyridin-2-yl | imidazol-1-yl | H |
| 3-Cl-5-CF₃Pyridin-2-yl | imidazol-1-yl | methyl |
| 3-Cl-5-CF₃Pyridin-2-yl | imidazol-1-yl | i-propyl |
| 3-Cl-5-CF₃Pyridin-2-yl | imidazol-1-yl | cyclopropyl |
| 3-Cl-5-CF₃Pyridin-2-yl | imidazol-1-yl | CF₃ |
| 3-Cl-5-CF₃Pyridin-2-yl | imidazol-4-yl | H |
| 3-Cl-5-CF₃Pyridin-2-yl | imidazol-4-yl | methyl |
| 3-Cl-5-CF₃Pyridin-2-yl | imidazol-4-yl | i-propyl |
| 3-Cl-5-CF₃Pyridin-2-yl | imidazol-4-yl | cyclopropyl |
| 3-Cl-5-CF₃Pyridin-2-yl | imidazol-4-yl | CF₃ |
| 3-Cl-5-CF₃Pyridin-2-yl | thiazol-2-yl | H |
| 3-Cl-5-CF₃Pyridin-2-yl | thiazol-2-yl | methyl |
| 3-Cl-5-CF₃Pyridin-2-yl | thiazol-2-yl | i-propyl |
| 3-Cl-5-CF₃Pyridin-2-yl | thiazol-2-yl | cyclopropyl |
| 3-Cl-5-CF₃Pyridin-2-yl | thiazol-2-yl | CF₃ |
| 3-Cl-5-CF₃Pyridin-2-yl | 4-methylthiazol-2-yl | H |
| 3-Cl-5-CF₃Pyridin-2-yl | 4-methylthiazol-2-yl | methyl |
| 3-Cl-5-CF₃Pyridin-2-yl | 4-methylthiazol-2-yl | i-propyl |
| 3-Cl-5-CF₃Pyridin-2-yl | 4-methylthiazol-2-yl | cyclopropyl |
| 3-Cl-5-CF₃Pyridin-2-yl | 4-methylthiazol-2-yl | CF₃ |
| 3-Cl-5-CF₃Pyridin-2-yl | oxazol-2-yl | H |
| 3-Cl-5-CF₃Pyridin-2-yl | oxazol-2-yl | methyl |
| 3-Cl-5-CF₃Pyridin-2-yl | oxazol-2-yl | i-propyl |
| 3-Cl-5-CF₃Pyridin-2-yl | oxazol-2-yl | cyclopropyl |
| 3-Cl-5-CF₃Pyridin-2-yl | oxazol-2-yl | CF₃ |
| 3-Cl-5-CF₃Pyridin-2-yl | 4,5-dimethyloxazol-2-yl | H |
| 3-Cl-5-CF₃Pyridin-2-yl | 4,5-dimethyloxazol-Z-yl | methyl |
| 3-Cl-5-CF₃Pyridin-2-yl | 4,5-dimethyloxazol-2-yl | i-propyl |
| 3-Cl-5-CF₃Pyridin-2-yl | 4,5-dimethyloxazol-2-yl | cyclopropyl |
| 3-Cl-5-CF₃Pyridin-2-yl | 4,5-dimethyloxazol-2-yl | CF₃ |
| 3-Cl-5-CF₃Pyridin-2-yl | 2-oxazolin-2-yl | H |
| 3-Cl-5-CF₃Pyridin-2-yl | 2-oxazolin-2-yl | methyl |
| 3-Cl-5-CF₃Pyridin-2-yl | 2-oxazolin-2-yl | i-propyl |
| 3-Cl-5-CF₃Pyridin-2-yl | 2-oxazolin-2-yl | cyclopropyl |
| 3-Cl-5-CF₃Pyridin-2-yl | 2-oxazolin-2-yl | CF₃ |
| 3-Cl-5-CF₃Pyridin-2-yl | 4,4-dimethyl-2-oxazolin-2-yl | H |
| 3-Cl-5-CF₃Pyridin-2-yl | 4,4-dimethyl-2-oxazolin-2-yl | methyl |
| 3-Cl-5-CF₃Pyridin-2-yl | 4,4-dimethyl-2-oxazolin-2-yl | i-propyl |
| 3-Cl-5-CF₃Pyridin-2-yl | 4,4-dimethyl-2-oxazolin-2-yl | cyclopropyl |
| 3-Cl-5-CF₃Pyridin-2-yl | 4,4-dimethyl-2-oxazolin-2-yl | CF₃ |
| 3-Cl-5-CF₃Pyridin-2-yl | 1,2,4-thiadiazol-5-yl | H |
| 3-Cl-5-CF₃Pyridin-2-yl | 1,2,4-thiadiazol-5-yl | methyl |
| 3-Cl-5-CF₃Pyridin-2-yl | 1,2,4-thiadiazol-5-yl | i-propyl |
| 3-Cl-5-CF₃Pyridin-2-yl | 1,2,4-thiadiazol-5-yl | cyclopropyl |
| 3-Cl-5-CF₃Pyridin-2-yl | 1,2,4-thiadiazol-5-yl | CF₃ |
| 3-Cl-5-CF₃Pyridin-2-yl | 3-methyl-1,2,4-thiadiazol-5-yl | H |
| 3-Cl-5-CF₃Pyridin-2-yl | 3-methyl-1,2,4-thiadiazol-5-yl | methyl |
| 3-Cl-5-CF₃Pyridin-2-yl | 3-methyl-1,2,4-thiadiazol-5-yl | i-propyl |
| 3-Cl-5-CF₃Pyridin-2-yl | 3-methyl-1,2,4-thiadiazol-5-yl | cyclopropyl |
| 3-Cl-5-CF₃Pyridin-2-yl | 3-methyl-1,2,4-thiadiazol-5-yl | CF₃ |
| 3-Cl-5-CF₃Pyridin-2-yl | 3-trifluoromethyl-1,2,4-thiadiazol-5-yl | H |
| 3-Cl-5-CF₃Pyridin-2-yl | 3-trifluoromethyl-1,2,4-thiadiazol-5-yl | methyl |
| 3-Cl-5-CF₃Pyridin-2-yl | 3-trifluoromethyl-1,2,4-thiadiazol-5-yl | i-propyl |
| 3-Cl-5-CF₃Pyridin-2-yl | 3-trifluoromethyl-1,2,4-thiadiazol-5-yl | cyclopropyl |
| 3-Cl-5-CF₃Pyridin-2-yl | 3-trifluoromethyl-1,2,4-thiadiazol-5-yl | CF₃ |
| 3-Cl-5-CF₃Pyridin-2-yl | 1,2,4-thiadiazol-3-yl | H |
| 3-Cl-5-CF₃Pyridin-2-yl | 1,2,4-thiadiazol-3-yl | methyl |
| 3-Cl-5-CF₃Pyridin-2-yl | 1,2,4-thiadiazol-3-yl | i-propyl |
| 3-Cl-5-CF₃Pyridin-2-yl | 1,2,4-thiadiazol-3-yl | cyclopropyl |
| 3-Cl-5-CF₃Pyridin-2-yl | 1,2,4-thiadiazol-3-yl | CF₃ |
| 3-Cl-5-CF₃Pyridin-2-yl | 5-methyl-1,2,4-thiadiazol-3-yl | H |
| 3-Cl-5-CF₃Pyridin-2-yl | 5-methyl-1,2,4-thiadiazol-3-yl | methyl |
| 3-Cl-5-CF₃Pyridin-2-yl | 5-methyl-1,2,4-thiadiazol-3-yl | i-propyl |
| 3-Cl-5-CF₃Pyridin-2-yl | 5-methyl-1,2,4-thiadiazol-3-yl | cyclopropyl |
| 3-Cl-5-CF₃Pyridin-2-yl | 5-methyl-1,2,4-thiadiazol-3-yl | CF₃ |
| 3-Cl-5-CF₃Pyridin-2-yl | 5-trifluoromethyl-1,2,4-thiadiazol-3-yl | H |
| 3-Cl-5-CF₃Pyridin-2-yl | 5-trifluoromethyl-1,2,4-thiadiazol-3-yl | methyl |
| 3-Cl-5-CF₃Pyridin-2-yl | 5-trifluoromethyl-1,2,4-thiadizol-3-yl | i-propyl |
| 3-Cl-5-CF₃Pyridin-2-yl | 5-tlifluorometllyl-1,2,4-thiadiazol-3-yl | cyclopropyl |
| 3-Cl-5-CF₃Pyridin-2-yl | 5-trifluoromethyl-1,2,4-thiadiazol-3-yl | CF₃ |
| 3-Cl-5-CF₃Pyridin-2-yl | 1,3,4-thiadiazol-2-yl | H |
| 3-Cl-5-CF₃Pyridin-2-yl | 1,3,4-thiadiazol-2-yl | methyl |
| 3-Cl-5-CF₃Pyridin-2-yl | 1,3,4-thiadiazol-2-yl | i-propyl |
| 3-Cl-5-CF₃Pyridin-2-yl | 1,3,4-thiadiazol-2-yl | cyclopropyl |
| 3-Cl-5-CF₃Pyridin-2-yl | 1,3,4-thiadiazol-2-yl | CF₃ |
| 3-Cl-5-CF₃Pyridin-2-yl | 5-methylsulfonyl-1,3,4-thiadizol-2-yl | H |
| 3-Cl-5-CF₃Pyridin-2-yl | 5-methylsulfonyl-1,3,4-thiadiazol-2-yl | methyl |
| 3-Cl-5-CF₃Pyridin 2-yl | 5-methylsulfonyl-1,3,4-thiadiazol-2-yl | i-propyl |
| 3-Cl-5-CF₃Pyridin-2-yl | 5-methylsulfonyl-1,3,4-thiadiazol-2-yl | cyclopropyl |
| 3-Cl-5-CF₃Pyridin-2-yl | 5-methylsulfonyl-1,3,4-thiadiazol-2-yl | CF₃ |
| 3-Cl-5-CF₃Pyridin-2-yl | 5-methyl-1,3,4-thiadiazol-2-yl | H |
| 3-Cl-5-CF₃Pyridin-2-yl | 5-methyl-1,3,4-thiadiazol-2-yl | methyl |
| 3-Cl-5-CF₃Pyridin-2-yl | 5-methyl-1,3,4-thiadiazol-2-yl | i-propyl |
| 3-Cl-5-CF₃Pyridin-2-yl | 5-methyl-1,3,4-thiadiazol-2-yl | cyclopropyl |
| 3-Cl-5-CF₃Pyridin-2-yl | 5-methyl-1,3,4-thiadiazol-2-yl | CF₃ |
| 3-Cl-5-CF₃Pyridin-2-yl | benzoxazol-2-yl | H |
| 3-Cl-5-CF₃Pyridin-2-yl | benzoxazol-2-yl | methyl |
| 3-Cl-5-CF₃Pyridin-2-yl | benzoxazol-2-yl | i-propyl |
| 3-Cl-5-CF₃Pyridin-2-yl | benzoxazol-2-yl | cyclopropyl |
| 3-Cl-5-CF₃Pyridin-2-yl | benzoxazol-2-yl | CF₃ |
| 3-Cl-5-CF₃Pyridin-2-yl | 6-methylbenzoxazol-2-yl | H |
| 3-Cl-5-CF₃Pyridin-2-yl | 6-methylbenzoxazol-2-yl | methyl |
| 3-Cl-5-CF₃Pyridin-2-yl | 6-methylbenzoxazol-2-yl | i-propyl |
| 3-Cl-5-CF₃Pyridin-2-yl | 6-methylbenzoxazol-2-yl | cyclopropyl |
| 3-Cl-5-CF₃Pyridin-2-yl | 6-methylbenzoxazol-2-yl | CF₃ |
| 3-Cl-5-CF₃Pyridin-2-yl | benzothiazol-2-yl | H |
| 3-Cl-5-CF₃Pyridin-2-yl | benzothiazol-2-yl | methyl |
| 3-Cl-5-CF₃Pyridin-2-yl | benzothiazol-2-yl | i-propyl |
| 3-Cl-5-CF₃Pyridin-2-yl | benzothiazol-2-yl | cyclopropyl |
| 3-Cl-5-CF₃Pyridin-2-yl | benzothiazol-2-yl | CF₃ |
| 3-Cl-5-CF₃Pyidin-2-yl | pyrazol-1-yl | H |
| 3-Cl-5-CF₃Pyridin-2-yl | pyrazol-1-yl | methyl |
| 3-Cl-5-CF₃Pyridin-2-yl | pyrazol-1-yl | i-propyl |
| 3-Cl-5-CF₃Pyridin-2-yl | pyrazol-1-yl | cyclopropyl |
| 3-Cl-5-CF₃Pyridin-2-yl | pyrazol-1-yl | CF₃ |
| 3-Cl-5-CF₃Pyridin-2-yl | pyrazol-3-yl | H |
| 3-Cl-5-CF₃Pyridin-2-yl | pyrazol-3-yl | methyl |
| 3-Cl-5-CF₃Pyridin-2-yl | pyrazol-3-yl | i-propyl |
| 3-Cl-5-CF₃Pyridin-2-yl | pyrazol-3-yl | cyclopropyl |
| 3-Cl-5-CF₃Pyridin-2-yl | pyrazol-3-yl | CF₃ |
| 3-Cl-5-CF₃Pyridin-2-yl | 1-methylpyrazol-3-yl | H |
| 3-Cl-5-CF₃Pyridin-2-yl | 1-methylpyrazol-3-yl | methyl |
| 3-Cl-5-CF₃Pyridin-2-yl | 1-methylpyrazol-3-yl | i-propyl |
| 3-Cl-5-CF₃Pyridin-2-yl | 1-methylpyrazol-3-yl | cyclopropyl |
| 3-Cl-5-CF₃Pyridin-2-yl | 1-methylpyrazol-3-yl | CF₃ |
| 3-Cl-5-CF₃Pyridin-2-yl | tetrazol-1-yl | H |
| 3-Cl-5-CF₃Pyridin-2-yl | tetrazol-1-yl | methyl |
| 3-Cl-5-CF₃Pyridin-2-yl | tetrazol-1-yl | i-propyl |
| 3-Cl-5-CF₃Pyridin-2-yl | tetrazol-1-yl | cyclopropyl |
| 3-Cl-5-CF₃Pyridin-2-yl | tetrazol-1-yl | CF₃ |
| 3-Cl-5-CF₃Pyridin-2-yl | 5-methyltetrazol-1-yl | H |
| 3-Cl-5-CF₃Pyridin-2-yl | 5-methyltetrazol-1-yl | methyl |
| 3-Cl-5-CF₃Pyridin-2-yl | 5-methyltetrazol-1-yl | i-propyl |
| 3-Cl-5-CF₃Pyridin-2-yl | 5-methyltetrazol-1-yl | cyclopropyl |
| 3-Cl-5-CF₃Pyridin-2-yl | 5-methyltetrazol-1-yl | CF₃ |
| 3-Cl-5-CF₃Pyridin-2-yl | tetrazol-2-yl | H |
| 3-Cl-5-CF₃Pyridin-yl | tetrazol-2-yl | methyl |
| 3-Cl-5-CF₃Pyridin-2-yl | tetrazol-2-yl | i-propyl |
| 3-Cl-5-CF₃Pyridin-2-yl | tetrazol-2-yl | cyclopropyl |
| 3-Cl-5-CF₃Pyridin-2-yl | tetrazol-2-yl | CF₃ |
| 3-Cl-5-CF₃Pyridin-2-yl | 5-methyltetrazol-2-yl | H |
| 3-Cl-5-CF₃Pyridin-2-yl | 5-methyltetrazol-2-yl | methyl |
| 3-Cl-5-CF₃Pyridin-2-yl | 5-methyltetrazol-2-yl | i-propyl |
| 3-Cl-5-CF₃Pyridin-2-yl | 5-methyltetrazol-2-yl | cyclopropyl |
| 3-Cl-5-CF₃Pyridin-2-yl | 5-methyltetrazol-2-yl | CF₃ |
| 3-Cl-5-CF₃Pyridin-2-yl | 1-methyltetrazol-5-yl | H |
| 3-Cl-5-CF₃Pyridin-2-yl | 1-methyltetrazol-5-yl | methyl |
| 3-Cl-5-CF₃Pyridin-2-yl | 1-methyltetrazol-5-yl | i-propyl |
| 3-Cl-5-CF₃Pyridin-2-yl | 1-methyltetrazol-5-yl | cyclopropyl |
| 3-Cl-5-CF₃Pyridin-2-yl | 1-methyltetrazol-5-yl | CF₃ |
| 3-Cl-5-CF₃Pyridin-2-yl | 2-methyltetrazol-5-yl | H |
| 3-Cl-5-CF₃Pyridin-2-yl | 2-methyltetrazol-5-yl | methyl |
| 3-Cl-5-CF₃Pyridin-2-yl | 2-methyltetrazol-5-yl | i-propyl |
| 3-Cl-5-CF₃Pyridin-2-yl | 2-methyltetrazol-5-yl | cyclopropyl |
| 3-Cl-5-CF₃Pyridin-2-yl | 2-methyltetrazol-5-yl | CF₃ |
| 3-Cl-5-CF₃Pyridin-2-yl | pyridin-2-yl | H |
| 3-Cl-5-CF₃Pyridin-2-yl | pyridin-2-yl | methyl |
| 3-Cl-5-CF₃Pyridin-2-yl | pyridin-2-yl | i-propyl |
| 3-Cl-5-CF₃Pyridin-2-yl | pyridin-2-yl | cyclopropyl |
| 3-Cl-5-CF₃Pyridin-2-yl | pyridin-2-yl | CF₃ |
| 3-Cl-5-CF₃Pyridin-2-yl | pyridin-4-yl | H |
| 3-Cl-5-CF₃Pyridin-2-yl | pyridin-4-yl | methyl |
| 3-Cl-5-CF₃Pyridin-2-yl | pyridin-4-yl | i-propyl |
| 3-Cl-5-CF₃Pyridin-2-yl | pyridin-4-yl | cyclopropyl |
| 3-Cl-5-CF₃Pyridin-2-yl | pyridin-4-yl | CF₃ |
| 3-Cl-5-CF₃Pyridin-2-yl | pyridin-3-yl | H |
| 3-Cl-5-CF₃Pyridin-2-yl | pyridin-3-yl | methyl |
| 3-Cl-5-CF₃Pyridin-2-yl | pyridin-3-yl | i-propyl |
| 3-Cl-5-CF₃Pyridin-2-yl | pyridin-3-yl | cyclopropyl |
| 3-Cl-5-CF₃Pyridin-2-yl | pyridin-3-yl | CF3 |
| 3-Cl-5-CF₃Pyridin-2-yl | 3-nitropyridin-4-yl | H |
| 3-Cl-5-CF₃Pyridin-2-yl | 3-nitropyridin-4-yl | methyl |
| 3-Cl-5-CF₃Pyridin-2-yl | 3-nitropyridin-4-yl | i-propyl |
| 3-Cl-5-CF₃Pyridin-2-yl | 3-nitropyridin-4-yl | cyclopropyl |
| 3-Cl-5-CF₃Pyridin-2-yl | 3-nitropyridin-4-yl | CF₃ |
| 3-Cl-5-CF₃Pyridin-2-yl | 5-cyanopyridin-2-yl | H |
| 3-Cl-5-CF₃Pyridin-2-yl | 5-cyanopyridin-2-yl | methyl |
| 3-Cl-5-CF₃Pyridin-2-yl | 5-cyanopyridin-2-yl | i-propyl |
| 3-Cl-5-CF₃Pyridin-2-yl | 5-cyanopyridin-2-yl | cyclopropyl |
| 3-Cl-5-CF₃Pyridin-2-yl | 5-cyanopyridin-2-yl | CF₃ |
| 3-Cl-5-CF₃Pyridin-2-yl | 5-trifluoromethylpyridin-2-yl | H |
| 3-Cl-5-CF₃Pyridin-2-yl | 5-trifluoromethylpyridin-2-yl | methyl |
| 3-Cl-5-CF₃Pyridin-2-yl | 5-trifluoromethylpyridin-2-yl | i-propyl |
| 3-Cl-5-CF₃Pyridin-2-yl | 5-trifluoromethylpyridin-2-yl | cyclopropyl |
| 3-Cl-5-CF₃Pyridin-2-yl | 5-trifluoromethylpyridin-2-yl | CF₃ |
| 3-Cl-5-CF₃Pyridin-2-yl | pyrimidin-2-yl | H |
| 3-Cl-5-CF₃Pyridin-2-yl | pyrimidin-2-yl | methyl |
| 3-Cl-5-CF₃Pyridin-2-yl | pyrimidin-2-yl | i-propyl |
| 3-Cl-5-CF₃Pyridin-2-yl | pyrimidin-2-yl | cyclopropyl |
| 3-Cl-5-CF₃Pyridin-2-yl | pyrimidin-2-yl | CF₃ |
| 3-Cl-5-CF₃Pyridin-2-yl | pyrimidin-4-yl | H |
| 3-Cl-5-CF₃Pyridin-2-yl | pyrimidin-4-yl | methyl |
| 3-Cl-5-CF₃Pyridin-2-yl | pyrimidin-4-yl | i-propyl |
| 3-Cl-5-CF₃Pyridin-2-yl | pyrimidin-4-yl | cyclopropyl |
| 3-Cl-5-CF₃Pyridin-2-yl | pyrimidin-4-yl | CF₃ |
| 3-Cl-5-CF₃Pyridin-2-yl | 6-chloropyrimidin-4-yl | methyl |
| 3-Cl-5-CF₃Pyridin-2-yl | 6-chloropyrimidin-4-yl | i-propyl |
| 3-Cl-5-CF₃Pyridin-2-yl | 6-chloropyrimidin-4-yl | cyclopropyl |
| 3-Cl-5-CF₃Pyridin-2-yl | 6-chloropyrimidin-4-yl | CF₃ |
| 3-Cl-5-CF₃Pyridin-2-yl | pyridazin-3-yl | H |
| 3-Cl-5-CF₃Pyridin-2-yl | pyridazin-3-yl | methyl |
| 3-Cl-5-CF₃Pyridin-2-yl | pyridazin-3-yl | i-propyl |
| 3-Cl-5-CF₃Pyridin-2-yl | pyridazin-3-yl | cyclopropyl |
| 3-Cl-5-CF₃Pyridin-2-yl | pyridazin-3-yl | CF₃ |
| 3-Cl-5-CF₃Pyridin-2-yl | 6-chloropyridazin-3-yl | methyl |
| 3-Cl-5-CF₃Pyridin-2-yl | 6-chloropyridazin-3-yl | i-propyl |
| 3-Cl-5-CF₃Pyridin-2-yl | 6-chloropyridazin-3-yl | cyclopropyl |
| 3-Cl-5-CF₃Pyridin-2-yl | 6-chloropyridazin-3-yl | CF₃ |
| 3-Cl-5-CF₃Pyridin-2-yl | pyrazin-2-yl | methyl |
| 3-Cl-5-CF₃Pyridin-2-yl | pyrazin-2-yl | i-propyl |
| 3-Cl-5-CF₃Pyridin-2-yl | pyrazin-2-yl | cyclopropyl |
| 3-Cl-5-CF₃Pyridin-2-yl | pyrazin-2-yl | CF₃ |
| 3-Cl-5-CF₃Pyridin-2-yl | triazin-2-yl | methyl |
| 3-Cl-5-CF₃Pyridin-2-yl | triazin-2-yl | i-propyl |
| 3-Cl-5-CF₃Pyridin-2-yl | triazin-2-yl | cyclopropyl |
| 3-Cl-5-CF₃Pyridin-2-yl | triazin-2-yl | CF₃ |
| 3-Cl-5-CF₃Pyridin-2-yl | quinolin-2-yl | methyl |
| 3-Cl-5-CF₃Pyridin-2-yl | quinolin-2-yl | i-propyl |
| 3-Cl-5-CF₃Pyridin-2-yl | quinolin-2-yl | cyclopropyl |
| 3-Cl-5-CF₃Pyridin-2-yl | quinolin-2-yl | CF₃ |
| 3-Cl-5-CF₃Pyridin-2-yl | 4,4,6-trimethyl-5,6-dihydro-1,3(4H)-oxazin-2-yl | H |
| 3-Cl-5-CF₃Pyridin-2-yl | 4,4,6-trimethyl-5,6-dihydro-1,3(4H)-oxazin-2-yl | methyl |
| 3-Cl-5-CF₃Pyridin-2-yl | 4,4,6-trimethyl-5,6-dihydro-1,3(4H)-oxazin-2-yl | i-propyl |
| 3-Cl-5-CF₃Pyridin-2-yl | 4,4,6-trimethyl-5,6-dihydro-1,3(4H)-oxazin-2-yl | cyclopropyl |
| 3-Cl-5-CF₃Pyridin-2-yl | 4,4,6-trimethyl-5,6-dihydro-1,3(4H)-oxazin-2-yl | CF₃ |
| 3-Cl-5-CF₃-Pyridin-2-yl | 2-oxazolidinon-3-yl | H |
| 3-Cl-5-CF₃Pyridin-2-yl | 2-oxazolidinon-3-yl | methyl |
| 3-Cl-5-CF₃Pyridin-2-yl | 2-oxazolidinon-3-yl | i-propyl |
| 3-Cl-5-CF₃Pyridin-2-yl | 2-oxazolidinon-3-yl | cyclopropyl |
| 3-Cl-5-CF₃Pyridin-2-yl | 2-oxazolidinon-3-yl | CF₃ |
| 3-Cl-5-CF₃Pyridin-2-yl | 2-pyrrolidinon-1-yl | methyl |
| 3-Cl-5-CF₃Pyridin-2-yl | 2-pyrrolidinon-1-yl | i-propyl |
| 3-Cl-5-CF₃Pyridin-2-yl | 2-pyrrolidinon-1-yl | cyclopropyl |
| 3-Cl-5-CF₃Pyridin-2-yl | 2-pyrrolidinon-1-yl | CF₃ |
| 3-Cl-5-CF₃Pyridin-2-yl | 3-methylisoxazol-5-yl | methyl |
| 3-Cl-5-CF₃Pyridin-2-yl | 3-methylisoxazol-5-yl | i-propyl |
| 3-Cl-5-CF₃Pyridin-2-yl | 3-methylisoxazol-5-yl | cyclopropyl |
| 3-Cl-5-CF₃Pyridin-2-yl | 3-methylisoxazol-5-yl | CF₃ |
| 2,4-(Me)₂Thiazol-5-yl | 1,2,4-oxadiazol-5-yl | H |
| 2,4-(Me)₂Thiazol-5-yl | 1,2,4-oxadiazol-5-yl | methyl |
| 2,4-(Me)₂Thiazol-5-yl | 1,2,4-oxadiazol-5-yl | i-propyl |
| 2,4-(Me)₂Thiazol-5-yl | 1,2,4-oxadiazol-5-yl | cyclopropyl |
| 2,4-(Me)₂Thiazol-5-yl | 1,2,4-oxadiazol-5-yl | CF₃ |
| 2,4-(Me)₂Thiazol-5-yl | 3-methyl-1,2,4-oxadiazol-5-yl | H |
| 2,4-(Me)₂Thiazol-5-yl | 3-methyl-1,2,4-oxadiazol-5-yl | methyl |
| 2,4-(Me)₂Thiazol-5-yl | 3-methyl-1,2,4-oxadiazol-5-yl | i-propyl |
| 2,4-(Me)₂Thiazol-5-yl | 3-methyl-1,2,4-oxadiazol-5-yi | cyclopropyl |
| 2,4-(Me)₂Thiazol-5-yl | 3-methyl-1,2,4-oxadiazol-5-yl | CF₃ |
| 2,4-(Me)₂Thiazol-5-yl | 3-trifluoromethyl-1,2,4-oxadiazol-5-yl | H |
| 2,4-(Me)₂Thiazol-5-yl | 3-trifluoromethyl-1,2,4-oxadiazol-5-yl | methyl |
| 2,4-(Me)₂Thiazol-5-yl | 3-trifluoromethyl-1,2,4-oxadiazol-5-yl | i-propyl |
| 2,4-(Me)₂Thiazol-5-yl | 3-trifluoromethyl-1,2,4-oxadiazol-5-yl | cyclopropyl |
| 2,4-(Me)₂Thiazol-5-yl | 3-trifluoromethyl-1,2,4-oxadiazol-5-yl | CF₃ |
| 2,4-(Me)₂Thiazol-5-yl | 1,2,4-oxadiazol-3-yl | H |
| 2,4-(Me)₂Thiazol-5-yl | 1,2,4-oxadiazol-3-yl | methyl |
| 2,4-(Me)₂Thiazol-5-yl | 1,2,4-oxadiazol-3-yl | i-propyl |
| 2,4-(Me)₂Thiazol-5-yl | 1,2,4-oxadiazol-3-yl | cyclopropyl |
| 2,4-(Me)₂Thiazol-5-yl | 1,2,4-oxadiazol-3-yl | CF₃ |
| 2,4-(Me)₂Thiazol-5-yl | 5-methyl-1,2,4-oxadiazol-3-yl | H |
| 2,4-(Me)₂Thiazol-5-yl | 5-methyl-1,2,4-oxadiazol-3-yl | methyl |
| 2,4-(Me)₂Thiazol-5-yl | 5-methyl-1,2,4-oxadiazol-3-yl | i-propyl |
| 2,4-(Me)₂Thiazol-5-yl | 5-methyl-1,2,4-oxadiazol-3-yl | cyclopropyl |
| 2,4-(Me)₂Thiazol-5-yl | 5-methyl-1,2,4-oxadiazol-3-yl | CF₃ |
| 2,4-(Me)₂Thiazol-5-yl | 5-trifluoromethyl-1,2,4-oxadiazol-3-yl | H |
| 2,4-(Me)₂Thiazol-5-yl | 5-trifluoromethyl-1,2,4-oxadiazol-3-yl | methyl |
| 2,4-(Me)₂Thiazol-5-yl | 5-trifluoromethyl-1,2,4-oxadiazol-3-yl | i-propyl |
| 2,4-(Me)₂Thiazol-5-yl | 5-trifluoromethyl-1,2,4-oxadiazol-3-yl | cyclopropyl |
| 2,4-(Me)₂Thiazol-5-yl | 5-trifluoromethyl-1,2,4-oxadiazol-3-yl | CF₃ |
| 2,4-(Me)₂Thiazol-5-yl | 5-chloro-1,2,4-oxadiazol-3-yl | H |
| 2,4-(Me)₂Thiazol-5-yl | 5-chloro-1,2,4-oxadiazol-3-yl | methyl |
| 2,4-(Me)₂Thiazol-5-yl | 5-chloro-1,2,4-oxadiazol-3-yl | i-propyl |
| 2,4-(Me)₂Thiazol-5-yl | 5-chloro-1,2,4-oxadiazol-3-yl | cyclopropyl |
| 2,4-(Me)₂Thiazol-5-yl | 5-chloro-1,2,4-oxadiazol-3-yl | CF₃ |
| 2,4-(Me)₂Thiazol-5-yl | 1,3,4-oxadiazol-2-yl | H |
| 2,4-(Me)₂Thiazol-5-yl | 1,3,4-oxadiazol-2-yl | methyl |
| 2,4-(Me)₂Thiazol-5-yl | 1,3,4-oxadiazol-2-yl | i-propyl |
| 2,4-(Me)₂Thiazol-5-yl | 1,3,4-oxadiazol-2-yl | cyclopropyl |
| 2,4-(Me)₂Thiazol-5-yl | 1,3,4-oxadiazol-2-yl | CF₃ |
| 2,4-(Me)₂Thiazol-5-yl | 5-methylsulfonyl-1,3,4-oxadiazol-2-yl | H |
| 2,4-(Me)₂Thiazol-5-yl | 5-methylsulfonyl-1,3,4-oxadiazol-2-yl | methyl |
| 2,4-(Me)₂Thiazol-5-yl | 5-methylsulfonyl-1,3,4-oxadiazol-2-yl | i-propyl |
| 2,4-(Me)₂Thiazol-5-yl | 5-methylsulfonyl-1,3,4-oxadiazol-2-yl | cyclopropyl |
| 2,4-(Me)₂Thiazol-5-yl | 5-methylsulfonyl-1,3,4-oxadiazol-2-yl | CF₃ |
| 2,4-(Me)₂Thiazol-5-yl | 5-methyl-1,3,4-oxadiazol-2-yl | H |
| 2,4-(Me)₂Thiazol-5-yl | 5-methyl-1,3,4-oxadiazol-2-yl | methyl |
| 2,4-(Me)₂Thiazol-5-yl | 5-methyl-1,3,4-oxadiazol-2-yl | i-propyl |
| 2,4-(Me)₂Thiazol-5-yl | 5-methyl-1,3,4-oxadiazol-2-yl | cyclopropyl |
| 2,4-(Me)₂Thiazol-5-yl | 5-methyl-l,3,4-oxadiazol-2-yl | CF₃ |
| 2,4-(Me)₂Thiazol-5-yl | 5-trifluoromethyl-1,3,4-oxadiazol-2-yl | H |
| 2,4-(Me)₂Thiazol-5-yl | 5-trifluoromethyl-1,3,4-oxadiazol-2-yl | methyl |
| 2,4-(Me)₂Thiazol-5-yl | 5-trifluoromethyl-1,3,4-oxadiazol-2-yl | i-propyl |
| 2,4-(Me)₂Thiazol-5-yl | 5-trifluoromethyl-1,3,4-oxadiazol-2-yl | cyclopropyl |
| 2,4-(Me)₂Thiazol-5-yl | 5-trifluoromethyl-1,3,4-oxadiazol-2-yl | CF₃ |
| 2,4-(Me)₂Thiazol-5-yl | 1,2,3-triazol-4-yl | H |
| 2,4-(Me)₂Thiazol-5-yl | 1,2,3-triazol-4-yl | methyl |
| 2,4-(Me)₂Thiazol-5-yl | 1,2,3-triazol-4-yl | i-propyl |
| 2,4-(Me)₂Thiazol-5-yl | 1,2,3-triazol-4-yl | cyclopropyl |
| 2,4-(Me)₂Thiazol-5-yl | 1,2,3-triazol-4-yl | CF₃ |
| 2,4-(Me)₂Thiazol-5-yl | 1-methyl-1,2,3-triazol-4-yl | H |
| 2,4-(Me)₂Thiazol-5-yl | 1-methyl-1,2,3-triazol-4-yl | methyl |
| 2,4-(Me)₂Thiazol-5-yl | 1-methyl-1,2,3-triazol-4-yl | i-propyl |
| 2,4-(Me)₂Thiazol-5-yl | 1-methyl-1,2,3-triazol-4-yl | cyclopropyl |
| 2,4-(Me)₂Thiazol-5-yl | 1-methyl-1,2,3-triazol-4-yl | CF₃ |
| 2,4-(Me)₂Thiazol-5-yl | 2-methyl-1,2,3-triazol-4-yl | H |
| 2,4-(Me)₂Thiazol-5-yl | 2-methyl-1,2,3-triazol-4-yl | methyl |
| 2,4-(Me)₂Thiazol-5-yl | 2-methyl-1,2,3-triazol-4-yl | i-propyl |
| 2,4-(Me)₂Thiazol-5-yl | 2-methyl-1,2,3-triazol-4-yl | cyclopropyl |
| 2,4-(Me)₂Thiazol-5-yl | 2-methyl-1,2,3-triazol-4-yl | CF₃ |
| 2,4-(Me)₂Thiazol-5-yl | 1,2,3-triazol-1-yl | H |
| 2,4-(Me)₂Thiazol-5-yl | 1,2,3-triazol-1-yl | methyl |
| 2,4-(Me)₂Thiazol-5-yl | 1,2,3-triazol-1-yl | i-propyl |
| 2,4-(Me)₂Thiazol-5-yl | 1,2,3-triazol-1-yl | cyclopropyl |
| 2,4-(Me)₂Thiazol-5-yl | 1,2,3-triazol-1-yl | CF₃ |
| 2,4-(Me)₂Thiazol-5-yl | 1,2,3-triazol-1-yl | H |
| 2,4-(Me)₂Thiazol-5-yl | 1,2,3-triazol-1-yl | methyl |
| 2,4-(Me)₂Thiazol-5-yl | 1,2,3 triazol-2-yl | i-propyl |
| 2,4-(Me)₂Thiazol-5-yl | 1,2,3-triazol-2-yl | cyclopropyl |
| 2,4-(Me)₂Thiazol-5-yl | 1,2,3-triazol-2-yl | CF₃ |
| 2,4-(Me)₂Thiazol-5-yl | 1,2,3-triazol-1-yl | H |
| 2,4-(Me)₂Thiazol-5-yl | 1,2,3-triazol-1-yl | methyl |
| 2,4-(Me)₂Thiazol-5-yl | 1,2,3-triazol-1-yl | i-propyl |
| 2,4-(Me)₂Thiazol-5-yl | 1,2,3-triazol-1-yl | cyclopropyl |
| 2,4-(Me)₂Thiazol-5-yl | 1,2,3-triazol-1-yl | CF₃ |
| 2,4-(Me)₂Thiazol-5-yl | imidazol-2-yl | H |
| 2,4-(Me)₂Thiazol-5-yl | imidazol-2-yl | methyl |
| 2,4-(Me)₂Thiazol-5-yl | imidazol-2-yl | i-propyl |
| 2,4-(Me)₂Thiazol-5-yl | imidazol-2-yl | cyclopropyl |
| 2,4-(Me)₂Thiazol-5-yl | imidazol-2-yl | CF₃ |
| 2,4-(Me)₂Thiazol-5-yl | imidazol-1-yl | H |
| 2,4-(Me)₂Thiazol-5-yl | imidazol-1-yl | methyl |
| 2,4-(Me)₂Thiazol-5-yl | imidazol-1-yl | i-propyl |
| 2,4-(Me)₂Thiazol-5-yl | imidazol-1-yi | cyclopropyl |
| 2,4-(Me)₂Thiazol-5-yl | imidazol-1-yl | CF₃ |
| 2,4-(Me)₂Thiazol-5-yl | imidazol-4-yl | H |
| 2,4-(Me)₂Thiazol-5-yl | imidazol-4-yl | methyl |
| 2,4-(Me)₂Thiazol-5-yl | imidazol-4-yl | i-propyl |
| 2,4-(Me)₂Thiazol-5-yl | imidazol-4-yl | cyclopropyl |
| 2,4-(Me)₂Thiazol-5-yl | imidazol-4-yl | CF₃ |
| 2,4-(Me)₂Thiazol-5-yl | thiazol-2-yl | H |
| 2,4-(Me)₂Thiazol-5-yl | thiazol-2-yl | methyl |
| 2,4-(Me)₂Thiazol-5-yl | thiazol-2-yl | i-propyl |
| 2,4-(Me)₂Thiazol-5-yl | thiazol-2-yl | cyclopropyl |
| 2,4-(Me)₂Thiazol-5-yl | thiazol-2-yl | CF₃ |
| 2,4-(Me)₂Thiazol-5-yl | 4-methylthiazol-2-yl | H |
| 2,4-(Me)₂Thiazol-5-yl | 4-methylthiazol-2-yl | methyl |
| 2,4-(Me)₂Thiazol-5-yl | 4-methylthiazol-2-yl | i-propyl |
| 2,4-(Me)₂Thiazol-5-yl | 4-methylthiazol-2-yl | cyclopropyl |
| 2,4-(Me)₂Thiazol-5-yl | 4-methylthiazol-2-yl | CF₃ |
| 2,4-(Me)₂Thiazol-5-yl | oxazol-2-yl | H |
| 2,4-(Me)₂Thiazol-5-yl | oxazol-2-yl | methyl |
| 2,4-(Me)₂Thiazol-5-yl | oxazol-2-yl | i-propyl |
| 2,4-(Me)₂Thiazol-5-yl | oxazol-2-yl | cyclopropyl |
| 2,4-(Me)₂Thiazol-5-yl | oxazol-2-yl | CF₃ |
| 2,4-(Me)₂Thiazol-5-yl | 4,5-dimethyloxazol-2-yl | H |
| 2,4-(Me)₂Thiazol-5-yl | 4,5-dimethyloxazol-2-yl | methyl |
| 2,4-(Me)₂Thiazol-5-yl | 4,5-dimethyloxazol-2-yl | i-propyl |
| 2,4-(Me)₂Thiazol-5-yl | 4,5-dimethyloxazol-2-yl | cyclopropyl |
| 2,4-(Me)₂Thiazol-5-yl | 4,5-dimethyloxazol-2-yl | CF₃ |
| 2,4-(Me)₂Thiazol-5-yl | 2-oxazolin-2-yl | H |
| 2,4-(Me)₂Thiazol-5-yl | 2-oxazolin-2-yl | methyl |
| 2,4-(Me)₂Thiazol-5-yl | 2-oxazolin-2-yl | i-propyl |
| 2,4-(Me)₂Thiazol-5-yl | 2-oxazolin-2-yl | cyclopropyl |
| 2,4-(Me)₂Thiazol-5-yl | 2-oxazolin-2-yl | CF₃ |
| 2,4-(Me)₂Thiazol-5-yl | 4,4-dimethyl-2-oxazolin-2-yl | H |
| 2,4-(Me)₂Thiazol-5-yl | 4,4-dimethyl-2-oxazolin-2-yl | methyl |
| 2,4-(Me)₂Thiazol-5-yl | 4,4-dimethyl-2-oxazolin-2-yl | i-propyl |
| 2,4-(Me)₂Thiazol-5-yl | 4,4-dimethyl-2-oxazolin-2-yl | cyclopropyl |
| 2,4-(Me)₂Thiazol-5-yl | 4,4-dimethyl-2-oxazolin-2-yl | CF₃ |
| 2,4-(Me)₂Thiazol-5-yl | 1,2,4-thiadiazol-5-yl | H |
| 2,4-(Me)₂Thiazol-5-yl | 1,2,4-thiadiazol-5-yl | methyl |
| 2,4-(Me)₂Thiazol-5-yl | 1,2,4-thiadiazol-5-yl | i-propyl |
| 2,4-(Me)₂Thiazol-5-yl | 1,2,4-thiadiazol-5-yl | cyclopropyl |
| 2,4-(Me)₂Thiazol-5-yl | 1,2,4-thiadiazol-5-yl | CF₃ |
| 2,4-(Me)₂Thiazol-5-yl | 3-methyl-1,2,4-thiadiazol-5-yl | H |
| 2,4-(Me)₂Thiazol-5-yl | 3-methyl-1,2,4-thiadiazol-5-yl | methyl |
| 2,4-(Me)₂Thiazol-5-yl | 3-methyl-1,2,4-thiadiazol-5-yl | i-propyl |
| 2,4-(Me)₂Thiazol-5-yl | 3-methyl-1,2,4-thiadiazol-5-yl | cyclopropyl |
| 2,4-(Me)₂Thiazol-5-yl | 3-methyl-1,2,4-thiadiazol-5-yl | CF₃ |
| 2,4-(Me)₂Thiazol-5-yl | 3-trifluoromethyl-1,2,4-thiadiazol-5-yl | H |
| 2,4-(Me)₂Thiazol-5-yl | 3-trifluoromethyl-1,2,4-thiadiazol-5-yl | methyl |
| 2,4-(Me)₂Thiazol-5-yl | 3-trifluoromethyl-1,2,4-thiadiazol-5-yl | i-propyl |
| 2,4-(Me)₂Thiazol-5-yl | 3-trifluoromethyl-1,2,4-thiadiazol-5-yl | cyclopropyl |
| 2,4-(Me)₂Thiazol-5-yi | 3-trifluoromethyl-1,2,4-thiadiazol-5-yl | CF₃ |
| 2,4-(Me)₂Thiazol-5-yl | 1,2,4-thiadiazol-3-yl | H |
| 2,4-(Me)₂Thiazol-5-yl | 1,2,4-thiadiazol-3-yl | methyl |
| 2,4-(Me)₂Thiazol-5-yl | 1,2,4-thiadiazol-3-yl | i-propyl |
| 2,4-(Me)₂Thiazol-5-yl | 1,2,4-thiadiazol-3-yl | cyclopropyl |
| 2,4-(Me)₂Thiazol-5-yl | 1,2,4-thiadiazol-3-yl | CF₃ |
| 2,4-(Me)₂Thiazol-5-yl | 5-methyl-1,2,4-thiadiazol-3-yl | H |
| 2,4-(Me)₂Thiazol-5-yl | 5-methyl-1,2,4-thiadiazol-3-yl | methyl |
| 2,4-(Me)₂Thiazol-5-yl | 5-methyl-1,2,4-thiadiazol-3-yl | i-propyl |
| 2,4-(Me)₂Thiazol-5-yl | 5-methyl-1,2,4-thiadiazol-3-yl | cyclopropyl |
| 2,4-(Me)₂Thiazol-5-yl | 5-methyl-1,2,4-thiadiazol-3-yl | CF₃ |
| 2,4-(Me)₂Thiazol-5-yl | 5-tifluoromethyl-1,2,4-thiadiazol-3-yl | H |
| 2,4-(Me)₂Thiazol-5-yl | 5-trifluoromethyl-1,2,4-thiadiazol-3-yl | methyl |
| 2,4-(Me)₂Thiazol-5-yl | 5-trifluoromethyl-1,2,4-thiadiazol-3-yl | i-propyl |
| 2,4-(Me)₂Thiazol-5-yl | 5-trifluoromethyl-1,2,4-thiadiazol-3-yl | cyclopropyl |
| 2,4-(Me)₂Thiazol-5-yl | 5-trifluoromethyl-1,2,4-thiadiazol-3-yl | CF₃ |
| 2,4-(Me)₂Thiazol-5-yl | 1,3,4-thiadiazol-2-yl | H |
| 2,4-(Me)₂Thiazol-5-yl | 1,3,4-thiadiazol-2-yl | methyl |
| 2,4-(Me)₂Thiazol-5-yl | 1,3,4-thiadiazol-2-yl | i-propyl |
| 2,4-(Me)₂Thiazol-5-yl | 1,3,4-thiadiazol-2-yl | cyclopropyl |
| 2,4-(Me)₂Thiazol-5-yl | 1,3,4-thiadiazol-2-yl | CF₃ |
| 2,4-(Me)₂Thiazol-5-yl | 5-methylsulfonyl-1,3,4-thiadiazol-2-yl | H |
| 2,4-(Me)₂Thiazol-5-yl | 5-methylsulfonyl-1,3,4-thiadiazol-2-yl | methyl |
| 2,4-(Me)₂Thiazol-5-yl | 5-methylsulfonyl-1,3,4-thiadiazol-2-yl | i-propyl |
| 2,4-(Me)₂Thiazol-5-yl | 5-methylsulfonyl-1,3,4-thiadiazol-2-yl | cyclopropyl |
| 2,4-(Me)₂Thiazol-5-yl | 5-methylsulfonyl-1,3,4-thiadiazol-2-yl | CF₃ |
| 2,4-(Me)₂Thiazol-5-yl | 5-methyl-1,3,4-thiadiazol-2-yl | H |
| 2,4-(Me)₂Thiazol-5-yl | 5-methylsulfonyl-1,3,4-thiadiazol-2-yl | methyl |
| 2,4-(Me)₂Thiazol-5-yl | 5-methyl-1,3,4-thiadiazol-2-yl | i-propyl |
| 2,4-(Me)₂Thiazol-5-yl | 5-methyl-1,3,4-thiadiazol-2-yl | cyclopropyl |
| 2,4-(Me)₂Thiazol-5-yl | 5-methyl-1,3,4-thiadiazol-2-yl | CF₃ |
| 2,4-(Me)₂Thiazol-5-yl | benzoxazol-2-yl | H |
| 2,4-(Me)₂Thiazol-5-yl | benzoxazol-2-yl | methyl |
| 2,4-(Me)₂Thiazol-5-yl | benzoxazol-2-yl | i-propyl |
| 2,4-(Me)₂Thiazol-5-yl | benzoxazol-2-yl | cyclopropyl |
| 2,4-(Me)₂Thiazol-5-yl | benzoxazol-2-yl | CF₃ |
| 2,4-(Me)₂Thiazol-5-yl | 6-methylbenzoxazol-2-yl | H |
| 2,4-(Me)₂Thiazol-5-yl | 6-methylbenzoxazol-2-yl | methyl |
| 2,4-(Me)₂Thiazol-5-yl | 6-methylbenzoxazol-2-yl | i-propyl |
| 2,4-(Me)₂Thiazol-5-yl | 6-methylbenzoxazol-2-yl | cyclopropyl |
| 2,4-(Me)₂Thiazol-5-yl | 6-methylbenzoxazol-2-yl | CF₃ |
| 2,4-(Me)₂Thiazol-5-yl | benzothiazol-2-yl | H |
| 2,4-(Me)₂Thiazol-5-yl | benzothiazol-2-yl | methyl |
| 2,4-(Me)₂Thiazol-5-yl | benzothiazol-2-yl | i-propyl |
| 2,4-(Me)₂Thiazol-5-yl | benzothiazol-2-yl | cyclopropyl |
| 2,4-(Me)₂Thiazol-5-yl | benzothiazol-2-yl | CF₃ |
| 2,4-(Me)₂Thiazol-5-yl | pyrazol-1-yl | H |
| 2,4-(Me)₂Thiazol-5-yl | pyrazol-1-yl | methyl |
| 2,4-(Me)₂Thiazol-5-yl | pyrazol-1-yl | i-propyl |
| 2,4-(Me)₂Thiazol-5-yl | pyrazol-1-yl | cyclopropyl |
| 2,4-(Me)₂Thiazol-5-yl | pyrazol-1-yl | CF₃ |
| 2,4-(Me)₂Thiazol-5-yl | pyrazol-3-yl | H |
| 2,4-(Me)₂Thiazol-5-yl | pyrazol-3-yl | methyl |
| 2,4-(Me)₂Thiazol-5-yl | pyrazol-3-yl | i-propyl |
| 2,4-(Me)₂Thiazol-5-yl | pyrazol-3-yl | cyclopropyl |
| 2,4-(Me)₂Thiazol-5-yl | pyrazol-3-yl | CF₃ |
| 2,4-(Me)₂Thiazol-5-yl | 1-methylpyrazol-3-yl | H |
| 2,4-(Me)₂Thiazol-5-yl | 1-methylpyrazol-3-yl | methyl |
| 2,4-(Me)₂Thiazol-5-yl | 1-methylpyrazol-3-yl | i-propyl |
| 2,4-(Me)₂Thiazol-5-yl | 1-methylpyrazol-3-yl | cyclopropyl |
| 2,4-(Me)₂Thiazol-5-yl | 1-medlylpyrazol-3-yl | CF₃ |
| 2,4-(Me)₂Thiazol-5-yl | tetrazol-1-yl | H |
| 2,4-(Me)₂Thiazol-5-yl | tetrazol-1-yl | methyl |
| 2,4-(Me)₂Thiazol-5-yl | tetrazol-1-yl | i-propyl |
| 2,4-(Me)₂Thiazol-5-yl | tetrazol-1-yl | cyclopropyl |
| 2,4-(Me)₂Thiazol-5-yl | tetrazol-1-yl | CF₃ |
| 2,4-(Me)₂Thiazol-5-yl | 5-methyltetrazol-1-yl | H |
| 2,4-(Me)₂Thiazol-5-yl | 5-methyltetrazol-1-yl | methyl |
| 2,4-(Me)₂Thiazol-5-yl | 5-methyltetrazol-1-yl | i-propyl |
| 2,4-(Me)₂Thiazol-5-yl | 5-methyltetrazol-1-yl | cyclopropyl |
| 2,4-(Me)₂Thiazol-5-yl | 5-methyltetrazol-1-yl | CF₃ |
| 2,4-(Me)₂Thiazol-5-yl | tetrazol-2-yl | H |
| 2,4-(Me)₂Thiazol-5-yl | tetrazol-2-yl | methyl |
| 2,4-(Me)₂Thiazol-5-yl | tetrazol-2-yl | i-propyl |
| 2,4-(Me)₂Thiazol-5-yl | tetrazol-2-yl | cyclopropyl |
| 2,4-(Me)₂Thiazol-5-yl | tetrazol-2-yl | CF₃ |
| 2,4-(Me)₂Thiazol-5-yl | 5-methyltetrazol-2-yl | H |
| 2,4-(Me)₂Thiazol-5-yl | 5-methyltetrazol-2-yl | methyl |
| 2,4-(Me)₂Thiazol-5-yl | 5-methyltetrazol-2-yl | i-propyl |
| 2,4-(Me)₂Thiazol-5-yl | 5-methyltetrazol-2-yl | cyclopropyl |
| 2,4-(Me)₂Thiazol-5-yl | 5-methyltetrazol-2-yl | CF₃ |
| 2,4-(Me)₂Thiazol-5-yl | 1-methyltetrazol-5-yl | H |
| 2,4-(Me)₂Thiazol-5-yl | 1-methyltetrazol-5-yl | methyl |
| 2,4-(Me)₂Thiazol-5-yl | 1-methyltetrazol-5-yl | i-propyl |
| 2,4-(Me)₂Thiazol-5-yl | 1-methyltetrazol-5-yl | cyclopropyl |
| 2,4-(Me)₂Thiaaol-S-yl | 1-methyltetrazol-5-yl | CF₃ |
| 2,4-(Me)₂Thiazol-5-yl | 2-methyltetrazol-5-yl | H |
| 2,4-(Me)₂Thiazol-5-yl | 2-methyltetrazol-5-yl | methyl |
| 2,4-(Me)₂Thiazol-5-yl | 2-methyltetrazol-5-yl | i-propyl |
| 2,4-(Me)₂Thiazol-5-yl | 2-methyltetrazol-5-yl | cyclopropyl |
| 2,4-(Me)₂Thiazol-5-yl | 2-methyltetrazol-5-yl | CF₃ |
| 2,4-(Me)₂Thiazol-5-yl | pyridin-2-yl | H |
| 2,4-(Me)₂Thiazol-5-yl | pyridin-2-yl | methyl |
| 2,4-(Me)₂Thiazol-5-yl | pyridin-2-yl | i-propyl |
| 2,4-(Me)₂Thiazol-5-yl | pyridin-2-yl | cyclopropyl |
| 2,4-(Me)₂Thiazol-5-yl | pyridin-2-yl | CF₃ |
| 2,4-(Me)₂Thiazol-5-yl | pyridin-4-yl | H |
| 2,4-(Me)₂Thiazol-5-yl | pyridin-4-yl | methyl |
| 2,4-(Me)₂Thiazol-5-yl | pyridin-4-yl | i-propyl |
| 2,4-(Me)₂Thiazol-5-yl | pyridin-4-yl | cyclopropyl |
| 2,4-(Me)₂Thiazol-5-yl | pyridin-4-yl | CF₃ |
| 2,4-(Me)₂Thiazol-5-yl | pyridin-3-yl | H |
| 2,4-(Me)₂Thiazol-5-yl | pyridin-3-yl | methyl |
| 2,4-(Me)₂Thiazol-5-yl | pyridin-3-yl | i-propyl |
| 2,4-(Me)₂Thiazol-5-yl | pyridin-3-yl | cyclopropyl |
| 2,4-(Me)₂Thiazol-5-yl | pyridin-3-yl | CF₃ |
| 2,4-(Me)₂Thiazol-5-yl | 3-nitropyridin-4-yl | H |
| 2,4-(Me)₂Thiazol-5-yl | 3-nitropyridin-4-yl | methyl |
| 2,4-(Me)₂Thiazol-5-yl | 3-nitropyridin-4-yl | i-propyl |
| 2,4-(Me)₂Thiazol-5-yl | 3-nitropyridin-4-yl | cyclopropyl |
| 2,4-(Me)₂Thiazol-5-yl | 3-nitropyridin-4-yl | CF₃ |
| 2,4-(Me)₂Thiazol-5-yl | 5-cyanopyridin-2-yl | H |
| 2,4-(Me)₂Thiazol-5-yl | 5-cyanopyridin-2-yl | methyl |
| 2,4-(Me)₂Thiazol-5-yl | 5-cyanopyridin-2-yl | i-propyl |
| 2,4-(Me)₂Thiazol-5-yl | 5-cyanopyridin-2-yl | cyclopropyl |
| 2,4-(Me)₂Thiazol-5-yl | 5-cyanopyridin-2-yl | CF₃ |
| 2,4-(Me)₂Thiazol-5-yl | 5-trifluoromethylpyridin-2-yl | H |
| 2,4-(Me)₂Thiazol-5-yl | 5-trifluoromethylpyridin-2-yl | methyl |
| 2,4-(Me)₂Thiazol-5-yl | 5-trifluoromethylpyridin-2-yl | i-propy |
| 2,4-(Me)₂Thiazol-5-yl | 5-trifluoromethylpyridin 2-yl | cyclopropyl |
| 2,4-(Me)₂Thiazol-5-yl | 5-trifluoromethylpyridin-2-yl | CF₃ |
| 2,4-(Me)₂Thiazol-5-yl | pyrimidin-2-yl | H |
| 2,4-(Me)₂Thiazol-5-yl | pyrimidin-2-yl | methyl |
| 2,4-(Me)₂Thiazol-5-yl | pyrimidin-2-yl | i-propyl |
| 2,4-(Me)₂Thiazol-5-yl | pyrimidin-2-yl | cyclopropyl |
| 2,4-(Me)₂Thiazol-5-yl | pyrimidin-2-yl | CF₃ |
| 2,4-(Me)₂Thiazol-5-yl | pyrimidin-4-yl | H |
| 2,4-(Me)₂Thiazol-5-yl | pyrimidin-4-yl | methyl |
| 2,4-(Me)₂Thiazol-5-yl | pyrimidin-4-yl | i-propyl |
| 2,4-(Me)₂Thiazol-5-yl | pyrimidin-4-yl | cyclopropyl |
| 2,4-(Me)₂Thiazol-5-yl | pyrimidin-4-yl | CF₃ |
| 2,4-(Me)₂Thiazol-5-yl | 6-chloropyrimidin-4-yl | methyl |
| 2,4-(Me)₂Thiazol-5-yl | 6-chloropyrimidin-4-yl | i-propyl |
| 2,4-(Me)₂Thiazol-5-yl | 6-chloropyrimidin-4-yl | cyclopropyl |
| 2,4-(Me)₂Thiazol-5-yl | 6-chloropyrimidin-4-yl | CF₃ |
| 2,4-(Me)₂Thiazol-5-yl | pyridazin-3-yl | H |
| 2,4-(Me)₂Thiazol-5-yl | pyridazin-3-yl | methyl |
| 2,4-(Me)₂Thiazol-5-yl | pyridazin-3-yl | i-propyl |
| 2,4-(Me)₂Thiazol-5-yl | pyridazin-3-yl | cyclopropyl |
| 2,4-(Me)₂Thiazol-5-yl | pyridazin-3-yl | CF₃ |
| 2,4-(Me)₂Thiazol-5-yl | 6-chloropyridazin-3-yl | methyl |
| 2,4-(Me)₂Thiazol-5-yl | 6-chloropyridazin-3-yl | i-propyl |
| 2,4-(Me)₂Thiazol-5-yl | 6-chloropyridazin-3-yl | cyclopropyl |
| 2,4-(Me)₂Thiazol-5-yl | 6-chloropyridazin-3-yl | CF₃ |
| 2,4-(Me)₂Thiazol-5-yl | pyrazin-2-yl | methyl |
| 2,4-(Me)₂Thiazol-5-yl | pyrazin-2-yl | i-propyl |
| 2,4-(Me)₂Thiazol-5-yl | pyrazin-2-yl | cyclopropyl |
| 2,4-(Me)₂Thiazol-5-yl | pyrazin-2-yl | CF₃ |
| 2,4-(Me)₂Thiazol-5-yl | triazin-2-yl | methyl |
| 2,4-(Me)₂Thiazol-5-yl | triazin-2-yl | i-propyl |
| 2,4-(Me)₂Thiazol-5-yl | triazin-2-yl | cyclopropyl |
| 2,4-(Me)₂Thiazol-5-yl | triazin-2-yl | CF₃ |
| 2,4-(Me)₂Thiazol-5-yl | quinolin-2-yl | methyl |
| 2,4-(Me)₂Thiazol-5-yl | quinolin-2-yl | i-propyl |
| 2,4-(Me)₂Thiazol-5-yl | quinolin-2-yl | cyclopropyl |
| 2,4-(Me)₂Thiazol-5-yl | quinolin-2-yl | CF₃ |
| 2,4-(Me)₂Thiazol-5-yl | 4,4,6-trimethyl-5,6-dihydro-1,3(4H)-oxazin-2-yl | H |
| 2,4-(Me)₂Thiazol-5-yl | 4,4,6-trimethyl-5,6-dihydro-1,3(4H)-oxazin-2-yl | methyl |
| 2,4-(Me)₂Thiazol-5-yl | 4,4,6-trimethyl-5,6-dihydro-1,3(4H)-oxazin-2-yl | i-propyl |
| 2,4-(Me)₂Thiazol-5-yl | 4,4,6-trimethyl-5,6-dihydro-1,3(4H)-oxazin-2-yl | cyclopropyl |
| 2,4-(Me)₂Thiazol-5-yl | 4,4,6-trimethyl-5,6-dihydro-1,3(4H)-oxazin-2-yl | CF₃ |
| 2,4-(Me)₂Thiazol-5-yl | 2-oxazolidinon-3-yl | H |
| 2,4-(Me)₂Thiazol-5-yl | 2-oxazolidinon-3-yl | methyl |
| 2,4-(Me)₂Thiazol-5-yl | 2-oxazolidinon-3-yl | i-propyl |
| 2,4-(Me)₂Thiazol-5-yl | 2-oxazolidinon-3-yl | cyclopropyl |
| 2,4-(Me)₂Thiazol-5-yl | 2-oxazolidinon-3-yl | CF₃ |
| 2,4-(Me)₂Thiazol-5-yl | 2-pyrrolidinon-1-yl | methyl |
| 2,4-(Me)₂Thiazol-5-yl | 2-pyrrolidinon-1-yl | i-propyl |
| 2,4-(Me)₂Thiazol-5-yl | 2-pyrrolidinon-1-yl | cyclopropyl |
| 2,4-(Me)₂Thiazol-5-yl | 2-pyrrolidinon-1-yl | CF₃ |
| 2,4-(Me)₂Thiazol-5-yl | 3-methylisoxazol-5-yl | methyl |
| 2,4-(Me)₂Thiazol-5-yl | 3-methylisoxazol-5-yl | i-propyl |
| 2,4-(Me)₂Thiazol-5-yl | 3-methylisoxazol-5-yl | cyclopropyl |
| 2,4-(Me)₂Thiazol-5-yl | 3-methylisoxazol-5-yl | CF₃ |
| 2-Me-4-SO₂Me-3-(4,5-dihydroisoxazol-3-yl)Ph | 1,2,4-oxadiazol-5-yl | H |
| 2-Me-4-SO₂Me-3-(4,5-dihydroisoxazol-3-yl)Ph | 1,2,4-oxadiazol-5-yl | methyl |
| 2-Me-4-SO₂Me-3-(4,5-dihydroisoxazol-3-yl)Ph | 1,2,4-oxadiazol-5-yl | i-propyl |
| 2-Me-4-SO₂Me-3-(4,5-dihydroisoxazol-3-yl)Ph | 1,2,4-oxadiazol-5-yl | cyclopropyl |
| 2-Me-4-SO₂Me-3-(4,5-dihydroisoxazol-3-yl)Ph | 1,2,4-oxadiazol-5-yl | CF₃ |
| 2-Me-4-SO₂Me-3-(4,5-dihydroisoxazol-3-yl)Ph | 3-methyl-1,2,4-oxadiazol-5-yl | H |
| 2-Me-4-SO₂Me-3-(4,5-dihydroisoxazol-3-yl)Ph | 3-methyl-1,2,4-oxadiazol-5-yl | methyl |
| 2-Me-4-SO₂Me-3-(4,5-dihydroisoxazol-3-yl)Ph | 3-methyl-1,2,4-oxadiazol-5-yl | i-propyl |
| 2-Me-4-SO₂Me-3-(4,5-dihydroisoxazol-3-yl)Ph | 3-methyl-1,2,4-oxadiazol-5-yl | cyclopropyl |
| 2-Me-4-SO₂Me-3-(4,5-dihydroisoxazol-3-yl)Ph | 3-methyl-1,2,4-oxadiazol-5-yl | CF₃ |
| 2-Me-4-SO₂Me-3-(4,5-dihydroisoxazol-3-yl)Ph | 3-trifluoromethyl-1,2,4-oxadiazol-5-yl | H |
| 2-Me-4-SO₂Me-3-(4,5-dihydroisoxazol-3-yl)Ph | 3-trifluoromethyl-1,2,4-oxadiazol-5-yl | methyl |
| 2-Me-4-SO₂Me-3-(4,5-dihydroisoxazol-3-yl)Ph | 3-trifluoromethyl-1,2,4-oxadiazol-5-yl | i-propyl |
| 2-Me-4-SO₂Me-3-(4,5-dihydroisoxazol-3-yl)Ph | 3-trifluoromethyl-1,2,4-oxadiazol-5-yl | cyclopropyl |
| 2-Me-4-SO₂Me-3-(4,5-dihydroisoxazol-3-yl)Ph | 3-trifluoromethyl-1,2,4-oxadiazol-5-yl | CF₃ |
| 2-Me-4-SO₂Me-3-(4,5-dihydroisoxazol-3-yl)Ph | 1,2,4-oxadiazol-3-yl | H |
| 2-Me-4-SO₂Me-3-(4,5-dihydroisoxazol-3-yl)Ph | 1,2,4-oxadiazol-3-yl | methyl |
| 2-Me-4-SO₂Me-3-(4,5-dihydroisoxazol-3-yl)Ph | 1,2,4-oxadiazol-3-yl | i-propyl |
| 2-Me-4-SO₂Me-3-(4,5-dihydroisoxazol-3-yl)Ph | 1,2,4-oxadiazol-3-yl | cyclopropyl |
| 2-Me-4-SO₂Me-3-(4,5-dihydroisoxazol-3-yl)Ph | 1,2,4-oxadiazol-3-yl | CF₃ |
| 2-Me-4-SO₂Me-3-(4,5-dihydroisoxazol-3-yl)Ph | 5-methyl-1,2,4-oxadiazol-3-yl | H |
| 2-Me-4-SO₂Me-3-(4,5-dihydroisoxazol-3-yl)Ph | 5-methyl-1,2,4-oxadiazol-3-yl | methyl |
| 2-Me-4-SO₂Me-3-(4,5-dihydroisoxazol-3-yl)Ph | 5-methyl-1,2,4-oxadiazol-3-yl | i-propyl |
| 2-Me-4-SO₂Me-3-(4,5-dihydroisoxazol-3-yl)Ph | 5-methyl-1,2,4-oxadiazol-3-yl | cyclopropyl |
| 2-Me-4-SO₂Me-3-(4,5-dihydroisoxazol-3-yl)Ph | 5-methyl-1,2,4-oxadiazol-3-yl | CF₃ |
| 2-Me-4-SO₂Me-3-(4,5-dihydroisoxazol-3-yl)Ph | 5-trifluoromethyl-1,2,4-oxadiazol-3-yl | H |
| 2-Me-4-SO₂Me-3-(4,5-dihydroisoxazol-3-yl)Ph | 5-trifluoromethyl-1,2,4-oxadiazol-3-yl | methyl |
| 2-Me-4-SO₂Me-3-(4,5-dihydroisoxazol-3-yl)Ph | 5-trifluoromethyl-1,2,4-oxadiazol-3-yl | i-propyl |
| 2-Me-4-SO₂Me-3-(4,5-dihydroisoxazol-3-yl)Ph | 5-trifluoromethyl-1,2,4-oxadiazol-3-yl | cyclopropyl |
| 2-Me-4-SO₂Me-3-(4,5-dihydroisoxazol-3-yl)Ph | 5-trifluoromethyl-1,2,4-oxadiazol-3-yl | CF₃ |
| 2-Me-4-SO₂Me-3-(4,5-dihydroisoxazol-3-yl)Ph | 5-chloro-1,2,4-oxadiazol-3-yl | H |
| 2-Me-4-SO₂Me-3-(4,5-dihydroisoxazol-3-yl)Ph | 5-chloro-1,2,4-oxadiazol-3-yl | methyl |
| 2-Me-4-SO₂Me-3-(4,5-dihydroisoxazol-3-yl)Ph | 5-chloro-1,2,4-oxadiazol-3-yl | i-propyl |
| 2-Me-4-SO₂Me-3-(4,5-dihydroisoxazol-3-yl)Ph | 5-chloro-1,2,4-oxadiazol-3-yl | cyclopropyl |
| 2-Me-4-SO₂Me-3-(4,5-dihydroisoxazol-3-yl)Ph | 5-chloro-1,2,4-oxadiazol-3-yl | CF₃ |
| 2-Me-4-SO₂Me-3-(4,5-dihydroisoxazol-3-yl)Ph | 1,3,4-oxadiazol-2-yl | H |
| 2-Me-4-SO₂Me-3-(4,5-dihydroisoxazol-3-yl)Ph | 1,3,4-oxadiazol-2-yl | methyl |
| 2-Me-4-SO₂Me-3-(4,5-dihydroisoxazol-3-yl)Ph | 1,3,4-oxadiazol-2-yl | i-propyl |
| 2-Me-4-SO₂Me-3-(4,5-dihydroisoxazol-3-yl)Ph | 1,3,4-oxadiazol-2-yl | cyclopropyl |
| 2-Me-4-SO₂Me-3-(4,5-dihydroisoxazol-3-yl)Ph | 1,3,4-oxadiazol-2-yl | CF₃ |
| 2-Me-4-SO₂Me-3-(4,5-dihydroisoxazol-3-yl)Ph | 5-methylsulfonyl-1,3,4-oxadiazol-2-yl | H |
| 2-Me-4-SO₂Me-3-(4,5-dihydroisoxazol-3-yl)Ph | 5-methylsulfonyl-1,3,4-oxadiazol-2-yl | methyl |
| 2-Me-4-SO₂Me-3-(4,5-dihydroisoxazol-3-yl)Ph | 5-methylsulfonyl-1,3,4-oxadiazol-2-yl | i-propyl |
| 2-Me-4-SO₂Me-3-(4,5-dihydroisoxazol-3-yl)Ph | 5-methylsulfonyl-1,3,4-oxadiazol-2-yl | cyclopropyl |
| 2-Me-4-SO₂Me-3-(4,5-dihydroisoxazol-3-yl)Ph | 5-methylsulfonyl-1,3,4-oxadiazol-2-yl | CF₃ |
| 2-Me-4-SO₂Me-3-(4,5-dihydroisoxazol-3-yl)Ph | 5-methyl-1,3,4-oxadiazol-2-yl | H |
| 2-Me-4-SO₂Me-3-(4,5-dihydroisoxazol-3-yl)Ph | 5-methyl-1,3,4-oxadiazol-2-yl | methyl |
| 2-Me-4-SO₂Me-3-(4,5-dihydroisoxazol-3-yl)Ph | 5-methyl-1,3,4-oxadiazol-2-yl | i-propyl |
| 2-Me-4-SO₂Me-3-(4,5-dihydroisoxazol-3-yl)Ph | 5-methyl-1,3,4-oxadiazol-2-yl | cyclopropyl |
| 2-Me-4-SO₂Me-3-(4,5-dihydroisoxazol-3-yl)Ph | 5-methyl-1,3,4-oxadiazol-2-yl | CF₃ |
| 2-Me-4-SO₂Me-3-(4,5-dihydroisoxazol-3-yl)Ph | 5-trifluoromethyl-1,3,4-oxadiazol-2-yl | H |
| 2-Me-4-SO₂Me-3-(4,5-dihydroisoxazol-3-yl)Ph | 5-trifluoromethyl-1,3,4-oxadiazol-2-yl | methyl |
| 2-Me-4-SO₂Me-3-(4,5-dihydroisoxazol-3-yl)Ph | 5-trifluoromethyl-1,3,4-oxadiazol-2-yl | i-propyl |
| 2-Me-4-SO₂Me-3-(4,5-dihydroisoxazol-3-yl)Ph | 5-tritluoromethyl-1,3,4-oxadiazol-2-yl | cyclopropyl |
| 2-Me-4-SO₂Me-3-(4,5-dihydroisoxazol-3-yl)Ph | 5-trifluoromethyl-1,3,4-oxadiazol-2-yl | CF₃ |
| 2-Me-4-SO₂Me-3-(4,5-dihydroisoxazol-3-yl)Ph | 1,2,3-triazol-4-yl | H |
| 2-Me-4-SO₂Me-3-(4,5-dihydroisoxazol-3-yl)Ph | 1,2,3-triazol-4-yl | methyl |
| 2-Me-4-SO₂Me-3-(4,5-dihydroisoxazol-3-yl)Ph | 1,2,3-triazol-4-yl | i-propyl |
| 2-Me-4-SO₂Me-3-(4,5-dihydroisoxazol-3-yl)Ph | 1,2,3-triazol-4-yl | cyclopropyl |
| 2-Me-4-SO₂Me-3-(4,5-dihydroisoxazol-3-yl)Ph | 1,2,3-triazol-4-yl | CF₃ |
| 2-Me-4-SO₂Me-3-(4,5-dihydroisoxazol-3-yl)Ph | 1-methyl-1,2,3-triazol-4-yl | H |
| 2-Me-4-SO₂Me-3-(4,5-dihydroisoxazol-3-yl)Ph | 1-methyl-1,2,3-triazol-4-yl | methyl |
| 2-Me-4-SO₂Me-3-(4,5-dihydroisoxazol-3-yl)Ph | 1-methyl-1,2,3-triazol-4-yl | i-propyl |
| 2-Me-4-SO₂Me-3-(4,5-dihydroisoxazol-3-yl)Ph | 1-methyl-1,2,3-triazol-4-yl | cyclopropyl |
| 2-Me-4-SO₂Me-3-(4,5-dihydroisoxazol-3-yl)Ph | 1-methyl-1,2,3-triazol-4-yl | CF₃ |
| 2-Me-4-SO₂Me-3-(4,5-dihydroisoxazol-3-yl)Ph | 2-methyl-1,2,3-triazol-4-yl | H |
| 2-Me-4-SO₂Me-3-(4,5-dihydroisoxazol-3-yl)Ph | 2-methyl-1,2,3-triazol-4-yl | methyl |
| 2-Me-4-SO₂Me-3-(4,5-dihydroisoxazol-3-yl)Ph | 2-methyl-1,2,3-triazol-4-yl | i-propyl |
| 2-Me-4-SO₂Me-3-(4,5-dihydroisoxazol-3-yl)Ph | 2-methyl-1,2,3-triazol-4-yl | cyclopropyl |
| 2-Me-4-SO₂Me-3-(4,5-dihydroisoxazol-3-yl)Ph | 2-methyl-1,2,3-triazol-4-yl | CF₃ |
| 2-Me-4-SO₂Me-3-(4,5-dihydroisoxazol-3-yl)Ph | 1,2,3-triazol-1-yl | H |
| 2-Me-4-SO₂Me-3-(4,5-dihydroisoxazol-3-yl)Ph | 1,2,3-triazol-1-yl | methyl |
| 2-Me-4-SO₂Me-3-(4,5-dihydroisoxazol-3-yl)Ph | 1,2,3-triazol-1-yl | i-propyl |
| 2-Me-4-SO₂Me-3-(4,5-dihydroisoxazol-3-yl)Ph | 1,2,3-triazol-1-yl | cyclopropyl |
| 2-Me-4-SO₂Me-3-(4,5-dihydroisoxazol-3-yl)Ph | 1,2,3 triazol-1-yl | CF₃ |
| 2-Me-4-SO₂Me-3-(4,5-dihydroisoxazol-3-yl)Ph | 1,2,3-triazol-2-yl | H |
| 2-Me-4-SO₂Me-3-(4,5-dihydroisoxazol-3-yl)Ph | 1,2,3-triazol-2-yl | methyl |
| 2-Me-4-SO₂Me-3-(4,5-dihydroisoxazol-3-yl)Ph | 1,2,3-triazol-2-yl | i-propyl |
| 2-Me-4-SO₂Me-3-(4,5-dihydroisoxazol-3-yl)Ph | 1,2,3-triazol-2-yl | cyclopropyl |
| 2-Me-4-SO₂Me-3-(4,5-dihydroisoxazol-3-yl)Ph | 1,2,3-triazol-2-yl | CF₃ |
| 2-Me-4-SO₂Me-3-(4,5-dihydroisoxazol-3-yl)Ph | 1,2,4-triazol-1-yl | H |
| 2-Me-4-SO₂Me-3-(4,5-dihydroisoxazol-3-yl)Ph | 1,2,4-triazol-1-yl | methyl |
| 2-Me-4-SO₂Me-3-(4,5-dihydroisoxazol-3-yl)Ph | 1,2,4-triazol-1-yl | i-propyl |
| 2-Me-4-SO₂Me-3-(4,5-dihydroisoxazol-3-yl)Ph | 1,2,4-triazol-1-yl | cyclopropyl |
| 2-Me-4-SO₂Me-3-(4,5-dihydroisoxazol-3-yl)Ph | 1,2,4-triazol-1-yl | CF₃ |
| 2-Me-4-SO₂Me-3-(4,5-dihydroisoxazol-3-yl)Ph | imidazol-2-yl | H |
| 2-Me-4-SO₂Me-3-(4,5-dihydroisoxazol-3-yl)Ph | imidazol-2-yl | methyl |
| 2-Me-4-SO₂Me-3-(4,5-dihydroisoxazol-3-yl)Ph | imidazol-2-yl | i-propyl |
| 2-Me-4-SO₂Me-3-(4,5-dihydroisoxazol-3-yl)Ph | imidazol-2-yl | cyclopropyl |
| 2-Me-4-SO₂Me-3-(4,5-dihydroisoxazol-3-yl)Ph | imidazol-2-yl | CF₃ |
| 2-Me-4-SO₂Me-3-(4,5-dihydroisoxazol-3-yl)Ph | imidazol-1-yl | H |
| 2-Me-4-SO₂Me-3-(4,5-dihydroisoxazol-3-yl)Ph | imidazol-1-yl | methyl |
| 2-Me-4-SO₂Me-3-(4,5-dihydroisoxazol-3-yl)Ph | imidazol-1-yl | i-propyl |
| 2-Me-4-SO₂Me-3-(4,5-dihydroisoxazol-3-yl)Ph | imidazol-1-yl | cyclopropyl |
| 2-Me-4-SO₂Me-3-(4,5-dihydroisoxazol-3-yl)Ph | imidazol-1-yl | CF₃ |
| 2-Me-4-SO₂Me-3-(4,5-dihydroisoxazol-3-yl)Ph | imidazol-4-yl | H |
| 2-Me-4-SO₂Me-3-(4,5-dihydroisoxazol-3-yl)Ph | imidazol-4-yl | methyl |
| 2-Me-4-SO₂Me-3-(4,5-dihydroisoxazol-3-yl)Ph | imidazol-4-yl | i-propyl |
| 2-Me-4-SO₂Me-3-(4,5-dihydroisoxazol-3-yl)Ph | imidazol-4-yl | cyclopropyl |
| 2-Me-4-SO₂Me-3-(4,5-dihydroisoxazol-3-yl)Ph | imidazol-4-yl | CF₃ |
| 2-Me-4-SO₂Me-3-(4,5-dihydroisoxazol-3-yl)Ph | thiazol-2-yl | H |
| 2-Me-4-SO₂Me-3-(4,5-dihydroisoxazol-3-yl)Ph | thiazol-2-yl | methyl |
| 2-Me-4-SO₂Me-3-(4,5-dihydroisoxazol-3-yl)Ph | thiazol-2-yl | i-propyl |
| 2-Me-4-SO₂Me-3-(4,5-dihydroisoxazol-3-yl)Ph | thiazol-2-yl | cyclopropyl |
| 2-Me-4-SO₂Me-3-(4,5-dihydroisoxazol-3-yl)Ph | thiazol-2-yl | CF₃ |
| 2-Me-4-SO₂Me-3-(4,5-dihydroisoxazol-3-yl)Ph | 4-methylthiazol-2-yl | H |
| 2-Me-4-SO₂Me-3-(4,5-dihydroisoxazol-3-yl)Ph | 4-methylthiazol-2-yl | methyl |
| 2-Me-4-SO₂Me-3-(4,5-dihydroisoxazol-3-yl)Ph | 4-methylthiazol-2-yl | i-propyl |
| 2-Me-4-SO₂Me-3-(4,5-dihydroisoxazol-3-yl)Ph | 4-methythiazol-2-yl | cyclopropyl |
| 2-Me-4-SO₂Me-3-(4,5-dihydroisoxazol-3-yl)Ph | 4-methylthiazol-2-yl | CF₃ |
| 2-Me-4-SO₂Me-3-(4,5-dihydroisoxazol-3-yl)Ph | oxazol-2-yl | H |
| 2-Me-4-SO₂Me-3-(4,5-dihydroisoxazol-3-yl)Ph | oxazol-2-yl | methyl |
| 2-Me-4-SO₂Me-3-(4,5-dihydroisoxazol-3-yl)Ph | oxazol-2-yl | i-propyl |
| 2-Me-4-SO₂Me-3-(4,5-dihydroisoxazol-3-yl)Ph | oxazol-2-yl | cyclopropyl |
| 2-Me-4-SO₂Me-3-(4,5-dihydroisoxazol-3-yl)Ph | oxazol-2-yl | CF₃ |
| 2-Me-4-SO₂Me-3-(4,5-dihydroisoxazol-3-yl)Ph | 4,5-dimethyloxazol-2-yl | H |
| 2-Me-4-SO₂Me-3-(4,5-dihydroisoxazol-3-yl)Ph | 4,5-dimethyloxazol-2-yl | methyl |
| 2-Me-4-SO₂Me-3-(4,5-dihydroisoxazol-3-yl)Ph | 4,5-dimethyloxazol-2-yl | i-propyl |
| 2-Me-4-SO₂Me-3-(4,5-dihydroisoxazol-3-yl)Ph | 4,5-dimethyloxazol-2-yl | cyclopropyl |
| 2-Me-4-SO₂Me-3-(4,5-dihydroisoxazol-3-yl)Ph | 4,5-dimethyloxazol-2-yl | CF₃ |
| 2-Me-4-SO₂Me-3-(4,5-dihydroisoxazol-3-yl)Ph | 2-oxazolin-2-yl | H |
| 2-Me-4-SO₂Me-3-(4,5-dihydroisoxazol-3-yl)Ph | 2-oxaolin-2-yl | methyl |
| 2-Me-4-SO₂Me-3-(4,5-dihydroisoxazol-3-yl)Ph | 2-oxazolin-2-yl | i-propyl |
| 2-Me-4-SO₂Me-3-(4,5-dihydroisoxazol-3-yl)Ph | 2-oxazolin-2-yl | cyclopropyl |
| 2-Me-4-SO₂Me-3-(4,5-dihydroisoxazol-3-yl)Ph | 2-oxazolin-2-yl | CF₃ |
| 2-Me-4-SO₂Me-3-(4,5-dihydroisoxazol-3-yl)Ph | 4,4-dimethyl-2-oxazolin-2-yl | H |
| 2-Me-4-SO₂Me-3-(4,5-dihydroisoxazol-3-yl)Ph | 4,4-dimethyl-2-oxazolin-2-yl | methyl |
| 2-Me-4-SO₂Me-3-(4,5-dihydroisoxazol-3-yl)Ph | 4,4-dimethyl-2-oxazolin-2-yl | i-propyl |
| 2-Me-4-SO₂Me-3-(4,5-dihydroisoxazol-3-yl)Ph | 4,4-dimethyl-2-oxazolin-2-yl | cyclopropyl |
| 2-Me-4-SO₂Me-3-(4,5-dihydroisoxazol-3-yl)Ph | 4,4-dimethyl-2-oxazolin-2-yl | CF₃ |
| 2-Me-4-SO₂Me-3-(4,5-dihydroisoxazol-3-yl)Ph | 1,2,4-thiadiazol-5-yl | H |
| 2-Me-4-SO₂Me-3-(4,5-dihydroisoxazol-3-yl)Ph | 1,2,4-thiadiazol-5-yl | methyl |
| 2-Me-4-SO₂Me-3-(4,5-dihydroisoxazol-3-yl)Ph | 1,2,4-thiadiazol-5-yl | i-propyl |
| 2-Me-4-SO₂Me-3-(4,5-dihydroisoxazol-3-yl)Ph | 1,2,4-thiadiazol-5-yl | cyclopropyl |
| 2-Me-4-SO₂Me-3-(4,5-dihydroisoxazol-3-yl)Ph | 1,2,4-thiadiazol-5-yl | CF₃ |
| 2-Me-4-SO₂Me-3-(4,5-dihydroisoxazol-3-yl)Ph | 3-methyl-1,2,4-thiadiazol-5-yl | H |
| 2-Me-4-SO₂Me-3-(4,5-dihydroisoxazol-3-yl)Ph | 3-methyl-1,2,4-thiadiazol-5-yl | methyl |
| 2-Me-4-SO₂Me-3-(4,5-dihydroisoxazol-3-yl)Ph | 3-methyl-1,2,4-thiadiazol-5-yl | i-propyl |
| 2-Me-4-SO₂Me-3-(4,5-dihydroisoxazol-3-yl)Ph | 3-methyl-1,2,4-thiadiazol-5-yl | cyclopropyl |
| 2-Me-4-SO₂Me-3-(4,5-dihydroisoxazol-3-yl)Ph | 3-methyl-1,2,4-thiadiazol-5-yl | CF₃ |
| 2-Me-4-SO₂Me-3-(4,5-dihydroisoxazol-3-yl)Ph | 3-trifluoromethyl-1,2,4-thiadiazol-5-yl | H |
| 2-Me-4-SO₂Me-3-(4,5-dihydroisoxazol-3-yl)Ph | 3-trifluoromethyl-1,2,4-thiadiazol-5-yl | methyl |
| 2-Me-4-SO₂Me-3-(4,5-dihydroisoxazol-3-yl)Ph | 3-trifluoromethyl-1,2,4-thiadiazol-5-yl | i-propyl |
| 2-Me-4-SO₂Me-3-(4,5-dihydroisoxazol-3-yl)Ph | 3-trifluoromethyl-1,2,4-thiadiazol-5-yl | cyclopropyl |
| 2-Me-4-SO₂Me-3-(4,5-dihydroisoxazol-3-yl)Ph | 3-trifluoromethyl-1,2,4-thiadiazol-5-yl | CF₃ |
| 2-Me-4-SO₂Me-3-(4,5-dihydroisoxazol-3-yl)Ph | 1,2,4-thiadiazol-3-yl | H |
| 2-Me-4-SO₂Me-3-(4,5-dihydroisoxazol-3-yl)Ph | 1,2,4-thiadiazol-3-yl | methyl |
| 2-Me-4-SO₂Me-3-(4,5-dihydroisoxazol-3-yl)Ph | 1,2,4-thiadiazol-3-yl | i-propyl |
| 2-Me-4-SO₂Me-3-(4,5-dihydroisoxazol-3-yl)Ph | 1,2,4-thiadiazol-3-yl | cyclopropyl |
| 2-Me-4-SO₂Me-3-(4,5-dihydroisoxazol-3-yl)Ph | 1,2,4-thiadiazol-3-yl | CF₃ |
| 2-Me-4-SO₂Me-3-(4,5-dihydroisoxazol-3-yl)Ph | 5-methyl-1,2,4-thiadiazol-3-yl | H |
| 2-Me-4-SO₂Me-3-(4,5-dihydroisoxazol-3-yl)Ph | 5-methyl-1,2,4-thiadiazol-3-yl | methyl |
| 2-Me-4-SO₂Me-3-(4,5-dihydroisoxazol-3-yl)Ph | 5-methyl-1,2,4-thiadiazol-3-yl | i-propyl |
| 2-Me-4-SO₂Me-3-(4,5-dihydroisoxazol-3-yl)Ph | 5-methyl-1,2,4-thiadiazol-3-yl | cyclopropyl |
| 2-Me-4-SO₂Me-3-(4,5-dihydroisoxazol-3-yl)Ph | 5-methyl-1,2,4-thiadiazol-3-yl | CF₃ |
| 2-Me-4-SO₂Me-3-(4,5-dihydroisoxazol-3-yl)Ph | 5-trifluoromethyl-1,2,4-thiadiazol-3-yl | H |
| 2-Me-4-SO₂Me-3-(4,5-dihydroisoxazol-3-yl)Ph | 5-trifluoromethyl-1,2,4-thiadiazol-3-yl | methyl |
| 2-Me-4-SO₂Me-3-(4,5-dihydroisoxazol-3-yl)Ph | 5-trifluoromethyl-1,2,4-thiadiazol-3-yl | i-propyl |
| 2-Me-4-SO₂Me-3-(4,5-dihydroisoxazol-3-yl)Ph | 5-trifluoromethyl-1,2,4-thiadiazol-3-yl | cyclopropyl |
| 2-Me-4-SO₂Me-3-(4,5-dihydroisoxazol-3-yl)Ph | 5-trifluoromethyl-1,2,4-thiadiazol-3-yl | CF₃ |
| 2-Me-4-SO₂Me-3-(4,5-dihydroisoxazol-3-yl)Ph | 1,3,4-thiadiazol-2-yl | H |
| 2-Me-4-SO₂Me-3-(4,5-dihydroisoxazol-3-yl)Ph | 1,3,4-thiadiazol-2-yl | methyl |
| 2-Me-4-SO₂Me-3-(4,5-dihydroisoxazol-3-yl)Ph | 1,3,4-thiadiazol-2-yl | i-propyl |
| 2-Me-4-SO₂Me-3-(4,5-dihydroisoxazol-3-yl)Ph | 1,3,4-thiadiazol-2-yl | cyclopropyl |
| 2-Me-4-SO₂Me-3-(4,5-dihydroisoxazol-3-yl)Ph | 1,3,4-thiadiazol-2-yl | CF₃ |
| 2-Me-4-SO₂Me-3-(4,5-dihydroisoxazol-3-yl)Ph | 5-methylsulfonyl-1,3,4-thiadiazol-2-yl | H |
| 2-Me-4-SO₂Me-3-(4,5-dihydroisoxazol-3-yl)Ph | 5-methylsulfonyl-1,3,4-thiadiazol-2-yl | methyl |
| 2-Me-4-SO₂Me-3-(4,5-dihydroisoxazol-3-yl)Ph | 5-methylsulfonyl-1,3,4-thiadiazol2-yl | i-propyl |
| 2-Me-4-SO₂Me-3-(4,5-dihydroisoxazol-3-yl)Ph | 5-methylsulfonyl-1,3,4-thiadiazol-2-yl | cyclopropyl |
| 2-Me-4-SO₂Me-3-(4,5-dihydroisoxazol-3-yl)Ph | 5-methylsulfonyl-1,3,4-thiadiazol-2-yl | CF₃ |
| 2-Me-4-SO₂Me-3-(4,5-dihydroisoxazol-3-yl)Ph | 5-methyl-1,3,4-thiadiazol-2-yl | H |
| 2-Me-4-SO₂Me-3-(4,5-dihydroisoxazol-3-yl)Ph | 5-methyl-1,3,4-thiadiazol-2-yl | methyl |
| 2-Me-4-SO₂Me-3-(4,5-dihydroisoxazol-3-yl)Ph | 5-methyl-1,3,4-thiadiazol-2-yl | i-propyl |
| 2-Me-4-SO₂Me-3-(4,5-dihydroisoxazol-3-yl)Ph | 5-methyl-1,3,4-thiadiazol-2-yl | cyclopropyl |
| 2-Me-4-SO₂Me-3-(4,5-dihydroisoxazol-3-yl)Ph | 5-methyl-1,3,4-thiadiazol-2-yl | CF₃ |
| 2-Me-4-SO₂Me-3-(4,5-dihydroisoxazol-3-yl)Ph | benzoxazol-2-yl | H |
| 2-Me-4-SO₂Me-3-(4,5-dihydroisoxazol-3-yl)Ph | benzoxazol-2-yl | methyl |
| 2-Me-4-SO₂Me-3-(4,5-dihydroisoxazol-3-yl)Ph | benzoxazol-2-yl | i-propyl |
| 2-Me-4-SO₂Me-3-(4,5-dihydroisoxazol-3-yl)Ph | benzoxazol-2-yl | cyclopropyl |
| 2-Me-4-SO₂Me-3-(4,5-dihydroisoxazol-3-yl)Ph | benzoxazol-2-yl | CF₃ |
| 2-Me-4-SO₂Me-3-(4,5-dihydroisoxazol-3-yl)Ph | 6-methylbenzoxazol-2-yl | H |
| 2-Me-4-SO₂Me-3-(4,5-dihydroisoxazol-3-yl)Ph | 6-methylbenzoxazol-2-yl | methyl |
| 2-Me-4-SO₂Me-3-(4,5-dihydroisoxazol-3-yl)Ph | 6-methylbenzoxazol-2-yl | i-propyl |
| 2-Me-4-SO₂Me-3-(4,5-dihydroisoxazol-3-yl)Ph | 6-methylbenzoxazol-2-yl | cyclopropyl |
| 2-Me-4-SO₂Me-3-(4,5-dihydroisoxazol-3-yl)Ph | 6-methylbenzoxazol-2-yl | CF₃ |
| 2-Me-4-SO₂Me-3-(4,5-dihydroisoxazol-3-yl)Ph | benzothiazol-2-yl | H |
| 2-Me-4-SO₂Me-3-(4,5-dihydroisoxazol-3-yl)Ph | benzothiazol-2-yl | methyl |
| 2-Me-4-SO₂Me-3-(4,5-dihydroisoxazol-3-yl)Ph | benzothiazol-2-yl | i-propyl |
| 2-Me-4-SO₂Me-3-(4,5-dihydroisoxazol-3-yl)Ph | benzothiazol-2-yl | cyclopropyl |
| 2-Me-4-SO₂Me-3-(4,5-dihydroisoxazol-3-yl)Ph | benzothiazol-2-yl | CF₃ |
| 2-Me-4-SO₂Me-3-(4,5-dihydroisoxazol-3-yl)Ph | pyrazol-1-yl | H |
| 2-Me-4-SO₂Me-3-(4,5-dihydroisoxazol-3-yl)Ph | pyrazol-1-yl | methyl |
| 2-Me-4-SO₂Me-3-(4,5-dihydroisoxazol-3-yl)Ph | pyrazol-1-yl | i-propyl |
| 2-Me-4-SO₂Me-3-(4,5-dihydroisoxazol-3-yl)Ph | pyrazol-1-yl | cyclopropyl |
| 2-Me-4-SO₂Me-3-(4,5-dihydroisoxazol-3-yl)Ph | pyrazol-1-yl | CF₃ |
| 2-Me-4-SO₂Me-3-(4,5-dihydroisoxazol-3-yl)Ph | pyrazol-3-yl | H |
| 2-Me-4-SO₂Me-3-(4,5-dihydroisoxazol-3-yl)Ph | pyrazol-3-yl | methyl |
| 2-Me-4-SO₂Me-3-(4,5-dihydroisoxazol-3-yl)Ph | pyrazol-3-yl | i-propyl |
| 2-Me-4-SO₂Me-3-(4,5-dihydroisoxazol-3-yl)Ph | pyrazol-3-yl | cyclopropyl |
| 2-Me-4-SO₂Me-3-(4,5-dihydroisoxazol-3-yl)Ph | pyrazol-3-yl | CF₃ |
| 2-Me-4-SO₂Me-3-(4,5-dihydroisoxazol-3-yl)Ph | 1-methylpyrazol-3-yl | H |
| 2-Me-4-SO₂Me-3-(4,5-dihydroisoxazol-3-yl)Ph | 1-methylpyrazol-3-yl | methyl |
| 2-Me-4-SO₂Me-3-(4,5-dihydroisoxazol-3-yl)Ph | 1-methylpyrazol-3-yl | i-propyl |
| 2-Me-4-SO₂Me-3-(4,5-dihydroisoxazol-3-yl)Ph | 1-methylpyrazol-3-yl | cyclopropyl |
| 2-Me-4-SO₂Me-3-(4,5-dihydroisoxazol-3-yl)Ph | 1-methylpyrazol-3-yl | CF₃ |
| 2-Me-4-SO₂Me-3-(4,5-dihydroisoxazol-3-yl)Ph | tetrazol-1-yl | H |
| 2-Me-4-SO₂Me-3-(4,5-dihydroisoxazol-3-yl)Ph | tetrazol-1-yl | methyl |
| 2-Me-4-SO₂Me-3-(4,5-dihydroisoxazol-3-yl)Ph | tetrazol-1-yl | i-propyl |
| 2-Me-4-SO₂Me-3-(4,5-dihydroisoxazol-3-yl)Ph | tetrazol-1-yl | cyclopropyl |
| 2-Me-4-SO₂Me-3-(4,5-dihydroisoxazol-3-yl)Ph | tetrazol-1-yl | CF₃ |
| 2-Me-4-SO₂Me-3-(4,5-dihydroisoxazol-3-yl)Ph | 5-methyltetrazol-1-yl | H |
| 2-Me-4-SO₂Me-3-(4,5-dihydroisoxazol-3-yl)Ph | 5-methyltetrazol-1-yl | methyl |
| 2-Me-4-SO₂Me-3-(4,5-dihydroisoxazol-3-yl)Ph | 5-methyltetrazol-1-yl | i-propyl |
| 2-Me-4-SO₂Me-3-(4,5-dihydroisoxazol-3-yl)Ph | 5-methyltetrazol-1-yl | cyclopropyl |
| 2-Me-4-SO₂Me-3-(4,5-dihydroisoxazol-3-yl)Ph | 5-methyltetrazol-1-yl | CF₃ |
| 2-Me-4-SO₂Me-3-(4,5-dihydroisoxazol-3-yl)Ph | tetrazol-2-yl | H |
| 2-Me-4-SO₂Me-3-(4,5-dihydroisoxazol-3-yl)Ph | tetrazol-2-yl | methyl |
| 2-Me-4-SO₂Me-3-(4,5-dihydroisoxazol-3-yl)Ph | tetrazol-2-yl | i-propyl |
| 2-Me-4-SO₂Me-3-(4,5-dihydroisoxazol-3-yl)Ph | tetrazol-2-yl | cyclopropyl |
| 2-Me-4-SO₂Me-3-(4,5-dihydroisoxazol-3-yl)Ph | tetrazol-2-yl | CF₃ |
| 2-Me-4-SO₂Me-3-(4,5-dihydroisoxazol-3-yl)Ph | 5-methyltetrazol-2-yl | H |
| 2-Me-4-SO₂Me-3-(4,5-dihydroisoxazol-3-yl)Ph | 5-methyhetrazol-2-yl | methyl |
| 2-Me-4-SO₂Me-3-(4,5-dihydroisoxazol-3-yl)Ph | 5-methyltetrazol-2-yl | i-propyl |
| 2-Me-4-SO₂Me-3-(4,5-dihydroisoxazol-3-yl)Ph | 5-methyltetrazol-2-yl | cyclopropyl |
| 2-Me-4-SO₂Me-3-(4,5-dihydroisoxazol-3-yl)Ph | 5-methyltetrazol-2-yl | CF₃ |
| 2-Me-4-SO₂Me-3-(4,5-dihydroisoxazol-3-yl)Ph | 1-methyltetrazol-5-yl | H |
| 2-Me-4-SO₂Me-3-(4,5-dihydroisoxazol-3-yl)Ph | 1-methyltetrazol-5-yl | methyl |
| 2-Me-4-SO₂Me-3-(4,5-dihydroisoxazol-3-yl)Ph | 1-methyltetrazol-5-yl | i-propyl |
| 2-Me-4-SO₂Me-3-(4,5-dihydroisoxazol-3-yl)Ph | 1-methyltetrazol-5-yl | cyclopropyl |
| 2-Me-4-SO₂Me-3-(4,5-dihydroisoxazol-3-yl)Ph | 1-methyltetrazol-5-yl | CF₃ |
| 2-Me-4-SO₂Me-3-(4,5-dihydroisoxazol-3-yl)Ph | 2-methyltetrazol-5-yl | H |
| 2-Me-4-SO₂Me-3-(4,5-dihydroisoxazol-3-yl)Ph | 2-methyltetrazol-5-yl | methyl |
| 2-Me-4-SO₂Me-3-(4,5-dihydroisoxazol-3-yl)Ph | 2-methyltetrazol-5-yl | i-propyl |
| 2-Me-4-SO₂Me-3-(4,5-dihydroisoxazol-3-yl)Ph | 2-methyltetrazol-5-yl | cyclopropyl |
| 2-Me-4-SO₂Me-3-(4,5-dihydroisoxazol-3-yl)Ph | 2-methyltetrazol-5-yl | CF₃ |
| 2 Me-4-SO_{z}Me-3-(4,5-dihydroisoxazol-3 yl)Ph | pyridin-2-yl | H |
| 2 Me-4-SO_{z}Me-3-(4,5-dihydroisoxazol-3 yl)Ph | pyridin-2-yl | methyl |
| 2 Me-4-SO_{z}Me-3-(4,5-dihydroisoxazol-3 yl)Ph | pyridin-2-yl | i-propyl |
| 2-Me-4-SO₂Me-3-(4,5-dihydroisoxazol-3-yl)Ph | pyridin-2-yl | cyclopropyl |
| 2-Me-4-SO₂Me-3-(4,5-dihydroisoxazol-3-yl)Ph | pyridin-2-yl | CF₃ |
| 2-Me-4-SO₂Me-3-(4,5-dihydroisoxazol-3-yl)Ph | pyridin-4-yl | H |
| 2-Me-4-SO₂Me-3-(4,5-dihydroisoxazol-3-yl)Ph | pyridin-4-yl | methyl |
| 2-Me-4-SO₂Me-3-(4,5-dihydroisoxazol-3-yl)Ph | pyridin-4-yl | i-propyl |
| 2-Me-4-SO₂Me-3-(4,5-dihydroisoxazol-3-yl)Ph | pyridin-4-yl | cyclopropyl |
| 2-Me-4-SO₂Me-3-(4,5-dihydroisoxazol-3-yl)Ph | pyridin-4-yl | CF₃ |
| 2-Me-4-SO₂Me-3-(4,5-dihydroisoxazol-3-yl)Ph | pyridin-3-yl | H |
| 2-Me-4-SO₂Me-3-(4,5-dihydroisoxazol-3-yl)Ph | pyridin-3-yl | methyl |
| 2-Me-4-SO₂Me-3-(4,5-dihydroisoxazol-3-yl)Ph | pyridin-3-yl | i-propyl |
| 2-Me-4-SO₂Me-3-(4,5-dihydroisoxazol-3-yl)Ph | pyridin-3-yl | cyclopropyl |
| 2-Me-4-SO₂Me-3-(4,5-dihydroisoxazol-3-yl)Ph | pyridin 3-yl | CF₃ |
| 2-Me-4-SO₂Me-3-(4,5-dihydroisoxazol-3-yl)Ph | 3-nitropyridin-4-yl | H |
| 2-Me-4-SO₂Me-3-(4,5-dihydroisoxazol-3-yl)Ph | 3-nitropyridin-4-yl | methyl |
| 2-Me-4-SO₂Me-3-(4,5-dihydroisoxazol-3-yl)Ph | 3-nitropyridin-4-yl | i-propyl |
| 2-Me-4-SO₂Me-3-(4,5-dihydroisoxazol-3-yl)Ph | 3-nitropyridin-4-yl | cyclopropyl |
| 2-Me-4-SO₂Me-3-(4,5-dihydroisoxazol-3-yl)Ph | 3-nitropyridin-4-yl | CF₃ |
| 2-Me-4-SO₂Me-3-(4,5-dihydroisoxazol-3-yl)Ph | 5-cyanopyridin-2-yl | H |
| 2-Me-4-SO₂Me-3-(4,5-dihydroisoxazol-3-yl)Ph | 5-cyanopyridin-2-yl | methyl |
| 2-Me-4-SO₂Me-3-(4,5-dihydroisoxazol-3-yl)Ph | 5-cyanopyridin-2-yl | i-propyl |
| 2-Me-4-SO₂Me-3-(4,5-dihydroisoxazol-3-yl)Ph | 5-cyanopyridin-2-yl | cyclopropyl |
| 2-Me-4-SO₂Me-3-(4,5-dihydroisoxazol-3-yl)Ph | 5-cyanopyridin-2-yl | CF₃ |
| 2-Me-4-SO₂Me-3-(4,5-dihydroisoxazol-3-yl)Ph | 5-trifluoromethylpyridin-2-yl | H |
| 2-Me-4-SO₂Me-3-(4,5-dihydroisoxazol-3-yl)Ph | 5-trifluoromethylpyridin-2-yl | methyl |
| 2-Me-4-SO₂Me-3-(4,5-dihydroisoxazol-3-yl)Ph | 5-trifluoromethylpyridin-2-yl | i-propyl |
| 2-Me-4-SO₂Me-3-(4,5-dihydroisoxazol-3-yl)Ph | 5-trifluoromethylpyridin-2-yl | cyclopropyl |
| 2-Me-4-SO₂Me-3-(4,5-dihydroisoxazol-3-yl)Ph | 5-trifluoromethylpyridin-2-yl | CF₃ |
| 2-Me-4-SO₂Me-3-(4,5-dihydroisoxazol-3-yl)Ph | pyrimidin-2-yl | H |
| 2-Me-4-SO₂Me-3-(4,5-dihydroisoxazol-3-yl)Ph | pyrimidin-2-yl | methyl |
| 2-Me-4-SO₂Me-3-(4,5-dihydroisoxazol-3-yl)Ph | pyrimidin-2-yl | i-propyl |
| 2-Me-4-SO₂Me-3-(4,5-dihydroisoxazol-3-yl)Ph | pyrimidin-2-yl | cyclopropyl |
| 2-Me-4-SO₂Me-3-(4,5-dihydroisoxazol-3-yl)Ph | pyrimidin-2-yl | CF₃ |
| 2-Me-4-SO₂Me-3-(4,5-dihydroisoxazol-3-yl)Ph | pyrimidin-4-yl | H |
| 2-Me-4-SO₂Me-3-(4,5-dihydroisoxazol-3-yl)Ph | pyrimidin-4-yl | methyl |
| 2-Me-4-SO₂Me-3-(4,5-dihydroisoxazol-3-yl)Ph | pyrimidin-4-yl | i-propyl |
| 2-Me-4-SO₂Me-3-(4,5-dihydroisoxazol-3-yl)Ph | pyrimidin-4-yl | cyclopropyl |
| 2-Me-4-SO₂Me-3-(4,5-dihydroisoxazol-3-yl)Ph | pyrimidin-4-yl | CF₃ |
| 2-Me-4-SO₂Me-3-(4,5-dihydroisoxazol-3-yl)Ph | 6-chloropyrimidin-4-yl | methyl |
| 2-Me-4-SO₂Me-3-(4,5-dihydroisoxazol-3-yl)Ph | 6-chloropyrimidin-4-yl | i-propyl |
| 2-Me-4-SO₂Me-3-(4,5-dihydroisoxazol-3-yl)Ph | 6-chloropyrimidin-4-yl | cyclopropyl |
| 2-Me-4-SO₂Me-3-(4,5-dihydroisoxazol-3-yl)Ph | 6-chloropyrimidin-4-yl | CF₃ |
| 2-Me-4-SO₂Me-3-(4,5-dihydroisoxazol-3-yl)Ph | pyridazin-3-yl | H |
| 2-Me-4-SO₂Me-3-(4,5-dihydroisoxazol-3-yl)Ph | pyridazin 3-yl | methyl |
| 2-Me-4-SO₂Me-3-(4,5-dihydroisoxazol-3-yl)Ph | pyridazin-3-yl | i-propyl |
| 2-Me-4-SO₂Me-3-(4,5-dihydroisoxazol-3-yl)Ph | pyridazin-3-yl | cyclopropyl |
| 2-Me-4-SO₂Me-3-(4,5-dihydroisoxazol-3-yl)Ph | pyridazin-3-yl | CF₃ |
| 2-Me-4-SO₂Me-3-(4,5-dihydroisoxazol-3-yl)Ph | 6-chloropyridazin-3-yl | methyl |
| 2-Me-4-SO₂Me-3-(4,5-dihydroisoxazol-3-yl)Ph | 6-chloropyridazin-3-yl | i-propyl |
| 2-Me-4-SO₂Me-3-(4,5-dihydroisoxazol-3-yl)Ph | 6-chloropyridazin-3-yl | cyclopropyl |
| 2-Me-4-SO₂Me-3-(4,5-dihydroisoxazol-3-yl)Ph | 6-chloropyridazin-3-yl | CF₃ |
| 2-Me-4-SO₂Me-3-(4,5-dihydroisoxazol-3-yl)Ph | pyrazin-2-yl | methyl |
| 2-Me-4-SO₂Me-3-(4,5-dihydroisoxazol-3-yl)Ph | pyrazin-2-yl | i-propyl |
| 2-Me-4-SO₂Me-3-(4,5-dihydroisoxazol-3-yl)Ph | pyrazin-2-yl | cyclopropyl |
| 2-Me-4-SO₂Me-3-(4,5-dihydroisoxazol-3-yl)Ph | pyrazin-2-yl | CF₃ |
| 2-Me-4-SO₂Me-3-(4,5-dihydroisoxazol-3-yl)Ph | triazin-2-yl | methyl |
| 2-Me-4-SO₂Me-3-(4,5-dihydroisoxazol-3-yl)Ph | triazin-2-yl | i-propyl |
| 2-Me-4-SO₂Me-3-(4,5-dihydroisoxazol-3-yl)Ph | triazin-2-yl | cyclopropyl |
| 2-Me-4-SO₂Me-3-(4,5-dihydroisoxazol-3-yl)Ph | triazin-2-yl | CF₃ |
| 2-Me-4-SO₂Me-3-(4,5-dihydroisoxazol-3-yl)Ph | quinolin-2-yl | methyl |
| 2-Me-4-SO₂Me-3-(4,5-dihydroisoxazol-3-yl)Ph | quinolin-2-yl | i-propyl |
| 2-Me-4-SO₂Me-3-(4,5-dihydroisoxazol-3-yl)Ph | quinolin-2-yl | cyclopropyl |
| 2-Me-4-SO₂Me-3-(4,5-dihydroisoxazol-3-yl)Ph | quinolin-2-yl | CF₃ |
| 2-Me-4-SO₂Me-3-(4,5-dihydroisoxazol-3-yl)Ph | 4,4,6-trimethyl-5,6-dihydro-1,3(4H)-oxazin-2-yl | H |
| 2-Me-4-SO₂Me-3-(4,5-dihydroisoxazol-3-yl)Ph | 4,4,6-trimethyl-5,6-dihydro-1,3(4H)-oxazin-2-yl | methyl |
| 2-Me-4-SO₂Me-3-(4,5-dihydroisoxazol-3-yl)Ph | 4,4,6-trimethyl-5,6-dihydro-1,3(4H)-oxazin-2-yl | i-propyl |
| 2-Me-4-SO₂Me-3-(4,5-dihydroisoxazol-3-yl)Ph | 4,4,6-trimethyl-5,6-dihydro-1,3(4H)-oxazin-2-yl | cyclopropyl |
| 2-Me-4-SO₂Me-3-(4,5-dihydroisoxazol-3-yl)Ph | 4,4,6-trimethyl-5,6-dihydro-1,3(4H)-oxazin-2-yl | CF₃ |
| 2-Me-4-SO₂Me-3-(4,5-dihydroisoxazol-3-yl)Ph | 2-oxazolidinon-3-yl | H |
| 2-Me-4-SO₂Me-3-(4,5-dihydroisoxazol-3-yl)Ph | 2-oxazolidinon-3-yl | methyl |
| 2-Me-4-SO₂Me-3-(4,5-dihydroisoxazol-3-yl)Ph | 2-oxazolidinon-3-yl | i-propyl |
| 2-Me-4-SO₂Me-3-(4,5-dihydroisoxazol-3-yl)Ph | 2-oxazolidinon-3-yl | cyclopropyl |
| 2-Me-4-SO₂Me-3-(4,5-dihydroisoxazol-3-yl)Ph | 2-oxazolidinon-3-yl | CF₃ |
| 2-Me-4-SO₂Me-3-(4,5-dihydroisoxazol-3-yl)Ph | 2-pyrrolidinon-1-yl | methyl |
| 2-Me-4-SO₂Me-3-(4,5-dihydroisoxazol-3-yl)Ph | 2-pyrrolidinon-1-yl | i-propyl |
| 2-Me-4-SO₂Me-3-(4,5-dihydroisoxazol-3-yl)Ph | 2-pyrrolidinon-1-yl | cyclopropyl |
| 2-Me-4-SO₂Me-3-(4,5-dihydroisoxazol-3-yl)Ph | 2-pyrrolidinon-1-yl | CF₃ |
| 2-Me-4-SO₂Me-3-(4,5-dihydroisoxazol-3-yl)Ph | 3-methylisoxazol-5-yl | methyl |
| 2-Me-4-SO₂Me-3-(4,5-dihydroisoxazol-3-yl)Ph | 3-methylisoxazol-5-yl | i-propyl |
| 2-Me-4-SO₂Me-3-(4,5-dihydroisoxazol-3-yl)Ph | 3-methylisoxazol-5-yl | cyclopropyl |
| 2-Me-4-SO₂Me-3-(4,5-dihydroisoxazol-3-yl)Ph | 3-methylisoxazol-5-yl | CF₃ |
| 4,4-dioxide-8-Me-2,3-dihydro-1,4-benzoxathiin-7-yl | 1,2,4-oxadiazol-5-yl | H |
| 4,4-dioxide-8-Me-2,3-dihydro-1,4-benzoxathiin-7-yl | 1,2,4-oxadiazol-5-yl | methyl |
| 4,4-dioxide-8-Me-2,3-dihydro-1,4-benzoxathiin-7-yl | 1,2,4-oxadiazol-5-yl | i-propyl |
| 4,4-dioxide-8-Me-2,3-dihydro-1,4-benzoxathiin-7-yl | 1,2,4-oxadiazol-5-yl | cyclopropyl |
| 4,4-dioxide-8-Me-2,3-dihydro-1,4-benzoxathiin-7-yl | 1,2,4-oxadiazol-5-yl | CF₃ |
| 4,4-dioxide-8-Me-2,3-dihydro-1,4-benzoxathiin-7-yl | 3-methyl-1,2,4-oxadiazol-5-yl | H |
| 4,4-dioxide-8-Me-2,3-dihydro-1,4-benzoxathiin-7-yl | 3-methyl-1,2,4-oxadiazol-5-yl | methyl |
| 4,4-dioxide-8-Me-2,3-dihydro-1,4-benzoxathiin-7-yl | 3-methyl-1,2,4-oxadiazol-5-yl | i-propyl |
| 4,4-dioxide-8-Me-2,3-dihydro-1,4-benzoxathiin-7-yl | 3-methyl-1,2,4-oxadiazol-5-yl | cyclopropyl |
| 4,4-dioxide-8-Me-2,3-dihydro-1,4-benzoxathiin-7-yl | 3-methyl-1,2,4-oxadiazol-5-yl | CF₃ |
| 4,4-dioxide-8-Me-2,3-dihydro-1,4-benzoxathiin-7-yl | 3-trifluoromethyl-1,2,4-oxadiazol-5-yl | H |
| 4,4-dioxide-8-Me-2,3-dihydro-1,4-benzoxathiin-7-yl | 3-trifluoromethyl-1,2,4-oxadiazol-5-yl | methyl |
| 4,4-dioxide-8-Me-2,3-dihydro-1,4-benzoxathiin-7-yl | 3-trifluoromethyl-1,2,4-oxadiazol-5-yl | i-propyl |
| 4,4-dioxide-8-Me-2,3-dihydro-1,4-benzoxathiin-7-yl | 3-trifluoromethyl-1,2,4-oxadiazol-5-yl | cyclopropyl |
| 4,4-dioxide-8-Me-2,3-dihydro-1,4-benzoxathiin-7-yl | 3-trifluoromethyl-1,2,4-oxadiazol-5-yl | CF₃ |
| 4,4-dioxide-8-Me-2,3-dihydro-1,4-benzoxathiin-7-yl | 1,2,4-oxadiazol-3-yl | H |
| 4,4-dioxide-8-Me-2,3-dihydro-1,4-benzoxathiin-7-yl | 1,2,4-oxadiazol-3-yl | methyl |
| 4,4-dioxide-8-Me-2,3-dihydro-1,4-benzoxathiin-7-yl | 1,2,4-oxadiazol-3-yl | i-propyl |
| 4,4-dioxide-8-Me-2,3-dihydro-1,4-benzoxathiin-7-yl | 1,2,4-oxadiazol-3-yl | cyclopropyl |
| 4,4-dioxide-8-Me-2,3-dihydro-1,4-benzoxathiin-7-yl | 1,2,4-oxadiazol-3-yl | CF₃ |
| 4,4-dioxide-8-Me-2,3-dihydro-1,4-benzoxathiin-7-yl | 5-methyl-1,2,4-oxadiazol-3-yl | H |
| 4,4-dioxide-8-Me-2,3-dihydro-1,4-benzoxathiin-7-yl | 5-methyl-1,2,4-oxadiazol-3-yl | methyl |
| 4,4-dioxide-8-Me-2,3-dihydro-1,4-benzoxathiin-7-yl | 5-methyl-1,2,4-oxadiazol-3-yl | i-propyl |
| 4,4-dioxide-8-Me-2,3-dihydro-1,4-benzoxathiin-7-yl | 5-methyl-1,2,4-oxadiazol-3-yl | cyclopropyl |
| 4,4-dioxide-8-Me-2,3-dihydro-1,4-benzoxathiin-7-yl | 5-methyl-1,2,4-oxadiazol-3-yl | CF₃ |
| 4,4-dioxide-8-Me-2,3-dihydro-1,4-benzoxathiin-7-yl | 5-trifluoromethyl-1,2,4-oxadiazol-3-yl | H |
| 4,4-dioxide-8-Me-2,3-dihydro-1,4-benzoxathiin-7-yl | 5-trifluoromethyl-1,2,4-oxadiazol-3-yl | methyl |
| 4,4-dioxide-8-Me-2,3-dihydro-1,4-benzoxathiin-7-yl | 5-trifluoromethyl-1,2,4-oxadiazol-3-yl | i-propyl |
| 4,4-dioxide-8-Me-2,3-dihydro-1,4-benzoxathiin-7-yl | 5-trifluoromethyl-1,2,4-oxadiazol-3-yl | cyclopropyl |
| 4,4-dioxide-8-Me-2,3-dihydro-1,4-benzoxathiin-7-yl | 5-trifluoromethyl-1,2,4-oxadiazol-3-yl | CF₃ |
| 4,4-dioxide-8-Me-2,3-dihydro-1,4-benzoxathiin-7-yl | 5-chloro-1,2,4-oxadiazol-3-yl | H |
| 4,4-dioxide-8-Me-2,3-dihydro-1,4-benzoxathiin-7-yl | 5-chloro-1,2,4-oxadiazol-3-yl | methyl |
| 4,4-dioxide-8-Me-2,3-dihydro-1,4-benzoxathiin-7-yl | 5-chloro-1,2,4-oxadiazol-3-yl | i-propyl |
| 4,4-dioxide-8-Me-2,3-dihydro-1,4-benzoxathiin-7-yl | 5-chloro-1,2,4-oxadiazol-3-yl | cyclopropyl |
| 4,4-dioxide-8-Me-2,3-dihydro-1,4-benzoxathiin-7-yl | 5-chloro-1,2,4-oxadiazol-3-yl | CF₃ |
| 4,4-dioxide-8-Me-2,3-dihydro-1,4-benzoxathiin-7-yl | 1,3,4-oxadiazol-2-yl | H |
| 4,4-dioxide-8-Me-2,3-dihydro-1,4-benzoxathiin-7-yl | 1,3,4-oxadiazol-2-yl | methyl |
| 4,4-dioxide-8-Me-2,3-dihydro-1,4-benzoxathiin-7-yl | 1,3,4-oxadiazol-2-yl | i-propyl |
| 4,4-dioxide-8-Me-2,3-dihydro-1,4-benzoxathiin-7-yl | 1,3,4-oxadiazol-2-yl | cyclopropyl |
| 4,4-dioxide-8-Me-2,3-dihydro-1,4-benzoxathiin-7-yl | 1,3,4-oxadinol-2-yl | CF₃ |
| 4,4-dioxide-8-Me-2,3-dihydro-1,4-benzoxathiin-7-yl | 5-methylsulfonyl-1,3,4-oxadiazol-2-yl | H |
| 4,4-dioxide-8-Me-2,3-dihydro-1,4-benzoxathiin-7-yl | 5-methylsulfonyl-1,3,4-oxadiazol-2-yl | methyl |
| 4,4-dioxide-8-Me-2,3-dihydro-1,4-benzoxathiin-7-yl | 5-methylsulfonyl-1,3,4-oxadiazol-2-yl | i-propyl |
| 4,4-dioxide-8-Me-2,3-dihydro-1,4-benzoxathiin-7-yl | 5-methylsulfonyl-1,3,4-oxadiazol-2-yl | cyclopropyl |
| 4,4-dioxide-8-Me-2,3-dihydro-1,4-benzoxathiin-7-yl | 5-methylsulfonyl-1,3,4-oxadiazol-2-yl | CF₃ |
| 4,4-dioxide-8-Me-2,3-dihydro-1,4-benzoxathiin-7-yl | 5-methyl-1,3,4-oxadiazol-2-yl | H |
| 4,4-dioxide-8-Me-2,3-dihydro-1,4-benzoxathiin-7-yl | 5-methyl-1,3,4-oxadiazol-2-yl | methyl |
| 4,4-dioxide-8-Me-2,3-dihydro-1,4-benzoxathiin-7-yl | 5-methyl-1,3,4-oxadiazol-2-yl | i-propyl |
| 4,4-dioxide-8-Me-2,3-dihydro-1,4-benzoxathiin-7-yl | 5-methyl-1,3,4-oxadiazol-2-yl | cyclopropyl |
| 4,4-dioxide-8-Me-2,3-dihydro-1,4-benzoxathiin-7-yl | 5-methyl-1,3,4-oxadiazol-2-yl | CF₃ |
| 4,4-dioxide-8-Me-2,3-dihydro-1,4-benzoxathiin-7-yl | 5-trifluoromethyl-1,3,4-oxadiazol-2-yl | H |
| 4,4-dioxide-8-Me-2,3-dihydro-1,4-benzoxathiin-7-yl | 5-trifluoromethyl-1,3,4-oxadiazol2-yl | methyl |
| 4,4-dioxide-8-Me-2,3-dihydro-1,4-benzoxathiin-7-yl | 5-trifluoromethyl-1,3,4-oxadiazol-2-yl | i-propyl |
| 4,4-dioxide-8-Me-2,3-dihydro-1,4-benzoxathiin-7-yl | 5-trifluoromethyl-1,3,4-oxadiazol-2-yl | cyclopropyl |
| 4,4-dioxide-8-Me-2,3-dihydro-1,4-benzoxathiin-7-yl | 5-trifluoromethyl-1,3,4-oxadiazol-2-yl | CF₃ |
| 4,4-dioxide-8-Me-2,3-dihydro-1,4-benzoxathiin-7-yl | 1,2,3-triazol-4-yl | H |
| 4,4-dioxide-8-Me-2,3-dihydro-1,4-benzoxathiin-7-yl | 1,2,3 triazol-4-yl | methyl |
| 4,4-dioxide-8-Me-2,3-dihydro-1,4-benzoxathiin-7-yl | 1,2,3-triazol-4-yl | i-propyl |
| 4,4-dioxide-8-Me-2,3-dihydro-1,4-benzoxathiin-7-yl | 1,2,3-triazol-4-yl | cyclopropyl |
| 4,4-dioxide-8-Me-2,3-dihydro-1,4-benzoxathiin-7-yl | 1,2,3-triazol-yl | CF₃ |
| 4,4-dioxide-8-Me-2,3-dihydro-1,4-benzoxathiin-7-yl | 1-methyl-1,2,3-tiazol-4-yl | H |
| 4,4-dioxide-8-Me-2,3-dihydro-1,4-benzoxathiin-7-yl | 1-methyl-1,2,3 triazol-4-yl | methyl |
| 4,4-dioxide-8-Me-2,3-dihydro-1,4-benzoxathiin-7-yl | 1-methyl-1,2,3-triazol-4-yl | i-propyl |
| 4,4-dioxide-8-Me-2,3-dihydro-1,4-benzoxathiin-7-yl | 1-methyl-1,2,3-triazol-4-yl | cyclopropyl |
| 4,4-dioxide-8-Me-2,3-dihydro-1,4-benzoxathiin-7-yl | 1-methyl-1,2,3-triazol-4-yl | CF₃ |
| 4,4-dioxide-8-Me-2,3-dihydro-1,4-benzoxathiin-7-yl | 2-methyl-1,2,3-triazol-4-yl | H |
| 4,4-dioxide-8-Me-2,3-dihydro-1,4-benzoxathiin-7-yl | 2-methyl-1,2,3-triazol-4-yl | methyl |
| 4,4-dioxide-8-Me-2,3-dihydro-1,4-benzoxathiin-7-yl | 2-methyl-1,2,3-triazol-4-yl | i-propyl |
| 4,4-dioxide-8-Me-2,3-dihydro-1,4-benzoxathiin-7-yl | 2-methyl-1,2,3-triazol-4-yl | cyclopropyl |
| 4,4-dioxide-8-Me-2,3-dihydro-1,4-benzoxathiin-7-yl | 2-methyl-1,2,3-triazol-4-yl | CF₃ |
| 4,4-dioxide-8-Me-2,3-dihydro-1,4-benzoxathiin-7-yl | 1,2,3-triazol-1-yl | H |
| 4,4-dioxide-8-Me-2,3-dihydro-1,4-benzoxathiin-7-yl | 1,2,3-triazol-1-yl | methyl |
| 4,4-dioxide-8-Me-2,3-dihydro-1,4-benzoxathiin-7-yl | 1,2,3-triazol-1-yl | i-propyl |
| 4,4-dioxide-8-Me-2,3-dihydro-1,4-benzoxathiin-7-yl | 1,2,3-triazol-1-yl | cyclopropyl |
| 4,4-dioxide-8-Me-2,3-dihydro-1,4-benzoxathiin-7-yl | 1,2,3-triazol-1-yl | CF₃ |
| 4,4-dioxide-8-Me-2,3-dihydro-1,4-benzoxathiin-7-yl | 1,2,3-triazol-2-yl | H |
| 4,4-dioxide-8-Me-2,3-dihydro-1,4-benzoxathiin-7-yl | 1,2,3-triazol-2-yl | methyl |
| 4,4-dioxide-8-Me-2,3-dihydro-1,4-benzoxathiin-7-yl | 1,2,3-triazol-2-yl | i-propyl |
| 4,4-dioxide-8-Me-2,3-dihydro-1,4-benzoxathiin-7-yl | 1,2,3-triazol-2-yl | cyclopropyl |
| 4,4-dioxide-8-Me-2,3-dihydro-1,4-benzoxathiin-7-yl | 1,2,3-triazol-2-yl | CF₃ |
| 4,4-dioxide-8-Me-2,3-dihydro-1,4-benzoxathiin-7-yl | 1,2,4-triazol-1-yl | H |
| 4,4-dioxide-8-Me-2,3-dihydro-1,4-benzoxathiin-7-yl | 1,2,4-triazol-1-yl | methyl |
| 4,4-dioxide-8-Me-2,3-dihydro-1,4-benzoxathiin-7-yl | 1,2,4-triazol-1-yl | i-propyl |
| 4,4-dioxide-8-Me-2,3-dihydro-1,4-benzoxathiin-7-yl | 1,2,4-triazol-1-yl | cyclopropyl |
| 4,4-dioxide-8-Me-2,3-dihydro-1,4-benzoxathiin-7-yl | 1,2,4-triazol-1-yl | CF₃ |
| 4,4-dioxide-8-Me-2,3-dihydro-1,4-benzoxathiin-7-yl | imidazol-2-yl | H |
| 4,4-dioxide-8-Me-2,3-dihydro-1,4-benzoxathiin-7-yl | imidazol-2-yl | methyl |
| 4,4-dioxide-8-Me-2,3-dihydro-1,4-benzoxathiin-7-yl | imidazol-2-yl | i-propyl |
| 4,4-dioxide-8-Me-2,3-dihydro-1,4-benzoxathiin-7-yl | imidazol-2-yl | cyclopropyl |
| 4,4-dioxide-8-Me-2,3-dihydro-1,4-benzoxathiin-7-yl | imidazol-2-yl | CF₃ |
| 4,4-dioxide-8-Me-2,3-dihydro-1,4-benzoxathiin-7-yl | imidazol-1-yl | H |
| 4,4-dioxide-8-Me-2,3-dihydro-1,4-benzoxathiin-7-yl | imidazol-1-yl | methyl |
| 4,4-dioxide-8-Me-2,3-dihydro-1,4-benzoxathiin-7-yl | imidazol-1-yl | i-propyl |
| 4,4-dioxide-8-Me-2,3-dihydro-1,4-benzoxathiin-7-yl | imidazol-1-yl | cyclopropyl |
| 4,4-dioxide-8-Me-2,3-dihydro-1,4-benzoxathiin-7-yl | imidazol-1-yl | CF₃ |
| 4,4-dioxide-8-Me-2,3-dihydro-1,4-benzoxathiin-7-yl | imidazol-4-yl | H |
| 4,4-dioxide-8-Me-2,3-dihydro-1,4-benzoxathiin-7-yl | imidazol-4-yl | methyl |
| 4,4-dioxide-8-Me-2,3-dihydro-1,4-benzoxathiin-7-yl | imidazol-4-yl | i-propyl |
| 4,4-dioxide-8-Me-2,3-dihydro-1,4-benzoxathiin-7-yl | imidazol-4-yl | cyclopropyl |
| 4,4-dioxide-8-Me-2,3-dihydro-1,4-benzoxathiin-7-yl | imidazol-4-yl | CF₃ |
| 4,4-dioxide-8-Me-2,3-dihydro-1,4-benzoxathiin-7-yl | thiazol-2-yl | H |
| 4,4-dioxide-8-Me-2,3-dihydro-1,4-benzoxathiin-7-yl | thiazol-2-yl | methyl |
| 4,4-dioxide-8-Me-2,3-dihydro-1,4-benzoxathiin-7-yl | thiazol-2-yl | i-propyl |
| 4,4-dioxide-8-Me-2,3-dihydro-1,4-benzoxathiin-7-yl | thiazol-2-yl | cyclopropyl |
| 4,4-dioxide-8-Me-2,3-dihydro-1,4-benzoxathiin-7-yl | thiazol-2-yl | CF₃ |
| 4,4-dioxide-8-Me-2,3-dihydro-1,4-benzoxathiin-7-yl | 4-methylthiazol-2-yl | H |
| 4,4-dioxide-8-Me-2,3-dihydro-1,4-benzoxathiin-7-yl | 4-methylthiazol-2-yl | methyl |
| 4,4-dioxide-8-Me-2,3-dihydro-1,4-benzoxathiin-7-yl | 4-methylthiazol-2-yl | i-propyl |
| 4,4-dioxide-8-Me-2,3-dihydro-1,4-benzoxathiin-7-yl | 4-methylthiazol-2-yl | cyclopropyl |
| 4,4-dioxide-8-Me-2,3-dihydro-1,4-benzoxathiin-7-yl | 4-methylthiazol-2-yl | CF₃ |
| 4,4-dioxide-8-Me-2,3-dihydro-1,4-benzoxathiin-7-yl | oxazol-2-yl | H |
| 4,4-dioxide-8-Me-2,3-dihydro-1,4-benzoxathiin-7-yl | oxazol-2-yl | methyl |
| 4,4-dioxide-8-Me-2,3-dihydro-1,4-benzoxathiin-7-yl | oxazol-2-yl | i-propyl |
| 4,4-dioxide-8-Me-2,3-dihydro-1,4-benzoxathiin-7-yl | oxazol-2-yl | cyclopropyl |
| 4,4-dioxide-8-Me-2,3-dihydro-1,4-benzoxathiin-7-yl | oxazol-2-yl | CF₃ |
| 4,4-dioxide-8-Me-2,3-dihydro-1,4-benzoxathiin-7-yl | 4,5-dimethyloxazol-2-yl | H |
| 4,4-dioxide-8-Me-2,3-dihydro-1,4-benzoxathiin-7-yl | 4,5-dimethyloxazol-2-yl | methyl |
| 4,4-dioxide-8-Me-2,3-dihydro-1,4-benzoxathiin-7-yl | 4,5-dimethyloxazol-2-yl | i-propyl |
| 4,4-dioxide-8-Me-2,3-dihydro-1,4-benzoxathiin-7-yl | 4,5-dimethyloxazol-2-yl | cyclopropyl |
| 4,4-dioxide-8-Me-2,3-dihydro-1,4-benzoxathiin-7-yl | 4,5-dimethyloxazol-2-yl | CF₃ |
| 4,4-dioxide-8-Me-2,3-dihydro-1,4-benzoxathiin-7-yl | 2-oxazolin-2-yl | H |
| 4,4-dioxide-8-Me-2,3-dihydro-1,4-benzoxathiin-7-yl | 2-oxazolin-2-yl | methyl |
| 4,4-dioxide-8-Me-2,3-dihydro-1,4-benzoxathiin-7-yl | 2-oxazolin-2-yl | i-propyl |
| 4,4-dioxide-8-Me-2,3-dihydro-1,4-benzoxathiin-7-yl | 2-oxazolin-2-yl | cyclopropyl |
| 4,4-dioxide-8-Me-2,3-dihydro-1,4-benzoxathiin-7-yl | 2-oxazolin-2-yl | CF₃ |
| 4,4-dioxide-8-Me-2,3-dihydro-1,4-benzoxathiin-7-yl | 4,4-dimethyl-2-oxazolin-2-yl | H |
| 4,4-dioxide-8-Me-2,3-dihydro-1,4-benzoxathiin-7-yl | 4,4-dimethyl-2-oxazolin-2-yi | methyl |
| 4,4-dioxide-8-Me-2,3-dihydro-1,4-benzoxathiin-7-yl | 4,4-dimethyl-2-oxazolin-2-yl | i-propyl |
| 4,4-dioxide-8-Me-2,3-dihydro-1,4-benzoxathiin-7-yl | 4,4-dimethyl-2-oxazolin-2-yl | cyclopropyl |
| 4,4-dioxide-8-Me-2,3-dihydro-1,4-benzoxathiin-7-yl | 4,4-dimethyl-2-oxazolin-2-yl | CF₃ |
| 4,4-dioxide-8-Me-2,3-dihydro-1,4-benzoxathiin-7-yl | 1,2,4-thiadiazol-5-yl | H |
| 4,4-dioxide-8-Me-2,3-dihydro-1,4-benzoxathiin-7-yl | 1,2,4-thiadiazol-5-yl | methyl |
| 4,4-dioxide-8-Me-2,3-dihydro-1,4-benzoxathiin-7-yl | 1,2,4-thiadiazol-5-yl | i-propyl |
| 4,4-dioxide-8-Me-2,3-dihydro-1,4-benzoxathiin-7-yl | 1,2,4-thiadiazol-5-yl | cyclopropyl |
| 4,4-dioxide-8-Me-2,3-dihydro-1,4-benzoxathiin-7-yl | 1,2,4-thiadiazol-5-yl | CF₃ |
| 4,4-dioxide-8-Me-2,3-dihydro-1,4-benzoxathiin-7-yl | 3-methyl-1,2,4-thiadiazol-5-yl | H |
| 4,4-dioxide-8-Me-2,3-dihydro-1,4-benzoxathiin-7-yl | 3-methyl-1,2,4-thiadiazol-5-yl | methyl |
| 4,4-dioxide-8-Me-2,3-dihydro-1,4-benzoxathiin-7-yl | 3-methyl-1,2,4-thiadiazol-5-yl | i-propyl |
| 4,4-dioxide-8-Me-2,3-dihydro-1,4-benzoxathiin-7-yl | 3-methyl-1,2,4-thiadiazol-5-yl | cyclopropyl |
| 4,4-dioxide-8-Me-2,3-dihydro-1,4-benzoxathiin-7-yl | 3-methyl-1,2,4-thiadiazol-5-yl | CF₃ |
| 4,4-dioxide-8-Me-2,3-dihydro-1,4-benzoxathiin-7-yl | 3-trifluoromethyl-1,2,4-thiadiazol-5-yl | H |
| 4,4-dioxide-8-Me-2,3-dihydro-1,4-benzoxathiin-7-yl | 3-trifluoromethyl-1,2,4-thiadiazol-5-yl | methyl |
| 4,4-dioxide-8-Me-2,3-dihydro-1,4-benzoxathiin-7-yl | 3-trifluoromethyl-1,2,4-thiadiazol-5-yl | i-propyl |
| 4,4-dioxide-8-Me-2,3-dihydro-1,4-benzoxathiin-7-yl | 3-trifluoromethyl-1,2,4-thiadiazol-5-yl | cyclopropyl |
| 4,4-dioxide-8-Me-2,3-dihydro-1,4-benzoxathiin-7-yl | 3-trifluoromethyl-1,2,4-thiadiazol-5-yl | CF₃ |
| 4,4-dioxide-8-Me-2,3-dihydro-1,4-benzoxathiin-7-yl | 1,2,4-thiadiazol-3-yl | H |
| 4,4-dioxide-8-Me-2,3-dihydro-1,4-benzoxathiin-7-yl | 1,2,4-thiadiazol-3-yl | methyl |
| 4,4-dioxide-8-Me-2,3-dihydro-1,4-benzoxathiin-7-yl | 1,2,4-thiadiazol-3-yl | i-propyl |
| 4,4-dioxide-8-Me-2,3-dihydro-1,4-benzoxathiin-7-yl | 1,2,4-thiadiazol-3-yl | cyclopropyl |
| 4,4-dioxide-8-Me-2,3-dihydro-1,4-benzoxathiin-7-yl | 1,2,4-thiadiazol-3-yl | CF₃ |
| 4,4-dioxide-8-Me-2,3-dihydro-1,4-benzoxathiin-7-yl | 5-methyl-1,2,4-thiadiazol-3-yl | H |
| 4,4-dioxide-8-Me-2,3-dihydro-1,4-benzoxathiin-7-yl | 5-methyl-1,2,4-thiadiazol-3-yl | methyl |
| 4,4-dioxide-8-Me-2,3-dihydro-1,4-benzoxathiin-7-yl | 5-methyl-1,2,4-thiadiazol-3-yl | i-propyl |
| 4,4-dioxide-8-Me-2,3-dihydro-1,4-benzoxathiin-7-yl | 5-methyl-1,2,4-thiadiazol-3-yl | cyclopropyl |
| 4,4-dioxide-8-Me-2,3-dihydro-1,4-benzoxathiin-7-yl | 5-methyl-1,2,4-thiadinol-3-yl | CF₃ |
| 4,4-dioxide-8-Me-2,3-dihydro-1,4-benzoxathiin-7-yl | 5-trifluoromethyl-1,2,4-thiadiazol-3-yl | H |
| 4,4-dioxide-8-Me-2,3-dihydro-1,4-benzoxathiin-7-yl | 5-trifluoromethyl-1,2,4-thiadiazol-3-yl | methyl |
| 4,4-dioxide-8-Me-2,3-dihydro-1,4-benzoxathiin-7-yl | 5-trifluoromethyl-1,2,4-thiadiazol-3-yl | i-propyl |
| 4,4-dioxide-8-Me-2,3-dihydro-1,4-benzoxathiin-7-yl | 5-trifluoromethyl-1,2,4-thiadiazol-3-yl | cyclopropyl |
| 4,4-dioxide-8-Me-2,3-dihydro-1,4-benzoxathiin-7-yl | 5-trifluoromethyl-1,2,4-thiadiazol-3-yl | CF₃ |
| 4,4-dioxide-8-Me-2,3-dihydro-1,4-benzoxathiin-7-yl | 1,3,4-thiadiazol-2-yl | H |
| 4,4-dioxide-8-Me-2,3-dihydro-1,4-benzoxathiin-7-yl | 1,3,4 thiadiazol-2-yl | methyl |
| 4,4-dioxide-8-Me-2,3-dihydro-1,4-benzoxathiin-7-yl | 1,3,4-thiadiazol-2-yl | i-propyl |
| 4,4-dioxide-8-Me-2,3-dihydro-1,4-benzoxathiin-7-yl | 1,3,4-thiadiazol-2-yl | cyclopropyl |
| 4,4-dioxide-8-Me-2,3-dihydro-1,4-benzoxathiin-7-yl | 1,3,4-thiadiazol-2-yl | CF₃ |
| 4,4-dioxide-8-Me-2,3-dihydro-1,4-benzoxathiin-7-yl | 5-methylsulfonyl-1,3,4-thiadiazol-2-yl | H |
| 4,4-dioxide-8-Me-2,3-dihydro-1,4-benzoxathiin-7-yl | 5-methylsulfonyl-1,3,4-thiadiazol-2-yl | methyl |
| 4,4-dioxide-8-Me-2,3-dihydro-1,4-benzoxathiin-7-yl | 5-methylsulfonyl-1,3,4-thiadiazol-2-yl | i-propyl |
| 4,4-dioxide-8-Me-2,3-dihydro-1,4-benzoxathiin-7-yl | 5-methylsulfonyl-1,3,4-thiadiazol-2-yl | cyclopropyl |
| 4,4-dioxide-8-Me-2,3-dihydro-1,4-benzoxathiin-7-yl | 5-methylsulfonyl-1,3,4-thiadiazol-2-yl | CF₃ |
| 4,4-dioxide-8-Me-2,3-dihydro-1,4-benzoxathiin-7-yl | 5-methyl-1,3,4-thiadiazol-2-yl | H |
| 4,4-dioxide-8-Me-2,3-dihydro-1,4-benzoxathiin-7-yl | 5-methyl-1,3,4-thiadiazol-2-yl | methyl |
| 4,4-dioxide-8-Me-2,3-dihydro-1,4-benzoxathiin-7-yl | 5-methyl-1,3,4-thiadiazol-2-yl | i-propyl |
| 4,4-dioxide-8-Me-2,3-dihydro-1,4-benzoxathiin-7-yl | 5-methyl-1,3,4-thiadiazol-2-yl | cyclopropyl |
| 4,4-dioxide-8-Me-2,3-dihydro-1,4-benzoxathiin-7-yl | 5-methyl-1,3,4-thiadiazol-2-yl | CF₃ |
| 4,4-dioxide-8-Me-2,3-dihydro-1,4-benzoxathiin-7-yl | benzoxazol-2-yl | H |
| 4,4-dioxide-8-Me-2,3-dihydro-1,4-benzoxathiin-7-yl | benzoxazol-2-yl | methyl |
| 4,4-dioxide-8-Me-2,3-dihydro-1,4-benzoxathiin-7-yl | benzoxazol-2-yl | i-propyl |
| 4,4-dioxide-8-Me-2,3-dihydro-1,4-benzoxathiin-7-yl | benzoxazol-2-yl | cyclopropyl |
| 4,4-dioxide-8-Me-2,3-dihydro-1,4-benzoxathiin-7-yl | benzoxazol-2-yl | CF₃ |
| 4,4-dioxide-8-Me-2,3-dihydro-1,4-benzoxathiin-7-yl | 6-methylbenzoxazol-2-yl | H |
| 4,4-dioxide-8-Me-2,3-dihydro-1,4-benzoxathiin-7-yl | 6-methylbenzoxazol-2-yl | methyl |
| 4,4-dioxide-8-Me-2,3-dihydro-1,4-benzoxathiin-7-yl | 6-methylbenzoxazol-2-yl | i-propyl |
| 4,4-dioxide-8-Me-2,3-dihydro-1,4-benzoxathiin-7-yl | 6-methylbenzoxazol-2-yl | cyclopropyl |
| 4,4-dioxide-8-Me-2,3-dihydro-1,4-benzoxathiin-7-yl | 6-methylbenzoxazol-2-yl | CF₃ |
| 4,4-dioxide-8-Me-2,3-dihydro-1,4-benzoxathiin-7-yl | benzothiazol-2-yl | H |
| 4,4-dioxide-8-Me-2,3-dihydro-1,4-benzoxathiin-7-yl | benzothiazol-2-yl | methyl |
| 4,4-dioxide-8-Me-2,3-dihydro-1,4-benzoxathiin-7-yl | benzothiazol-2-yl | i-propyl |
| 4,4-dioxide-8-Me-2,3-dihydro-1,4-benzoxathiin-7-yl | benzothiazol-2-yl | cyclopropyl |
| 4,4-dioxide-8-Me-2,3-dihydro-1,4-benzoxathiin-7-yl | benzothiazol-2-yl | CF₃ |
| 4,4-dioxide-8-Me-2,3-dihydro-1,4-benzoxathiin-7-yl | pyrazol-1-yl | H |
| 4,4-dioxide-8-Me-2,3-dihydro-1,4-benzoxathiin-7-yl | pyrazol-1-yl | methyl |
| 4,4-dioxide-8-Me-2,3-dihydro-1,4-benzoxathiin-7-yl | pyrazol-1-yl | i-propyl |
| 4,4-dioxide-8-Me-2,3-dihydro-1,4-benzoxathiin-7-yl | pyrazol-1-yl | cyclopropyl |
| 4,4-dioxide-8-Me-2,3-dihydro-1,4-benzoxathiin-7-yl | pyrazol-1-yl | CF₃ |
| 4,4-dioxide-8-Me-2,3-dihydro-1,4-benzoxathiin-7-yl | pyrazol-3-yl | H |
| 4,4-dioxide-8-Me-2,3-dihydro-1,4-benzoxathiin-7-yl | pyrazol-3-yl | methyl |
| 4,4-dioxide-8-Me-2,3-dihydro-1,4-benzoxathiin-7-yl | pyrazol-3-yl | i-propyl |
| 4,4-dioxide-8-Me-2,3-dihydro-1,4-benzoxathiin-7-yl | pyrazol-3-yl | cyclopropyl |
| 4,4-dioxide-8-Me-2,3-dihydro-1,4-benzoxathiin-7-yl | pyrazol-3-yl | CF₃ |
| 4,4-dioxide-8-Me-2,3-dihydro-1,4-benzoxathiin-7-yl | 1-methylpyrazol-3-yl | H |
| 4,4-dioxide-8-Me-2,3-dihydro-1,4-benzoxathiin-7-yl | 1-methylpyrazol-3-yl | methyl |
| 4,4-dioxide-8-Me-2,3-dihydro-1,4-benzoxathiin-7-yl | 1-methylpymzol-3-yl | i-propyl |
| 4,4-dioxide-8-Me-2,3-dihydro-1,4-benzoxathiin-7-yl | 1-methylpyrazol-3-yl | cyclopropyl |
| 4,4-dioxide-8-Me-2,3-dihydro-1,4-benzoxathiin-7-yl | 1-methylpyrazol-3-yl | CF₃ |
| 4,4-dioxide-8-Me-2,3-dihydro-1,4-benzoxathiin-7-yl | tetrazol-1-yl | H |
| 4,4-dioxide-8-Me-2,3-dihydro-1,4-benzoxathiin-7-yl | tetrazol-1-yl | methyl |
| 4,4-dioxide-8-Me-2,3-dihydro-1,4-benzoxathiin-7-yl | tetrazol-1-yl | i-propyl |
| 4,4-dioxide-8-Me-2,3-dihydro-1,4-benzoxathiin-7-yl | tetrazol-1-yl | cyclopropyl |
| 4,4-dioxide-8-Me-2,3-dihydro-1,4-benzoxathiin-7-yl | tetrazol-1-yl | CF₃ |
| 4,4-dioxide-8-Me-2,3-dihydro-1,4-benzoxathiin-7-yl | 5-methyltetrazol-1-yl | H |
| 4,4-dioxide-8-Me-2,3-dihydro-1,4-benzoxathiin-7-yl | 5-methyltetrazol-1-yl | methyl |
| 4,4-dioxide-8-Me-2,3-dihydro-1,4-benzoxathiin-7-yl | 5-methyltetrazol-1-yl | i-propyl |
| 4,4-dioxide-8-Me-2,3-dihydro-1,4-benzoxathiin-7-yl | 5-methyltetrazol-1-yl | cyclopropyl |
| 4,4-dioxide-8-Me-2,3-dihydro-1,4-benzoxathiin-7-yl | 5-methyltetrazol-1-yl | CF₃ |
| 4,4-dioxide-8-Me-2,3-dihydro-1,4-benzoxathiin-7-yl | tetrazol-2-yl | H |
| 4,4-dioxide-8-Me-2,3-dihydro-1,4-benzoxathiin-7-yl | tetrazol-2-yl | methyl |
| 4,4-dioxide-8-Me-2,3-dihydro-1,4-benzoxathiin-7-yl | tetrazol-2-yl | i-propyl |
| 4,4-dioxide-8-Me-2,3-dihydro-1,4-benzoxathiin-7-yl | tetrazol-2-yl | cyclopropyl |
| 4,4-dioxide-8-Me-2,3-dihydro-1,4-benzoxathiin-7-yl | tetrazol-2-yl | CF₃ |
| 4,4-dioxide-8-Me-2,3-dihydro-1,4-benzoxathiin-7-yl | 5-methyltetrazol-2-yl | H |
| 4,4-dioxide-8-Me-2,3-dihydro-1,4-benzoxathiin-7-yl | 5-methyltetrazol-2-yl | methyl |
| 4,4-dioxide-8-Me-2,3-dihydro-1,4-benzoxathiin-7-yl | 5-methyltetrazol-2-yl | i-propyl |
| 4,4-dioxide-8-Me-2,3-dihydro-1,4-benzoxathiin-7-yl | 5-methyltetrazol-2-yl | cyclopropyl |
| 4,4-dioxide-8-Me-2,3-dihydro-1,4-benzoxathiin-7-yl | 5-methyltetrazol-2-yl | CF₃ |
| 4,4-dioxide-8-Me-2,3-dihydro-1,4-benzoxathiin-7-yl | 1-methyltetrazol-5-yl | H |
| 4,4-dioxide-8-Me-2,3-dihydro-1,4-benzoxathiin-7-yl | 1-methyltetrazol-5-yl | methyl |
| 4,4-dioxide-8-Me-2,3-dihydro-1,4-benzoxathiin-7-yl | 1-methyltetrazol-5-yl | i-propyl |
| 4,4-dioxide-8-Me-2,3-dihydro-1,4-benzoxathiin-7-yl | 1-methyltetrazol-5-yl | cyclopropyl |
| 4,4-dioxide-8-Me-2,3-dihydro-1,4-benzoxathiin-7-yl | 1-methyltetrazol-5-yl | CF₃ |
| 4,4-dioxide-8-Me-2,3-dihydro-1,4-benzoxathiin-7-yl | 2-methyltetrazol-5-yl | H |
| 4,4-dioxide-8-Me-2,3-dihydro-1,4-benzoxathiin-7-yl | 2-methyltetrazol-5-yl | methyl |
| 4,4-dioxide-8-Me-2,3-dihydro-1,4-benzoxathiin-7-yl | 2-methyltetrazol-5-yl | i-propyl |
| 4,4-dioxide-8-Me-2,3-dihydro-1,4-benzoxathiin-7-yl | 2-methyltetrazol-5-yl | cyclopropyl |
| 4,4-dioxide-8-Me-2,3-dihydro-1,4-benzoxathiin-7-yl | 2-methyltetrazol-5-yl | CF₃ |
| 4,4-dioxide-8-Me-2,3-dihydro-1,4-benzoxathiin-7-yl | pyridin-2-yl | H |
| 4,4-dioxide-8-Me-2,3-dihydro-1,4-benzoxathiin-7-yl | pyridin-2-yl | methyl |
| 4,4-dioxide-8-Me-2,3-dihydro-1,4-benzoxathiin-7-yl | pyridin-2-yl | i-propyl |
| 4,4-dioxide-8-Me-2,3-dihydro-1,4-benzoxathiin-7-yl | pyridin-2-yl | cyclopropyl |
| 4,4-dioxide-8-Me-2,3-dihydro-1,4-benzoxathiin-7-yl | pyridin-2-yl | CF₃ |
| 4,4-dioxide-8-Me-2,3-dihydro-1,4-benzoxathiin-7-yl | pyridin-4-yl | H |
| 4,4-dioxide-8-Me-2,3-dihydro-1,4-benzoxathiin-7-yl | pyridin-4-yl | methyl |
| 4,4-dioxide-8-Me-2,3-dihydro-1,4-benzoxathiin-7-yl | pyridin-4-yl | i-propyl |
| 4,4-dioxide-8-Me-2,3-dihydro-1,4-benzoxathiin-7-yl | pyridin-4-yl | cyclopropyl |
| 4,4-dioxide-8-Me-2,3-dihydro-1,4-benzoxathiin-7-yl | pyridin-4-yl | CF₃ |
| 4,4-dioxide-8-Me-2,3-dihydro-1,4-benzoxathiin-7-yl | pyridin-3-yl | H |
| 4,4-dioxide-8-Me-2,3-dihydro-1,4-benzoxathiin-7-yl | pyridin-3-yl | methyl |
| 4,4-dioxide-8-Me-2,3-dihydro-1,4-benzoxathiin-7-yl | pyridin-3-yl | i-propyl |
| 4,4-dioxide-8-Me-2,3-dihydro-1,4-benzoxathiin-7-yl | pyridin-3-yl | cyclopropyl |
| 4,4-dioxide-8-Me-2,3-dihydro-1,4-benzoxathiin-7-yl | pyridin-3-yl | CF₃ |
| 4,4-dioxide-8-Me-2,3-dihydro-1,4-benzoxathiin-7-yl | 3-nitropyridin-4-yl | H |
| 4,4-dioxide-8-Me-2,3-dihydro-1,4-benzoxathiin-7-yl | 3-nitropyridin-4-yl | methyl |
| 4,4-dioxide-8-Me-2,3-dihydro-1,4-benzoxathiin-7-yl | 3-nitropyridin-4-yl | i-propyl |
| 4,4-dioxide-8-Me-2,3-dihydro-1,4-benzoxathiin-7-yl | 3-nitropyridin-4-yl | cyclopropyl |
| 4,4-dioxide-8-Me-2,3-dihydro-1,4-benzoxathiin-7-yl | 3-nitropyridin-4-yl | CF₃ |
| 4,4-dioxide-8-Me-2,3-dihydro-1,4-benzoxathiin-7-yl | 5-cyanopyndin-2-yl | H |
| 4,4-dioxide-8-Me-2,3-dihydro-1,4-benzoxathiin-7-yl | 5-cyanopyridin-2-yl | methyl |
| 4,4-dioxide-8-Me-2,3-dihydro-1,4-benzoxathiin-7-yl | 5-cyanopyridin-2-yl | i-propyl |
| 4,4-dioxide-8-Me-2,3-dihydro-1,4-benzoxathiin-7-yl | 5-cyanopyridin-2-yl | cyclopropyl |
| 4,4-dioxide-8-Me-2,3-dihydro-1,4-benzoxathiin-7-yl | 5-cyanopyridin-2-yl | CF₃ |
| 4,4-dioxide-8-Me-2,3-dihydro-1,4-benzoxathiin-7-yl | 5-trifluoromethylpyridin-2-yl | H |
| 4,4-dioxide-8-Me-2,3-dihydro-1,4-benzoxathiin-7-yl | 5-trifluoromethylpyridin-2-yl | methyl |
| 4,4-dioxide-8-Me-2,3-dihydro-1,4-benzoxathiin-7-yl | 5-trifluoromethylpyridin-2-yl | i-propyl |
| 4,4-dioxide-8-Me-2,3-dihydro-1,4-benzoxathiin-7-yl | 5-trifluoromethylpyridin-2-yl | cyclopropyl |
| 4,4-dioxide-8-Me-2,3-dihydro-1,4-benzoxathiin-7-yl | 5-trifluoromethylpyridin-2-yl | CF₃ |
| 4,4-dioxide-8-Me-2,3-dihydro-1,4-benzoxathiin-7-yl | pyrimidin-2-yl | H |
| 4,4-dioxide-8-Me-2,3-dihydro-1,4-benzoxathiin-7-yl | pyrimidin-2-yl | methyl |
| 4,4-dioxide-8-Me-2,3-dihydro-1,4-benzoxathiin-7-yl | pyrimidin-2-yl | i-propyl |
| 4,4-dioxide-8-Me-2,3-dihydro-1,4-benzoxathiin-7-yl | pyrimidin-2-yl | cyclopropyl |
| 4,4-dioxide-8-Me-2,3-dihydro-1,4-benzoxathiin-7-yl | pyrimidin-2-yl | CF₃ |
| 4,4-dioxide-8-Me-2,3-dihydro-1,4-benzoxathiin-7-yl | pyrimidin-4-yl | H |
| 4,4-dioxide-8-Me-2,3-dihydro-1,4-benzoxathiin-7-yl | pyrimidin-4-yl | methyl |
| 4,4-dioxide-8-Me-2,3-dihydro-1,4-benzoxathiin-7-yl | pyrimidin-4-yl | i-propyl |
| 4,4-dioxide-8-Me-2,3-dihydro-1,4-benzoxathiin-7-yl | pyrimidin-4-yl | cyclopropyl |
| 4,4-dioxide-8-Me-2,3-dihydro-1,4-benzoxathiin-7-yl | pyrimidin-4-yl | CF₃ |
| 4,4-dioxide-8-Me-2,3-dihydro-1,4-benzoxathiin-7-yl | 6-chloropyrimidin-4-yl | methyl |
| 4,4-dioxide-8-Me-2,3-dihydro-1,4-benzoxathiin-7-yl | 6-chloropyrimidin-4-yl | i-propyl |
| 4,4-dioxide-8-Me-2,3-dihydro-1,4-benzoxathiin-7-yl | 6-chloropyrimidin-4-yl | cyclopropyl |
| 4,4-dioxide-8-Me-2,3-dihydro-1,4-benzoxathiin-7-yl | 6-chloropyrimidin-4-yl | CF₃ |
| 4,4-dioxide-8-Me-2,3-dihydro-1,4-benzoxathiin-7-yl | pyridazin-3-yl | H |
| 4,4-dioxide-8-Me-2,3-dihydro-1,4-benzoxathiin-7-yl | pyridazin-3-yl | methyl |
| 4,4-dioxide-8-Me-2,3-dihydro-1,4-benzoxathiin-7-yl | pyridazin-3-yl | i-propyl |
| 4,4-dioxide-8-Me-2,3-dihydro-1,4-benzoxathiin-7-yl | pyridazin-3-yl | cyclopropyl |
| 4,4-dioxide-8-Me-2,3-dihydro-1,4-benzoxathiin-7-yl | pyridazin-3-yl | CF₃ |
| 4,4-dioxide-8-Me-2,3-dihydro-1,4-benzoxathiin-7-yl | 6-chloropyridazin-3-yl | methyl |
| 4,4-dioxide-8-Me-2,3-dihydro-1,4-benzoxathiin-7-yl | 6-chloropyridazin-3-yl | i-propyl |
| 4,4-dioxide-8-Me-2,3-dihydro-1,4-benzoxathiin-7-yl | 6-chloropyridazin 3-yl | cyclopropyl |
| 4,4-dioxide-8-Me-2,3-dihydro-1,4-benzoxathiin-7-yl | 6-chloropyridazin-3-yl | CF₃ |
| 4,4-dioxide-8-Me-2,3-dihydro-1,4-benzoxathiin-7-yl | pyrazin-2-yl | methyl |
| 4,4-dioxide-8-Me-2,3-dihydro-1,4-benzoxathiin-7-yl | pyrazin-2-yl | i-propyl |
| 4,4-dioxide-8-Me-2,3-dihydro-1,4-benzoxathiin-7-yl | pyrazin-2-yl | cyclopropyl |
| 4,4-dioxide-8-Me-2,3-dihydro-1,4-benzoxathiin-7-yl | pyrazin-2-yl | CF₃ |
| 4,4-dioxide-8-Me-2,3-dihydro-1,4-benzoxathiin-7-yl | triazin-2-yl | methyl |
| 4,4-dioxide-8-Me-2,3-dihydro-1,4-benzoxathiin-7-yl | triazin-2-yl | i-propyl |
| 4,4-dioxide-8-Me-2,3-dihydro-1,4-benzoxathiin-7-yl | triazin-2-yl | cyclopropyl |
| 4,4-dioxide-8-Me-2,3-dihydro-1,4-benzoxathiin-7-yl | tiazin-2-yl | CF₃ |
| 4,4-dioxide-8-Me-2,3-dihydro-1,4-benzoxathiin-7-yl | quinolin-2-yl | methyl |
| 4,4-dioxide-8-Me-2,3-dihydro-1,4-benzoxathiin-7-yl | quinolin-2-yl | i-propyl |
| 4,4-dioxide-8-Me-2,3-dihydro-1,4-benzoxathiin-7-yl | quinolin-2-yl | cyclopropyl |
| 4,4-dioxide-8-Me-2,3-dihydro-1,4-benzoxathiin-7-yl | quinolin-2-yl | CF₃ |
| 4,4-dioxide-8-Me-2,3-dihydro-1,4-benzoxathiin-7-yl | 4,4,6-trimethyl-5,6-dihydro-1,3(4H)-oxazin-2-yl | H |
| 4,4-dioxide-8-Me-2,3-dihydro-1,4-benzoxathiin-7-yl | 4,4,6-trimethyl-5,6-dihydro-1,3(4H)-oxazin-2-yl | methyl |
| 4,4-dioxide-8-Me-2,3-dihydro-1,4-benzoxathiin-7-yl | 4,4,6-trimethyl-5,6-dihydro-1,3(4H)-oxazin-2-yl | i-propyl |
| 4,4-dioxide-8-Me-2,3-dihydro-1,4-benzoxathiin-7-yl | 4,4,6-trimethyl-5,6-dihydro-1,3(4H)-oxazin-2-yl | cyclopropyl |
| 4,4-dioxide-8-Me-2,3-dihydro-1,4-benzoxathiin-7-yl | 4,4,6-trimethyl-5,6-dihydro-1,3(4H)-oxazin-2-yl | CF₃ |
| 4,4-dioxide-8-Me-2,3-dihydro-1,4-benzoxathiin-7-yl | 2-oxazolidinon-3-yl | H |
| 4,4-dioxide-8-Me-2,3-dihydro-1,4-benzoxathiin-7-yl | 2-oxazolidinon-3-yl | methyl |
| 4,4-dioxide-8-Me-2,3-dihydro-1,4-benzoxathiin-7-yl | 2-oxazolidinon-3-yl | i-propyl |
| 4,4-dioxide-8-Me-2,3-dihydro-1,4-benzoxathiin-7-yl | 2-oxazolidinon-3-yl | cyclopropyl |
| 4,4-dioxide-8-Me-2,3-dihydro-1,4-benzoxathiin-7-yl | 2-oxazolidinon-3-yl | CF₃ |
| 4,4-dioxide-8-Me-2,3-dihydro-1,4-benzoxathiin-7-yl | 2-pyrrolidinon-1-yl | methyl |
| 4,4-dioxide-8-Me-2,3-dihydro-1,4-benzoxathiin-7-yl | 2-pyrrolidinon-1-yl | i-propyl |
| 4,4-dioxide-8-Me-2,3-dihydro-1,4-benzoxathiin-7-yl | 2-pyrrolidinon-1-yl | cyclopropyl |
| 4,4-dioxide-8-Me-2,3-dihydro-1,4-benzoxathiin-7-yl | 2-pyrrolidinon-1-yl | CF₃ |
| 4,4-dioxide-8-Mo-2,3-dihydro-l,4-benzoxathiin-7-yl | 3-methylisoxazol-5-yl | methyl |
| 4,4-dioxide-8-Me-2,3-dihydro-1,4-benzoxathiin-7-yl | 3-methylisoxazol-5-yl | i-propyl |
| 4,4-dioxide-8-Me-2,3-dihydro-1,4-benzoxathiin-7-yl | 3-methylisoxazol-5-yl | cyclopropyl |
| 4,4-dioxide-8-Me-2,3-dihydro-1,4-benzoxathiin-7-yl | 3-methylisoxazol-5-yl | CF₃ |
| 2-Cl-4-SO₂MePh | 2-trifluoromethyl-1,3,4-thiadiazol-5-yl | cyclopropyl |
| 2-Cl-4-SO₂MePh | 1,1-dioxido-3-oxo-1,2-benzisothiazol-2(3H)-yl | cyclopropyl |
| 4-Cl-Ph | 2-t-butyl-1,3,4-oxadiazol-5-yl | CF3 |
| 2-Me-6-CF₃Pyridin-3-yl | 2-methyltetrazol-5-yl | cyclopropyl |
| 2-[(2-methoxyethoxy)methyl]-6-CF₃Pyridin-3-yl | 2-methyltetrazol-5-yl | cyclopropyl |
| 2-Cl-4-SO₂MePh | 2,5-dioxopyrrolidin-1-yl | cyclopropyl |
| 2-Cl-4-SO₂MePh | 2-oxopyndin-1(2B)-yl | cyclopropyl |
| 2-Cl-4-SO₂MePh | 2-oxoquinolin-1(2H)-yl | cyclopropyl |
| 2-Cl-4-SO₂MePh | 1,2-benzisoxazol-3-yl | cyclopropyl |
| 2-Cl-4-SO₂MePh | 2-oxo-1,3-benzoxazol-3(2H)-yl | cyclopropyl |
| 2-Cl-4-SO₂MePh | 3-oxo-2,3-dihydro-4H-1,4-benzoxazin-4-yl | cyclopropyl |
| 2-Cl-4-SO₂MePh | 2-oxopyrimidin-1(2H)-yl | cyclopropyl |
| 2-Cl-4-SO₂MePh | 1H-1,2,3-benzotriazol-1-yl | cyclopropyl |
| 2-NO₂4-SO₂MePh | 2,5-dioxopyrrolidin-1-yl | cyclopropyl |
| 2-NO₂4-SO₂MePh | 2-oxopyridin-1(2H)-yl | cyclopropyl |
| 2-NO₂-4-SO₂MePh | 2-oxoquinolin-1(2H)-yl | cyclopropyl |
| 2-NO₂-4-SO₂MePh | 1,2-benzisoxazol-3-yl | cyclopropyl |
| 2-NO₂-4-SO₂MePh | 2-oxo-1,3-benzoxazol-3(2H)-yl | cyclopropyl |
| 2-NO₂4-SO₂MePh | 3-oxo-2,3-dihydro-4H-1,4-benzoxazin-4-yl | cyclopropyl |
| 2-NO₂4-SO₂MePh | 2-oxopyrimidin-1(2H)-yl | cyclopropyl |
| 2-NO₂4-SO₂MePh | 1H-1,2,3-benzotriazol-1-yl | cyclopropyl |

The compounds having general formula (I') can be prepared by the reaction of a carbonyl compound having general formula (II) with a compound having general formula (III) according to reaction scheme 1.

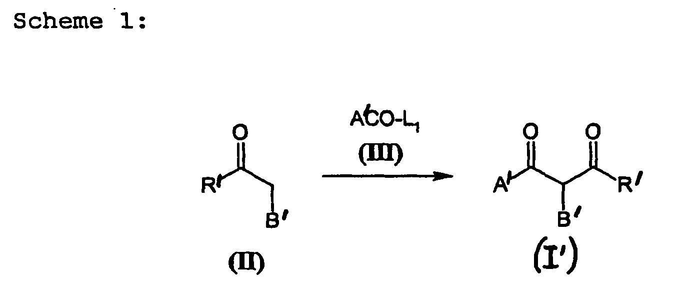

In the general formulae indicated in this reaction scheme:
- A', B' and R' have the meanings previously defined;
- L₁ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or an A' group.

The reaction between the compounds having general formula (II) and the compounds having general formula (III) is preferably carried out in the presence of an inert organic solvent and in the presence of an organic or inorganic base, at a temperature ranging from -80°C to the boiling point of the reaction mixture. The reaction can also be carried out in two distinct phases. In the latter case, in the first phase, the compounds having general formula (II) are reacted with a base. The intermediate obtained is reacted, in the subsequent phase, with an acylating compound.

Examples of solvents which can be used for the above reaction comprise aromatic hydrocarbons (benzene, toluene, xylene, chlorobenzene, etc.), ethers (diethyl ether, diisopropyl ether, dimethoxyethane, dioxane, tetrahydrofuran, etc.), aprotic dipolar solvents (dimethylformamide, dimethylacetamide, hexamethylphosphoramide, N-methylpyrrolidone, etc.).

Inorganic bases which can be used for the purpose are, for example, sodium and potassium hydrides, hydroxides and carbonates, sodium amide.

Organic bases which can be used for the purpose are, for example, sodium, potassium and magnesium alcoholates, phenyllithium, butyllithium, lithium diisopropylamide, triethylamine, pyridine, 4-N,N-dimethylaminopyridine, N,N-dimethylaniline, N-methyl piperidine, lutidine, diazabicyclooctane (DABCO), diazabicyclononene (DBN), diazabicycloundecene (DBU).

The compounds having general formula (I') can also be prepared by the reaction of a carbonyl compound having general formula (IV) with a compound having general formula (V) according to reaction scheme 2.

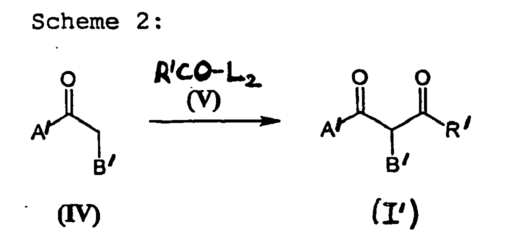

In the general formulae indicated in this reaction scheme:
- A', B' and R' have the meanings previously defined;
- L₂ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or an R' group.

The reaction between the compounds having general formula (IV) and the compounds having general formula (V) is preferably carried out in the presence of an inert organic solvent and in the presence of an organic or preferably inorganic base, at a temperature ranging from -80°C to the boiling point of the reaction mixture. The reaction can also be carried out in two distinct phases. In the latter case, in the first phase, the compounds having general formula (IV) are reacted with a base. The intermediate obtained is reacted, in the subsequent phase, with an acylating compound.

Examples of solvents which can be used for the above reaction comprise aromatic hydrocarbons (benzene, toluene, xylene, chlorobenzene, etc.), ethers (diethyl ether, diisopropyl ether, dimethoxyethane, dioxane, tetrahydrofuran, etc.), aprotic dipolar solvents (dimethylformamide, dimethylacetamide, hexamethylphosphoramide, N-methylpyrrolidone, etc.).

Inorganic bases which can be used for the purpose are, for example, sodium and potassium hydrides, hydroxides and carbonates, sodium amide.

Organic bases which can be used for the purpose are, for example, sodium, potassium and magnesium alcoholates, phenyllithium, butyllithium, lithium diisopropylamide, triethylamine, pyridine, 4-N,N-dimethylaminopyridine, N,N-dimethylaniline, N-methyl piperidine, lutidine, diazabicyclooctane (DABCO), diazabicyclononene (DBN), diazabicycloundecene (DBU).

The compounds having general formula (I') can also be prepared by the reaction of a 1,3-dicarbonyl compound having general formula (VI) with a compound having general formula (VII) according to reaction scheme 3.

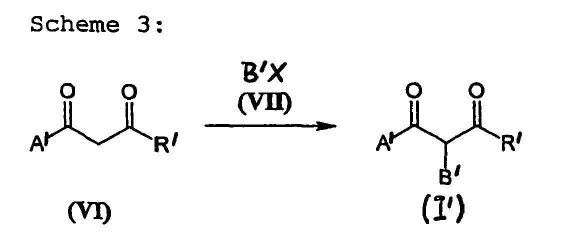

In the general formulae indicated in this reaction scheme:
- A', B' and R' have the meanings previously defined;
- X represents a halo.gen atom, an R_{L2}SO₂O- group, wherein R_{L2} represents a C₁-C₄ alkyl or haloalkyl group, a phenyl group optionally substituted by C₁-C₄ alkyl groups, or it represents an R_{L3}SO₂- group, wherein R_{L3} represents a C₁-C₉ alkyl or haloalkyl group.

The reaction between the compounds having general formula (VI) and the compounds having general formula (VII) is preferably carried out in the presence of one or more inert organic solvents and in the presence of an organic or inorganic base, at a temperature ranging from -80°C to the boiling point of the reaction mixture.

Organic solvents which can be used for the purpose are, for example, aromatic hydrocarbons (benzene, toluene, xylene, chlorobenzene, etc.), ethers (diethyl ether, diisopropyl ether, dimethoxyethane, dioxane, tetrahydrofuran, etc.), alcohols and glycols (methanol, ethanol, methyl cellosolve, ethylene glycol, etc.), ketones (acetone, methyl ethyl ketone, methyl propyl ketone, methyl isobutyl ketone, etc.), nitriles (acetonitrile, benzonitrile, etc.), aprotic dipolar solvents (dimethylformamide, dimethylacetamide, hexamethylphosphoramide, dimethylsulfoxide, sulfolane, N-methylpyrrolidone, etc.).

Organic bases which can be used for the purpose are, for example, sodium, potassium and magnesium alcoholates, phenyllithium, butyllithium, lithium diisopropylamide, triethylamine, pyridine, 4-N,N-dimethylaminopyridine, N,N-dimethylaniline, N-methyl piperidine, lutidine, diazabicyclooctane (DABCO), diazabicyclononene (DBN), diazabicycloundecene (DBU).

Inorganic bases which can be used for the purpose are, for example, sodium or potassium hydrides, hydroxides and carbonates, sodium amide.

The reaction can also be carried out using suitable catalysts based on transition metals, such as, for example, Cu and Pd.

Examples of these reactions are described in Chem. Pharm. Bull. (1987), vol. 35, pages 4972-4976 and J. Chem. Soc., Perkin 1 (1976), vol. 6, pages 592-594.

The 1,3-dicarbonyl compounds having general formula (VI) can be prepared by the acylation of ketones according to what is described, for example, in Organic Reaction (1954), vol. 8, pages 59-196, or in Tetrahedron Letters (2002), vol. 43, pages 2945-2948.

The compounds having general formula (II) can be prepared by the reaction of a compound having general formula (VIII) with an acylating compound having general formula (V) according to reaction scheme 4.

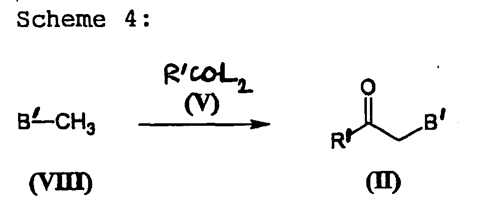

In the general formulae indicated in this reaction scheme:
- B' and R' have the meanings previously defined;
- L₂ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or an R' group.

The reaction between the compounds having general formula (VIII) and the compounds having general formula (V) is preferably carried out in the presence of an inert organic solvent and in the presence of an organic or preferably inorganic base, at a temperature ranging from -80°C to the boiling point of the reaction mixture. The reaction can also be carried out in two distinct phases. In the latter case, in the first phase, the compounds having general formula (VIII) are reacted with a base. The intermediate obtained is reacted, in the subsequent phase, with an acylating compound.

Examples of solvents which can be used for the above reaction comprise aromatic hydrocarbons (benzene, toluene, xylene, chlorobenzene, etc.), ethers (diethyl ether, diisopropyl ether, dimethoxyethane, dioxane, tetrahydrofuran, etc.), aprotic dipolar solvents (dimethylformamide, dimethylacetamide, hexamethylphosphoramide, N-methylpyrrolidone, etc.).

Inorganic bases which can be used for the purpose are, for example, sodium and potassium hydrides, hydroxides and carbonates, sodium amide.

Organic bases which can be used for the purpose are, for example, sodium, potassium and magnesium alcoholates, phenyllithium, butyllithium, lithium diisopropylamide, triethylamine, pyridine, 4-N,N-dimethylaminopyridine, N,N-dimethylaniline, N-methyl piperidine, lutidine, diazabicyclooctane (DABCO), diazabicyclononene (DBN), diazabicycloundecene (DBU).

The compounds having general formula (IV) can be prepared by the reaction of a compound having general formula (VIII) with an acylating compound having general formula (III) according to reaction scheme 5.

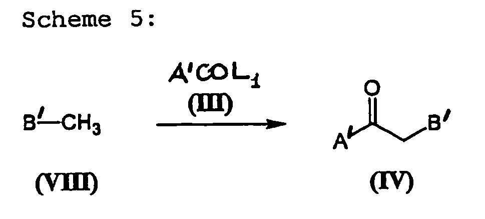

In the general formulae indicated in this reaction scheme:
- B' and A' have the meanings previously defined;
- L₁ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or an A' group.

The reaction between the compounds having general formula (VIII) and the compounds having general formula (III) is preferably carried out in the presence of an inert organic solvent and in the presence of an organic or inorganic base, at a temperature ranging from -80°C to the boiling point of the reaction mixture. The reaction can also be carried out in two distinct phases. In the latter case, in the first phase, the compounds having general formula (VIII) are reacted with a base. The intermediate obtained is reacted, in the subsequent phase, with an acylating compound.

Examples of solvents which can be used for the above reaction comprise aromatic hydrocarbons (benzene, toluene, xylene, chlorobenzene, etc.), ethers (diethyl ether, diisopropyl ether, dimethoxyethane, dioxane, tetrahydrofuran, etc.), aprotic dipolar solvents (dimethylformamide, dimethylacetamide, hexamethylphosphoramide, N--methylpyrrolidone, etc.).

Inorganic bases which can be used for the purpose are, for example, sodium and potassium hydrides, hydroxides and carbonates, sodium amide.

Organic bases which can be used for the purpose are, for example, sodium, potassium and magnesium alcoholates, phenyllithium, butyllithium, lithium diisopropylamide, triethylamine, pyridine, 4-N,N-dimethylaminopyridine, N,N-dimethylaniline, N-methyl piperidine, lutidine, diazabicyclooctane (DABCO), diazabicyclononene (DBN), diazabicycloundecene (DBU).

The compounds having general formula (IV) can also be prepared by the reaction of a compound having general formula (IX) with an acylating compound having general formula (III) in the presence of a base. The reaction provides intermediate compounds having general formula (X) which then undergo a hydrolysis and decarboxylation process according to reaction scheme 6.

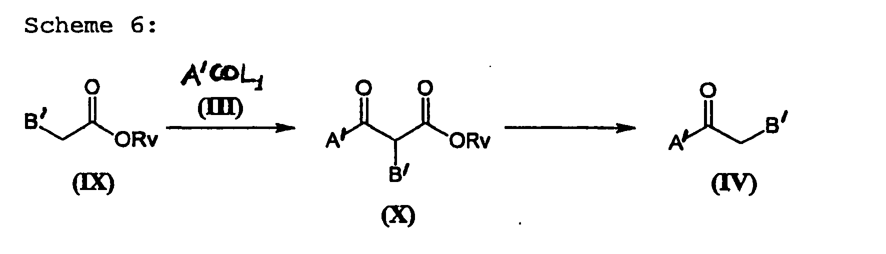

In the general formulae indicated in this reaction scheme:
- B' and A' have the meanings previously defined;
- L₁ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or one of the A' groups.
- Rv represents a C₁-C₅ alkyl or haloalkyl group, an arylalkyl or aryl group.

The compounds having general formula (II) can also be prepared by the reaction of a compound having general formula (IX) with an acylating compound having general formula (V) in the presence of a base. The reaction provides intermediate compounds having general formula (XI) which then undergo a hydrolysis and decarboxylation process according to reaction scheme 7.

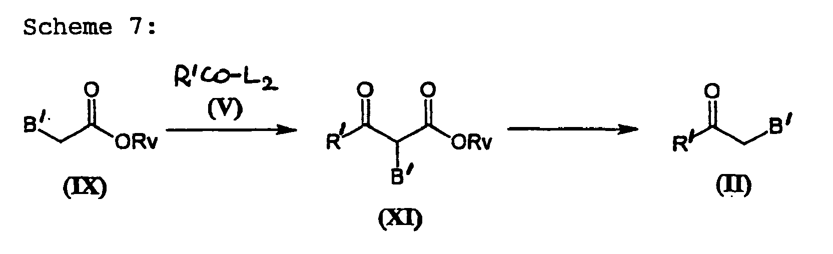

In the general formulae indicated in this reaction scheme:
- B' and R' have the meanings previously defined;
- L₂ represents a suitable leaving group such as, for example, a halogen atom, a CN group, an imidazol-1-yl group, an R_{L}O- group wherein R_{L} represents a C₁-C₄ alkyl group or a phenyl group optionally substituted, or it represents an R_{L1}COO- group wherein R_{L1} represents a hydrogen atom, a C₁-C₄ alkyl or haloalkyl group, a phenyl group optionally substituted or one of the R' groups.
- Rv represents a C₁-C₅ alkyl or haloalkyl group, an arylalkyl or aryl group.

The reactions indicated in reaction schemes 6 and 7 can be carried out, for example, according to the methods described in J. Am. Chem. Soc. (1950), vol. 72, pages 1352-1356 and in J. Am. Chem. Soc. (1987), vol . 109, pages 4717-4718.

The compounds having general formula (II) wherein R' has the meanings previously defined and B' represents a 1,2,4-oxadiazol-5-yl, compounds (IIa), can be prepared, for example, starting from compounds having general formula (XII) by reaction with an amidoxime having general formula (XIII) according to reaction scheme 8.

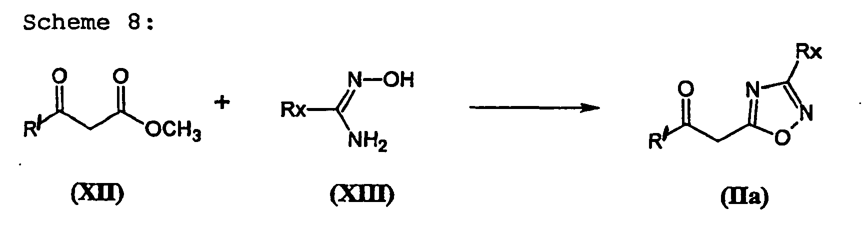

The above reaction can be carried out according to the method described for example in Bull. Soc. Chim. Belges (1949), vol. 58, pages 58-65.

The compounds having general formula (II) wherein R' has the meanings previously defined and B' represents tetrazol-5-yl (D' = tetrazole, Rₓ = H), compounds (IIb), can be prepared, for example, starting from compounds having general formula (XIV) by transforming the cyano group into tetrazole, for example by heating with trimethylsilylazide, in toluene, catalyzed by dibutyltin oxide, according to what is described in J. Org. Chem. (1933), vol. 58, pages 4139-4141, or by heating with sodium azide in water with the catalysis of ZnBr₂, as described in J. Org. Chem. (2001), vol. 66, pages 7945-7950.

The above transformation is indicated in reaction scheme 9.

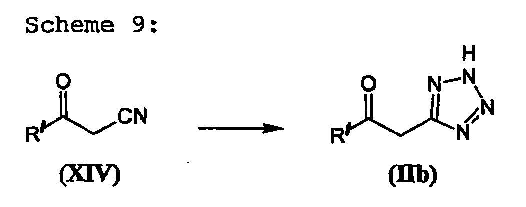

The intermediates having general formulae (III), (V), (VII), (VIII), (IX), (XII), (XIII) and (XIV), when not already known as such, can be easily prepared according to methods known in organic chemistry practice.

In some cases, the compounds having general formula (I') can be obtained in the form of two or more optic or geometric or position isomers. Compounds having general formula (I') which are isomerically pure, and also mixtures of these, possibly obtained during the preparation of the compounds having general formula (I') or deriving from an incomplete separation of the isomers themselves, in any proportion, are therefore considered as being included within the scope of the present invention.

As already mentioned, the compounds having general formula (I') have a high herbicidal activity which makes them suitable for use in the agrarian field in the defense of useful crops from weeds.

In particular, the compounds, object of the present invention, are effective in the control, in both pre-emergence and post-emergence, of numerous monocotyledon and dicotyledon weeds. At the same time, these compounds show compatibility or the absence of toxic effects with respect to useful crops in pre- and/or post-emergence treatments.

The compounds of the present invention can act as total or selective herbicides also in relation to the quantity of active principle used.

Examples of weeds which can be effectively controlled using the compounds having general formula (I') are: Abutilon theofrasti, Alisma plantago, Amaranthus spp., Amni maius, Capsella bursa pastoris, Chenopodium album, Convolvulus sepium, Galium aparine, Geranium dissectum, Ipomea spp., Matricaria spp., Papaver rhoaes, Phaseolus aureus, Polygonum persicaria, Portulaca oleracea, Sida spinosa, Sinapsis arvensis, Solanum nigrum, Stellaria media, Veronica spp., Viola spp., Xanthium spp., Alopercurus myosuroides, Avena fatua, Cyperus spp., Digitaria sanguinalis, Echinocloa spp., Heleocaris avicularis, Heteranthera spp., Panicum spp., Poa spp., Scirpus spp., Sorghum spp., etc.

With the doses of use suitable for agrarian applications, many of the above compounds showed no toxic effects towards one or more important agrarian crops such as corn (Zea mays), wheat (Triticum sp.), barley (Hordeum vulgare), soybean (Glycine max), rice (Oryza sativa).

A further object of the present invention relates to a method for controlling weeds in cultivated areas by the application of the compounds having general formula (I').

The quantity of compound to be applied for obtaining the desired effect can vary in relation to various factors such as, for example, the compound used, the crop to be preserved, the weed to be fought, the degree of infestation, the climatic conditions, the characteristics of the soil, the application method, etc.

Doses of compound ranging from 1 g to 4,000 g per hectare generally provide a sufficient control.

For use in agriculture, it is often advantageous to adopt compositions with a herbicidal activity containing, as active substance, one or more compounds having general formula (I'), optionally also as a mixture of tautomers and/or optic or geometric or position isomers.

Compositions can be used in the form of dry powders, wettable powders, emulsifiable concentrates, micro-emulsions, pastes, granulates, solutions, suspensions, etc.: the selection of the type of composition depends on the specific use.

The compositions are prepared according to known methods, for example by diluting or dissolving the active substance by means of a solvent medium and/or solid diluent, possibly in the presence of surface-active agents.

Kaolin, alumina, silica, talc, bentonite, chalk, quartz, dolomite, attapulgite, montmorillonite, diatomaceous earth, cellulose, starch, etc.., can be used as inert solid diluents, or carriers.

Inert liquid diluents which can be used, are water or organic solvents such as aromatic hydrocarbons (xylols, blends of alkyl benzenes, etc..), aliphatic hydrocarbons (hexane, cyclohexane, etc..) halogenated aromatic hydrocarbons (chlorobenzene, etc..), alcohols (methanol, propanol, butanol, octanol, etc..), esters (isobutyl acetate, etc..), ketones (acetone, cyclohexanone, acetophenone, isophorone, ethylamylketone etc..), or vegetable and mineral oils or mixtures thereof, etc..

Surfactants which can be used are wetting and emulsifying agents, of the non-ionic type (polyethoxylated alkyl phenols, polyethoxylated fatty alcohols, etc..), of the anionic type (alkylbenzenesulphonates, alkylsulphonates, etc..), of the cationic type (alkyl ammonium quaternary salts, etc..).

Dispersing agents can also be added (for example lignin and its salts, cellulose derivatives, alginates, etc..), stabilizers (for example antioxidants, UV absorbers, etc..).

In order to enlarge the action range of the above compositions, it is possible to add active ingredients, such as, for example, other herbicides, fungicides, insecticides, acaricides, fertilizers, etc..

Examples of other herbicides which can be added to the compositions containing one or more compounds having general formula (I), are the following: Acetochlor, acifluorfen, aclonifen, AKH-7088, alachlor, alloxydim, ametryn, amicarbazone, amidosulfuron, amitrole, anilofos, asulam, atrazine, azafenidin, azimsulfuron, aziprotryne, BAS 670 H, BAY MKH 6561, beflubutamid, benazolin, benfluralin, benfuresate, bensulfuron, bensulide, bentazone, benzfendizone, benzobicyclon, benzofenap, benzthiazuron, bifenox, bilanafos, bispyribac-sodium, bromacil, bromobutide, bromofenoxim, bromoxynil, butachlor, butafenacil, butamifos, butenachlor, butralin, butroxydim, butylate, cafenstrole, carbetamide, carfentrazone-ethyl, chlomethoxyfen, chloramben, chlorbromuron, chlorbufam, chlorflurenol, chloridazon, chlorimuron, chlornitrofen, chlorotoluron, chloroxuron, chlorpropham, chlorsulfuron, chlorthal, chlorthiamid, cinidon ethyl, cinmethylin, cinosulfuron, clethodim, clodinafop, clomazone, clomeprop, clopyralid, cloransulam-methyl, cumyluron (JC-940), cyanazine, cycloate, cyclosulfamuron, cycloxydim, cyhalofopbutyl, 2,4-D, 2,4-DB, daimuron, dalapon, desmedipham, desmetryn, dicamba, dichlobenil, dichlorprop, dichlorprop-P, diclofop, diclosulam, diethatyl, difenoxuron, difenzoquat, diflufenican, diflufenzopyr, dimefuron, dimepiperate, dimethachlor, dimethametryn, dimethenamid, dinitramine, dinosseb, dinoseb acetate, dinoterb, diphenamid, dipropetryn, diquat, dithiopyr, 1-diuron, eglinazine, endothal, EPTC, espropcarb, ethalfluralin, ethametsulfuron-methyl, ethidimuron, ethiozin (SMY 1500), ethofumesate, ethoxyfen-ethyl (HC-252), ethoxysulfuron, etobenzanid (HW 52), fenoxaprop, fenoxaprop-P, fentrazamide, fenuron, flamprop, flamprop-M, flazasulfuron, florasulam, fluazifop, fluazifop-P, fluazolate (JV 485), flucarbazone-sodium, fluchloralin, flufenacet, flufenpyr ethyl, flumetsulam, flumiclorac-pentyl, flumioxazin, flumipropin, fluometuron, fluoroglycofen, fluoronitrofen, flupoxam, fluproanate, flupyrsulfuron, flurenol, fluridone, flurochloridone, fluroxypyr, flurtamone, fluthiacet-methyl, fomesafen, foramsulfuron, fosamine, furyloxyfen, glufosinate, glyphosate, halosulfuron-methyl, haloxyfop, haloxyfop-P-methyl, hexazinone, imazamethabenz, imazamox, imazapic, imazapyr, imazaquin, imazethapyr, imazosulfuron, indanofan, iodosulfuron, ioxynil, isopropalin, isoproturon, isouron, isoxaben, isoxachlortole, isoxaflutole, isoxapyrifop, KPP-421, lactofen, lenacil, linuron, LS830556, MCPA, MCPA-thioethyl, MCPB, mecoprop, mecoprop-P, mefenacet, mesosulfuron, mesotrione, metamitron, metazachlor, methabenzthiazuron, methazole, methoprotryne, methyldymron, metobenzuron, metobromuron, metolachlor, S-metolachlor, metosulam, metoxuron, metribuzin, metsulfuron, molinate, monalide, monolinuron, naproanilide, napropamide, naptalam, NC-330, neburon, nicosulfuron, nipyraclofen, norflurazon, orbencarb, oryzalin, oxadiargyl, oxadiazon, oxasulfuron, oxaziclomefone, oxyfluorfen, paraquat, pebulate, pendimethalin, penoxsulam, pentanochlor, pentoxazone, pethoxamid,, phenmedipham, picloram, picolinafen, piperophos, pretilachlor, primisulfuron, prodiamine, profluazol, proglinazine, prometon, prometryne, propachlor, propanil, propaquizafop, propazine, propham, propisochlor, propyzamide, prosulfocarb, prosulfuron, pyraclonil, pyraflufen-ethyl, pyrazogyl (HAS-961), pyrazolynate, pyrazosulfuron, pyrazoxyfen, pyribenzoxim, pyributicarb, pyridafol, pyridate, pyriftalid, pyriminobac-methyl, pyrithiobac-sodium, quinclorac, quinmerac, quizalofop, quizalofop-P, rimsulfuron, sethoxydim, siduron, simazine, simetryn, sulcotrione, sulfentrazone, sulfometuron-methyl, sulfosulfuron, 2,3,6-TBA, TCA-sodium, tebutam, tebuthiuron, tepraloxydim, terbacil, terbumeton, terbuthyl-azine, terbutryn, thenylchlor, thiazafluron, thiazopyr, thidiazimin, thifensulfuron-methyl, thiobencarb, tiocarbazil, tioclorim, tralkoxydim, tri-allate, triasulfuron, triaziflam, tribenuron, triclopyr, trietazine, trifloxysulfuron, trifluralin, triflusulfuron-methyl, tritosulfuron, UBI-C4874, vernolate.

The concentration of active substance in the above compositions can vary within a wide range, depending on the active compound, the applications to which they are destined, the environmental conditions and the type of formulation adopted. In general, the concentration of active substance preferably ranges from 1 to 90%.

Some examples are now provided for illustrative and non-limiting purposes of the present invention.

### EXAMPLE 1

### Synthesis of 3,3-dimethyl-1-(tatrazol-5-yl)butane-2-one.

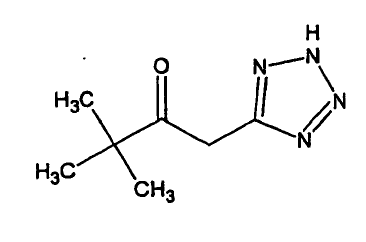

NaN₃ (1.71 g) and ZnBr₂ (5.40 g) are added to a suspension of 4,4-dimethyl-3-oxopentanenitrile (3.00 g) in 50 ml of water and 4 ml of isopropylic alcohol and the resulting mixture is stirred at 90°C for 12 hours.

After completion of the reaction, 15 ml of 10% HCl are added, then the mixture is extracted two times with ethyl acetate; the organic phase is then evaporated under reduced pressure. The residue is stirred with 100 ml of 10% NaOH for 20 minutes, then cooled with an ice bath and acidified with concentrated HCl: the white precipitate is extracted two times with ethyl acetate, which is then dried with Na₂SO₄ and evaporated.

The resulting solid is purified by washing with dichloromethane to obtain 2.75 g of pure product (yield: 68%) .

### EXAMPLE 2

### Synthesis of 3,3-dimethyl-1-(2-methyl-2H-tetrazol-5-yl)butane-2-one and 3,3-dimethyl-1-(1-methyl-1H-tetrazol-5-yl)butane-2-one.

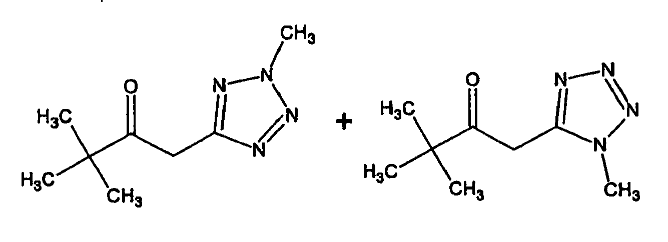

K₂CO₃ (1.40 g) and CH₃I (1.32 g) are added to a solution of 3,3-dimethyl-1-(tetrazol-5-yl)butan-2-one (1.42 g) in 35 ml of acetone under an inert atmosphere,; the mixture is stirred at room temperature for 20 hours.

The solvent is then evaporated, the residue is taken up with water and extracted two times with ethyl acetate, which is then washed with water, dried with Na₂SO₄ and evaporated.

The raw product is purified by flash chromatography, isolating the two isomers 3,3-dimethyl-1-(2-methyl-2*H-*tetrazol-5-yl)butan-2-one (0.60 g, yield: 39%) and 3,3-dimethyl-1-(1-methyl-1*H*-tetrazol-5-yl)butan-2-one (0.64 g, yield: 42%). The structure of each isomer was assigned according to the NMR spectra.
**¹H-NMR (CDCl₃) :**
- (2-methyl isomer) - δ 1.24 (s, 9H, t-butyl), 4.12 (s, 2H, CH₂), 4.32 (s, 3H, N-CH₃)
- (1-methyl isomer) - δ 1.19 (s, 9H, t-butyl), 3.90 (s, 3H, N-CH₃), 4.17 (s, 2H, CH₂)

### EXAMPLE 3

### Synthesis of 1-[2-chloro-4-(methylsulphonyl)phenyl]-4,4-dimethyl-2-(1-methyl-1H-tetrazol-5-yl)pentane-1,3-dione (Compound N° 1).

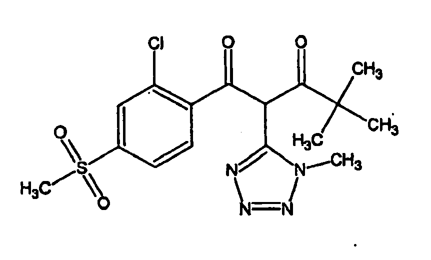

Under an inert atmosphere, Mg(OEt)₂ (0.279 g) is added to a solution of 3,3-dimethyl-1-(1-methyl-1*H-*tetrazol-5-yl)butan-2-one (0.64 g) in 16 ml of dry tetrahydrofuran; the stirred mixture is refluxed for 3 hours, then completely evaporated under reduced pressure.

The residue is taken up with 16 ml of dry tetrahydrofuran, under an inert atmosphere, then a solution of 2-chloro-4-(methylsulphonyl)benzoyl chloride (1.04 g) in dry tetrahydrofuran is added; the stirred mixture is refluxed for 3 more hours.

After completion of the reaction, the solvent is evaporated and the residue is taken up with water and ethyl acetate; after acidification with 10% HCl the organic phase is recovered and extracted three times with aqueous NaHCO₃ saturated solution. The combined basic aqueous phases are acidified and extracted three times with ethyl acetate, which is then dried with Na₂SO₄ and evaporated, obtaining an off-white solid.

The raw product is purified by filtration over silica gel eluting with dichloromethane/methanol 8:2, then by washing the obtained solid with acetone, thus obtaining 0.60 g of product as a white solid (yield: 45%, m.p. 195-200°C).
**¹H-NMR (CDCl₃) :** δ 1.07 (s, 9H, t-butyl), 3.01 (s, 3H, SO₂CH₃), 3.76 (s, 3H, N-CH₃), 7.30-7.94 (m, 3H, arom. H's)

### EXAMPLE 4

### Synthesis of 1-(2,4-dichlorophenyl)-4,4-dimethyl-2-(2-methyl-2H-tetrazol-5-yl)pentane-1,3-dione (Compound N° 2).

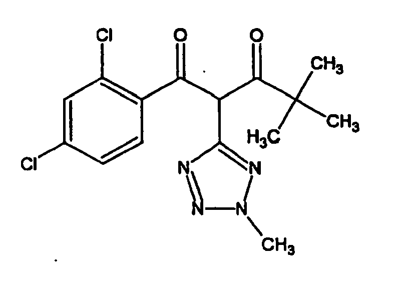

Under an inert atmosphere, Mg(OEt)₂ (0.257 g) is added to a solution of starting 3,3-dimethyl-1-(2-methyl-2*H-*tetrazol-5-yl)butan-2-one (0.59 g) in 16 ml of dry tetrahydrofuran; the stirred mixture is refluxed for 3 hours, then completely evaporated under reduced pressure.

The residue is taken up with 16 ml of dry tetrahydrofuran, under an inert atmosphere, then a solution of 2,4-dichlorobenzoyl chloride (0.746 g) in dry tetrahydrofuran is added; the stirred mixture is refluxed for 3 more hours.

After completion of the reaction, the solvent is evaporated and the residue is taken up with water and extracted with ethyl acetate; the organic phase is washed with diluted HCl, with brine, then dried with Na₂SO₄ and evaporated.

The raw product is purified by flash chromatography to obtain 0.49 g of product (yield: 43%).
**¹H-NMR (CDCl₃) :** (mixture of two keto-enolic tautomers) δ 1.05, 1.10 (2s, 9H, t-butyl), 4.19, 4.33 (2s, 3H, N-CH₃), 6.62 (s, 1H, CO -CH-CO), 7.04-7.50 (m, 3H, arom. H's)

### EXAMPLE 5

### Synthesis of 2-(5-tert-butyl-1,3,4-oxadiazol-2-yl)-1-(4-chlorophenyl)-3-(cyclopropyl)propane-1,3-dione (Compound N° 3).

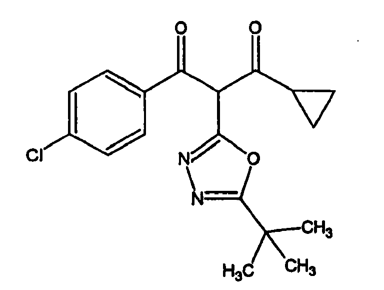

Under an inert atmosphere, Mg(OEt)₂ (0.209 g) is added to a solution of 2-(5-*tert*-butyl-1,3,4-oxadiazol-2-yl)-1-(4-chlorophenyl)ethanone (0.50 g) in 10 ml of dry tetrahydrofuran; the stirred mixture is refluxed for 3 hours, then completely evaporated under reduced pressure.

The residue is taken up with 10 ml of dry tetrahydrofuran, under an invert atmosphere, then cyclopropanecarbonylchloride (0.208 g) is added; the stirred mixture is refluxed for 3 more hours.

After completion of then reaction, the solvent is evaporated and the residue is taken up with water and extracted with ethyls acetate; the organic phase is washed with diluted HCl, with brine, then dried with Na₂SO₄ and evaporated.

The raw product is purified by flash chromatography to obtain 0.28 g of pure product (yield: 44%).
**¹H-NMR (CDCl₃) :** δ 1.01-1.43 (m, 4H, CH₂-CH₂), 1.20 (s, 9H, t-butyl), 2.12-2.22 (m, 1H, CH), 7.26 (s, 4H, arom. H's)

### EXAMPLE 6

### Synthesis of 1-(4-chlorophenyl)-2-(2H-tetrazol-5-yl) ethanone.

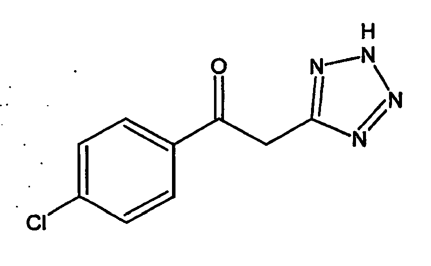

NaN₃ (1.19 g) and ZnBr (3.76 g) are added to a suspension of 3-(4-chlorophenyl)-3-oxopropanenitrile (3.00 g) in 30 ml of H₂O and 4 ml of isopropylic acid and the resulting mixture is stirred at 90°C for 12 hours.

After completion of the reaction, 15 ml of 10% HCl are added, then the mixture is extracted two times with ethyl acetate; the combined organic phases are then evaporated under reduced pressure. The residue is stirred with 100 ml of 10% NaOH for 6 hours, then the mixture is cooled with an ice bath and acidified with concentrated HCl : the white precipitate is extracted two times with ethyl acetate, which is then dried with Na₂SO₄ and evaporated.

The resulting solid is purified by digestion in ethyl acetate to obtain 1.76 g of pure product (yield: 47%) **¹H-NMR (acetone-*d*₆) :** δ 4.98 (s, 2H, CH₂), 7.60-3.20 (m, 4H, arom. H's)

### EXAMPLE 7

### Synthesis of 1-(4-chlorophenyl)-2-(2-methyl-2H-tetrazol-5-yl)ethanone and 1-(4-chlorophenyl)-2-(1-methyl-1H-tetrazol-5-yl)ethanone.

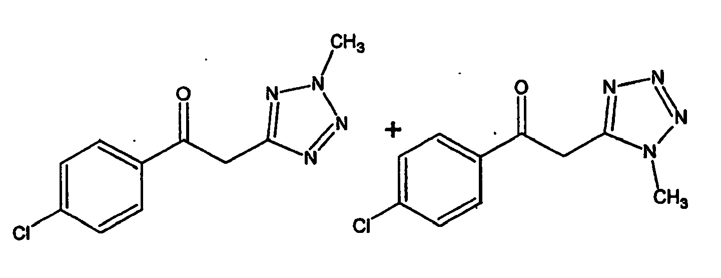

K₂CO₃ (0.47 g) and CH₃I (0.32 g) are added to a solution of 1-(4-chlorophenyl)-2-(2*H*-tetrazol-5-yl)ethanone (0.50 g) in 15 ml of acetone, under an inert atmosphere; the mixture is stirred at room temperature for 20 hours.

The solvent is then evaporated, the residue is taken up with water and extracted two times with ethyl acetate, which is then washed with water, dried with Na₂SO₄ and evaporated, thus obtaining a solid raw product (0.56 g) containing the two isomers ( 1-(4-chlorophenyl)-2-(2-methyl-2*H*-tetrazol-5-yl)ethanone and 1-(4-chlorophenyl)-2-(1-methyl-1*H*-tetrazol-5-yl)ethanone), which is used for the following reaction.

### EXAMPLE 8

### Synthesis of 1-(4-chlorophenyl)-3-cyclopropyl-2-(2-methyl-2H-tetrazol-5-yl)propane-1,3-dione (Compound N° 4) and 1-(4-ahlorophenyl)-3-cyalopropyl-2-(1-methyl-1H-tetrazol-5-yl)propane-1,3-dione (Compound N° 5).

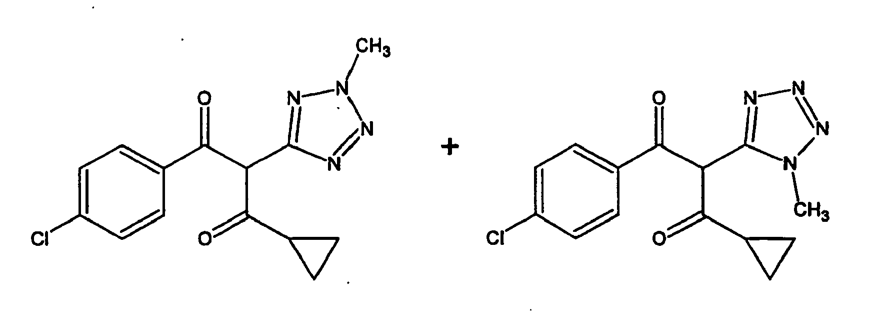

Under an inert atmosphere, Mg(OEt)₂ (0.263 g) is added to a solution of the starting mixture of 1-(4-chlorophenyl)-2-(2-methyl-2*H*-tetrazol-5-yl)ethanone and 1-(4-chlorophenyl)-2-(1-methyl-1*H*-tetrazol-5-yl)ethanone (0.53 g) in 10 ml of dry tetrahydrofuran; the stirred mixture is refluxed for 3 hours, then completely evaporated under reduced pressure.

The residue is taken up with 10 ml of dry tetrahydrofuran, under an inert atmosphere, then cyclopropanecarbonylchloride (0.235 g) is added; the stirred mixture is refluxed for 3 more hours.

After completion of the reaction, the solvent is evaporated and the residue is taken up with water and extracted with ethyl acetate; the organic phase is washed with diluted HCl, with brine, then dried with Na₂SO₄ and evaporated.

The raw product is purified by flash chromatography to obtain 0.26 g of 2-methyl isomer (yield: 37%) and 0.17 g of 1-methyl isomer (yield: 24%).
**¹H-NMR (CDCl₃) :**
- (2-methyl isomer) - δ 0.90-1.67 (m, 5H, ciclopropyl), 4.29 (s, 3H, N-CH₃), 7.18 (s, 4H, arom. H's)
- (1-methyl isomer) - δ 0.9-1.61 (m, 5H, ciclopropyl), 3.49 (s, 3H, N-CH₃), 7.12-7.27 (m, 4H, arom. H's)

### EXAMPLE 9

### Synthesis of 1-ayclopropyl-2-(tetrazol-5-yl)ethanone

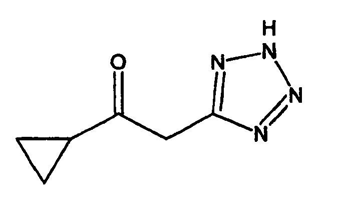

NaN₃ (5.0 g) and ZnBr (14.5 g) are added to a suspension of 3-cyclopropyl-3-oxopropanenitrile (7.0 g) in 130 ml of water and 10 ml of isopropylic alcohol and the resulting mixture is stirred at 100°C for 12 hours.

After completion of the reaction, 60 ml of 10% HCl are added, then the mixture is extracted three times with ethyl acetate; the organic phase is then evaporated under reduced pressure. The residue is stirred with 400 ml of 1% NaOH for 20 hours, cooled with an ice bath and acidified with 10% HCl; the mixture is extracted three times with ethyl acetate, which is then dried with Na₂SO₄ and evaporated.

The resulting solid is purified by washing with CH₂Cl₂ to obtain 3.6 g of pure product (yield: 37%).

### EXAMPLE 10

### Synthesis of 1-cyclopropyl-2-(2-methyl-2H-tetrazol-5-yl)ethanone and 1-cyclopropyl-2-(1-methyl-1H-tetrazol-5-yl)ethanone

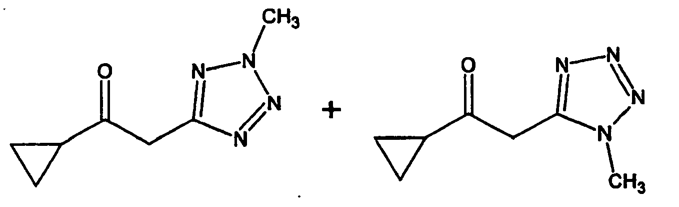

K₂CO₃ (4.85 g) and CH₃I (3.99 g) are added to a solution of 1-cyclopropyl-2-(tetrazol-5-yl)ethanone (3.56 g) in 90 ml of acetone, under an inert atmosphere; the mixture is then stirred at room temperature for 20 hours.

The solvent is then evaporated, the residue is taken up with water/ethyl acetate and the mixture is acidified to pH 1-2 with HCl 10%; the aqueous phase is extracted two more times with ethyl acetate; the combined organic phases are then washed with brine, dried with Na₂SO₄ and evaporated.

The raw product is purified by flash chromatography, isolating the two isomers 2-methyl (1.95 g, yield: 50%) and 1-methyl (1.13 g, yield: 29%).
**¹H-NMR (CDCl₃) :**
- (2-methyl isomer) - δ 0.90-1.16 (m, 4H, CH₂-CH₂), 2.06 (m, 1H, COCH), 4.15 (s, 2H, COCH₂), 4.33 (s, 3H, N-CH₃)
- (1-methyl isomer) - δ 0.98-1.18 (m, 4H, CH₂-CH₂), 2.07 (m, 1H, COCH), 3.96 (s, 2H, COCH₂), 4.25 (s, 3H, N-CH₃)

### EXAMPLE 11

### Synthesis of 1-[2-chloro-4-(methylsulphonyl)phenyl]-3-cyclopropyl-2-(2-methyl-2H-tetrazol-5-yl)propane-1,3-dione (Compound N° 6)

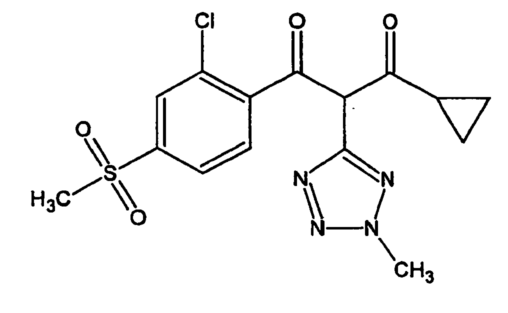

Under an inert atmosphere, Mg(OEt)₂ (0.383 g) is added to a solution of 1-cyclopropyl-2-(2-methyl-2*H-*tetrazol-5-yl)ethanone (0.80 g) in 22 ml of dry tetrahydrofuran; the stirred mixture is refluxed for 3 hours, then completely evaporated under reduced pressure.

The residue is taken up with 15 ml of dry tetarhydrofuran, under an inert atmosphere, then a suspension of 2-chloro-4-(methylsulphonyl)benzoyl chloride (0.96 g) in 20 ml of dry tetrahydrofuran is added ; the stirred mixture is refluxed for 5 more hours.

After completion of the reaction, the solvent is evaporated and the residue is taken up with water and ethyl acetate; after acidification with 10% HCl the organic phase is recovered and extracted three times with aqueous NaHCO₃. The combined basic aqueous phases are acidified and extracted three times with ethyl acetate, which is then dried with Na₂SO₄ and evaporated.

The raw product is purified by washing with warm ethyl acetate, to obtain 0.58 g of product as an orange solid (yield: 40%; m.p.: 220°C).

**¹H-NMR (CDCl₃):** δ 1.02-1.96 (m, 5H, cyclopropyl), 3.03 (s, 3H, SO₂CH₃), 4.21 (s, 3H, N-CH₃), 7.42-7.86 (m, 3H, arom. H's), 17.52 (s, 1H, OH).

### EXAMPLE 12

### Synthesis of 1-[2-chloro-9-(methylsulphonyl)phenyl]-3-clcpropyl-2-(1-methyl-1H-tetrazol-5-yl)propane-1,3-dione (Compound N° 7)

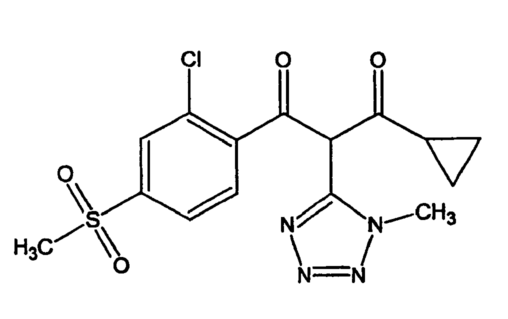

Under an inert atmosphere, Mg(OEt)₂ (0.278 g) is added to a solution of starting 1-cyclopropyl-2-(1-methyl-1*H*-tetrazol5-yl)ethanone (0.58 g) in 15 ml of dry tetrahydrofuran; the stirred mixture is refluxed for 3 hours, then completely evaporated under reduced pressure.

The residue is taken up with 2 ml of dry tetrahydrofuran, under an inert atmosphere, then a suspension of 2-chloro-4(methylsulphonyl)benzoyl chloride (0.97 g) in 16 ml of dry tetrahydrofuran is added; the stirred mixture is refluxed for 5 more hours.

The solvent is then evaporated and the residue is taken up with water and ethyl acetate; after acidification with 10% HCl the organic phase is collected and extracted two times with aqueous NaHCO₃. The combined basic aqueous phases are acidified and extracted three times with ethyl acetate, which is then dried with Na₂SO₄ and evaporated.

The raw product is purified by flash chromatography, to obtain 0.81 g of product as an orange solid (yield: 61%; m.p.: 104°C).

**¹H-NMR (CDCl₃):** δ 1.09-1.42 (m, 5H, cyclopropyl), 3.02 (s, 3H, SO₂CH₃), 3.91 (s, 3H, N-CH₃), 7.47-7.89 (m, 3H, arom. H's), 17.44 (s, 1H, OH).

### EXAMPLE 13

### Synthesis of 1-(4-chloro-2-nitrophenyl)-2-3-cyclopropyl-(1-methyl-1A-tetrazol-5-yl)propane-1,3-dione (Compound N° 8)

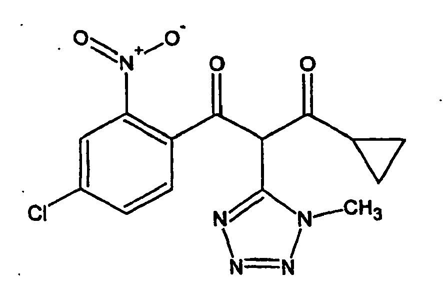

Under an inert atmosphere, Mg(OEt)₂ (0.263 g) is added to a solution of 1-cyclopropyl-2-(1-methyl-1*H-*tetrazol5-yl)ethanone (0.55 g) in 15 ml of dry tetrahydrofuran; the stirred mixture is refluxed for 3 hours, then completely evaporated under reduced pressure.

The residue is taken up with 7 ml of dry tetrahydrofuran, under an inert atmosphere, then a solution of the 4-chloro-2-nitrobenzoyl chloride (0.80 g) in 8 ml of dry tetrahydrofuran is added; the stirred mixture is refluxed for 3 more hours.

The solvent is then evaporated and the residue is taken up with water and ethyl acetate; after acidification with 10% HCl the organic phase is collected and extracted two times with aqueous NaHCO₃. The combined basic aqueous phases are acidified and extracted three times with ethyl acetate, which is then dried with Na₂SO₄ and evaporated.

The raw product is purified by flash chromatography, to obtain 0.72 g of product as an orange solid (yield: 61%; m.p.: 152°C).

**¹H-NMR (CDCl₃) :** δ 1.05-1.52 (m, 5H, cyclopropyl), 3.92 (s, 3H, N-CH₃), 7.39-7.93 (m, 3H, arom. H's), 17.07 (s, 1H, OH) .

### EXAMPLE 14

### Synthesis of 3-cyclopropyl-1-[4-(methylsulphonyl)-2-nitrophenyl]-2-(2-methyl-2H-tetrazol-5-yl)propane-1,3-dione (Compound N° 9)

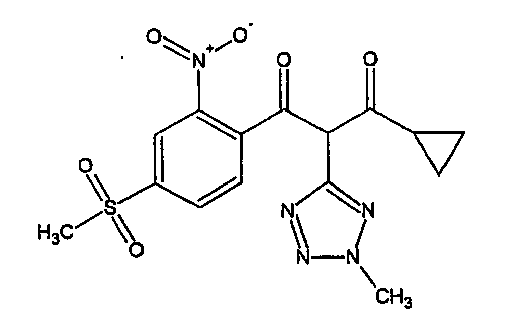

Under an inert atmosphere, Mg(OEt)₂ (0.171 g) is added to a solution of 1-cyclopropyl-2-(2-methyl-2*H-*tetrazol5-yl)ethanone (0.35 g) in 9 ml of dry tetrahydrofuran; the stirred mixture is refluxed for 3 hours, then completely evaporated under reduced pressure.

The residue is taken up with 3 ml of dry tetrahydrofuran, under an inert atmosphere, then a solution of 4-methylsulphonyl-2-nitrobenzoyl chloride (0.61 g) in 6 ml of dry tetrahydrofuran is added; the stirred mixture is refluxed for 3 more hours.

The solvent is then evaporated and the residue is taken up with water and ethyl acetate; after acidification with 10% HCl the organic phase is collected and extracted three times with aqueous NaHCO₃. The combined basic aqueous phases are slowly acidified to pH 5 and extracted with ethyl acetate, which is then washed three times with pH 5 buffered solution until all the benzoic acid is eliminated, dried with Na₂SO₄ and evaporated.

The resulting solid is purified by filtration over silica gel eluting with ethyl acetate to obtain 0.24 g of pure product as a light brown solid (yield: 61%; m.p 186°C, decomposition).

**¹R-NMR (CDCl₃) :** δ 1.08-1.99 (m, 5H, cyclopropyl), 3.09 (s, 3H, SO₂CH₃), 4.17 (s, 3H, N-CH₃), 7.47-8.62 (m, 3H, carom. H's), 17.19 (s, 1H, OH).

### EXAMPLE 15

### Synthesis of 1-cyclopropyl-2-(4-methyl-1,3-thiazol-2-yl)ethanone.

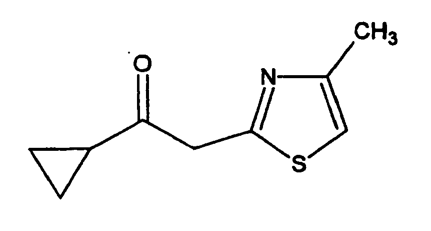

Under an inert atmosphere and in dried glassware, 2,4-dimethyl-1,3-thiazole (3.15 g) is dissolved in 90 ml of anhydrous tetrahydrofuran; 17.4 ml of 1.6 M butyllithium solution in hexanes are then added dropwise: the solution temperature rises to about 40 °C, after the addition, the mixture is stirred for 30 minutes, allowing the temperature to return to about 25°C.

The reaction mixture is then cooled in an ice bath and a solution of ethyl cyclopropanecarboxylate (3.17 g) in 15 ml of anhydrous tetrahydrofuran is quickly added; the ice bath is removed and the resulting solution is stirred at 50°C for 3 hours.

After completion of the reaction, the solvent is removed at reduced pressure and the residue taken up with 5% HCl, which is washed with a little portion of diethyl ether, then slowly neutralized to pH 7-7.5 and extracted three times with diethyl ether.

The combined organic phases are dried with Na₂SO₄ and evaporated, yielding a dark oil, which is purified by flash chromatography to obtain 0.72 g of desired product as an oil (yield: 14%).
**¹H-NMR (CDCl₃) :** δ 0.89-1.24 (m, 4H, CH₂CH₂), 2.06 (m, 1H, CH), 2.43 (s, 3H, CH₃), 4.23 (s, 2H, CH₂), 6.83 (s, 1H, thiazole H)

### EXAMPLE 16

### Synthesis of 1-[2-chloro-4-(methylsulphonyl)phenyl]-3-cyclopropyl-2-(4-methyl-1,3-thiazol-2-yl)propane-1,3-dione (Compound N° 10)

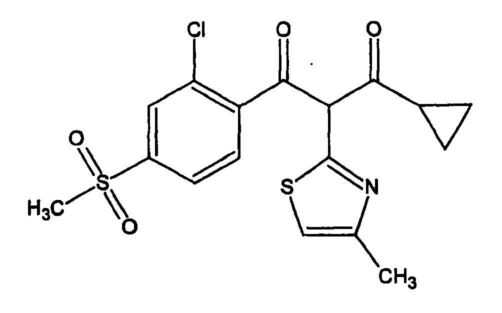

Under an inert atmosphere, Mg(OEt)₂ (0.316 g) is added to a solution of 1-cyclopropyl-2-(4-methyl-1,3-thiazol-2-yl)ethanone (0.72 g) in 18 ml of dry tetrahydrofuran; the stirred mixture is refluxed for 3 hours, then completely evaporated under reduced pressure.

The residue is taken up with 3 ml of dry tetrahydrofuran, under an inert atmosphere, then a suspension of 2-chloro-4(methylsulphonyl)benzoyl chloride (1.11 g) in 15 ml of dry tetrahydrofuran is added ; the stirred mixture is refluxed for 3 more hours.

After completion of the reaction, the solvent is evaporated and the residue is taken up with ethyl acetate and 1% HCl, then the mixture is neutralized with NaHCO₃ and extracted three times with ethyl acetate; the combined organic phases are washed with brine, dried with Na₂SO₄ and evaporated.

The resulting solid is purified by washing with diethyl ether to obtain 1.06 g of pure product as an off-white solid (yield: 67%; m.p.: 199°C).
**¹H-MMR (CDCl₃) :** δ 0.51-1.35 (m, 5H, cyclopropyl), 2.43 (2s, 3H, Ar-CH₃), 3.07 (s, 3H, SO₂CH₃), 6.59 (2s, 1H, thiazole H), 7.58-8.02 (m, 3H, arom. H's), 14.78 (s, 1H, OH) .
**MS-DCI:** m/z 398 (M+1).

### EXAMPLE 17

### Synthesis of 1-cyclopropyl-2-(3-methyl-1,2,4-oxadiazol-5-yl)ethanone

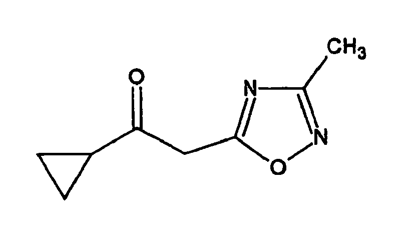

Under an inert atmosphere, acetamidoxime (1.56 g) is added to a solution of methyl 3-cyclopropyl-3-oxopropanoate (3.0 g) in 50 ml of toluene; the stirred mixture is heated to 130°C while distilling off the solvent and methanol formed in the reaction.

When all the solvent has been removed, 50 ml of toluene and 1.56 g of acetamidoxime are added again to the residue and the distillation continued until all of this second portion of solvent has been removed.

The residue is then purified by flash chromatography to obtain 1.48 g of pure product as a violet oil (yield: 42%).

**¹H-NMR (CDCl₃) :** δ 0.95-1.18 (m, 4H, CH₂CH₂), 2.00 (m, 1H, CH), 2.40 (s, 3H, CH₃), 4.14 (s, 2H, CH₂).

### EXAMPLE 18

### Synthesis of 1-[2-chloro-4-(methylsulphonyl)phenyl]-3-cyclopropyl-2-(3-methyl-1,2,4-oxadiazol-5-yl)propane-1,3-dione (Compound N° 11)

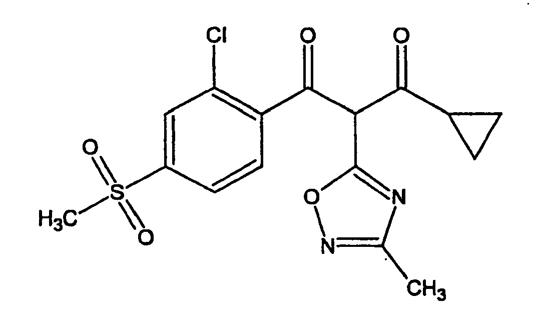

Under an inert atmosphere, Mg(OEt)₂ (0.239 g) is added to a solution of 1-cyclopropyl-2-(3-methyl-1,2,4-oxadiazol-5-y1)ethanone (0.50 g) in 13 ml of dry tetrahydrofuran; the stirred mixture is refluxed for 3 hours, then completely evaporated under reduced pressure.

The residue is taken up with 3 ml of dry tetrahydrofuran, under an inert atmosphere, then a suspension of 2-chloro-4-(methylsulphonyl)benzoyl chloride (0.84 g) in 10 ml of dry tetrahydrofuran is added ; the stirred mixture is refluxed for 3 more hours.

After completion of the reaction, the solvent is evaporated and the residue is taken up with ethyl acetate and 1% HCl, then neutralized with aqueous NaHCO₃ and extracted three times with 5% NaOH; the combined basic aqueous phases are acidified and extracted three times with ethyl acetate, which is then washed with brine, dried with Na₂SO₄ and evaporated.

The resulting solid is purified by washing with diethyl ether to obtain 0.90 g of pure product as an off-white solid (yield: 78%; m.p.: 188°C).

**¹H-NMR (CDCl₃) :** (δ 1.19-1.48 (m, 4H, CH₂CH₂), 2.29 (2s, 3H, Ar-CH₃), 2.55 (m, 1H, CH), 3.06 (s, 3H, SO₂CH₃), 7.46-7.93 (m, 3H, arom. H's), 17.93 (bs, 1H, OH).

### EXAMPLE 19

### Synthesis of 1-(4-chloro-2-nitrophenil)-3-cyclopropyl-2-(3-methyl-1,2,4-oxadiazol-5-yl)propane-1,3-dione (Compound N° 12)

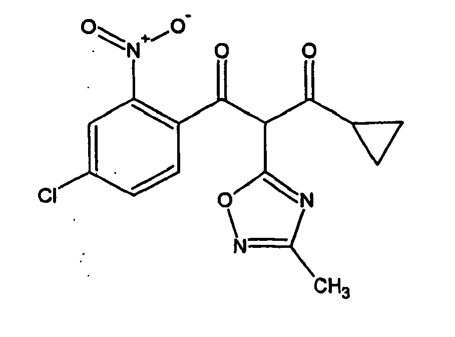

Under an inert atmosphere, Mg(OEt)₂ (0.215 g) is added to a solution of 1-ciclopropyl-2-(3-methyl-1,2,4-oxadiazol-5-yl)ethanone (0.45 g) in 12 ml of dry tetrahydrofuran; the stirred mixture is refluxed for 3 hours, then completely evaporated under reduced pressure.

The residue is taken up with 6 ml of dry tetrahydrofuran, under an inert atmosphere, then a solution of 4-chloro-2-nitrobenzoyl chloride (0.66 g) in 6 ml of dry tetrahydrofuran is added; the stirred mixture is refluxed for 3 more hours.

After completion of the reaction, the solvent is evaporated and the residue is taken up with ethyl acetate and 1% HCl, then neutralized with aqueous NaHCO₃ and extracted three times with 5% NaOH; the combined basic aqueous phases are acidified and extracted three times with ethyl acetate, which is then washed with brine, dried with Na₂SO₄ and evaporated.

The resulting solid is purified by washing with a little portion of diethyl ether to obtain 0.51 g of pure product as an off-white solid (yield: 54%; m.p.: 127°C).

**¹H-NMR (CDCl₃) :** δ 1.18-1.49 (m, 4H, CH₂CH₂), 2.25 (2s, 3H, Ar-CH₃), 2.47 (m, 1H, CH), 7.16-8.15 (m, 3H, arom. H's), 17.61 (bs, 1H, OH).

### EXAMPLE 20

### Synthesis of 1-cyclopropyl-2-(pyridin-2-yl)ethanone

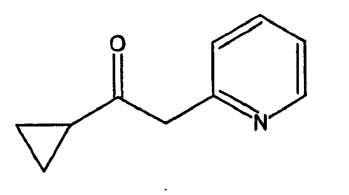

Under an inert atmosphere and in dried glassware, 2-picoline (9.43 g) is dissolved in 95 ml of anhydrous tetrahydrofuran; 63.1 ml of 1.6 M buthyllithium solution in hexanes are then added: the solution temperature rises to about 40 °C: after the addition, the mixture is stirred for 30 minutes at 40°C.

A solution of methyl cyclopropanecarboxylate (5.07 g) in 5 ml of anhydrous tetrahydrofuran is then quickly added and the mixture is stirred for 1 h at 40°C.

The mixture is then cautiously diluted with water and the organic solvent evaporated at reduced pressure; the residue is taken up with ether and a mixture of 10% HCl and ice; the organic phase is extracted 4 times with HCl 10%.

The combined aqueous acid phases are cautiously treated with 50% NaOH until slightly acid, then basified to pH 8 with solid NaHCO₃; the mixture is then saturated with NaCl and extracted three times with ethyl acetate, which is then dried with Na₂SO₄ and evaporated.

The resulting dark oil is purified by flash chromatography to obtain 5.08 g of desired product as a yellow oil (yield: 31%).
**¹H-NMR (CDCl₃) :** δ 0.82-1.11 (m, 4H, CH₂CH₂), 2.05 (m, 1H, CH), 4.03 (s, 2H, CH₂), 7.19, 7.63, 8.55 (3m, 4H, arom. H's)

### EXAMPLE 21

### Synthesis of 1-[2-chloro-4-(methylsulphonyl)phenyl]-3-cyclopropyl-2-(pyridin-2-yl)propane-1,3-dione (Compound N° 13)

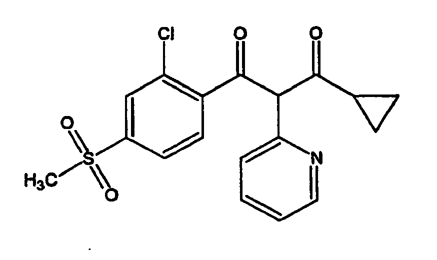

Under an inert atmosphere, Mg(OEt)₂ (0.152 g) is added to a solution of 1-ciclopropyl-2-(pyridin-2-yl)ethanone (0.30 g) in 8 ml of dry tetrahydrofuran; the stirred mixture is refluxed for 3 hours, then completely evaporated under reduced pressure.

The residue is taken up with 2 ml of dry tetrahydrofuran, under an inert atmosphere, then a suspension of 2-chloro-4-(methylsulfonyl)benzoyl chloride (0.52 g) in 6 ml of dry tetrahydrofuran is added; the stirred mixture is refluxed for 3 more hours.

After completion of the reaction, the mixture is diluted with methanol to have an homogeneous solution, then the solvent is evaporated. The residue is taken up with water and extracted three times with ethyl acetate, which is then washed with brine, dried with Na₂SO₄ and evaporated.

The resulting solid is purified by flash chromatography to obtain 0.36 g of product ass a yellow amorphous solid (yield: 51%).

**¹H-NMR (CDCl₃) :** δ 0.82-1.70 (m, 5H, cyclopropyl), 3.06 (s, 3H, SO₂CH₃), 7.06-8.21 (m, 7H, arom. H's), 18.05 (bs, 1H, OH).

### EXAMPLE 22

### Synthesis of 1-[2-chloro-4-(methylsulphonyl)phenyl]-3-(cyclopropyl) propane-1,3-dione.

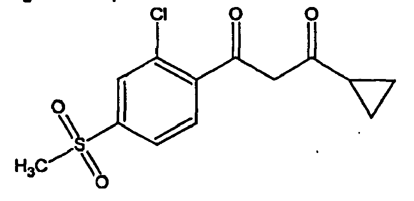

Under an inert atmosphere, Mg(OEt)₂ (1.29 g) is added to a solution of t-butyl 3-cyclopropyl-3-oxopropanoate (3.0 g) in 75 ml of dry tetrahydrofuran; the stirred mixture is refluxed for 3 hours, then completely evaporated under reduced pressure.

The residue is taken up with 20 ml of dry tetrahydrofuran, under an inert atmosphere, then a suspension of 2-chloro-4-(methylsulfonyl)benzoyl chloride (4.52 g) in 55 ml of dry tetrahydrofuran is added; the stirred mixture is refluxed for 3 more hours.

After completion of the reaction, the solvent is evaporated under reduced pressure; the residue is taken up with 30 ml of toluene and p-toluenesulphonic acid (1.13 g) is added, then the stirred mixture is refluxed for 8 hours.

The solid precipitate is filtered off and the solution is evaporated under reduced pressure; the oily residue is purified by flash chromatography to obtain 2.64 g of solid product (yield: 54%).
**¹H-NMR (CDCl₃) :** δ 0.98-1.80 (m, 5H, cyclopropyl), 3.07 (s, 3H, SO₂CH₃), 6.13 (s, 1H, enolic form =CH-), 7.74-8.00 (m, 3H, arom. H's).

### EXAMPLE 23

### Synthesis of 1-[2-chloro-4-(methylsulphonyl)phenyl]-3-cyclopropyl-2-(5-trifluoromethyl-1,3,4-thiadiazol-2-yl)propane-1,3-dione (Compound N° 2550).

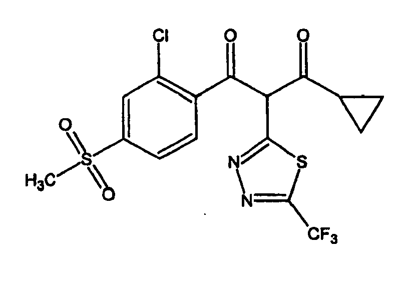

Under an inert atmosphere, NaH (60% suspension in mineral oil, 0.27 g) is suspended in 10 ml of dry tetrahydrofuran; a solution of 1-[2-chloro-4-(methylsulphonyl)phenyl]-3-(cyclopropyl)propan-1,3-dione (1.76 g) in 15 ml of dry tetrahydrofuran is then slowly added dropwise.

The mixture is stirred for 1 hour, then a solution of 2-methylsulphonyl-5-trifluoromethyl-1,3,4-thiadiazole (1.97 g) in 13 ml of dry tetrahydrofuran is added dropwise.

The stirred mixture is refluxed for 3 hours, then the solvent is evaporated under reduced pressure; the residue is taken up with diethyl ether and extracted two times with aqueous NaHCO₃; the combined aqueous phases are slowly acidified to pH 2-3 and extracted with ethyl acetate, which is then dried with Na₂SO₄ and evaporated.

The residue is purified by flash chromatography, then by washing witch diethyl ether, to obtain 0.83 g of product as a white solid (yield: 31%; m.p.: 185°C).
**¹H-NMR (CDCl₃) :** (mixture of two tautomers) δ 0.50-1.40 (m, 5H, cyclopropyl), 3.10 (s, 3H, SO₂CH₃), 7.60-8.06 (m, 3H, arom. H's), 15.23, 15.39 (2 bs, 1H, OH).
**¹⁹F-NNMR (CDCl₃) :** (mixture of two tautomers) δ -60.52, -60.68 (2 s, CF₃) .

### EXAMPLE 24

### synthesis of 2-bromo-1-[2-chloro-4-(methylsulphonyl)phenyl]-3-(cyclopropyl)propane-1,3-dione

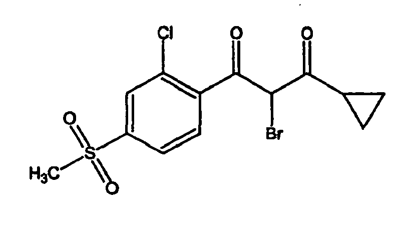

Under an inert atmosphere, bromine (0.24 g) is slowly added dropwise to a solution of (1-[2-chloro-4-(methylsulphonyl)phenyl]-3-(cyclopropyl)propan-1,3-dione (0.43 g) in 25 ml of dichloromethane cooled to 5°C, then the mixture is stirred overnight at room temperature.

The solvent is then completely evaporated under reduced pressure and the residue (0.57 g) is used without purification for the following reaction.
**¹H-NMR (CDCl₃) :** δ 1.14-1.34 (m, 4H, cyclic CH₂-CH₂), 2.61 (m, 1H, cyclic CH), 3.10 (s, 3H, SO₂CH₃), 7.52-8. 05 (m, 3H, arom. H's), 16.02 (bs, 1H, OH).

### EXAMPLE 25

### Synthesis of 1-[2-chloro-4-(methylsulphonyl)phenyl]-3-cyclopropyl-2-(1,2,4-triazol-1-yl)propane-1,3-dione (Compound N° 413)

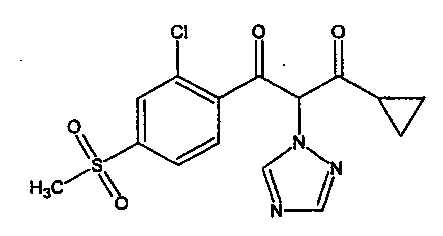

Under an inert atmosphere, NaH (60% suspension in mineral oil, 0.133 g) is suspended in 2 ml of dry tetrahydrofuran cooled in a water bath, then 1,2,4-triazole (0.23 g) is added.

After stirring for 30 minutes at room temperature, a solution of 2-bromo-1-[2-chloro-4-(methylsulphonyl)phenyl]-3-(cyclopropyl)propan-1,3-dione (0.63 g) in 5 ml of dry tetrahydrofuran is added, then the mixture is heated to 50°C for 8 hours.

After completion of the reaction, the mixture is diluted with water, acidified and extracted three times with ethyl acetate, which is then washed with brine, dried with Na₂SO₄ and evaporated.

The residue is purified by flash chromatography, then by washing with ethyl ether to obtain 0.23 g of solid product (yield: 38%; m.p.: 162°C).
**¹H-M4R (CDCl₃) :** δ 1.09-1.40 (m, 5H, cyclopropyl), 3.01 (s, 3H, SO₂CH₃), 7.38-8.05 (m, 5H, arom. H's), 16.23 (bs, 1H, OH).

### EXAMPLE 26

### Synthesis of 1-[2-Chloro-4-(methylsulphonyl)phenyl]-3-cyclopropyl-2-[1,1-dioxido-3-oxo-1,2-benzisothiazol-2(3H)-yl]propane-1,3-dione (Compound N° 2551)

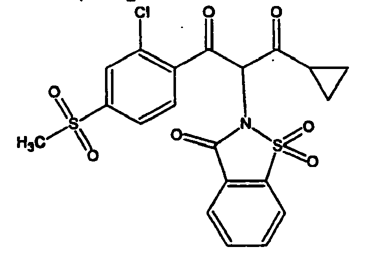

Under an inert atmosphere, NaH (60% suspension in mineral oil, 0.114 g) is suspended in 3 ml of dry tetrahydrofuran cooled in a water bath, then saccharine (0.52 g) is added.

After stirring for 30 minutes at room temperature, a solution of 2-bromo-1-[2-chloro-4-(methylsulphonyl)phenyl]-3-(cyclopropyl)propan-1,3-dione (0.54 g) in 8 ml of dry tetrahydrofuran is added, then the mixture is heated to 50°C for 6 hours.

After completion of the reaction, the solvent is evaporated; the residue is taken up with water, acidified and extracted three times with ethyl acetate, which is then washed with brine, dried with Na₂SO₄ and evaporated.

The residue is purified by flash chromatography, then by washing with ethyl ether to obtain 0.31 g of product as an amorphous solid (yield: 45%).
**¹H-NMR (CDCl₃) :** δ 1.09-1.45 (m, 4H, cyclic CH₂-CH₂), 1.86 (m, 1H, cyclic CH), 2.99 (s, 3H, SO₂CH₃), 7.51-8.08 (m, 7H, arom. H's), 17.19 (bs, 1H, OH).

### Example 27

### Synthesis of 1,1,1-trifluoro-3-pyridin-2-ylacetone

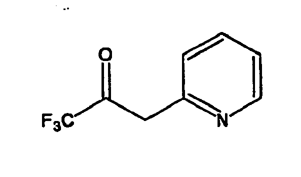

Under an inert atmosphere, 2-picoline (4.72 g) and pyridine (20.0 g) are dissolved in 130 ml of toluene cooled in an ice bath; trifluoroacetic anhydride (31.9 g) is then slowly added dropwise and the mixture is stirred at room temperature for 48 hours.

The mixture is then cautiously poured into 500 ml of 3% Na₂CO₃, and extracted three times with ethyl acetate; the combined organic phases are extracted two times with 5% NaOH, then these combined basic aqueous phases are acidified to pH 6.5.and extracted with ethyl acetate, which is washed with brine, dried with Na₂SO₄ and evaporated.

The raw product is purified by flash chromatography, then by washing with diethyl ether to obtain 4.24 g of product as a yellow solid (yield: 44%).
**¹H-NMR (CDCl₃) :** δ 5.82 (s, 1H, enolic form CH), 6.99-8.10 (m, 4H, arom. H's), 15.88 (bs, 1H, OH).
**¹⁹F-NMR (CDCl₃) :** δ -74.94 (s, CF₃) .

### EXAMPLE 28

### Synthesis of 1-[2-chloro-4-(methylsulphonyl)phenyl]-2-(pyridin-2-yl)-4,4,4-trifluorobutane-1,3-dione (Compound N° 565).

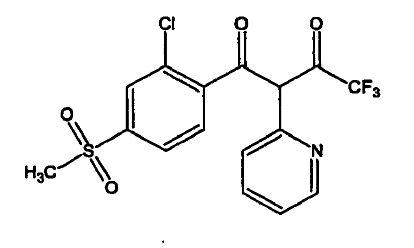

Under an inert atmosphere, Mg(OEt)₂ (0.336 g) is added to a solution of 1,1,1-trifluoro-3-pyridin-2-ylacetone (0.80 g) in 17 ml of dry tetrahydrofuran; the stirred mixture is stirred at room temperature for 3 hours, then completely evaporated under reduced pressure.

The residue is taken up with 5 ml of dry tetrahydrofuran, under an inert atmosphere, then a suspension of 2-chloro-4-(methylsulfonyl)benzoyl chloride (1.17 g) in 12 ml of dry tetrahydrofuran is added ; the mixture is stirred overnight at room temperature.

After completion of the reaction, the mixture is diluted with ethyl acetate, quickly washed with NH₄Cl saturated solution, with brine, then dried with Na₂SO₄ and evaporated.

The residue is taken up with a mixture of diethyl ether and hexane which causes the product to precipitate: 0.24 g of solid are recovered by filtration (yield: 14%; m.p.: 189°C, with decomposition)
**¹H-NMR (acetone-*d*₆) :** δ 3.09 (s, 3H, SO₂CH₃), 7.07-8.37 (m, 7H, arom. H's).
**¹⁹F-NMR (acetone-*d*₆) :** δ -67.43 (s, CF₃)

### EXAMPLE 29

Following the suitable procedures, some of which are detailed in the examples above, the following compounds, listed in Table 2, have been prepared and identified by elemental analysis and/or ¹H-NMR:

| | | | | |
|---|---|---|---|---|
| 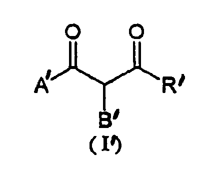 | | | | |

| **Compound N** | **A'** | **B'** | **R'** | **m.p. (°C)** |
|---|---|---|---|---|
| 14 | 2-NO₂-4-SO₂MePh | 1,2,4-oxadiazol-5-yl | H | |
| 15 | 2-NO₂-4-SO₂MePh | 1,2,4-oxadiazol-5-yl | methyl | |
| 16 | 2-NO₂-4-SO₂MePh | 1,2,4-oxadiazol-5-yl | i-propyl | |
| 17 | 2-NO₂-4-SO₂MePh | 1,2,4-oxadiazol-5-yl | cyclopropyl | |
| 18 | 2-NO₂-4-SO₂MePh | 1,2,4-oxadiazol-5-yl | CF₃ | |
| 19 | 2-NO₂-4-SO₂MePh | 3-methyl-1,2,4-oxadiazol-5-yl | H | |
| 20 | 2-NO₂-4-SO₂MePh | 3-methyl-1,2,4-oxadiazol-5-yl | methyl | |
| 21 | 2-NO₂-4-SO₂MePh | 3-methyl-1,2,4-oxadiazol-5-yl | i-propyl | |
| 22 | 2-NO₂-4-SO₂MePh | 3-methyl-1,2,4-oxadiazol-5-yl | cyclopropyl | |
| 23 | 2-NO₂-4-SO₂MePh | 3-methyl-1,2,4-oxadiazol-5-yl | CF₃ | |
| 24 | 2-NO₂-4-SO₂MePh | 3-trifluoromethyl-1,2,4-oxadiazol-5-yl | H | |
| 25 | 2-NO₂-4-SO₂MePh | 3-trifluoromethyl-1,2,4-oxadiazol-5-yl | methyl | |
| 26 | 2-NO₂-4-SO₂MePh | 3-trifluoromethyl-1,2,4-oxadiazol-5-yl | i-propyl | |
| 27 | 2-NO₂-4-SO₂MePh | 3-trifluoromethyl-1,2,4-oxadiazol-5-yl | cyclopropyl | |
| 28 | 2-NO₂-4-SO₂MePh | 3-trifluoromethyl-1,2,4-oxadiazol-5-yl | CF₃ | |
| 29 | 2-NO₂-4-SO₂MePh | 1,2,4-oxadiazol-3-yl | H | |
| 30 | 2-NO₂-4-SO₂MePh | 1,2,4-oxadiazol-3-yl | methyl | |
| 31 | 2-NO₂-4-SO₂MePh | 1,2,4-oxadiazol-3-yl | i-propyl | |
| 32 | 2-NO₂-4-SO₂MePh | 1,2,4-oxadiazol-3-yl | cyclopropyl | |
| 33 | 2-NO₂-4-SO₂MePh | 1,2,4-oxadiazol-3-yl | CF₃ | |
| 34 | 2-NO₂-4-SO₂MePh | 5-methyl-1,2,4-oxadiazol-3-yl | H | |
| 35 | 2-NO₂-4-SO₂MePh | 5-methyl-1,2,4-oxadiazol-3-yl | methyl | |
| 36 | 2-NO₂-4-SO₂MePh | 5-methyl-1,2,4-oxadiazol-3-yl | i-propyl | |
| 37 | 2-NO₂-4-SO₂MePh | 5-methyl-1,2,4-oxadiazol-3-yl | cyclopropyl | |
| 38 | 2-NO₂-4-SO₂MePh | 5-methyl-1,2,4-oxadiazol-3-yl | CF₃ | |
| 39 | 2-NO₂-4-SO₂MePh | 5-trifluoromethyl-1,2,4-oxadiazol-3-yl | H | |
| 40 | 2-NO₂-4-SO₂MePh | 5-trifluoromethyl-1,2,4-oxadiazol-3-yl | methyl | |
| 41 | 2-NO₂-4-SO₂MePh | 5-trifluoromethyl-1,2,4-oxadiazol-3-yl | i-propyl | |
| 42 | 2-NO₂-4-SO₂MePh | 5-trifluoromethyl-1,2,4-oxadiazol-3-yl | cyclopropyl | |
| 43 | 2-NO₂-4-SO₂MePh | 5-trifluoromethyl-1,2,4-oxadiazol-3-yl | CF₃ | |
| 44 | 2-NO₂-4-SO₂MePh | 5-chloro-1,2,4-oxadiazol-3-yl | H | |
| 45 | 2-NO₂-4-SO₂MePh | 5-chloro-1,2,4-oxadiazol-3-yl | methyl | |
| 46 | 2-NO₂-4-SO₂MePh | 5-chloro-1,2,4-oxadiazol-3-yl | i-propyl | |
| 47 | 2-NO₂-4-SO₂MePh | 5-chloro-1,2,4-oxadiazol-3-yl | cyclopropyl | |
| 48 | 2-NO₂-4-SO₂MePh | 5-chloro-1,2,4-oxadiazol-3-yl | CF₃ | |
| 49 | 2-NO₂-4-SO₂MePh | 1,3,4-oxadiazol-2-yl | H | |
| 50 | 2-NO₂-4-SO₂MePh | 1,3,4-oxadiazol-2-yl | methyl | |
| 51 | 2-NO₂-4-SO₂MePh | 1,3,4-oxadiazol-2-yl | i-propyl | |
| 52 | 2-NO₂-4-SO₂MePh | 1,3,4-oxadiazol-2-yl | cyclopropyl | |
| 53 | 2-NO₂-4-SO₂MePh | 1,3,4-oxadiazol-2-yl | CF₃ | |
| 54 | 2-NO₂-4-SO₂MePh | 5-methylsulfonyl-1,3,4-oxadiazol-2-yl | H | |
| 55 | 2-NO₂-4-SO₂MePh | 5-methylsulfonyl-1,3,4-oxadiazol-2-yl | methyl | |
| 56 | 2-NO₂-4-SO₂MePh | 5-methylsulfonyl-1,3,4-oxadiazol-2-yl | i-propyl | |
| 57 | 2-NO₂-4-SO₂MePh | 5-methylsulfonyl-1,3,4-oxadiazol-2-yl | cyclopropyl | |
| 58 | 2-NO₂-4-SO₂MePh | 5-methylsulfonyl-1,3,4-oxadiazol-2-yl | CF₃ | |
| 59 | 2-NO₂-4-SO₂MePh | 5-methyl-1,3,4-oxadiazol-2-yl | H | |
| 60 | 2-NO₂-4-SO₂MePh | 5-methyl1,3,4-oxadiazol-2-yl | methyl | |
| 61 | 2-NO₂-4-SO₂MePh | 5-methyl-1,3,4-oxadiazol-2-yl | i-propyl | |
| 62 | 2-NO₂-4-SO₂MePh | 5-methyl-1,3,4-oxadiazol-2-yl | cyclopropyl | |
| 63 | 2-NO₂-4-SO₂MePh | 5-methyl-1,3,4-oxadiazol-2-yl | CF₃ | |
| 64 | 2-NO₂-4-SO₂MePh | 5-trifluoromethyl-1,3,4-oxadiazol-2-yl | H | |
| 65 | 2-NO₂-4-SO₂MePh | 5-methylsulfonyl-1,3,4-oxadiazol-2-yl | methyl | |
| 66 | 2-NO₂-4-SO₂MePh | 5-trifluoromethyl-1,3,4-oxadiazol-2-yl | i-propyl | |
| 67 | 2-NO₂-4-SO₂MePh | 5-trifluoromethyl-1,3,4-oxadiazol-2-yl | cyclopropyl | |
| 68 | 2-NO₂-4-SO₂MePh | 5-trifluoromethyl-1,3,4-oxadiazol-2-yl | CF₃ | |
| 69 | 2-NO₂-4-SO₂MePh | 1,2,3-triazol-4-yl | H | |
| 70 | 2-NO₂-4-SO₂MePh | 1,2,3-triazol-4-yl | methyl | |
| 71 | 2-NO₂-4-SO₂MePh | 1,2,3-triazol-4-yl | i-propyl | |
| 72 | 2-NO₂-4-SO₂MePh | 1,2,3-triazol-4-yl | cyclopropyl | |
| 73 | 2-NO₂-4-SO₂MePh | 1,2,3-triazol-4-yl | CF₃ | |
| 74 | 2-NO₂-4-SO₂MePh | 1-methyl-1,2,3-triazol-4-yl | H | |
| 75 | 2-NO₂-4-SO₂MePh | 1-methyl-1,2,3-triazol-4-yl | methyl | |
| 76 | 2-NO₂-4-SO₂MePh | 1-methyl-1,2,3-triazol-4-yl | i-propyl | |
| 77 | 2-NO₂-4-SO₂MePh | 1-methyl-1,2,3-triazol-4-yl | cyclopropyl | |
| 78 | 2-NO₂-4-SO₂MePh | 1-methyl-1,2,3-triazol-4-yl | CF₃ | |
| 79 | 2-NO₂-4-SO₂MePh | 2-methyl-1,2,3-triazol-4-yl | H | |
| 80 | 2-NO₂-4-SO₂MePh | 2-methyl-1,2,3-triazol-4-yl | methyl | |
| 81 | 2-NO₂-4-SO₂MePh | 2-methyl-1,2,3-triazol-4-yl | i-propyl | |
| 82 | 2-NO₂-4-SO₂MePh | 2-methyl-1,2,3-triazol-4-yl | cyclopropyl | |
| 83 | 2-NO₂-4-SO₂MePh | 2-methyl-1,2,3-triazol-4-yl | CF₃ | |
| 84 | 2-NO₂-4-SO₂MePh | 1,2,3-triazol-1-yl | H | |
| 85 | 2-NO₂-4-SO₂MePh | 1,2,3-triazol-1-yl | methyl | |
| 86 | 2-NO₂-4-SO₂MePh | 1,2,3-triazol-1-yl | i-propyl | |
| 87 | 2-NO₂-4-SO₂MePh | 1,2,3-triazol-1-yl | cyclopropyl | |
| 88 | 2-NO₂-4-SO₂MePh | 1,2,3-triazol-1-yl | CF₃ | |
| 89 | 2-NO₂-4-SO₂MePh | 1,2,3-triazol-2-yl | H | |
| 90 | 2-NO₂-4-SO₂MePh | 1,2,3-triazol-2-yl | methyl | |
| 91 | 2-NO₂-4-SO₂MePh | 1,2,3-triazol-2-yl | i-propyl | |
| 92 | 2-NO₂-4-SO₂MePh | 1,2,3-triazol-2-yl | cyclopropyl | |
| 93 | 2-NO₂-4-SO₂MePh | 1,2,3-triazol-1-yl | CF₃ | |
| 94 | 2-NO₂-4-SO₂MePh | 1,2,3-triazol-1-yl | H | |
| 95 | 2-NO₂-4-SO₂MePh | 1,2,3-triazol-1-yl | methyl | |
| 96 | 2-NO₂-4-SO₂MePh | 1,2,3-triazol-1-yl | i-propyl | |
| 97 | 2-NO₂-4-SO₂MePh | 1,2,3-triazol-1-yl | cyclopropyl | |
| 98 | 2-NO₂-4-SO₂MePh | 1,2,3-triazol-1-yl | CF₃ | |
| 99 | 2-NO₂-4-SO₂MePh | imidazol-2-yl | H | |
| 100 | 2-NO₂-4-SO₂MePh | imidazol-2-yl | methyl | |
| 101 | 2-NO₂-4-SO₂MePh | imidazol-2-yl | i-propyl | |
| 102 | 2-NO₂-4-SO₂MePh | imidazol-2-yl | cyclopropyl | |
| 103 | 2-NO₂-4-SO₂MePh | imidazol-2-yl | CF₃ | |
| 104 | 2-NO₂-4-SO₂MePh | imidazol-1-yl | H | |
| 105 | 2-NO₂-4-SO₂MePh | imidazol-1-yl | methyl | |
| 106 | 2-NO₂-4-SO₂MePh | imidazol-1-yl | i-propyl | |
| 107 | 2-NO₂-4-SO₂MePh | imidazol-1-yl | cyclopropyl | |
| 108 | 2-NO₂-4-SO₂MePh | imidazol-1-yl | CF₃ | |
| 109 | 2-NO₂-4-SO₂MePh | imidazol-4-yl | H | |
| 110 | 2-NO₂-4-SO₂MePh | imidazol-4-yl | methyl | |
| 111 | 2-NO₂-4-SO₂MePh | imidazol-4-yl | i-propyl | |
| 112 | 2-NO₂-4-SO₂MePh | imidazol-4-yl | cyclopropyl | |
| 113 | 2-NO₂-4-SO₂MePh | imidazol-4-yl | CF₃ | |
| 114 | 2-NO₂-4-SO₂MePh | thiazol-2-yl | H | |
| 115 | 2-NO₂-4-SO₂MePh | thiazol-2-yl | methyl | |
| 116 | 2-NO₂-4-SO₂MePh | thiazol-2-yl | i-propyl | |
| 117 | 2-NO₂-4-SO₂MePh | thiazol-2-yl | cyclopropyl | |
| 118 | 2-NO₂-4-SO₂MePh | thiazol-2-yl | CF₃ | |
| 119 | 2-NO₂-4-SO₂MePh | 4-methylthiazol-2-yl | H | |
| 120 | 2-NO₂-4-SO₂MePh | 4-methylthiazol-2-yl | methyl | |
| 121 | 2-NO₂-4-SO₂MePh | 4-methylthiazol-2-yl | i-propyl | |
| 122 | 2-NO₂-4-SO₂MePh | 4-methylthiazol-2-yl | cyclopropyl | |
| 123 | 2-NO₂-4-SO₂MePh | 4-methylthiazol-2-yl | CF₃ | |
| 124 | 2-NO₂-4-SO₂MePh | oxazol-2-yl | H | |
| 125 | 2-NO₂-4-SO₂MePh | oxazol-2-yl | methyl | |
| 126 | 2-NO₂-4-SO₂MePh | oxazol-2-yl | i-propyl | |
| 127 | 2-NO₂-4-SO₂MePh | oxazol-2-yl | cyclopropyl | |
| 128 | 2-NO₂-4-SO₂MePh | oxazol-2-yl | CF₃ | |
| 129 | 2-NO₂-4-SO₂MePh | 4,5-dimethyloxazol-2-yl | H | |
| 130 | 2-NO₂-4-SO₂MePh | 4,5-methylthiazol-2-yl | methyl | |
| 131 | 2-NO₂-4-SO₂MePh | 4,5-dimethyloxazol-2-yl | i-propyl | |
| 132 | 2-NO₂-4-SO₂MePh | 4,5-dimethyloxazol-2-yl | cyclopropyl | |
| 133 | 2-NO₂-4-SO₂MePh | 4,5-dimethyloxazol-2-yl | CF₃ | |
| 134 | 2-NO₂-4-SO₂MePh | 2-oxazolin-2-yl | H | |
| 135 | 2-NO₂-4-SO₂MePh | 2-oxazolin-2-yl | methyl | |
| 136 | 2-NO₂-4-SO₂MePh | 2-oxazolin-2-yl | i-propyl | |
| 137 | 2-NO₂-4-SO₂MePh | 2-oxazolin-2-yl | cyclopropyl | |
| 138 | 2-NO₂-4-SO₂MePh | 2-oxazolin-2-yl | CF₃ | |
| 139 | 2-NO₂-4-SO₂MePh | 4,4-dimethyl-2-oxazolin-2-yl | H | |
| 144 | 2-NO₂-4-SO₂MePh | 4,4-dimethyl-2-oxazolin-2-yl | methyl | |
| 141 | 2-NO₂-4-SO₂MePh | 4,4-dimethyl-2-oxazolin-2-yl | i-propyl | |
| 142 | 2-NO₂-4-SO₂MePh | 4,4-dimethyl-2-oxazolin-2-yl | cyclopropyl | |
| 143 | 2-NO₂-4-SO₂MePh | 4,4-dimethyl-2-oxazolin-2-yl | CF₃ | |
| 144 | 2-NO₂-4-SO₂MePh | 1,2,4-thiadiazol-5-yl | H | |
| 145 | 2-NO₂-4-SO₂MePh | 1,2,4-thiadiazol-5-yl | methyl | |
| 146 | 2-NO₂-4-SO₂MePh | 1,2,4-thiadiazol-5-yl | i-propyl | |
| 147 | 2-NO₂4-SO₂MePh | 1,2,4-thiadiazol-5-yl | cyclopropyl | |
| 148 | 2-NO₂-4-SO₂MePh | 1,2,4-thiadiazol-5-yl | CF₃ | |
| 149 | 2-NO₂-4-SO₂MePh | 3-methyl-1,2,4-thiadiazol-5-yl | H | |
| 150 | 2-NO₂-4-SO₂MePh | 3-methyl-1,2,4-thiadiazol-5-yl | methyl | |
| 151 | 2-NO₂-4-SO₂MePh | 3-methyl-1,2,4-thiadiazol-5-yl | i-propyl | |
| 152 | 2-NO₂-4-SO₂MePh | 3-methyl-1,2,4-thiadiazol-5-yl | cyclopropyl | |
| 153 | 2-NO₂-4-SO₂MePh | 3-methyl-1,2,4-thiadiazol-5-yl | CF₃ | |
| 154 | 2-NO₂-4-SO₂MePh | 3-trifluoromethyl-1,2,4-thiadiazol-5-yl | H | |
| 155 | 2-NO₂-4-SO₂MePh | 3-trifluoromethyl-1,2,4-thiadiazol-5-yl | methyl | |
| 156 | 2-NO₂-4-SO₂MePh | 3-trifluoromethyl-1,2,4-thiadiazol-5-yl | i-propyl | |
| 157 | 2-NO₂-4-SO₂MePh | 3-trifluoromethyl-1,2,4-thiadiazol-5-yl | cyclopropyl | |
| 158 | 2-NO₂-4-SO₂MePh | 3-trifluoromethyl-1,2,4-thiadiazol-5-yl | CF₃ | |
| 159 | 2-NO₂-4-SO₂MePh | 1,2,4-thiadiazol-3-yl | H | |
| 160 | 2-NO₂-4-SO₂MePh | 1,2,4-thiadiazol-3-yl | methyl | |
| 161 | 2-NO₂-4-SO₂MePh | 1,2,4-thiadiazol-3-yl | i-propyl | |
| 162 | 2-NO₂-4-SO₂MePh | 1,2,4-thiadiazol-3-yl | cyclopropyl | |
| 163 | 2-NO₂-4-SO₂MePh | 1,2,4-thiadiazol-3-yl | CF₃ | |
| 164 | 2-NO₂-4-SO₂MePh | 5-methyl-1,2,4-thiadiazol-3-yl | H | |
| 165 | 2-NO₂-4-SO₂MePh | 5-methyl-1,2,4-thiadiazol-3-yl | methyl | |
| 166 | 2-NO₂-4-SO₂MePh | 5-methyl-1,2,4-thiadiazol-3-yl | i-propyl | |
| 167 | 2-NO₂-4-SO₂MePh | 5-methyl-1,2,4-thiadiazol-3-yl | cyclopropyl | |
| 168 | 2-NO₂-4-SO₂MePh | 5-methyl-1,2,4-thiadiazol-3-yl | CF₃ | |
| 169 | 2-NO₂-4-SO₂MePh | 5-trifluoromethyl-1,2,4-thiadiazol-3-yl | H | |
| 170 | 2-NO₂-4-SO₂MePh | 5-trifluoromethyl-1,2,4-thiadiazol-3-yl | methyl | |
| 171 | 2-NO₂-4-SO₂MePh | 5-trifluoromethyl-1,2,4-thiadiazol-3-yl | i-propyl | |
| 172 | 2-NO₂-4-SO₂MePh | 5-trifluoromethyl-1,2,4-thiadiazol-3-yl | cyclopropyl | |
| 173 | 2-NO₂-4-SO₂MePh | 5-trifluoromethyl-1,2,4-thiadiazol-3-yl | CF₃ | |
| 174 | 2-NO₂-4-SO₂MePh | 1,3,4-thiadiazol-2-yl | H | |
| 175 | 2-NO₂-4-SO₂MePh | 1,3,4-thiadiazol-2-yl | methyl | |
| 176 | 2-NO₂-4-SO₂MePh | 1,3,4-thiadiazol-2-yl | i-propyl | |
| 177 | 2-NO₂-4-SO₂MePh | 1,3,4-thiadiazol-2-yl | cyclopropyl | |
| 178. | 2-NO₂-4-SO₂MePh | 1,3,4-thiadiazol-2-yl | CF₃ | |
| 179 | 2-NO₂-4-SO₂MePh | 5-methylsulfonyl-1,3,4-thiadiazol-2-yl | H | |
| 180 | 2-NO₂-4-SO₂MePh | 5-methylsulfonyl-1,3,4-thiadiazol-2-yl | methyl | |
| 181 | 2-NO₂-4-SO₂MePh | 5-methylsulfonyl-1,3,4-thiadiazol-2-yl | i-propyl | |
| 182 | 2-NO₂-4-SO₂MePh | 5-methylsulfonyl-1,3,4-thiadiazol-2-yl | cyclopropyl | |
| 183 | 2-NO₂-4-SO₂MePh | 5-methylsulfonyl-1,3,4-thiadiazol-2-yl | CF₃ | |
| 184 | 2-NO₂-4-SO₂MePh | 5-methyl-1,3,4-thiadiazol-2-yl | H | |
| 185 | 2-NO₂-4-SO₂MePh | 5-methyl-1,3,4-thiadiazol-2-yl | methyl | |
| 186 | 2-NO₂-4-SO₂MePh | 5-methyl-1,3,4-thiadiazol-2-yl | i-propyl | |
| 187 | 2-NO₂-4-SO₂MePh | 5-methyl-1,3,4-thiadiazol-2-yl | cyclopropyl | |
| 188 | 2-NO₂-4-SO₂MePh | 5-methyl-1,3,4-thiadiazol-2-yl | CF₃ | |
| 189 | 2-NO₂-4-SO₂MePh | benzoxazol-2-yl | H | |
| 190 | 2-NO₂-4-SO₂MePh | benzoxazol-2-yl | methyl | |
| 191 | 2-NO₂-4-SO₂MePh | benzoxazol-2-yl | i-propyl | |
| 192 | 2-NO₂-4-SO₂MePh | benzoxazol-2-yl | cyclopropyl | |
| 193 | 2-NO₂-4-SO₂MePh | benzoxazol-2-yl | CF₃ | |
| 194 | 2-NO₂-4-SO₂MePh | 6-methylbenzoxazol-2-yl | H | |
| 195 | 2-NO₂-4-SO₂MePh | 6-methylbenzoxazol-2-yl | methyl | |
| 196 | 2-NO₂-4-SO₂MePh | 6-methylbenzoxazol-2-yl | i-propyl | |
| 197 | 2-NO₂-4-SO₂MePh | 6-methylbenzoxazol-2-yl | cyclopropyl | |
| 198 | 2-NO₂-4-SO₂MePh | 6-methylbenzoxazol-2-yl | CF₃ | |
| 199 | 2-NO₂-4-SO₂MePh | benzothiazol-2-yl | H | |
| 200 | 2-NO₂-4-SO₂MePh | benzothiazol-2-yl | methyl | |
| 201 | 2-NO₂-4-SO₂MePh | benzothiazol-2-yl | i-propyl | |
| 202 | 2-NO₂-4-SO₂MePh | benzothiazol-2-yl | cyclopropyl | |
| 203 | 2-NO₂-4-SO₂MePh | benzothiazol-2-yl | CF₃ | |
| 204 | 2-NO₂-4-SO₂MePh | pyrazol-1-yl | H | |
| 205 | 2-NO₂-4-SO₂MePh | pyrazol-1-yl | methyl | |
| 206 | 2-NO₂-4-SO₂MePh | pyrazol-1-yl | i-propyl | |
| 207 | 2-NO₂-4-SO₂MePh | pyrazol-1-yl | cyclopropyl | |
| 208 | 2-NO₂-4-SO₂MePh | pyrazol-1-yl | CF₃ | |
| 209 | 2-NO₂-4-SO₂MePh | pyrazol-3-yl | H | |
| 210 | 2-NO₂-4-SO₂MePh | pyrazol-3-yl | methyl | |
| 211 | 2-NO₂-4-SO₂MePh | pyrazol-3-yl | i-propyl | |
| 212 | 2-NO₂-4-SO₂MePh | pyrazol-3-yl | cyclopropyl | |
| 213 | 2-NO₂-4-SO₂MePh | pyrazol-3-yl | CF₃ | |
| 214 | 2-NO₂-4-SO₂MePh | 1-methylpyrazol-3-yl | H | |
| 215 | 2-NO₂-4-SO₂MePh | 1-methylpyrazol-3-yl | methyl | |
| 216 | 2-NO₂-4-SO₂MePh | 1-methylpyrazol-3-yl | i-propyl | |
| 217 | 2-NO₂-4-SO₂MePh | 1-methylpyrazol-3-yl | cyclopropyl | |
| 218 | 2-NO₂-4-SO₂MePh | 1-methylpyrazol-3-yl | CF₃ | |
| 219 | 2-NO₂-4-SO₂MePh | tetrazol-1-yl | H | |
| 220 | 2-NO₂-4-SO₂MePh | tetrazol-1-yl | methyl | |
| 221 | 2-NO₂-4-SO₂MePh | tetrazol-1-yl | i-propyl | |
| 222 | 2-NO₂-4-SO₂MePh | tetrazol-1-yl | cyclopropyl | |
| 223 | 2-NO₂-4-SO₂MePh | tetrazol-1-yl | CF₃ | |
| 224 | 2-NO₂-4-SO₂MePh | 5-methyltetrazol-1-yl | H | |
| 225 | 2-NO₂-4-SO₂MePh | 5-methyltetrazol-1-yl | methyl | |
| 226 | 2-NO₂-4-SO₂MePh | 5-methyltetrazol-1-yl | i-propyl | |
| 227 | 2-NO₂-4-SO₂MePh | 5-methyltetrazol-1-yl | cyclopropyl | |
| 228 | 2-NO₂-4-SO₂MePh | 5-methyltetrazol-1-yl | CF₃ | |
| 229 | 2-NO₂-4-SO₂MePh | tetrazol-2-yl | H | |
| 230 | 2-NO₂-4-SO₂MePh | tetrazol-2-yl | methyl | |
| 231 | 2-NO₂-4-SO₂MePh | tetrazol-2-yl | i-propyl | |
| 232 | 2-NO₂-4-SO₂MePh | tetrazol-2-yl | cyclopropyl | |
| 233 | 2-NO₂-4-SO₂MePh | tetrazol-2-yl | CF₃ | |
| 234 | 2-NO₂-4-SO₂MePh | 5-methyltetrazol-2-yl | H | |
| 235 | 2-NO₂-4-SO₂MePh | 5-methyltetrazol-2-yl | methyl | |
| 236 | 2-NO₂-4-SO₂MePh | 5-methyltetrazol-2-yl | i-propyl | |
| 237 | 2-NO₂-4-SO₂MePh | 5-methyltetrazol-2-yl | cyclopropyl | |
| 238 | 2-NO₂-4-SO₂MePh | 5-methyltetrazol-2-yl | CF₃ | |
| 239 | 2-NO₂-4-SO₂MePh | 1-methyltetrazol-5-yl | H | |
| 240 | 2-NO₂-4-SO₂MePh | 1-methyltetrazol-5-yl | methyl | |
| 241 | 2-NO₂-4-SO₂MePh | 1-methyltetrazol-5-yl | i-propyl | |
| 242 | 2-NO₂-4-SO₂MePh | 1-methyltetrazol-5-yl | cyclopropyl | |
| 243 | 2-NO₂-4-SO₂MePh | 1-methyltetrazol-5-yl | CF₃ | |
| 244 | 2-NO₂-4-SO₂MePh | 2-methyltetrazol-5-yl | H | |
| 245 | 2-NO₂-4-SO₂MePh | 2-methyltetrazol-5-yl | methyl | |
| 246 | 2-NO₂-4-SO₂MePh | 2-methyltetrazol-5-yl | i-propyl | |
| 247 | 2-NO₂-4-SO₂MePh | 2-methyltetrazol-5-yl | CF₃ | |
| 248 | 2-NO₂-4-SO₂MePh | pyridin-2-yl | H | |
| 249 | 2-NO₂-4-SO₂MePh | pyridin-2-yl | methyl | |
| 250 | 2-NO₂-4-SO₂MePh | pyridin-2-yl | i-propyl | |
| 251 | 2-NO₂-4-SO₂MePh | pyridin-2-yl | cyclopropyl | |
| 252 | 2-NO₂-4-SO₂MePh | pyridin-2-yl | CF₃ | |
| 253 | 2-NO₂-4-SO₂MePh | pyridin-4-yl | H | |
| 254 | 2-NO₂-4-SO₂MePh | pyridin-4-yl | methyl | |
| 255 | 2-NO₂-4-SO₂MePh | pyridin-4-yl | i-propyl | |
| 256 | 2-NO₂-4-SO₂MePh | pyridin-4-yl | cyclopropyl | |
| 257 | 2-NO₂-4-SO₂MePh | pyridin-4-yl | CF₃ | |
| 258 | 2-NO₂-4-SO₂MePh | pyridin-3-yl | H | |
| 259 | 2-NO₂-4-SO₂MePh | pyridin-3-yl | methyl | |
| 260 | 2-NO₂-4-SO₂MePh | pyridin-4-yl | i-propyl | |
| 261 | 2-NO₂-4-SO₂MePh | pyridin-3-yl | cyclopropyl | |
| 262 | 2-NO₂-4-SO₂MePh | pyridin-3-yl | CF₃ | |
| 263 | 2-NO₂-4-SO₂MePh | 3-nitropyridin-4-yl | H | |
| 264 | 2-NO₂-4-SO₂MePh | 3-nitropyridin-4-yl | methyl | |
| 265 | 2-NO₂-4-SO₂MePh | 3-nitropyridin-4-yl | i-propyl | |
| 266 | 2-NO₂-4-SO₂MePh | 3-nitropyridin-4-yl | cyclopropyl | |
| 267 | 2-NO₂-4-SO₂MePh | 3-nitropyridin-4-yl | CF₃ | |
| 268 | 2-NO₂-4-SO₂MePh | 5-cyanopyridin-2-yl | H | |
| 269 | 2-NO₂-4-SO₂MePh | 5-cyanopyridin-2-yl | methyl | |
| 270 | 2-NO₂-4-SO₂MePh | 5-cyanopyridin-2-yl | i-propyl | |
| 271 | 2-NO₂-4-SO₂MePh | 5-cyanopyridin-2-yl | cyclopropyl | |
| 272 | 2-NO₂-4-SO2MePh | 5-cyanopyridin-2-yl | CF₃ | |
| 273 | 2-NO₂-4-SO₂MePh | 5-trifluoromethylpyridin-2-yl | H | |
| 274 | 2-NO₂-4-SO₂MePh | 5-trifluoromethylpyridin-2-yl | methyl | |
| 275 | 2-NO₂-4-SO₂MePh | 5-trifluoromethylpyridin-2-yl | i-propyl | |
| 276 | 2-NO₂-4-SO₂MePh | 5-trifluoromethylpyridin-2-yl | cyclopropyl | |
| 277 | 2-NO₂-4-SO₂MePh | 5-trifluoromethylpyridin-2-yl | CF₃ | |
| 278 | 2-NO₂-4-SO₂MePh | pyrimidin-2-yl | H | |
| 279 | 2-NO₂-4-SO₂MePh | pyrimidin-2-yl | methyl | |
| 280 | 2-NO₂-4-SO₂MePh | pyrimidin-2-yl | i-propyl | |
| 281 | 2-NO₂-4-SO₂MePh | pyrimidin-2-yl | cyclopropyl | |
| 282 | 2-NO₂-4-SO₂MePh | pyrimidin-2-yl | CF₃ | |
| 283 | 2-NO₂-4-SO₂MePh | pyrimidin-4-yl | H | |
| 284 | 2-NO₂-4-SO₂MePh | pyrimidin-4-yl | methyl | |
| 285 | 2-NO₂-4-SO₂MePh | pyrididin-4-yl | i-propyl | |
| 286 | 2-NO₂-4-SO₂MePh | pyrimidin-4-yl | cyclopropyl | |
| 287 | 2-NO₂-4-SO₂MePh | pyrimidin-4-yl | CF₃ | |
| 288 | 2-NO₂-4-SO₂MePh | 6-chloropyrimidin-4-yl | methyl | |
| 289 | 2-NO₂-4-SO₂MePh | 6-chloropyrimidin-4-yl | i-propyl | |
| 290 | 2-NO₂-4-SO₂MePh | 6-chloropyrimidin-4-yl | cyclopropyl | |
| 291 | 2-NO₂-4-SO₂MePh | 6-chloropyrimidin-4-yl | CF₃ | |
| 292 | 2-NO₂-4-SO₂MePh | pyridazin-3-yl | H | |
| 293 | 2-NO₂-4-SO₂MePh | pyridazin-3-yl | methyl | |
| 294 | 2-NO₂-4-SO₂MePh | pyridazin-3-yl | i-propyl | |
| 295 | 2-NO₂-4-SO₂MePh | pyridazin-3-yl | cyclopropyl | |
| 296 | 2-NO₂-4-SO₂MePh | pyridazin-3-yl | CF₃ | |
| 297 | 2-NO₂-4-SO₂MePh | 6-chloropyridazin-3-yl | methyl | |
| 298 | 2-NO₂-4-SO₂MePh | 6-chloropyridazin-3-yl | i-propyl | |
| 299 | 2-NO₂-4-SO₂MePh | 6-chloropyridazin-3-yl | cyclopropyl | |
| 300 | 2-NO₂-4-SO₂MePh | 6-chloropyridazin-3-yl | CF₃ | |
| 301 | 2-NO₂-4-SO₂MePh | pyrazin-2-yl | methyl | |
| 302 | 2-NO₂-4-SO₂MePh | pyrazin-2-yl | i-propyl | |
| 303 | 2-NO₂-4-SO₂MePh | pyrazin-2-yl | cyclopropyl | |
| 304 | 2-NO₂-4-SO₂MePh | pyrazin-2-yl | CF₃ | |
| 305 | 2-NO₂-4-SO₂MePh | triazin-2-yl | methyl | |
| 306 | 2-NO₂-4-SO₂MePh | triazin-2-yl | i-propyl | |
| 307 | 2-NO₂-4-SO₂MePh | triazin-2-yl | cyclopropyl | |
| 308 | 2-NO₂-4-SO₂MePh | triazin-2-yl | CF₃ | |
| 309 | 2-NO₂-4-SO₂MePh | quinolin-2-yl | methyl | |
| 310 | 2-NO₂-4-SO₂MePh | quinolin-2-yl | i-propyl | |
| 311 | 2-NO₂-4-SO₂MePh | quinolin-2-yl | cyclopropyl | |
| 312 | 2-NO₂-4-SO₂MePh | quinolin-2-yl | CF₃ | |
| 313 | 2-NO₂-4-SO₂MePh | 4,4,6-trimethyl-5,6.dihydro-1,3(4H)-oxazin-2-yl | H | |
| 314 | 2-NO₂-4-SO₂MePh | 4,4,6-trimethyl-5,6.dihydro-1,3(4H)-oxazin-2-yl | methyl | |
| 315 | 2-NO₂-4-SO₂MePh | 4,4,6-trimethyl-5,6.dihydro-1,3(4H)-oxazin-2-yl | i-propyl | |
| 316 | 2-NO₂-4-SO₂MePh | 4,4,6-trimethyl-5,6.dihydro-1,3(4H)-oxazin-2-yl | cyclopropyl | |
| 317 | 2-NO₂-4-SO₂MePh | 4,4,6-trimethyl-5,6.dihydro-1,3(4H)-oxazin-2-yl | CF₃ | |
| 318 | 2-NO₂-4-SO₂MePh | 2-oxazolidinon-3-yl | H | |
| 319 | 2-NO₂-4-SO₂MePh | 2-oxazolidinon-3-yl | methyl | |
| 320 | 2-NO₂-4-SO₂MePh | 2-oxazolidinon-3-yl | i-propyl | |
| 321 | 2-NO₂-4-SO₂MePh | 2-oxazolidinon-3-yl | cyclopropyl | |
| 322 | 2-NO₂-4-SO₂MePh | 2-oxazolidinon-3-yl | CF₃ | |
| 323 | 2-NO₂-4-SO₂MePh | 2-pyrrolidinon-1-yl | methyl | |
| 324 | 2-NO₂-4-SO₂MePh | 2-pyrrolidinon-1-yl | i-prapyl | |
| 325 | 2-NO₂-4-SO₂MePh | 2-pyrrolidinon-1-yl | cyclopropyl | |
| 326 | 2-NO₂-4-SO₂MePh | 2-pyrrolidinon-1-yl | CF₃ | |
| 327 | 2-NO₂-4-SO₂MePh | 3-methylisoxazol-5-yl | methyl | |
| 328 | 2-NO₂-4-SO₂MePh | 3-methylisoxazol-5-yl | i-propyl | |
| 329 | 2-NO₂-4-SO₂MePh | 3-methylisoxazol-5-yl | cyclopropyl | |
| 330 | 2-NO₂-4-SO₂MePh | 3-methylisoxazol-5-yl | CF₃ | |
| 331 | 2-Cl-4-SO₂MePh | 1,2,4-oxadiazol-5-yl | H | |
| 332 | 2-Cl-4-SO₂MePh | 1,2,4-oxadiazol-5-yl | methyl | |
| 333 | 2-Cl-4-SO₂MePh | 1,2,4-oxadiazol-5-yl | i-propyl | |
| 334 | 2-Cl-4-SO₂MePh | 1,2,4-oxadiazol-5-yl | cyclopropyl | |
| 335 | 2-Cl-4-SO₂MePh | 1,2,4-oxadiazol-5-yl | CF₃ | |
| 336 | 2-Cl-4-SO₂MePh | 3-methyl-1,2,4-oxadiazol-5-yl | H | |
| 337 | 2-Cl-4-SO₂MePh | 3-methyl-1,2,4-oxadiazol-5-yl | methyl | oil |
| 338 | 2-Cl-4-SO₂MePh | 3-methyl-1,2,4-oxadiazol-5-yl | i-propyl | 174 |
| 339 | 2-Cl-4-SO₂MePh | 3-methyl-1,2,4-oxadiazol-5-yl | CF₃ | |
| 340 | 2-Cl-4-SO₂MePh | 3-trifluoromethyl-1,2,4-oxadiazol-5-yl | H | |
| 341 | 2-Cl-4-SO₂MePh | 3-trifluoromethyl-1,2,4-oxadiazol-5-yl | methyl | |
| 342 | 2-Cl-4-SO₂MePh | 3-trifluoromethyl-1,2,4-oxadiazol-5-yl | i-propyl | |
| 343 | 2-Cl-4-SO₂MePh | 3-trifluoromethyl-1,2,4-oxadiazol-5-yl | cyclopropyl | |
| 344 | 2-Cl-4-SO₂MePh | 3-trifluoromethyl-1,2,4-oxadiazol-5-yl | CF₃ | |
| 345 | 2-Cl-4-SO₂MePh | 1,2,4-oxadiazol-3-yl | H | |
| 346 | 2-Cl-4-SO₂MePh | 1,2,4-oxadiazol-3-yl | methyl | |
| 347 | 2-Cl-4-SO₂MePh | 1,2,4-oxadiazol-3-yl | i-propyl | |
| 348 | 2-Cl-4-SO₂MePh | 1,2,4-oxadiazol-3-yl | cyclopropyl | |
| 349 | 2-Cl-4-SO₂MePh | 1,2,4-oxadiazol-3-yl | CF₃ | |
| 350 | 2-Cl-4-SO₂MePh | 5-methyl-1,2,4-oxadiazol-3-yl | H | |
| 351 | 2-Cl-4-SO₂MePh | 5-methyl-1,2,4-oxadiazol-3-yl | methyl | |
| 352 | 2-Cl-4-SO₂MePh | 5-methyl-1,2,4-oxadiazol-3-yl | i-propyl | |
| 353 | 2-Cl-4-SO₂MePh | 5-methyl-1,2,4-oxadiazol-3-yl | cyclopropyl | |
| 354 | 2-Cl-4-SO₂MePh | 5-methyl-1,2,4-oxadiazol-3-yl | CF₃ | |
| 355 | 2-Cl-4-SO₂MePh | 5-trifluoromethyl-1,2,4-oxadiazol-3-yl | H | |
| 356 | 2-Cl-4-SO₂MePh | 5-trifluoromethyl-1,2,4-oxadiazol-3-yl | methyl | |
| 357 | 2-Cl-4-SO₂MePh | 5-trifluoromethyl-1,2,4-oxadiazol-3-yl | i-propyl | |
| 358 | 2-Cl-4-SO₂MePh | 5-trifluoromethyl-1,2,4-oxadiazol-3-yl | cyclopropyl | |
| 359 | 2-Cl-4-SO₂MePh | 5-trifluoromethyl-1,2,4-oxadiazol-3-yl | CF₃ | |
| 360 | 2-Cl-4-SO₂MePh | 5-chloro-1,2,4-oxadiazol-3-yl | H | |
| 361 | 2-Cl-4-SO₂MePh | 5-chloro-1,2,4-oxadiazol-3-yl | methyl | |
| 362 | 2-Cl-4-SO₂MePh | 5-chloro-1,2,4-oxadiazol-3-yl | i-propyl | |
| 363 | 2-Cl-4-SO₂MePh | 5-chloro-1,2,4-oxadiazol-3-yl | cyclopropyl | |
| 364 | 2-Cl-4-SO₂MePh | 5-chloro-1,2,4-oxadiazol-3-yl | CF₃ | |
| 365 | 2-Cl-4-SO₂MePh | 1,3,4-oxadiazol-2-yl | H | |
| 366 | 2-Cl-4-SO₂MePh | 1,3,4-oxadiazol-2-yl | methyl | |
| 367 | 2-Cl-4-SO₂MePh | 1,3,4-oxadiazol-2-yl | i-propyl | |
| 348 | 2-Cl-4-SO₂MePh | 1,3,4-oxadiazol-2-yl | cyclopropyl | |
| 369 | 2-Cl-4-SO₂MePh | 1,3,4-oxadiazol-2-yl | CF₃ | |
| 370 | 2-Cl-4-SO₂MePh | 5-methylsulfonyl-1,3,4-oxadiazol-2-yl | H | |
| 371 | 2-Cl-4-SO₂MePh | 5-methylsulfonyl-1,3,4-oxadiazol-2-yl | methyl | |
| 372 | 2-Cl-4-SO₂MePh | 5-methylsulfonyl-1,3,4-oxadiazol-2-yl | i-propyl | |
| 373 | 2-Cl-4-SO₂MePh | 5-methylsulfonyl-1,3,4-oxadiazol-2-yl | cyclopropyl | |
| 374 | 2-Cl-4-SO₂MePh | 5-methylsulfonyl-1,3,4-oxadiazol-2-yl | CF₃ | |
| 375 | 2-Cl-4-SO₂MePh | 5-methyl-1,3,4-oxadiazol-2-yl | H | |
| 376 | 2-Cl-4-SO₂MePh | 5-methyl-1,3,4-oxadiazol-2-yl | methyl | |
| 377 | 2-Cl-4-SO₂MePh | 5-methyl-1,3,4-oxadiazol-2-yl | i-propyl | |
| 378 | 2-Cl-4-SO₂MePh | 5-methy-1-1,3,4-oxadiazol-2-yl | cyclopropyl | |
| 379 | 2-Cl-4-SO₂MePh | 5-methyl-1,3,4-oxadiazol-2-yl | CF₃ | |
| 380 | 2-Cl-4-SO₂MePh | 5-trifluoromethyl-1,3,4-oxadiazol-2-yl | H | |
| 381 | 2-Cl-4-SO₂MePh | 5-trifluoromethyl-1,3,4-oxadiazol-2-yl | methyl | |
| 382 | 2-Cl-4-SO₂MePh | 5-trifluoromethyl-1,3,4-oxadiazol-2-yl | i-propyl | |
| 383 | 2-Cl-4-SO₂MePh | 5-trifluoromethyl-1,3,4-oxadiazol-2-yl | cyclopropyl | |
| 384 | 2-Cl-4-SO₂MePh | 5-trifluoromethyl-1,3,4-oxadiazol-2-yl | CF₃ | |
| 385 | 2-Cl-4-SO₂MePh | 1,2,3-triazol-4-yl | H | |
| 386 | 2-Cl-4-SO₂MePh | 1,2,3-triazol-4-yl | methyl | |
| 387 | 2-Cl-4-SO₂MePh | 1,2,3-triazol-4-yl | i-propyl | |
| 388 | 2-Cl-4-SO₂MePh | 1,2,3-triazol-4-yl | cyclopropyl | |
| 389 | 2-Cl-4-SO₂MePh | 1,2,3-triazol-4-yl | CF₃ | |
| 390 | 2-Cl-4-SO₂MePh | 1-methyl-1,2,3 triazol-4-yl | H | |
| 391 | 2-Cl-4-SO₂MePh | 1-methyl-1,2,3 triazol-4-yl | methyl | |
| 392 | 2-Cl-4-SO₂MePh | 1-methyl-1,2,3 triazol-4-yl | i-propyl | |
| 393 | 2-Cl-4-SO₂MePh | 1-methyl-1,2,3 triazol-4-yl | cyclopropyl | |
| 394 | 2-Cl-4-SO₂MePh | 1-methyl-1,2,3 triazol-4-yl | CF₃ | |
| 395 | 2-Cl-4-SO₂MePh | 2-methyl-1,2,3 triazol-4-yl | H | |
| 396 | 2-Cl-4-SO₂MePh | 2-methyl-1,2,3-triazol-4-yl | methyl | |
| 397 | 2-Cl-4-SO₂MePh | 2-methyl-1,2,3-triazol-4-yl | i-propyl | |
| 398 | 2-Cl-4-SO₂MePh | 2-methyl-1,2,3-triazol-4-yl | cyclopropyl | |
| 399 | 2-Cl-4-SO₂MePh | 2-methyl-1,2,3-triazol-4-yl | CF₃ | |
| 400 | 2-Cl-4-SO₂MePh | 1,2,3-triazol-1-yl | H | |
| 401 | 2-Cl-4-SO₂MePh | 1,2,3-triazol-1-yl | methyl | |
| 402 | 2-Cl-4-SO₂MePh | 1,2,3-triazol-1-yl | i-propyl | |
| 403 | 2-Cl-4-SO₂MePh | 1,2,3-triazol-1-yl | cyclopropyl | |
| 404 | 2-Cl-4-SO₂MePh | 1,2,3-triazol-1-yl | CF₃ | |
| 405 | 2-Cl-4-SO₂MePh | 1,2,3-triazol-2-yl | H | |
| 406 | 2-Cl-4-SO₂MePh | 1,2,3-triazol-2-yl | methyl | |
| 407 | 2-Cl-4-SO₂MePh | 1,2,3-triazol-1-yl | i-propyl | |
| 408 | 2-Cl-4-SO₂MePh | 1,2,3-triazol-2-yl | cyclopropyl | |
| 409 | 2-Cl-4-SO₂MePh | 1,2,3-triazol-1-yl | CF₃ | |
| 410 | 2-Cl-4-SO₂MePh | 1,2,4-triazol-1-yl | H | |
| 411 | 2-Cl-4-SO₂MePh | 1,2,3-triazol-1-yl | methyl | |
| 412 | 2-Cl-4-SO₂MePh | 1,2,4-triazol-1-yl | i-propyl | |
| 413 | 2-Cl-4-SO₂MePh | 1,2,4-triazol-1-yl | cyclopropyl | 162 |
| 414 | 2-Cl-4-SO₂MePh | 1,2,4-triazol-1-yl | CF₃ | |
| 415 | 2-Cl-4-SO₂MePh | imidazol-2-yl | H | |
| 416 | 2-Cl-4-SO₂MePh | imidazol-2-yl | methyl | |
| 417 | 2-Cl-4-SO₂MePh | imidazol-2-yl | i-propyl | |
| 418 | 2-Cl-4-SO₂MePh | imidazol-2-yl | cyclopropyl | |
| 419 | 2-Cl-4-SO₂MePh | imidazol-2-yl | CF₃ | |
| 420 | 2-Cl-4-SO₂MePh | imidazol-1-yl | H | |
| 421 | 2-Cl-4-SO₂MePh | imidazol-1-yl | methyl | |
| 422 | 2-Cl-4-SO₂MePh | imidazol-1-yl | i-propyl | |
| 423 | 2-Cl-4-SO₂MePh | imidazol-1-yl | cyclopropyl | |
| 424 | 2-Cl-4-SO₂MePh | imidazol-1-yl | CF₃ | |
| 425 | 2-Cl-4-SO₂MePh | imidazol-4-yl | H | |
| 426 | 2-Cl-4-SO₂MePh | imidazol-4-yl | methyl | |
| 427 | 2-Cl-4-SO₂MePh | imidazol-4-yl | i-propyl | |
| 428 | 2-Cl-4-SO₂MePh | imidazol-4-yl | cyclopropyl | |
| 429 | 2-Cl-4-SO₂MePh | imidazol-4-yl | CF₃ | |
| 430 | 2-Cl-4-SO₂MePh | thiazol-2-yl | H | |
| 431 | 2-Cl-4-SO₂MePh | thiazol-2-yl | methyl | |
| 432 | 2-Cl-4-SO₂MePh | thiazol-2-yl | i-propyl | |
| 433 | 2-Cl-4-SO₂MePh | thiazol-2-yl | cyclopropyl | |
| 434 | 2-Cl-4-SO₂MePh | thiazol-2-yl | CF₃ | |
| 435 | 2-Cl-4-SO₂MePh | 4-methylthiazol-2-yl | H | |
| 436 | 2-Cl-4-SO₂MePh | 4-methylthiazol-2-yl | methyl | |
| 437 | 2-Cl-4-SO₂MePh | 4-methylthiazol-2-yl | i-propyl | |
| 438 | 2-Cl-4-SO₂MePh | 4-methylthiazol-2-yl | CF₃ | |
| 439 | 2-Cl-4-SO₂MePh | oxazol-2-yl | H | |
| 440 | 2-Cl-4-SO₂MePh | oxazol-2-yl | methyl | |
| 441 | 2-Cl-4-SO₂MePh | oxazol-2-yl | i-propyl | |
| 442 | 2-Cl-4-SO₂MePh | oxazol-2-yl | cyclopropyl | |
| 443 | 2-Cl-4-SO₂MePh | oxazol-2-yl | CF₃ | |
| 444 | 2-Cl-4-SO₂MePh | 4,5-dimethyloxazol-2-yl | H | |
| 445 | 2-Cl-4-SO₂MePh | 4,5-dimethyloxazol-2-yl | methyl | |
| 446 | 2-Cl-4-SO₂MePh | 4,5-dimethyloxazol-2-yl | i-propyl | |
| 447 | 2-Cl-4-SO₂MePh | 4,5-dimethyloxazol-2-yl | cyclopropyl | |
| 448 | 2-Cl-4-SO₂MePh | 4,5-dimethyloxazol-2-yl | CF₃ | |
| 449 | 2-Cl-4-SO₂MePh | 2-oxazolin-2-yl | H | |
| 450 | 2-Cl-4-SO₂MePh | 2-oxazolin-2-yl | methyl | |
| 451 | 2-Cl-4-SO₂MePh | 2-oxazolin-2-yl | i-propyl | |
| 452 | 2-Cl-4-SO₂MePh | 2-oxazolin-2-yl | cyclopropyl | |
| 453 | 2-Cl-4-SO₂MePh | 2-oxazolin-2-yl | CF₃ | |
| 454 | 2-Cl-4-SO₂MePh | 4,4-dimethyl-2-oxazolin-2-yl | H | |
| 455 | 2-Cl-4-SO₂MePh | 4,4-dimethyl-2-oxazolin-2-yl | methyl | |
| 456 | 2-Cl-4-SO₂MePh | 4,4-dimethyl-2-oxazolin-2-yl | i-propyl | |
| 457 | 2-Cl-4-SO₂MePh | 4,4-dimethyl-2-oxazolin-2-yl | cyclopropyl | |
| 458 | 2-Cl-4-SO₂MePh | 4,4-dimethyl-2-oxazolin-2-yl | CF₃ | |
| 459 | 2-Cl-4-SO₂MePh | 1,2,4-thiadiazol-5-yl | H | |
| 460 | 2-Cl-4-SO₂MePh | 1,2,4-thiadiazol-5-yl | methyl | |
| 461 | 2-Cl-4-SO₂MePh | 1,2,4-thiadiazol-5-yl | i-propyl | |
| 462 | 2-Cl-4-SO₂MePh | 1,2,4-thiadiazol-5-yl | cyclopropyl | |
| 463 | 2-Cl-4-SO₂MePh | 1,2,4-thiadiazol-5-yl | CF₃ | |
| 464 | 2-Cl-4-SO₂MePh | 3-methyl-1,2,4-thiadiazol-5-yl | H | |
| 465 | 2-Cl-4-SO₂MePh | 3-methyl-1,2,4-thiadiazol-5-yl | methyl | |
| 466 | 2-Cl-4-SO₂MePh | 3-methyl-1,2,4-thiadiazol-5-yl | i-propyl | |
| 467 | 2-Cl-4-SO₂MePh | 3-methyl-1,2,4-thiadiazol-5-yl | cyclopropyl | |
| 468 | 2-Cl-4-SO₂MePh | 3-methyl-1,2,4-thiadiazol-5-yl | CF₃ | |
| 469 | 2-Cl-4-SO₂MePh | 3-trifluoromethyl-1,2,4-thiadiazol-5-yl | H | |
| 470 | 2-Cl-4-SO₂MePh | 3-trifluoromethyl-1,2,4-thiadiazol-5-yl | methyl | |
| 471 | 2-Cl-4-SO₂MePh | 3-trifluoromethyl-1,2,4-thiadiazol-5-yl | i-propyl | |
| 472 | 2-Cl-4-SO₂MePh | 3-trifluoromethyl-1,2,4-thiadiazol-5-yl | cyclopropyl | |
| 473 | 2-Cl-4-SO₂MePh | 3-trifluoromethyl-1,2,4-thiadiazol-5-yl | CF₃ | |
| 474 | 2-Cl-4-SO₂MePh | 12,4-thiadiazol-3-yl | H | |
| 475 | 2-Cl-4-SO₂MePh | 1,2,4-thiadiazol-3-l | methyl | |
| 476 | 2-Cl-4-SO₂MePh | 1,2,4-thiadiazol-3-yl | i-propyl | |
| 477 | 2-Cl-4-SO₂MePh | 12,4-thiadiazol-3-yl | cyclopropyl | |
| 478 | 2-Cl-4-SO₂MePh | 1,2,4-thiadiazol-3-yl | CF₃ | |
| 479 | 2-Cl-4-SO₂MePh | 5-methyl-1,2,4-thiadiazol-3-yl | H | |
| 480 | 2-Cl-4-SO₂MePh | 5-methyl-12,4-thiadiazol-3-yl | methyl | |
| 481 | 2-Cl-4-SO₂MePh | 5-methyl-1,2,4-thiadiazol-3-yl | i-propyl | |
| 482 | 2-Cl-4-SO₂MePh | 5-methyl-1,2,4-thiadiazol-3-yl | cyclopropyl | |
| 483 | 2-Cl-4-SO₂MePh | 5-methyl-1,2,4-thiadiazol-3-yl | CF₃ | |
| 484 | 2-Cl-4-SO₂MePh | 5-trifluoromethyl-1,2,4-thiadiazol-3-yl | H | |
| 485 | 2-Cl-4-SO₂MePh | 5-trifluoromethyl-1,2,4-thiadiazol-3-yl | methyl | |
| 486 | 2-Cl-4-SO₂MePh | 5-trifluoromethyl-1,2,4-thiadiazol-3-yl | i-propyl | |
| 487 | 2-Cl-4-SO₂MePh | 5-trifluoromethyl-1,2,4-thiadiazol-3-yl | cyclopropyl | |
| 488 | 2-Cl-4-SO₂MePh | 5-trifluoromethyl-1,2,4-thiadiazol-3-yl | CF₃ | |
| 489 | 2-Cl-4-SO₂MePh | 1,3,4-thiadiazol-2-yl | H | |
| 490 | 2-Cl-4-SO₂MePh | 1,3,4-thiadiazol-2-yl | methyl | |
| 491 | 2-Cl-4-SO₂MePh | 1,3,4-thiadiazol-2-yl | i-propyl | |
| 492 | 2-Cl-4-SO₂MePh | 1,3,4-thiadiazol-2-yl | cyclopropyl | |
| 493 | 2-Cl-4-SO₂MePh | 1,3,4-thiadiazol-2-yl | CF₃ | |
| 494 | 2-Cl-4-SO₂MePh | 5-methylsulfonyl-1,3,4-thiadiazol-2-yl | H | |
| 495 | 2-Cl-4-SO₂MePh | 5-methylsulfonyl-1,3,4-thiadiazol-2-yl | methyl | |
| 496 | 2-Cl-4-SO₂MePh | 5-methylsulfonyl-1,3,4-thiadiazol-2-yl | i-propyl | |
| 497 | 2-Cl-4-SO₂MePh | 5-methylsulfonyl-1,3,4-thiadiazol-2-yl | cyclopropyl | |
| 498 | 2-Cl-4-SO₂MePh | 5-methylsulfonyl-1,3,4-thiadiazol-2-yl | CF₃ | |
| 499 | 2-Cl-4-SO₂MePh | 5-methyl-1,3,4-thiadiazol-2-yl | H | |
| 500 | 2-Cl-4-SO₂MePh | 5-methyl-1,3,4-thiadiazol-2-yl | methyl | |
| 501 | 2-Cl-4-SO₂MePh | 5-methyl-1,3,4-thiadiazol-2-yl | i-propyl | |
| 502 | 2-Cl-4-SO₂MePh | 5-methyl-1,3,4-thiadiazol-2-yl | cyclopropyl | |
| 503 | 2-Cl-4-SO₂MePh | 5-methyl-1,3,4-thiadiazol-2-yl | CF₃ | |
| 504 | 2-Cl-4-SO₂MePh | benzoxazol-2-yl | H | |
| 505 | 2-Cl-4-SO₂MePh | benzoxazol-2-yl | methyl | |
| 506 | 2-Cl-4-SO₂MePh | benzoxazol-2-yl | i-propyl | |
| 507 | 2-Cl-4-SO₂MePh | benzoxazol-2-yl | cyclopropyl | |
| 508 | 2-Cl-4-SO₂MePh | benzoxazol-2-yl | CF₃ | |
| 509 | 2-Cl-4-SO₂MePh | 6-methylbenzoxazol-2-yl | H | |
| 510 | 2-Cl-4-SO₂MePh | 6-methylbenzoxazol-2-yl | methyl | |
| 511 | 2-Cl-4-SO₂MePh | 6-methylbenzoxazol-2-yl | i-propyl | |
| 512 | 2-Cl-4-SO₂MePh | 6-methylbenzoxazol-2-yl | cyclopropyl | |
| 513 | 2-Cl-4-SO₂MePh | 6-methylbenzoxazol-2-yl | CF₃ | |
| 514 | 2-Cl-4-SO₂MePh | benzothiazol-2-yl | H | |
| 515 | 2-Cl-4-SO₂MePh | benzothiazol-2-yl | methyl | |
| 516 | 2-Cl-4-SO₂MePh | benzothiazol-2-yl | i-propyl | |
| 517 | 2-Cl-4-SO₂MePh | benzothiazol-2-yl | cyclopropyl | |
| 518 | 2-Cl-4-SO₂MePh | benzothiazol-2-yl | CF₃ | |
| 519 | 2-Cl-4-SO₂MePh | pyrazol-1-yl | H | |
| 520 | 2-Cl-4-SO₂MePh | pyrazol-1-yl | methyl | |
| 521 | 2-Cl-4-SO₂MePh | pyrazol-1-yl | i-propyl | |
| 522 | 2-Cl-4-SO₂MePh | pyrazol-1-yl | cyclopropyl | |
| 523 | 2-Cl-4-SO₂MePh | pyrazol-1-yl | CF₃ | |
| 524 | 2-Cl-4-SO₂MePh | pyrazol-3-yl | H | |
| 525 | 2-Cl-4-SO₂MePh | pyrazol-3-yl | methyl | |
| 526 | 2-Cl-4-SO₂MePh | pyrazol-3-yl | i-propyl | |
| 527 | 2-Cl-4-SO₂MePh | pyrazol-3-yl | cyclopropyl | |
| 528 | 2-Cl-4-S0₂MePh | pyrazol-3-yl | CF₃ | |
| 529 | 2-Cl-4-SO₂MePh | 1-methylpyrazol-3-yl | H | |
| 530 | 2-Cl-4-SO₂MePh | 1-methylpyrazol-3-yl | methyl | |
| 531 | 2-Cl-4-SO₂MePh | 1-methylpyrazol-3-yl | i-propyl | |
| 532 | 2-Cl-4-SO₂MePh | 1-methylpyrazol-3-yl | cyclopropyl | |
| 533 | 2-Cl-4-SO₂MePh | 1-methylpyrazol-3-yl | CF₃ | |
| 534 | 2-Cl-4-SO₂MePh | tetrazol-1-yl | H | |
| 535 | 2-Cl-4-SO₂MePh | tetrazol-1-yl | methyl | |
| 536 | 2-Cl-4-SO₂MePh | tetrazol-1-yl | i-propyl | |
| 537 | 2-Cl-4-SO₂MePh | tetrazol-1-yl | cyclopropyl | |
| 538 | 2-Cl-4-SO₂MePh | tetrazol-1-yl | CF₃ | |
| 539 | 2-Cl-4-SO₂MePh | 5-methyltetrazol-1-yl | H | |
| 540 | 2-Cl-4-SO₂MePh | 5-methyltetrazol-1-yl | methyl | |
| 541 | 2-Cl-4-SO₂MePh | 5-methyltetrazol-1-yl | i-propyl | |
| 542 | 2-Cl-4-SO₂MePh | 5-methyltetrazol-1-yl | cyclopropyl | |
| 543 | 2-Cl-4-SO₂MePh | 5-methyltetrazol-1-yl | CF₃ | |
| 544 | 2-Cl-4-SO₂MePh | tetrazol-2-yl | H | |
| 545 | 2-Cl-4-SO₂MePh | tetrazol-2-yl | methyl | |
| 546 | 2-Cl-4-SO₂MePh | tetrazol-2-yl | i-propyl | |
| 547 | 2-Cl-4-SO₂MePh | tetrazol-2-yl | cyclopropyl | |
| 548 | 2-Cl-4-SO₂MePh | tetrazol-2-yl | CF₃ | |
| 549 | 2-Cl-4-SO₂MePh | 5-methyltetrazol-2-yl | H | |
| 550 | 2-Cl-4-SO₂MePh | 5-methyltetrazol-2-yl | methyl | |
| 551 | 2-Cl-4-SO₂MePh | 5-methyltetrazol-2-yl | i-propyl | |
| 552 | 2-Cl-4-SO₂MePh | 5-methyltetrazol-2-yl | cyclopropyl | |
| 553 | 2-Cl-4-SO₂MePh | 5-methyltetrazol-2-yl | CF₃ | |
| 554 | 2-Cl-4-SO₂MePh | 1-methyltetrazol-5-yl | H | |
| 555 | 2-Cl-4-SO₂MePh | 1-methyltetrazol-5-yl | methyl | |
| 556 | 2-Cl-4-SO₂MePh | 1-methyltetrazol-5-yl | i-propyl | |
| 557 | 2-Cl-4-SO₂MePh | 1-methyltetrazol-5-yl | CF₃ | |
| 558 | 2-Cl-4-SO₂MePh | 2-methyltetrazol-5-yl | t-butile | oil |
| 559 | 2-Cl-4-SO₂MePh | 2-methyltetrazol-5-yl | methyl | |
| 560 | 2-Cl-4-SO₂MePh | 2-methyltetrazol-5-yl | i-propyl | 210 |
| 561 | 2-Cl-4-SO₂MePh | 2-methyltetrazol-5-yl | CF₃ | |
| 562 | 2-Cl-4-SO₂MePh | pyridin-2-yl | H | |
| 563 | 2-Cl-4-SO₂MePh | pyridin-2-yl | methyl | |
| 564 | 2-Cl-4-SO₂MePh | pyridin-2-yl | i-propyl | |
| 565 | 2-Cl-4-SO₂MePh | pyridin-2-yl | CF₃ | 189 |
| 566 | 2-Cl-4-SO₂MePh | pyridin-4-yl | H | |
| 567 | 2-Cl-4-SO₂MePh | pyridin-4-yl | methyl | |
| 568 | 2-Cl-4-SO₂MePh | pyridin-4-yl | i-propyl | |
| 569 | 2-Cl-4-SO₂MePh | pyridin-4-yl | cyclopropyl | |
| 570 | 2-Cl-4-SO₂MePh | pyridin-4-yl | CF₃ | |
| 571 | 2-Cl-4-SO₂MePh | pyridin-3-yl | H | |
| 572 | 2-Cl-4-SO₂MePh | pyridin-3-yl | methyl | |
| 573 | 2-Cl-4-SO₂MePh | pyridin-3-yl | i-propyl | |
| 574 | 2-Cl-4-SO₂MePh | pyridin-3-yl | cyclopropyl | |
| 575 | 2-Cl-4-SO₂MePh | pyridin-3-yl | CF₃ | |
| 576 | 2-Cl-4-SO₂MePh | 3-nitropyridin-4-yl | H | |
| 577 | 2-Cl-4-SO₂MePh | 3-nitropyridin-4-yl | methyl | |
| 578 | 2-Cl-4-SO₂MePh | 3-nitropyridin-4-yl | i-propyl | |
| 579 | 2-Cl-4-SO₂MePh | 3-nitropyridin-4-yl | cyclopropyl | |
| 580 | 2-Cl-4-SO₂MePh | 3-nitropyridin-4-yl | CF₃ | |
| 581 | 2-Cl-4-SO₂MePh | 5-cyanopyridin-2-yl | H | |
| 582 | 2-Cl-4-SO₂MePh | 5-cyanopyridin-2-yl | methyl | |
| 583 | 2-Cl-4-SO₂MePh | 5-cyanopyridin-2-yl | i-propyl | |
| 584 | 2-Cl-4-SO₂MePh | 5-cyanopyridin-2-yl | cyclopropyl | |
| 585 | 2-Cl-4-SO₂MePh | 5-cyanopyridin-2-yl | CF₃ | |
| 586 | 2-Cl-4-SO₂MePh | 5-trifluoromethylpyridin-2-yl | H | |
| 587 | 2-Cl-4-SO₂MePh | 5-trifluoromethylpyridin-2-yl | methyl | |
| 588 | 2-Cl-4-SO₂MePh | 5-trifluoromethylpyridin-2-yl | i-propyl | |
| 589 | 2-Cl-4-SO₂MePh | 5-trifluoromethylpyridin-2-yl | cyclopropyl | |
| 590 | 2-Cl-4-SO₂MePh | 5-trifluoromethylpyridin-2-yl | CF₃ | |
| 591 | 2-Cl-4-SO₂MePh | pyrimidin-2-yl | H | |
| 592 | 2-Cl-4-SO₂MePh | pyrimidin-2-yl | methyl | |
| 593 | 2-Cl-4-SO₂MePh | pyrimidin-2-yl | i-propyl | |
| 594 | 2-Cl-4-SO₂MePh | pyrimidin-2-yl | cyclopropyl | |
| 595 | 2-Cl-4-SO₂MePh | pyrimidin-2-yl | CF₃ | |
| 596 | 2-Cl-4-SO₂MePh | pyrimidin-4-yl | H | |
| 597 | 2-Cl-4-SO₂MePh | pyrimidin-4-yl | methyl | |
| 598 | 2-Cl-4-SO₂MePh | pyrimidin-4-yl | i-propyl | |
| 599 | 2-Cl-4-SO₂MePh | pyrimidin-4-yl | cyclopropyl | |
| 600 | 2-Cl-4-SO₂MePh | pyrimidin-4-yl | CF₃ | |
| 601 | 2-Cl-4-SO₂MePh | 6-chloropyrimidin-4-yl | methyl | |
| 602 | 2-Cl-4-SO₂MePh | 6-chloropyrimidin-4-yl | i-propyl | |
| 603 | 2-Cl-4-SO₂MePh | 6-chloropyrimidin-4-yl | cyclopropyl | |
| 604 | 2-Cl-4-SO₂MePh | 6-chloropyrimidin-4-yl | CF₃ | |
| 605 | 2=Cl-4-SO₂MePh | pyridazin-3-yl | H | |
| 606 | 2-Cl-4-SO₂MePh | pyridazin-3-yl | methyl | |
| 607 | 2-Cl-4-SO₂MePh | pyridazin-3-yl | i-propyl | |
| 608 | 2-Cl-4-SO₂MePh | pyridazin-3-yl | cyclopropyl | |
| 602 | 2-Cl-4-SO₂MePh | pyridazin-3-yl | CF₃ | |
| 610 | 2-Cl-4-SO₂MePh | 6-chloropyridazin-3-yl | methyl | |
| 611 | 2-Cl-4-SO₂MePh | 6-chloropyridazin-3-yl | i-propyl | |
| 612 | 2-Cl-4-SO₂MePh | 6-chloropyridazin-3-yl | cyclopropyl | |
| 613 | 2-Cl-4-SO₂MePh | 6-chloropyridazin-3-yl | CF₃ | |
| 614 | 2-Cl-4-SO₂MePh | pyrazin-2-yl | methyl | |
| 615 | 2-Cl-4-SO₂MePh | pyrazin 2-yl | i-propyl | |
| 616 | 2-Cl-4-SmO₂MePh | pyrazin-2-yl | cyclopropyl | |
| 617 | 2-Cl-4-SO₂MePh | pyrazin-2-yl | CF₃ | |
| 618 | 2-Cl-4-SO₂MePh | triazin-2-yl | methyl | |
| 619 | 2-Cl-4-sO₂MePh | triazin-2-yl | i-propyl | |
| 620 | 2-Cl-4-SO₂MePh | triazin-2-yl | cyclopropyl | |
| 621 | 2-Cl-4-SO₂MePh | triazin-2-yl | CF₃ | |
| 622 | 2-Cl-4-SO₂MePh | quinolin-2-yl | methyl | |
| 623 | 2-Cl-4-SO₂MePh | quinolin-2-yl | i-propyl | |
| 624 | 2-Cl-4-SO₂MePh | quinolin-2-yl | cyclopropyl | |
| 625 | 2-Cl-4-SO₂MePh | quinolin-2-yl | CF₃ | |
| 626 | 2-Cl-4-SO₂MePh | 4,4,6-trimethyl-5,6-dihydro-1,3(4H)-2-yl | H | |
| 627 | 2-Cl-4-SO₂MePh | 4,4,6-trimethyl-5,6-dihydro-1,3(4H)-oxazin-2-yl | methyl | |
| 628 | 2-Cl-4-SO₂MePh | 4,4,6-trimethyl-5,6-dihydro-1,3(4H)-oxazin-2-yl | i-propyl | |
| 629 | 2-Cl-4-SO₂MePh | 4,4,6-trimethyl-5,6-dihydro-1,3(4H)-oxazin-2-yl | cyclopropyl | |
| 630 | 2-Cl-4-SO₂MePh | 4,4,6-trimethyl-5,6-dihydro-1,3(4H)-oxazin-2-yl | CF₃ | |
| 631 | 2-Cl-4-SO₂MePh | 2-oxazolidinon-3-yl | H | |
| 632 | 2-Cl-4-SO₂MePh | 2-oxazolidinon-3-yl | methyl | |
| 633 | 2-Cl-4-SO₂MePh | 2-oxazolidinon-3-yl | i-propyl | |
| 634 | 2-Cl-4-SO₂MePh | 2-oxazolidinon-3-yl | cyclopropyl | |
| 635 | 2-Cl-4-SO₂MePh | 2-oxazolidinon-3-yl | CF₃ | |
| 636 | 2-Cl-4-SO₂MePh | 2-pyrrolidinon-1-yl | methyl | |
| 637 | 2-Cl-4-SO₂MePh | 2-pyrrolidinon-1-yl | i-propyl | |
| 638 | 2-Cl-4-SO₂MePh | 2-pyrrolidinon-1-yl | cyclopropyl | |
| 639 | 2-Cl-4-SO₂MePh | 2-pyrrolidinon-1-yl | CF₃ | |
| 640 | 2-Cl-4-SO₂MePh | 3-methylisoxazol-5-yl | methyl | |
| 641 | 2-Cl-4-SO₂MePh | 3-methylisoxazol-5-yl | i-propyl | |
| 642 | 2-Cl-4-SO₂MePh | 3-methylisoxazol-5-yl | cyclopropyl | |
| 643 | 2-Cl-4-SO₂MePh | 3-methylisoxazol-5-yl | CF₃ | |
| 644 | 4-Cl-2-NO₂Ph | 1,2,4-oxadiazol-5-yl | H | |
| 645 | 4-Cl-2-NO₂Ph | 1,2,4-oxadiazol-5-yl | methyl | |
| 646 | 4-Cl-2-NO₂Ph | 1,2,4-oxadiazol-5-yl | i-propyl | |
| 647 | 4-Cl-2-NO₂Ph | 1,2,4-oxadiazol-5-yl | cyclopropyl | |
| 648 | 4-Cl-2-NO₂Ph | 1,2,4-oxadiazol-5-yl | CF₃ | |
| 649 | 4-Cl-2-NO₂Ph | 3-methyl-1,2,4-oxadiazol-5-yl | H | |
| 650 | 4-Cl-2-NO₂Ph | 3-methyl-1,2,4-oxadiazol-5-yl | methyl | |
| 651 | 4-Cl-2-NO₂Ph | 3-methyl-1,2,4-oxadiazol-5-yl | i-propyl | |
| 652 | 4-Cl-2-NO₂Ph | 3-methyl-1,2,4-oxadiazol-5-yl | CF₃ | |
| 653 | 4-Cl-2-NO₂Ph | 3-trifluoromethyl-1,2,4-oxadiazol-5-yl | H | |
| 654 | 4-Cl-2-NO₂Ph | 3-trifluoromethyl-1,2,4-oxadiazol-5-yl | methyl | |
| 655 | 4-Cl-2-NO₂Ph | 3-trifluoromethyl-1,2,4-oxadiazol-5-yl | i-propyl | |
| 656 | 4-Cl-2-NO₂Ph | 3-trifluoromethyl-1,2,4-oxadiazol-5-yl | cyclopropyl | |
| 657 | 4-Cl-2-NO₂Ph | 3-trifluoromethyl-1,2,4-oxadiazol-5-yl | CF₃ | |
| 658 | 4-Cl-2-NO₂Ph | 1,2,4-oxadiazol-3-yl | H | |
| 659 | 4-Cl-2-NO₂Ph | 1,2,4-oxadiazol-3-yl | methyl | |
| 660 | 4-Cl-2-NO₂Ph | 1,2,4-oxadiazol-3-yl | i-propyl | |
| 661 | 4-Cl-2-NO₂Ph | 1,2,4-oxadiazol-3-yl | cyclopropyl | |
| 662 | 4-Cl-2-NO₂Ph | 1,2,4-oxadiazol-3-yl | CF₃ | |
| 663 | 4-Cl-2-NO₂Ph | 5-methyl-1,2,4-oxadiazol-3-yl | H | |
| 664 | 4-Cl-2-NO₂Ph | 5-methyl-1,2,4-oxadiazol-3-yl | methyl | |
| 665 | 4-Cl-2-NO₂Ph | 5-methyl-1,2,4-oxadiazol-3-yl | i-propyl | |
| 666 | 4-Cl-2-NO₂Ph | 5-methyl-1,2,4-oxadiazol-3-yl | cyclopropyl | |
| 667 | 4-Cl-2-NO₂Ph | 5-methyl-1,2,4-oxadiazol-3-yl | CF₃ | |
| 668 | 4-Cl-2-NO₂Ph | 5-trifluoromethyl-1,2,4-oxadiazol-3-yl | H | |
| 669 | 4-Cl-2-NO₂Ph | 5-trifluoromethyl-1,2,4-oxadiazol-3-yl | methyl | |
| 670 | 4-Cl-2-NO₂Ph | 5-trifluoromethyl-1,2,4-oxadiazol-3-yl | i-propyl | |
| 671 | 4-Cl-2-NO₂Ph | 5-trifluoromethyl-1,2,4-oxadiazol-3-yl | cyclopropyl | |
| 672 | 4-Cl-2-NO₂Ph | 5-tritluoromethyl-1,2,4-oxadiazol-3-yl | CF₃ | |
| 673 | 4-Cl-2-NO₂Ph | 5-chloro-1,2,4-oxadiazol-3-yl | H | |
| 674 | 4-Cl-2-NO₂Ph | 5-chloro-1,2,4-oxadiazol-3-yl | methyl | |
| 675 | 4-Cl-2-NO₂Ph | 5-chloro-1,2,4-oxadiazol-3-yl | i-propyl | |
| 676 | 4-Cl-2-NO₂Ph | 5-chloro-1,2,4-oxadiazol-3-yl | cyclopropyl | |
| 677 | 4-Cl-2-NO₂Ph | 5-chloro-1,2,4-oxadiazol-3-yl | CF₃ | |
| 678 | 4-Cl-2-NO₂Ph | 1,3,4-oxadiazol-2-yl | H | |
| 679 | 4-Cl-2-NO₂Ph | 1,3,4-oxadiazol-2-yl | methyl | |
| 680 | 4-Cl-2-NO₂Ph | 1,3,4-oxadiazol-2-yl | i-propyl | |
| 681 | 4-Cl-2-NO₂Ph | 1,3,4-oxadiazol-2-yl | cyclopropyl | |
| 682 | 4-Cl-2-NO₂Ph | 1,3,4-oxadiazol-2-yl | CF₃ | |
| 683 | 4-Cl-2-NO₂Ph | 5-methylsulfonyl-1,3,4-oxadiazol-2-yl | H | |
| 684 | 4-Cl-2-NO₂Ph | 5-methylsulfonyl-1,3,4-oxadiazol-2-yl | methyl | |
| 685 | 4-Cl-2-NO₂h | 5-methylsulfonyl-1,3,4-oxadiazol-2-yl | i-propyl | |
| 686 | 4-Cl-2-NO₂Ph | 5-methylsulfonyl-1,3,4-oxadiazol-2-yl | cyclopropyl | |
| 687 | 4-Cl-2-NO₂Ph | 5-methylsulfonyl-1,3,4-oxadiazol-2-yl | CF₃ | |
| 688 | 4-Cl-2-NO₂Ph | 5-methyl-1,3,4-oxadiazol-2-yl | H | |
| 689 | 4-Cl-2-NO₂Ph | 5-methyl-1,3,4-oxadiazol-2-yl | methyl | |
| 690 | 4-Cl-2-NO₂Ph | 5-methyl-1,3,4-oxadiazol-2-yl | i-propyl | |
| 691 | 4-Cl-2-NO₂Ph | 5-methyl-1,3,4-oxadiazol-2-yl | cyclopropyl | |
| 692 | 4-Cl-2-NO₂Ph | 5-methyl-1,3,4-oxadiazol-2-yl | CF₃ | |
| 693 | 4-Cl-2-NO₂Ph | 5-trifluoromethyl-1,3,4-oxadiazol-2-yl | H | |
| 694 | 4-Cl-2-NO₂Ph | 5-trifluoromethyl-1,3,4-oxadiazol-2-yl | methyl | |
| 695 | 4-Cl-2-NO₂Ph | 5-trifluoromethyl-1,3,4-oxadiazol-2-yl | i-propyl | |
| 696 | 4-Cl-2-NO₂Ph | 5-trifluoromethyl-1,3,4-oxadiazol-2-yl | cyclopropyl | |
| 697 | 4-Cl-2-NO₂Ph | 5-trifluoromethyl-1,3,4-oxadiazol-2-yl | CF₃ | |
| 698 | 4-Cl-2-NO₂Ph | 1,2,3-triazol-4-yl | H | |
| 699 | 4-Cl-2-NO₂Ph | 1,2,3-triazol-4-yl | methyl | |
| 700 | 4-Cl-2-NO₂Ph | 1,2,3-triazol-4-yl | i-propyl | |
| 701 | 4-Cl-2-NO₂Ph | 1,2,3-triazol-4-yl | cyclopropyl | |
| 702 | 4-Cl-2-NO₂Ph | 1,2,3-triazol-4-yl | CF₃ | |
| 703 | 4-Cl-2-NO₂Ph | 1-methyl-1,2,3-triazol-4-yl | H | |
| 704 | 4-Cl-2-NO₂Ph | 1-methyl-1,2,3-tiazol-4-yl | methyl | |
| 705 | 4-Cl-2-NO₂Ph | 1-methyl-1,2,3-triazol-4-yl | i-propyl | |
| 706 | 4-Cl-2-NO₂Ph | 1-methyl-1,2,3-triazol-4-yl | cyclopropyl | |
| 707 | 4-Cl-2-NO₂Ph | 1-methyl-1,2,3-triazol-4-yl | CF₃ | |
| 708 | 4-Cl-2-NO₂Ph | 2-methyl-1,2,3-triazol-4-yl | H | |
| 709 | 4-Cl-2-NO₂Ph | 2-methyl-1,2,3-triazol-4-yl | methyl | |
| 710 | 4-Cl-2-NO₂Ph | 2-methyl-1,2,3-triazol-4-yl | i-propyl | |
| 711 | 4-Cl-2-NO₂Ph | 2-methyl-1,2,3-triazol-4-yl | cyclopropyl | |
| 712 | 4-Cl-2-NO₂Ph | 2-methyl-1,2,3-triazol-4-yl | CF₃ | |
| 713 | 4-Cl-2-NO₂Ph | 1,2,3-triazol-1-yl | H | |
| 714 | 4-Cl-2-NO₂Ph | 1,2,3-triazol-1-yl | methyl | |
| 715 | 4-Cl-2-NO₂Ph | 1,2,3-triazol-1-yl | i-propyl | |
| 716 | 4-Cl-2-NO₂Ph | 1,2,3-triazol-1-yl | cyclopropyl | |
| 717 | 4-Cl-2-NO₂Ph | 1,2,3-triazol-1-yl | CF₃ | |
| 718 | 4-Cl-2-NO₂Ph | 1,2,3-triazol-2-yl | H | |
| 719 | 4-Cl-2-NO₂Ph | 1,2,3-triazol-2-yl | methyl | |
| 720 | 4-Cl-2-NO₂Ph | 1,2,3-triazol-2-yl | i-propyl | |
| 721 | 4-Cl-2-NO₂Ph | 1,2,3-triazol-2-yl | cyclopropyl | |
| 722 | 4-Cl-2-NO₂Ph | 1,2,3-triazol-2-yl | CF₃ | |
| 723 | 4-Cl-2-NO₂Ph | 1,2,4-triazol-1-yl | H | |
| 724 | 4-Cl-2-NO₂Ph | 1,2,4-triazol-1-yl | methyl | |
| 725 | 4-Cl-2-NO₂Ph | 1,2,4-triazol-1-yl | i-propyl | |
| 726 | 4-Cl-2-NO₂Ph | 1,2,4-triazol-l-yl | cyclopropyl | |
| 727 | 4-Cl-2-NO₂Ph | 1,2,4-triazol-1-yl | CF₃ | |
| 728 | 4-Cl-2-NO₂Ph | imidazol-2-yl | H | |
| 729 | 4-Cl-2-NO₂Ph | imidazol-2-yl | methyl | |
| 730 | 4-Cl-2-NO₂Ph | imidazol-2-yl | i-propyl | |
| 731 | 4-Cl-2-NO₂Ph | imidazol-2-yl | cyclopropyl | |
| 732 | 4-Cl-2-NO₂Ph | imidazol-2-yl | CF₃ | |
| 733 | 4-Cl-2-NO₂Ph | imidazol-1-yl | H | |
| 734 | 4-Cl-2-NO₂Ph | imidazol-1-yl | methyl | |
| 735 | 4-Cl-2-NO₂Ph | imidazol-1-yl | i-propyl | |
| 736 | 4-Cl-2-NO₂Ph | imidazol-1-yl | cyclopropyl | |
| 737 | 4-Cl-2-NO₂Ph | imidazol-1-yl | CF₃ | |
| 738 | 4-Cl-2-NO₂Ph | imidazol-4-yl | H | |
| 739 | 4-Cl-2-NO₂Ph | imidazol-4-yl | methyl | |
| 740 | 4-Cl-2-NO₂Ph | imidazol-4-yl | i-propyl | |
| 741 | 4-Cl-2-NO₂Ph | imidazol-4-yl | cyclopropyl | |
| 742 | 4-Cl-2-NO₂Ph | imidazol-4-yl | CF₃ | |
| 743 | 4-Cl-2-NO₂Ph | thiazol-2-yl | H | |
| 744 | 4-Cl-2-NO₂Ph | thiazol-2-yl | methyl | |
| 745 | 4-Cl-2-NO₂Ph | thiazol-2-yl | i-propyl | |
| 746 | 4-Cl-2-NO₂Ph | thiazole-2-yl | cyclopropyl | |
| 747 | 4-Cl-2-NO₂Ph | thiazol-2-yl | CF₃ | |
| 748 | 4-Cl-2-NO₂Ph | 4-methylthiazol-2-yl | H | |
| 749 | 4-Cl-2-NO₂Ph | 4-methylthiazol-2-yl | methyl | |
| 750 | 4-Cl-2-NO₂Ph | 4-methyltlziazol-2-yl | i-propyl | |
| 751 | 4-Cl-2-NO₂Ph | 4-methylthiazol-2-yl | cyclopropyl | |
| 752 | 4-Cl-2-NO₂Ph | 4-methylthiazol-2-yl | CF₃ | |
| 753 | 4-Cl-2-NO₂Ph | oxazol-2-yl | H | |
| 754 | 4-Cl-2-NO₂Ph | oxazol-2-yl | methyl | |
| 755 | 4-Cl-2-NO₂Ph | oxazol-2-yl | i-propyl | |
| 756 | 4-Cl-2-NO₂Ph | oxazol-2-yl | cyclopropyl | |
| 757 | 4-Cl-2-NO₂Ph | oxazol-2-yl | CF₃ | |
| 758 | 4-Cl-2-NO₂Ph | 4,5-dimethyloxazol-2-yl | H | |
| 759 | 4-Cl-2-NO₂Ph | 4,5-dimethyloxazol-2-yl | methyl | |
| 760 | 4-Cl-2-NO₂Ph | 4,5-dimethyloxazol-2-yl | i-propyl | |
| 761 | 4-Cl-2-NO₂Ph | 4,5-dimethyloxazol-2-yl | cyclopropyl | |
| 762 | 4-Cl-2-NO₂Ph | 4,5-dimethyloxazol-2-yl | CF₃ | |
| 763 | 4-Cl-2-NO₂Ph | 2-oxazolin-2-yl | H | |
| 764 | 4-Cl-2-NO₂Ph | 2-oxazolin-2-yl | methyl | |
| 765 | 4-Cl-2-NO₂Ph | 2-oxazolin-2-yl | i-propyl | |
| 766 | 4-Cl-2-NO₂Ph | 2-oxazolin-2-yl | cyclopropyl | |
| 767 | 4-Cl-2-NO₂Ph | 2-oxazolin-2-yl | CF₃ | |
| 768 | 4-Cl-2-NO₂Ph | 4,4-dimethyl-2-oxazolin-2-yl | H | |
| 769 | 4-Cl-2-NO₂Ph | 4,4-dimethyl-2-oxazolin-2-yl | methyl | |
| 770 | 4-Cl-2-NO₂Ph | 4,4-dimethyl-2-oxazolin-2-yl | i-propyl | |
| 771 | 4-Cl-2-NO₂Ph | 4,4-dimethyl-2-oxazolin-2-yl | cyclopropyl | |
| 772 | 4-Cl-2-NO₂Ph | 4,4-dimethyl-2-oxazolin-2-yl | CF₃ | |
| 773 | 4-Cl-2-NO₂Ph | 1,2,4-thiadiazol-5-yl | H | |
| 774 | 4-Cl-2-NO₂Ph | 1,2,4-thiadiazol-5-yl | methyl | |
| 775 | 4-Cl-2-NO₂Ph | 1,2,4-thiadiazol-5-yl | i-propyl | |
| 776 | 4-Cl-2-NO₂Ph | 1,2,4-thiadiazol-5-yl | cyclopropyl | |
| 777 | 4-Cl-2-NO₂Ph | 1,2,4-thiadiazol-5-yl | CF₃ | |
| 778 | 4-Cl-2-NO₂Ph | 3-methyl-1,2,4-thiadiazol-5-yl | H | |
| 779 | 4-Cl-2-NO₂Ph | 3-methyl-1,2,4-thiadiazol-5-yl | methyl | |
| 780 | 4-Cl-2-NO₂Ph | 3-methyl-1,2,4-thiadiazol-5-yl | i-propyl | |
| 781 | 4-Cl-2-NO₂Ph | 3-methyl-1,2,4-thiadiazol-5-yl | cyclopropyl | |
| 782 | 4-Cl-2-NO₂Ph | 3-methyl-1,2,4-thiadiazol-5-yl | CF₃ | |
| 783 | 4-Cl-2-NO₂Ph | 3-trifluoromethyl-1,2,4-thiadiazol-5-yl | H | |
| 784 | 4-Cl-2-NO₂Ph | 3-trifluoromemyl-1,2,4-thiadiazol-5-yl | methyl | |
| 785 | 4-Cl-2-NO₂Ph | 3-trifluoromethyl-1,2,4-thiadiazol-5-yl | i-propyl | |
| 786 | 4-Cl-2-NO₂Ph | 3-trifluoromethyl-1,2,4-thiadiazol-5-yl | cyclopropyl | |
| 787 | 4-Cl-2-NO₂Ph | 3-trifluoromethyl-1,2,4-thiadiazol-5-yl | CF₃ | |
| 788 | 4-Cl-2-NO₂Ph | 1,2,4-thiadiazol-3-yl | H | |
| 789 | 4-Cl-2-NO₂Ph | 1,2,4-thiadiazol-3-yl | methyl | |
| 790 | 4-Cl-2-NO₂Ph | 1,2,4-thiadiazol-3-yl | i-propyl | |
| 791 | 4-Cl-2-NO₂Ph | 1,2,4-thiadiazol-3-yl | cyclopropyl | |
| 792 | 4-Cl-2-NO₂Ph | 1,2,4-thiadiazol-3-yl | CF₃ | |
| 793 | 4-Cl-2-NO₂Ph | 5-methyl-1,2,4-thiadiazol-3-yl | H | |
| 794 | 4-Cl-2-NO₂Ph | 5-methyl-1,2,4-thiadiazol-3-yl | methyl | |
| 795 | 4-Cl-2-NO₂Ph | 5-methyl-1,2,4-thiadiazol-3-yl | i-propyl | |
| 796 | 4-Cl-2-NO₂Ph | 5-methyl-1,2,4-thiadiazol-3-yl | cyclopropyl | |
| 797 | 4-Cl-2-NO₂Ph | 5-methyl-1,2,4-thiadiazol-3-yl | CF₃ | |
| 798 | 4-Cl-2-NO₂Ph | 5-tritluoromethyl-1,2,4-thiadiazol-3-yl | H | |
| 799 | 4-Cl-2-NO₂Ph | 5-trifluoromethyl-1,2,4-thiadiazol-3-yl | methyl | |
| 800 | 4-Cl-2-NO₂Ph | 5-trifluoromethyl-1,2,4-thiadiazol-3-yl | i-propyl | |
| 801 | 4-Cl-2-NO₂Ph | 5-trifluoromethyl-1,2,4-thiadiazol-3-yl | cyclopropyl | |
| 802 | 4-Cl-2-NO₂Ph | 5-trifluoromethyl-1,2,4-thiadiazol-3-yl | CF₃ | |
| 803 | 4-Cl-2-NO₂Ph | 1,3,4-thiadiazol-2-yl | H | |
| 804 | 4-Cl-2-NO₂Ph | 1,3,4-thiadiazol-2-yl | methyl | |
| 805 | 4-Cl-2-NO₂Ph | 1,3,4-thiadiazol-2-yl | i-propyl | |
| 806 | 4-Cl-2-NO₂Ph | 1,3,4-thiadiazol-2-yl | cyclopropyl | |
| 807 | 4-Cl-2-NO₂Ph | 1,3,4-thiadiazol-2-yl | CF₃ | |
| 808 | 4-Cl-2-NO₂Ph | 5-methylsulfonyl-1,3,4-thiadiazol-2-yl | H | |
| 809 | 4-Cl-2-NO₂Ph | 5-methylsulfonyl-1,3,4-thiadiazol-2-yl | methyl | |
| 810 | 4-Cl-2-NO₂Ph | 5-methylsulfonyl-1,3,4-thiadiazol-2-yl | i-propyl | |
| 811 | 4-Cl-2-NO₂Ph | 5-methylsulfonyl-1,3,4-thiadiazol-2-yl | cyclopropyl | |
| 812 | 4-Cl-2-NO₂Ph | 5-methylsulfonyl-1,3,4-thiadiazol-2-yl | CF₃ | |
| 813 | 4-Cl-2-NO₂Ph | 5-methyl-1,3,4-thiadiazol-2-yl | H | |
| 814 | 4-Cl-2-NO₂Ph | 5-methyl-1,3,4-thiadiazol-2-yl | methyl | |
| 815 | 4-Cl-2-NO₂Ph | 5-methyl-1,3,4-thiadiazol-2-yl | i-propyl | |
| 816 | 4-Cl-2-NO₂Ph | 5-methyl-1,3,4-thiadiazol-2-yl | cyclopropyl | |
| 817 | 4-Cl-2-NO₂Ph | 5-methyl-1,3,4-thiadiazol-2-yl | CF₃ | |
| 818 | 4-Cl-2-NO₂Ph | benzoxazol-2-yl | H | |
| 819 | 4-Cl-2-NO₂Ph | benzoxazol-2-yl | methyl | |
| 820 | 4-Cl-2-NO₂Ph | benzoxazol-2-yl | i-propyl | |
| 821 | 4-Cl-2-NO₂Ph | benzoxazol-2-yl | cyclopropyl | |
| 822 | 4-Cl-2-NO₂Ph | benzoxazol-2-yl | CF₃ | |
| 823 | 4-Cl-2-NO₂Ph | 6-methylbenzoxazol-2-yl | H | |
| 824 | 4-Cl-2-NO₂Ph | 6-methylbenzoxazol-2-yl | methyl | |
| 825 | 4-Cl-2-NO₂Ph | 6-methylbenzoxazol-2-yl | i-propyl | |
| 826 | 4-Cl-2-NO₂Ph | 6-methylbenzoxazol-2-yl | cyclopropyl | |
| 827 | 4-Cl-2-NO₂Ph | 6-methylbenzoxazol-2-yl | CF₃ | |
| 828 | 4-Cl-2-NO₂Ph | benzothiazol-2-yl | H | |
| 829 | 4-Cl-2-NO₂Ph | benzothiazol-2-yl | methyl | |
| 830 | 4-Cl-2-NO₂Ph | benzothiazol-2-yl | i-propyl | |
| 831 | 4-Cl-2-NO₂Ph | benzothiazol-2-yl | cyclopropyl | |
| 832 | 4-Cl-2-NO₂Ph | benzothiazol-2-yl | CF₃ | |
| 833 | 4-Cl-2-NO₂Ph | pyrazol-1-yl | H | |
| 834 | 4-Cl-2-NO₂Ph | pyrazol-1-yl | methyl | |
| 835 | 4-Cl-2-NO₂Ph | pyrazol-1-yl | i-propyl | |
| 836 | 4-Cl-2-NO₂Ph | pyrazol-1-yl | cyclopropyl | |
| 837 | 4-Cl-2-NO₂Ph | pyrazol-1-yl | CF₃ | |
| 838 | 4-Cl-2-NO₂Ph | pyrazol-3-yl | H | |
| 839 | 4-Cl-2-NO₂Ph | pyrazol-3-yl | methyl | |
| 840 | 4-Cl-2-NO₂Ph | pyrazol-3-yl | i-propyl | |
| 841 | 4-Cl-2-NO₂Ph | pyrazol-3-yl | cyclopropyl | |
| 842 | 4-Cl-2-NO₂Ph | pyrazol-3-yl | CF₃ | |
| 843 | 4-Cl-2-NO₂Ph | 1-methylpyrazol-3-yl | H | |
| 844 | 4-Cl-2-NO₂Ph | 1-methylpyrazol-3-yl | methyl | |
| 845 | 4-Cl-2-NO₂Ph | 1-methylpyrazol-3-yl | i-propyl | |
| 846 | 4-Cl-2-NO₂Ph | 1-methylpyrazol-3-yl | cyclopropyl | |
| 847 | 4-Cl-2-NO₂Ph | 1-methylpyrazol-3-yl | CF₃ | |
| 848 | 4-Cl-2-NO₂Ph | tetrazol-1-yl | H | |
| 849 | 4-Cl-2-NO₂Ph | tetrazol-1-yl | methyl | |
| 850 | 4-Cl-2-NO₂Ph | tebuol-1-yl | i-propyl | |
| 851 | 4-Cl-2-NO₂Ph | tetrazol-1-yl | cyclopropyl | |
| 852 | 4-Cl-2-NO₂Ph | tetrazol-1-yl | CF₃ | |
| 853 | 4-Cl-2-NO₂Ph | 5-methyltetrazol-1-yl | H | |
| 854 | 4-Cl-2-NO₂Ph | 5-methyltebrazol-1-yl | methyl | |
| 855 | 4-Cl-2-NO₂Ph | 5-methyltetrazol-1-yl | i-propyl | |
| 856 | 4-Cl-2-NO₂Ph | 5-methyltetrazol-1-yl | cyclopropyl | |
| 857 | 4-Cl-2-NO₂Ph | 5-methyltetrazol-1-yl | CF₃ | |
| 858 | 4-Cl-2-NO₂Ph | tetrazol-2-yl | H | |
| 859 | 4-Cl-2-NO₂Ph | tetrazol-2-yl | methyl | |
| 860 | 4-Cl-2-NO₂Ph | tetrazol-2-yl | i-propyl | |
| 861 | 4-Cl-2-NO₂Ph | tetrazol-2-yl | cyclopropyl | |
| 862 | 4-Cl-2-NO₂Ph | tetrazol-2-yl | CF₃ | |
| 863 | 4-Cl-2-NO₂Ph | 5-methyltetrazol-2-yl | H | |
| 864 | 4-Cl-2-NO₂Ph | 5-methyltetrazol-2-yl | methyl | |
| 865 | 4-Cl-2-NO₂Ph | 5-methyltetrazol-2-yl | i-propyl | |
| 866 | 4-Cl-2-NO₂Ph | 5-methyltetrazol-2-yl | cyclopropyl | |
| 867 | 4-Cl-2-NO₂Ph | 5-methyltetrazol-2-yl | CF₃ | |
| 868 | 4-Cl-2-NO₂Ph | 1-methyltetrazol-5-yl | H | |
| 869 | 4-Cl-2-NO₂Ph | 1-methyltetrazol-5-yl | methyl | |
| 870 | 4-Cl-2-NO₂Ph | 1-methyltetrazol-5-yl | i-propyl | |
| 871 | 4-Cl-2-NO₂Ph | 1-methyltetrazol-5-yl | CF₃ | |
| 872 | 2-Cl-4-NO₂Ph | 2-methyltetrazol-5-yl | cyclopropyl | 137 |
| 873 | 4-Cl-2-NO₂Ph | 2-methyltetrazol-5-yl | methyl | |
| 874 | 4-Cl-2-NO₂Ph | 2-methyltetrazol-5-yl | i-propyl | |
| 875 | 4-Cl-2-NO₂Ph | 2-methyltetrazol-5-yl | cyclopropyl | 126 |
| 876 | 4-Cl-2-NO₂Ph | 2-methyltetrazol-5-yl | CF₃ | |
| 877 | 2,4-(NO₂)₂Ph | 2-methyltetrazol-5-yl | cyclopropyl | 144 |
| 878 | 4-Cl-2-NO₂Ph | pyridin-2-yl | methyl | |
| 879 | 4-Cl-2-NO₂Ph | pyridin-2-yl | i-propyl | |
| 880 | 4-Cl-2-NO₂h | pyridin-2-yl | cyclopropyl | |
| 881 | 4-Cl-2-NO₂Ph | pyridin-2-yl | CF₃ | |
| 882 | 4-Cl-2-NO₂Ph | pyridin-4-yl | H | |
| 883 | 4-Cl-2-NO₂Ph | pyridin-4-yl | methyl | |
| 884 | 4-Cl-2-NO₂Ph | pyridin-4-yl | i-propyl | |
| 885 | 4-Cl-2-NO₂Ph | pyridin-4-yl | cyclopropyl | |
| 886 | 4-Cl-2-NO₂Ph | pyridin-4-yl | CF₃ | |
| 887 | 4-Cl-2-NO₂Ph | pyridin-3-yl | H | |
| 888 | 4-Cl-2-NO₂Ph | pyridin-3-yl | methyl | |
| 889 | 4-Cl-2-NO₂Ph | pyridin-3-yl | i-propyl | |
| 890 | 4-Cl-2-NO₂Ph | pyridin-3-yl | cyclopropyl | |
| 891 | 4-Cl-2-NO₂Ph | pyridin-3-yl | CF₃ | |
| 892 | 4-Cl-2-NO₂Ph | 3-nitropyndin-4-yl | H | |
| 893 | 4-Cl-2-NO₂Ph | 3-nitropyndin-4-yl | methyl | |
| 894 | 4-Cl-2-NO₂Ph | 3-nitropyridin-4-yl | i-propyl | |
| 895 | 4-Cl-2-NO₂Ph | 3-nitropyridin-4-yl | cyclopropyl | |
| 896 | 4-Cl-2-NO₂Ph | 3-nitropyridin-4-yl | CF₃ | |
| 897 | 4-Cl-2-NO₂Ph | 5-cyanopyridin-2-yl | H | |
| 898 | 4-Cl-2-NO₂Ph | 5-cyanopyridin-2-yl | methyl | |
| 899 | 4-Cl-2-NO₂Ph | 5-cyanopyridin-2-yl | i-propyl | |
| 900 | 4-Cl-2-NO₂Ph | 5-cyanopyridin-2-yl | cyclopropyl | |
| 901 | 4-Cl-2-NO₂Ph | 5-cyanopyridin-2-yl | CF₃ | |
| 902 | 4-Cl-2-NO₂Ph | 5-trifluoromethylpyridin-2-yl | H | |
| 903 | 4-Cl-2-NO₂Ph | 5-trifluoromethylpyridin-2-yl | methyl | |
| 904 | 4-Cl-2-NO₂Ph | 5-trifluoromethylpyridin-2-yl | i-propyl | |
| 905 | 4-Cl-2-NO₂Ph | 5-trifluoromethylpyridin-2-yl | cyclopropyl | |
| 906 | 4-Cl-2-NO₂Ph | 5-trifluoromethylpyridin-2-yl | CF₃ | |
| 907 | 4-Cl-2-NO₂Ph | pyrimidin-2-yl | H | |
| 908 | 4-Cl-2-NO₂Ph | pyrimidin-2-yl | methyl | |
| 909 | 4-Cl-2-NO₂Ph | pyrimidin-2-yl | i-propyl | |
| 910 | 4-Cl-2-NO₂Ph | pyrimidin-2-yl | cyclopropyl | |
| 911 | 4-Cl-2-NO₂Ph | pyrimidin-2-yl | CF₃ | |
| 912 | 4-Cl-2-NO₂Ph | pyrimidin-4-yl | H | |
| 913 | 4-Cl-2-NO₂Ph | pyrimidin-4-yl | methyl | |
| 914 | 4-Cl-2-NO₂Ph | pyrimidin-4-yl | i-propyl | |
| 915 | 4-Cl-2-NO₂Ph | pyrimidin4-yl | cyclopropyl | |
| 916 | 4-Cl-2-NO₂Ph | pyrimidin-4-yl | CF₃ | |
| 917 | 4-Cl-2-NO₂Ph | 6-chloropyrimidin-4-yl | methyl | |
| 918 | 4-Cl-2-NO₂Ph | 6-chloropyrimidin-4-yl | i-propyl | |
| 919 | 4-Cl-2-NO₂Ph | 6-chloropyrimidin-4-yl | cyclopropyl | |
| 920 | 4-Cl-2-NO₂Ph | 6-chloropyrimidin-4-yl | CF₃ | |
| 921 | 2,4-(Cl)₂Ph | 1-methyltetrazol-5-yl | t-butil | 124 |
| 922 | 4-Cl-2-NO₂Ph | pyridazin-3-yl | methyl | |
| 923 | 4-Cl-2-NO₂Ph | pyridazin-3-yl | i-propyl | |
| 924 | 4-Cl-2-NO₂Ph | pyridazin-3-yl | cyclopropyl | |
| 925 | 4-Cl-2-NO₂Ph | pyridazin-3-yl | CF₃ | |
| 926 | 4-Cl-2-NO₂Ph | 6-chloropyridazin-3-yl | methyl | |
| 927 | 4-Cl-2-NO₂Ph | 6-chloropyridazin-3-yl | i-propyl | |
| 928 | 4-Cl-2-NO₂Ph | 6-chloropyridazin-3-yl | cyclopropyl | |
| 929 | 4-Cl-2-NO₂Ph | 6-chloropyridazin-3-yl | CF₃ | |
| 930 | 4-Cl-2-NO₂Ph | pyrazin-2-yl | methyl | |
| 931 | 4-Cl-2-NO₂Ph | pyrazin-2-yl | i-propyl | |
| 932 | 4-Cl-2 NO₂Ph | pyrazin-2-yl | cyclopropyl | |
| 933 | 4-Cl-2-NO₂Ph | pyrazin-2-yl | CF₃ | |
| 934 | 4-Cl-2-NO₂Ph | triazin-2-yl | methyl | |
| 935 | 4-Cl-2-NO₂Ph | triazin-2-yl | i-propyl | |
| 936 | 4-Cl-2-NO₂Ph | triazin-2-yl | cyclopropyl | |
| 937 | 4-Cl-2-NO₂Ph | triazin-2-yl | CF₃ | |
| 938 | 4-Cl-2-NO₂Ph | quinolin-2-yl | methyl | |
| 939 | 4-Cl-2-NO₂Ph | quinolin-2-yl | i-propyl | |
| 940 | 4-Cl-2-NO₂Ph | quinolin-2-yl | cyclopropyl | |
| 941 | 4-Cl-2-NO₂Ph | quinolin-2-yl | CF₃ | |
| 942 | 4-Cl-2-NO₂Ph | 4,4,6-trimethyl-5,6-dihydro-1,3(4H)-oxazin-2-yl | H | |
| 943 | 4-Cl-2-NO₂Ph | 4,4,6-trimethyl-5,6-dihydro-1,3(4H)-oxazin-2-yl | methyl | |
| 944 | 4-Cl-2-NO₂Ph | 4,4,6-trimethyl-5,6-dihydro-1,3(4H)-oxazin-2-yl | i-propyl | |
| 945 | 4-Cl-2-NO₂Ph | 4,4,6-trimethyl-5,6-dihydro-1,3(4H)-oxazin-2-yl | cyclopropyl | |
| 946 | 4-Cl-2-NO₂Ph | 4,4,6-trimethyl-5,6-dihydro-1,3(4H)-oxazin-2-yl | CF₃ | |
| 947 | 4-Cl-2-NO₂Ph | 2-oxazolidinon-3-yl | H | |
| 948 | 4-Cl-2-NO₂Ph | 2-oxazolidinon-3-yl | methyl | |
| 949 | 4-Cl-2-NO₂Ph | 2-oxazolidinon-3-yl | i-propyl | |
| 950 | 4-Cl-2-NO₂Ph | 2-oxazolidinon-3-yl | cyclopropyl | |
| 951 | 4-Cl-2-NO₂Ph | 2-oxazolidinon-3-yl | CF₃ | |
| 952 | 4-Cl-2-NO₂Ph | 2-oxazolidinon-1-yl | methyl | |
| 953 | 4-Cl-2-NO₂Ph | 2-oxazolidinon-1-yl | i-propyl | |
| 954 | 4-Cl-2-NO₂Ph | 2-pyrrolidinon-1-yl | cyclopropyl | |
| 955 | 4-Cl-2-NO₂Ph | 2-pyrrolidinon-1-yl | CF₃ | |
| 956 | 4-Cl-2-NO₂Ph | 3-methylisoxazol-5-yl | methyl | |
| 957 | 4-Cl-2-NO₂Ph | 3-methylisoxazol-5-yl | i-propyl | |
| 958 | 4-Cl-2-NO₂Ph | 3-methylisoxazol-5-yl | cyclopropyl | |
| 959 | 4-Cl-2-NO₂Ph | 3-methylisoxazol-5-yl | CF₃ | |
| 960 | 2-SO₂Me-4-CF₃Ph | 1,2,4-oxadiazol-5-yl | H | |
| 961 | 2-SO₂Me-4-CF₃Ph | 1,2,4-oxadiazol-5-yl | methyl | |
| 962 | 2-SO₂Me-4-CF₃Ph | 1,2,4-oxadiazol-5-yl | i-propyl | |
| 963 | 2-SO₂Me-4-CF₃Ph | 1,2,4-oxadiazol-5-yl | cyclopropyl | |
| 964 | 2-SO₂Me-4-CF₃Ph | 1,2,4-oxadiazol-5-yl | CF₃ | |
| 965 | 2-SO₂Me-4-CF₃Ph | 3-methyl-1,2,4-oxadiazol-5-yl | H | |
| 966 | 2-SO₂Me-4-CF₃Ph | 3-methyl-1,2,4-oxadiazol-5-yl | methyl | |
| 967 | 2-SO₂Me-4-CF₃Ph | 3-methyl-1,2,4-oxadiazol-5-yl | i-propyl | |
| 968 | 2-SO₂Me-4-CF₃Ph | 3-methyl-1,2,4-oxadiazol-5-yl | cyclopropyl | |
| 969 | 2-SO₂Me-4-CF₃Ph | 3-methyl-1,2,4-oxadiazol-5-yl | CF₃ | |
| 970 | 2-SO₂Me-4-CF₃Ph | 3-trifluoromethyl-1,2,4-oxadiazol-5-yl | H | |
| 971 | 2-SO₂Me-4-CF₃Ph | 3-trifluoromethyl-1,2,4-oxadiazol-5-yl | methyl | |
| 972 | 2-SO₂Me-4-CF₃Ph | 3-trifluoromethyl-1,2,4-oxadiazol-5-yl | i-propyl | |
| 973 | 2-SO₂Me-4-CF₃Ph | 3-trifluoromethyl-1,2,4-oxadiazol-5-yl | cyclopropyl | |
| 974 | 2-SO₂Me-4-CF₃Ph | 3 trifluoromethyl-1,2,4-oxadiazol-5-yl | CF₃ | |
| 975 | 2-SO₂Me-4-CF₃Ph | 1,2,4-oxadiazol-3-yl | H | |
| 976 | 2-SO₂Me-4-CF₃Ph | 1,2,4-oxadiazol-3-yl | methyl | |
| 977 | 2-SO₂Me-4-CF₃Ph | 1,2,4-oxadiazol-3-yl | i-propyl | |
| 978 | 2-SO₂Me-4-CF₃Ph | 1,2,4-oxadiazol-3-yl | cyclopropyl | |
| 979 | 2-SO₂Me-4-CF₃Ph | 1,2,4-oxadiazol-3-yl | CF₃ | |
| 980 | 2-SO₂Me-4-CF₃Ph | 5-methyl-1,2,4-oxadiazol-3-yl | H | |
| 981 | 2-SO₂Me-4-CF₃Ph | 5-methyl-1,2,4-oxadiazol-3-yl | methyl | |
| 982 | 2-SO₂Me-4-CF₃Ph | 5-methyl-1,2,4-oxadiazol-3-yl | i-propyl | |
| 983 | 2-SO₂Me-4-CF₃Ph | 5-methyl-1,2,4-oxadiazol-3-yl | cyclopropyl | |
| 984 | 2-SO₂Me-4-CF₃Ph | 5-methyl-1,2,4-oxadiazol-3-yl | CF₃ | |
| 985 | 2-SO₂Me-4-CF₃Ph | 5-trifluoromethyl-1,2,4-oxadiazol-3-yl | H | |
| 986 | 2-SO₂Me-4-CF₃Ph | 5 trifluoromethyl-1,2,4-oxadiazol-3-yl | methyl | |
| 987 | 2-SO₂Me-4-CF₃Ph | 5-trifluoromethyl-1,2,4-oxadiazol-3-yl | i-propyl | |
| 988 | 2-SO₂Me-4-CF₃Ph | 5-trifluoromethyl-1.2,4-oxadiazol-3-yl | cyclopropyl | |
| 989 | 2-SO₂Me-4-CF₃Ph | 5-tritluoromethyl-1,2,4-oxadiazol-3-yl | CF₃ | |
| 990 | 2-SO₂Me-4-CF₃Ph | 5-chloro-1,2,4-oxadiazol-3-yl | H | |
| 991 | 2-SO₂Me-4-CF₃Ph | 5-chloro-1,2,4-oxadiazol-3-yl | methyl | |
| 992 | 2-SO₂Me-4-CF₃Ph | 5-chloro-1,2,4-oxadiazol-3-yl | i-propyl | |
| 993 | 2-SO₂Me-4-CF₃Ph | 5-chloro-1,2,4-oxadiazol-3-yl | cyclopropyl | |
| 994 | 2-SO₂Me-4-CF₃Ph | 5-chloro-1,2,4-oxadiazol-3-yl | CF₃ | |
| 995 | 2-SO₂Me-4-CF₃Ph | 1,3,4-oxadiazol-2-yl | H | |
| 996 | 2-SO₂Me-4-CF₃Ph | 1,3,4-oxadiazol-2-yl | methyl | |
| 997 | 2-SO₂Me-4-CF₃Ph | 1,3,4-oxadiazol-2-yl | i-propyl | |
| 998 | 2-SO₂Me-4-CF₃Ph | 1,3,4-oxadiazol-2-yl | cyclopropyl | |
| 999 | 2-SO₂Me-4-CF₃Ph | 1,3,4-oxadiazol-2-yl | CF₃ | |
| 1000 | 2-SO₂Me-4-CF₃Ph | 5-methylsulfonyl-1,3,4-oxadiazol-2-yl | H | |
| 1001 | 2-SO₂Me4-CF₃Ph | 5-methylsulfonyl-1,3,4-oxadiazol-2-yl | methyl | |
| 1002 | 2-SO₂Me-4-CF₃Ph | 5-methylsulfonyl-1,3,4-oxadiazol-2-yl | i-propyl | |
| 1003 | 2-SO₂Me-4-CF₃Ph | 5-methylsulfonyl-1,3,4-oxadiazol-2-yl | cyclopropyl | |
| 1004 | 2-SO₂Me-4-CF₃Ph | 5-methylsulfonyl-1,3,4-oxadiazol-2-yl | CF₃ | |
| 1005 | 2-SO₂Me-4-CF₃Ph | 5-methyl-1,3,4-oxadiazol-2-yl | H | |
| 1006 | 2-SO₂Me-4-CF₃Ph | 5-methyl-1,3,4-oxadiazol-2-yl | methyl | |
| 1007 | 2-SO₂Me-4-CF₃Ph | 5-methyl-1,3,4-oxadiazol-2-yl | i-propyl | |
| 1008 | 2-SO₂Me-4-CF₃Ph | 5-methyl-1,3,4-oxadiazol-2-yl | cyclopropyl | |
| 1009 | 2-SO₂Me-4-CF₃Ph | 5-methyl-1,3,4-oxadiazol-2-yl | CF₃ | |
| 1010 | 2-SO₂Me-4-CF₃Ph | 5-trifluoromethyl-1,3,4-oxadiazol-2-yl | H | |
| 1011 | 2-SO₂Me-4-CF₃Ph | 5-trifluoromethyl-1,3,4-oxadiazol-2-yl | methyl | |
| 1012 | 2-SO₂Me-4-CF₃Ph | 5-trifluoromethyl-1,3,4-oxadiazol-2-yl | i-propyl | |
| 1013 | 2-SO₂Me-4-CF₃Ph | 5-trifluoromethyl-1,3,4-oxadiazol-2-yl | cyclopropyl | |
| 1014 | 2-SO₂Me-4-CF₃Ph | 5-trifluoromethyl-1,3,4-oxadiazol-2-yl | CF₃ | |
| 1015 | 2-SO₂Me-4-CF₃Ph | 1,2,3-triazol-4-yl | H | |
| 1016 | 2-SO₂Me-4-CF₃Ph | 1,2,3-triazol-4-yl | methyl | |
| 1017 | 2-SO₂Me-4-CF₃Ph | 1,2,3-triazol-4-yl | i-propyl | |
| 1018 | 2-SO₂Me-4-CF₃Ph | 1,2,3-triazol-4-yl | cyclopropyl | |
| 1019 | 2-SO₂Me-4-CF₃Ph | 1,2,3-triazol-4-yl | CF₃ | |
| 1020 | 2-SO₂Me-4-CF₃Ph | 1-methyl-1,2,3-triazol-4-yl | H | |
| 1021 | 2-SO₂Me-4-CF₃Ph | 1-methyl-1,2,3-triazol-4-yl | methyl | |
| 1022 | 2-SO₂Me-4-CF₃Ph | 1-methyl-1,2,3-triazol-4-yl | i-propyl | |
| 1023 | 2-SO₂Me-4-CF₃Ph | 1-methyl-1,2,3-triazol-4-yl | cyclopropyl | |
| 1024 | 2-SO₂Me-4-CF₃Ph | 1-methyl-1,2,3-triazol-4-yl | CF₃ | |
| 1025 | 2-SO₂Me-4-CF₃Ph | 2-methyl-1,2,3-triazol-4-yl | H | |
| 1026 | 2-SO₂Me-4-CF₃Ph | 2-methyl-1,2,3-triazol-4-yl | methyl | |
| 1027 | 2-SO₂Me-4-CF₃Ph | 2-methyl-1,2,3-triazol-4-yl | i-propyl | |
| 1028 | 2-SO₂Me-4-CF₃Ph | 2-methyl-1,2,3-triazol-4-yl | cyclopropyl | |
| 1029 | 2-SO₂Me-4-CF₃Ph | 2-methyl-1,2,3-triazol-4-yl | CF₃ | |
| 1030 | 2-SO₂Me-4-CF₃Ph | 1,2,3-triazol-1-yl | H | |
| 1031 | 2-SO₂Me-4-CF₃Ph | 1,2,3-triazol-1-yl | methyl | |
| 1032 | 2-SO₂Me-4-CF₃Ph | 1,2,3-triazol-1-yl | i-propyl | |
| 1033 | 2-SO₂Me-4-CF₃Ph | 1,2,3-triazol-1-yl | cyclopropyl | |
| 1034 | 2-SO₂Me-4-CF₃Ph | 1,2,3-triazol-1-yl | CF₃ | |
| 1035 | 2-SO₂Me-4-CF₃Ph | 1,2,3-triazol-2-yl | H | |
| 1036 | 2-SO₂Me-4-CF₃Ph | 1,2,3-triazol-2-yl | methyl | |
| 1037 | 2-SO₂Me-4-CF₃Ph | 1,2,3-triazol-2-yl | i-propyl | |
| 1038 | 2-SO₂Me-4-CF₃Ph | 1,2,3-triazol-2-yl | cyclopropyl | |
| 1039 | 2-SO₂Me-4-CF₃Ph | 1,2,3-triazol-2-yl | CF₃ | |
| 1040 | 2-SO₂Me-4-CF₃Ph | 1,2,4-triazol-1-yl | H | |
| 1041 | 2-SO₂Me-4-CF₃Ph | 1,2,4-triazol-1-yl | methyl | |
| 1042 | 2-SO₂Me-4-CF₃Ph | 1,2,4-triazol-1-yl | i-propyl | |
| 1043 | 2-SO₂Me-4-CF₃Ph | 1,2,4-triazol-1-yl | cyclopropyl | |
| 1044 | 2-SO₂Me-4-CF₃Ph | 1,2,4-triazol-1-yl | CF₃ | |
| 1045 | 2-SO₂Me-4-CF₃Ph | imidazol-2-yl | H | |
| 1046 | 2-SO₂Me-4-CF₃Ph | imidazol-2-yl | methyl | |
| 1047 | 2-SO₂Me-4-CF₃Ph | imidazol-2-yl | i-propyl | |
| 1048 | 2-SO₂Me-4-CF₃Ph | imidazol-2-yl | cyclopropyl | |
| 1049 | 2-SO₂Me-4-CF₃Ph | imidazol-2-yl | CF₃ | |
| 1050 | 2-SO₂Me-4-CF₃Ph | imidazol-1-yl | H | |
| 1051 | 2-SO₂Me-4-CF₃Ph | imidazol-1-yl | methyl | |
| 1052 | 2-SO₂Me-4-CF₃Ph | imidazol-1-yl | i-propyl | |
| 1053 | 2-SO₂Me-4-CF₃Ph | imidazol-1-yl | cyclopropyl | |
| 1054 | 2-SO₂Me-4-CF₃Ph | imidazol-1-yl | CF₃ | |
| 1055 | 2-SO₂Me-4-CF₃Ph | imidazol-4-yl | H | |
| 1056 | 2-SO₂Me-4-CF₃Ph | imidazol-4-yl | methyl | |
| 1057 | 2-SO₂Me-4-CF₃Ph | imidazol-4-yl | i-propyl | |
| 1058 | 2-SO₂Me-4-CF₃Ph | imidazol-4-yl | cyclopropyl | |
| 1059 | 2-SO₂Me-4-CF₃Ph | imidazol-4-yl | CF₃ | |
| 1060 | 2-SO₂Me-4-CF₃Ph | thiazol-2-yl | H | |
| 1061 | 2-SO₂Me-4-CF₃Ph | thizazol-2-yl | methyl | |
| 1062 | 2-SO₂Me-4-CF₃Ph | thiazol-2-yl | i-propyl | |
| 1063 | 2-SO₂Me-4-CF₃Ph | thiazol-2-yl | cyclopropyl | |
| 1064 | 2-SO₂Me-4-CF₃Ph | thiazol-2-yl | CF₃ | |
| 1065 | 2-SO₂Me-4-CF₃Ph | 4-methylthiazol-2-yl | H | |
| 1066 | 2-SO₂Me-4-CF₃Ph | 4-methylthiazol-2-yl | methyl | |
| 1067 | 2-SO₂Me-4-CF₃Ph | 4-methylthiazol-2-yl | i-propyl | |
| 1068 | 2-SO₂Me-4-CF₃Ph | 4-methylthiazol-2-yl | cyclopropyl | |
| 1069 | 2-SO₂Me-4-CF₃Ph | 4-methylthiazol-2-yl | CF₃ | |
| 1070 | 2-SO₂Me-4-CF₃Ph | oxazol-2-yl | H | |
| 1071 | 2-SO₂Me-4-CF₃Ph | oxazol-2-yl | methyl | |
| 1072 | 2-SO₂Me-4-CF₃Ph | oxazol-2-yl | i-propyl | |
| 1073 | 2-SO₂Me-4-CF₃Ph | oxazol-2-yl | cyclopropyl | |
| 1074 | 2-SO₂Me-4-CF₃Ph | oxazol-2-yl | CF₃ | |
| 1075 | 2-SO₂Me-4-CF₃Ph | 4,5-dimethyloxazol-2-yl | H | |
| 1076 | 2-SO₂Me-4-CF₃Ph | 4,5-dimethyloxazol-2-yl | methyl | |
| 1077 | 2-SO₂Me-4-CF₃Ph | 4,5-dimethyloxazol-2-yl | i-propyl | |
| 1078 | 2-SO₂Me-4-CF₃Ph | 4,5-dimethyloxazol-2-yl | cyclopropyl | |
| 1079 | 2-SO₂Me-4-CF₃Ph | 4,5-dimethyloxazol-2-yl | CF₃ | |
| 1080 | 2-SO₂Me-4-CF₃Ph | 2-oxazolin-2-yl | H | |
| 1081 | 2-SO₂Me-4-CF₃Ph | 2-oxazolin-2-yl | methyl | |
| 1082 | 2-SO₂Me-4-CF₃Ph | 2-oxazolin-2-yl | i-propyl | |
| 1083 | 2-SO₂Me-4-CF₃Ph | 2-oxazolin-2-yl | cyclopropyl | |
| 1084 | 2-SO₂Me-4-CF₃Ph | 2-oxazolin-2-yl | CF₃ | |
| 1085 | 2-SO₂Me-4-CF₃Ph | 4,4-dimethyl-2-oxazolin-2-yl | H | |
| 1086 | 2-SO₂Me-4-CF₃Ph | 4,4-dimethyl-2-oxazolin-2-yl | methyl | |
| 1087 | 2-SO₂Me-4-CF₃Ph | 4,4-dimethyl-2-oxazolin-2-yl | i-propyl | |
| 1088 | 2-SO₂Me-4-CF₃Ph | 4,4-dimethyl-2-oxazolin-2-yl | cyclopropyl | |
| 1089 | 2-SO₂Me-4-CF₃Ph | 4,4-dimethyl-2-oxazolin-2-yl | CF₃ | |
| 1090 | 2-SO₂Me-4-CF₃Ph | 1,2,4-thiadiazol-5-yl | H | |
| 1091 | 2-SO₂Me-4-CF₃Ph | 1,2,4-thiadiazol-5-yl | methyl | |
| 1092 | 2-SO₂Me-4-CF₃Ph | 1,2,4-thiadiazol-5-yl | i-propyl | |
| 1093 | 2-SO₂Me-4-CF₃Ph | 1,2,4-thiadiazol-5-yl | cyclopropyl | |
| 1094 | 2-SO₂Me-4-CF₃Ph | 1,2,4-thiadiazol-5-yl | CF₃ | |
| 1095 | 2-SO₂Me-4-CF₃Ph | 3-methyl-1,2,4-thiadiazol-5-yl | H | |
| 1096 | 2-SO₂Me-4-CF₃Ph | 3-methyl-1,2,4-thiadiazol-5-yl | methyl | |
| 1097 | 2-SO₂Me-4-CF₃Ph | 3-methyl-1,2,4-thiadiazol-5-yl | i-propyl | |
| 1098 | 2-SO₂Me-4-CF₃Ph | 3-methyl-1,2,4-thiadiazol-5-yl | cyclopropyl | |
| 1099 | 2-SO₂Me-4-CF₃Ph | 3-methyl-1,2,4-thiadiazol-5-yl | CF₃ | |
| 1100 | 2-SO₂Me-4-CF₃Ph | 3-triffuoromethyl-1,2,4-thiadiazol=5-yl | H | |
| 1101 | 2-SO₂Me-4-CF₃Ph | 3-triffuoromethyl-1,2,4-thiadiazol-5-yl | methyl | |
| 1102 | 2-SO₂Me-4-CF₃Ph | 3-trifluoromethyl-1,2,4-thiadiazol-5-yl | i-propyl | |
| 1103 | 2-SO₂Me-4-CF₃Ph | 3-trifluoromethyl-1,2,4-thiadiazol-5-yl | cyclopropyl | |
| 1104 | 2-SO₂Me-4-CF₃Ph | 3-trifluoromethyl-1,2,4-thiadiazol-5-yl | CF₃ | |
| 1105 | 2-SO₂Me-4-CF₃Ph | 1,2,4-thiadiazol-3-yl | H | |
| 1106 | 2-SO₂Me-4-CF₃Ph | 1,2,4-thiadiazol-3-yl | methyl | |
| 1107 | 2-SO₂Me-4-CF₃Ph | 1,2,4-thiadiazol-3-yl | i-propyl | |
| 1108 | 2-SO₂Me-4-CF₃Ph | 1,2,4-thiadiazol-3-yl | cyclopropyl | |
| 1109 | 2-SO₂Me-4-CF₃Ph | 1,2,4-thiadiazol-3-yl | CF₃ | |
| 1110 | 2-SO₂Me-4-CF₃Ph | 5-methyl-1,2,4-thiadiazol-3-yl | H | |
| 1111 | 2-SO₂Me-4-CF₃Ph | 5-methyl-1,2,4-thiadiazol-3-yl | methyl | |
| 1112 | 2-SO₂Me-4-CF₃Ph | 5-methyl-1,2,4-thiadiazol-3-yl | i-propyl | |
| 1113 | 2-SO₂Me-4-CF₃Ph | 5-methyl-1,2,4-thiadiazol-3-yl | cyclopropyl | |
| 1114 | 2-SO₂Me-4-CF₃Ph | 5-methyl-1,2,4-thiadiazol-3-yl | CF₃ | |
| 1115 | 2-SO₂Me-4-CF₃Ph | 5-trifluoromethyl-1,2,4-thiadiazol-3-yl | H | |
| 1116 | 2-SO₂Me-4-CF₃Ph | 5-trifluoromethyl-1,2,4-thianazol-3-yl | methyl | |
| 1117 | 2-SO₂Me-4-CF₃Ph | 5-trifluoromethyl-1,2,4-thiadiazol-3-yl | i-propyl | |
| 1118 | 2-SO₂Me-4-CF₃Ph | 5-trifluoromethyl-1,2,4-thiadiazol-3-yl | cyclopropyl, | |
| 1119 | 2-SO₂Me-4-CF₃Ph | 5-trifluoromethyl-1,2,4-thiadiazol-3-yl | CF₃ | |
| 1120 | 2-SO₂Me-4-CF₃Ph | 1,3,4-thiadiazol-2-yl | H | |
| 1121 | 2-SO₂Me-4-CF₃Ph | 1,3,4-thiadiazol-2-yl | methyl | |
| 1122 | 2-SO₂Me-4-CF₃Ph | 1,3,4-thiadiazol-2-yl | i-propyl | |
| 1123 | 2-SO₂Me-4-CF₃Ph | 1,3,4-thiadiazol-2-yl | cyclopropyl | |
| 1124 | 2-SO₂Me-4-CF₃Ph | 1,3,4-thiadiazol-2-yl | CF₃ | |
| 1125 | 2-SO₂Me-4-CF₃Ph | 5-methylsulfonyl-1,3,4-thiadiazol-2-yl | H | |
| 1126 | 2-SO₂Me-4-CF₃Ph | 5-methylsulfonyl-1,3,4-thiadiazol-2-yl | methyl | |
| 1127 | 2-SO₂Me-4-CF₃Ph | 5-methylsulfonyl-1,3,4-thiadiazol-2-yl | i-propyl | |
| 1128 | 2-SO₂Me-4-CF₃Ph | 5-methylsulfonyl-1,3,4-thiadiazol-2-yl | cyclopropyl | |
| 1129 | 2-SO₂Me-4-CF₃Ph | 5-methylsulfonyl-1,3,4-thiadiazol-2-yl | CF₃ | |
| 1130 | 2-SO₂Me-4-CF₃Ph | 5-methyl-1,3,4-thiadiazol-2-yl | H | |
| 1131 | 2-SO₂Me-4-CF₃Ph | 5-methyl-1,3,4-thiadiazol-2-yl | methyl | |
| 1132 | 2-SO₂Me-4-CF₃Ph | 5-methyl-1,3,4-thiadiazol-2-yl | i-propyl | |
| 1133 | 2-SO₂Me-4-CF₃Ph | 5-methyl-1,3,4-thiadiazol-2-yl | cyclopropyl | |
| 1134 | 2-SO₂Me-4-CF₃Ph | 5-methyl-1,3,4-thiadiazol-2-yl | CF₃ | |
| 1135 | 2-SO₂Me-4-CF₃Ph | benzoxazol-2-yl | H | |
| 1136 | 2-SO₂Me-4-CF₃Ph | benzoxazol-2-yl | methyl | |
| 1137 | 2-SO₂Me-4-CF₃Ph | benzoxazol-2-yl | i-propyl | |
| 1138 | 2-SO₂Me-4-CF₃Ph | benzoxazol-2-yl | cyclopropyl | |
| 1139 | 2-SO₂Me-4-CF₃Ph | benzoxazol-2-yl | CF₃ | |
| 1140 | 2-SO₂Me-4-CF₃Ph | 6-methylbenzoxazol-2-yl | H | |
| 1141 | 2-SO₂Me-4-CF₃Ph | 6-methylbenzoxazol-2-yl | methyl | |
| 1142 | 2-SO₂Me-4-CF₃Ph | 6-methylbenzoxazol-2-yl | i-propyl | |
| 1143 | 2-SO₂Me-4-CF₃Ph | 6-methylbenzoxazol-2-yl | cyclopropyl | |
| 1144 | 2-SO₂Me-4-CF₃Ph | 6-methylbenzoxazol-2-yl | CF₃ | |
| 1145 | 2-SO₂Me-4-CF₃Ph | benzothiazol-2-yl | H | |
| 1146 | 2-S0₂Me-4-CF₃Ph | benzothiazol-2-yl | methyl | |
| 1147 | 2-SO₂Me-4-CF₃Ph | benzotbiazol-2-yl | i-propyl | |
| 1148 | 2-SO₂Me-4-CF₃Ph | benzothiazol-2-yl | cyclopropyl | |
| 1149 | 2-SO₂Me-4-CF₃Ph | benzothiazol-2-yl | CF₃ | |
| 1150 | 2-SO₂Me-4-CF₃Ph | pyrazol-1-yl | H | |
| 1151 | 2-SO₂Me-4-CF₃Ph | pyrazol-1-yl | methyl | |
| 1152 | 2-SO₂Me-4-CF₃Ph | pyrazol-1-yl | i-propyl | |
| 1153 | 2-SO₂Me-4-CF₃Ph | pyrazol-1-yl | cyclopropyl | |
| 1154 | 2-SO₂Me-4-CF₃Ph | pyrazol-1-yl | CF₃ | |
| 1155 | 2-SO₂Me-4-CF₃Ph | pyrazol-3-yl | H | |
| 1156 | 2-SO₂Me-4-CF₃Ph | pyrazol-3-yl | methyl | |
| 1157 | 2-SO₂Me-4-CF₃Ph | pyrazol-3-yl | i-propyl | |
| 1158 | 2-SO₂Me-4-CF₃Ph | pyrazol-3-yl | cyclopropyl | |
| 1159 | 2-SO₂Me-4-CF₃Ph | pyrazol-3-yl | CF₃ | |
| 1160 | 2-SO₂Me-4-CF₃Ph | I-methylpyrazol-3-yl | H | |
| 1161 | 2-SO₂Me-4-CF₃Ph | 1-methylpyrazol-3-yl | methyl | |
| 1162 | 2-SO₂Me-4-CF₃Ph | 1-methylpyrazol-3-yl | i-propyl | |
| 1163 | 2-SO₂Me-4-CF₃Ph | 1-methylpyrazol-3-yl | cyclopropyl | |
| 1164 | 2-SO₂Me-4-CF₃Ph | 1-methylpyrazol-3-yl | CF₃ | |
| 1165 | 2-SO₂Me-4-CF₃Ph | tetrazol-1-yl | H | |
| 1166 | ¨2-SO₂Me-4-CF₃Ph | tetrazol-1-yl | methyl | |
| 1167 | 2-SO₂Me-4-CF₃Ph | tetrazol-1-yl | i-propyl | |
| 1168 | 2-SO₂Me-4-CF₃Ph | tetrazol-1-yl | cyclopropyl | |
| 1169 | 2-SO₂Me-4-CF₃Ph | tetrazol-1-yl | CF₃ | |
| 1170 | 2-SO₂Me-4-CF₃Ph | 5-methyltetrazol-1-yl | H | |
| 1171 | 2-SO₂Me-4-CF₃Ph | 5-methyltetrazol-1-yl | methyl | |
| 1172 | 2-SO₂Me-4-CF₃Ph | 5-methyltetrazol-1-yl | i-propyl | |
| 1173 | 2-SO₂Me-4-CF₃Ph | 5-methyltetrazol-1-yl | cyclopropyl | |
| 1174 | 2-SO₂Me-4-CF₃Ph | 5-methyltetrazol-1-yl | CF₃ | |
| 1175 | 2-SO₂Me-4-CF₃Ph | tetrazol-2-yl | H | |
| 1176 | 2-SO₂Me-4-CF₃Ph | tetrazol-2-yl | methyl | |
| 1177 | 2 SO₂Me-4-CF₃Ph | tetrazol-2-yl | i-propyl | |
| 1178 | 2-SO₂Me-4-CF₃Ph | tetrazol-2-yl | cyclopropyl | |
| 1179 | 2-SO₂Me-4-CF₃Ph | tetrazol-2-yl | CF₃ | |
| 1180 | 2-SO₂Me-4-CF₃Ph | 5-methyltetrazol-2-yl | H | |
| 1181 | 2-SO₂Me-4-CF₃Ph | 5-methyltetrazol-2-yl | methyl | |
| 1182 | 2-SO₂Me-4-CF₃Ph | 5-methyltetrazol-2-yl | i-propyl | |
| 1183 | 2-SO₂Me-4-CF₃Ph | 5-methyltetrazol-2-yl | cyclopropyl | |
| 1184 | 2-SO₂Me-4-CF₃Ph | 5-methyltetrazol-2-yl | CF₃ | |
| 1185 | 2-SO₂Me-4-CF₃Ph | 1-methyltetrazol-5-yl | H | |
| 1186 | 2-SO₂Me-4-CF₃Ph | 1-methyltetrazol-5-yl | methyl | |
| 1187 | 2-SO₂Me-4-CF₃Ph | 1-methyltetrazol-5-yl | i-propyl | |
| 1188 | 2-SO₂Me-4-CF₃Ph | 1-methyltetrazol-5-yl | cyclopropyl | |
| 1189 | 2-SO₂Me-4-CF₃Ph | 1-methyltetraaol-5-yl | CF₃ | |
| 1190 | 2-SO₂Me-4-CF₃Ph | 2-methyltetrazol-5-yl | H | |
| 1191 | 2-SO₂Me-4-CF₃Ph | 2-methyltetrazol-5-yl | methyl | |
| 1192 | 2-SO₂Me-4-CF₃Ph | 2-methyltetrazol-5-yl | i-propyl | |
| 1193 | 2-SO₂Me-4-CF₃Ph | 2-methyltetrazol-5-yl | cyclopropyl | 157 |
| 1194 | 2-SO₂Me-4-CF₃Ph | 2-methyltetrazol-5-yl | CF₃ | |
| 1195 | 2-SO₂Me-4-CF₃Ph | pyridin-2-yl | H | |
| 1196 | 2-SO₂Me-4-CF₃Ph | pyridin-2-yl | methyl | |
| 1197 | 2-SO₂Me-4-CF₃Ph | pyridin-2-yl | i-propyl | |
| 1198 | 2-SO₂Me-4-CF₃Ph | pyridin-2-yl | cyclopropyl | |
| 1199 | 2-SO₂Me-4-CF₃Ph | pyridin-2-yl | CF₃ | |
| 1200 | 2-SO₂Me-4-CF₃Ph | pyridin-4-yl | H | |
| 1201 | 2-SO₂Me-4-CF₃Ph | pyridin-4-yl | methyl | |
| 1202 | 2-SO₂Me-4-CF₃Ph | pyridin-4-yl | i-propyl | |
| 1203 | 2-SO₂Me-4-CF₃Ph | pyridin-4-yl | cyclopropyl | |
| 1204 | 2-SO₂Me-4-CF₃Ph | pyridin-4-yl | CF₃ | |
| 1205 | 2-SO₂Me-4-CF₃Ph | pyridin-3-yl | H | |
| 1206 | 2-SO₂Me-4-CF₃Ph | pyridin-3-yl | methyl | |
| 1207 | 2-SO₂Me-4-CF₃Ph | pyridin-3-yl | i-propyl | |
| 1208 | 2-SO₂Me-4-CF₃Ph | pyridin-3-yl | cyclopropyl | |
| 1209 | 2-SO₂Me-4-CF₃Ph | pyridin-3-yl | CF₃ | |
| 1210 | 2-SO₂Me-4-CF₃Ph | 3 nitropyridin-4-yl | H | |
| 1211 | 2-SO₂Me-4-CF₃Ph | 3-nitropyndin-4-yl | methyl | |
| 1212 | 2-SO₂Me-4-CF₃Ph | 3 nitropyridin-4-yl | i-propyl | |
| 1213 | 2-SO₂Me-4-CF₃Ph | 3-nitropyridin-4-yl | cyclopropyl | |
| 1214 | 2-SO₂Me-4-CF₃Ph | 3-nitropyridin-4-yl | CF₃ | |
| 1215 | 2-SO₂Me-4-CF₃Ph | 5-cyanopyridin-2-yl | H | |
| 1216 | 2-SO₂Me-4-CF₃Ph | 5-cyanopyridin-2-yl | methyl | |
| 1217 | 2-SO₂Me-4-CF₃Ph | 5-cyanopyridin-2-yl | i-propyl | |
| 1218 | 2-SO₂Me-4-CF₂Ph | 5-cyanopyridin-2-yl | cyclopropyl | |
| 1219 | 2-SO₂Me-4-CF₃Ph | 5-cyanopyridin-2-yl | CF₃ | |
| 1220 | 2-SO₂Me-4-CF₃Ph | 5-trifluoromethylpyridin-2-yl | H | |
| 1221 | 2-SO₂Me-4-CF₃Ph | 5-trifluoromethylpyridin-2-yl | methyl | |
| 1222 | 2-SO₂Me-4-CF₃Ph | 5-triffuoromethylpyridin-2-yl | i-propyl | |
| 1223 | 2-SO2Me-4-CF₃Ph | 5-trifluoromethylpyridin-2-yl | cyclopropyl | |
| 1224 | 2-SO₂Me-4-CF₃Ph | 5-trifluoromethylpyridin-2-yl | CF₃ | |
| 1225 | 2-SO₂Me-4-CF₃Ph | pytimidin-2-yl | H | |
| 1226 | 2-SO₂Me-4-CF₃Ph | pyrimidin-2-yl | methyl | |
| 1227 | 2-SO₂Me-4-CF₃Ph | pyrimidin-2-yl | i-propyl | |
| 1228 | 2-SO₂Me-4-CF₃Ph | pyrimidin-2-yl | cyclopropyl | |
| 1229 | 2-S0₂Me-4-CF₃Ph | pyrimidin-2-yl | CF₃ | |
| 1230 | 2-SO₂Me-4-CF₃Ph | pyrimidin-4-yl | H | |
| 1231 | 2-SO₂Me-4-CF₃Ph | pyrimidin-4-yl | methyl | |
| 1232 | 2-SO₂Me-4-CF₃Ph | pyrimidin-4-yl | i-propyl | |
| 1233 | 2-SO₂Me-4-CF₃Ph | pyrimidin-4-yl | cyclopropyl | |
| 1234 | 2-SO₂Me-4-CF₃Ph | pyrimidin-4-yl | CF₃ | |
| 1235 | 2-SO₂Me-4-CF₃Ph | 6-chloropyrimidin-4-yl | methyl | |
| 1236 | 2-SO₂Me-4-CF₃Ph | 6-chloropyrimidin-4-yl | i-propyl | |
| 1237 | 2-SO₂Me-4-CF₃Ph | 6-chloropyrimiciin4-yl | cyclopropyl | |
| 1238 | 2-SO₂Me-4-CF₃Ph | 6-chloropyrimidin-4-yl | CF₃ | |
| 1239 | 2-SO₂Me-4-CF₃Ph | pyridazin 3-yl | H | |
| 1240 | 2-SO₂Me-4-CF₃Ph | pyridazin-3-yl | methyl | |
| 1241 | 2-SO₂Me-4-CF₃Ph | pyridazin-3-yl | i-propyl | |
| 1242 | 2-SO₂Me-4-CF₃Ph | pyridazin-3-yl | cyclopropyl | |
| 1243 | 2-SO₂Me-4-CF₃Ph | pyridazin-3-yl | CF₃ | |
| 1244 | 2-SO₂Me-4-CF₃Ph | 6-chloropyridazin-3-yl | methyl | |
| 1245 | 2-SO₂Me-4-CF₃Ph | 6-chloropyridazin-3-yl | i-propyl | |
| 1246 | 2-SO₂Me-4-CF₃Ph | 6-chloropyridazin-3-yl | cyclopropyl | |
| 1247 | 2-SO₂Me-4-CF₃Ph | 6-chloropyridazin-3-yl | CF₃ | |
| 1248 | 2-SO₂Me-4-CF₃Ph | pyrazin-2-yl | methyl | |
| 1249 | 2-SO₂Me-4-CF₃Ph | pyrazin-2-yl | i-propyl | |
| 1250 | 2-SO₂Me-4-CF₃Ph | pyrazin-2-yl | cyclopropyl | |
| 1251 | 2-SO₂Me-4-CF₃Ph | pyrazin-2-yl | CF₃ | |
| 1252 | 2-SO₂Me-4-CF₃Ph | triazin-2-yl | methyl | |
| 1253 | 2-SO₂Me-4-CF₃Ph | triazin-2-yl | i-propyl | |
| 1254 | 2-SO₂Me-4-CF₃Ph | triazin-2-yl | cyclopropyl | |
| 1255 | 2-SO₂Me-4-CF₃Ph | triazin-2-yl | CF₃ | |
| 1256 | 2-SO₂Me-4-CF₃Ph | quinolin-2-yl | methyl | |
| 1257 | 2-SO₂Me-4-CF₃Ph | quinolin-2-yl | i-propyl | |
| 1258 | 2-SO₂Me-4-CF₃Ph | quinolin-2-yl | cyclopropyl | |
| 1259 | 2-SO₂Me-4-CF₃Ph | quinolin-2-yl | CF₃ | |
| 1260 | 2-SO₂Me-4-CF₃Ph | 4,4,6-trimethyl-5,6-dihydro-1,3(4H)-oxazin-2-yl | H | |
| 1261 | 2-SO₂Me-4-CF₃Ph | 4,4,6-trimethyl-5,6-dihydro-1,3(4H)-oxazin-2-yl | methyl | |
| 1262 | 2-SO₂Me-4-CF₃Ph | 4,4,6-trimethyl-5,6-dihydro-1,3(4H)-oxazin-2-yl | i-propyl | |
| 1263 | 2-SO₂Me-4-CF₃Ph | 4,4,6-trimethyl-5,6-dihydro-1,3(4H)-oxazin-2-yl | cyclopropyl | |
| 1264 | 2-SO₂Me-4-CF₃Ph | 4,4,6-trimethyl-5,6-dihydro-1,3(4H)-oxazin-2-yl | CF₃ | |
| 1265 | 2-SO₂Me-4-CF₃Ph | 2-oxazolidinon-3-yl | H | |
| 1266 | 2-SO₂Me-4-CF₃Ph | 2-oxazolidinon-3-yl | methyl | |
| 1267 | 2-SO₂Me-4-CF₃Ph | 2-oxazolidinon-3-yl | i-propyl | |
| 1268 | 2-SO₂Me-4-CF₃Ph | 2-oxazolidinon-3-yl | cyclopropyl | |
| 1269 | 2-SO₂Me-4-CF₃Ph | 2-oxazolidinon-3-yl | CF₃ | |
| 1270 | 2-SO₂Me-4-CF₃Ph | 2-pyrrolidinon-1-yl | methyl | |
| 1271 | 2-SO₂Me-4-CF₃Ph | 2-pyrrolidinon-1-yl | i-propyl | |
| 1272 | 2-SO₂Me-4-CF₃Ph | 2-pyrrolidinon-1-yl | cyclopropyl | |
| 1273 | 2-SO₂Me-4-CF₃Ph | 2-pyrrolidinon-1-yl | CF₃ | |
| 1274 | 2-SO₂Me-4-CF₃Ph | 3-methylisoxazol-5-yl | methyl | |
| 1275 | 2-SO₂Me-4-CF₃Ph | 3-methylisoxazol-5-yl | i-propyl | |
| 1276 | 2-SO₂Me-4-CF₃Ph | 3-methylisoxazol-5-yl | cyclopropyl | |
| 1277 | 2-SO₂Me-4-CF₃Ph | 3-methylisoxazol-5-yl | CF₃ | |
| 1278 | 3-Cl-5-CF₃Pyridin-2-yl | 1,2,4-oxadiazol-5-yl | H | |
| 1279 | 3-Cl-5-CF₃Pyridin-2-yl | 1,2,4-oxadiazol-5-yl | methyl | |
| 1280 | 3-Cl-5-CF₃Pyridin-2-yl | 1,2,4-oxadiazol-5-yl | i-propyl | |
| 1281 | 3-Cl-5-CF₃Pyridin-2-yl | 1,2,4-oxadiazol-5-yl | cyclopropyl | |
| 1282 | 3-Cl-5-CF₃Pyridin-2-yl | 1,2,4-oxadiazol-5-yl | CF₃ | |
| 1283 | 3-Cl-5-CF₃Pyridin-2-yl | 3-methyl-1,2,4-oxadiazol-5-yl | H | |
| 1284 | 3-Cl-5-CF₃Pyridin-2-yl | 3-methyl-1,2,4-oxadiazol-5-yl | methyl | |
| 1285 | 3-Cl-5-CF₃Piridin-2-yl | 3-methyl-1,2,4-oxadiazol-5-yl | i-propyl | |
| 1286 | 3-Cl-5-CF₃Pyridin-2-yl | 3-methyl-1,2,4-oxadiazol-5-yl | cyclopropyl | |
| 1287 | 3-Cl-5-CF₃Pyridin-2-yl | 3-methyl-1,2,4-oxadiazol-5-yl | CF₃ | |
| 1288 | 3-Cl-5-CF₃Pyridin-2-yl | 3-trifluoromethyl-1,2,4-oxadiazol-5-yl | H | |
| 1289 | 3-Cl-5-CF₃Pyridin-2-yl | 3-trifluoromethyl-1,2,4-oxadiazol-5-yl | methyl | |
| 1290 | 3-Cl-5-CF₃Pyridin-2-yl | 3-trifluoromethyl-1,2,4-oxadiazol-5-yl | i-propyl | |
| 1291 | 3-Cl-5-CF₃Pyridin-2-yl | 3-trifluoromethyl-1,2,4-oxadiazol-5-yl | cyclopropyl | |
| 1292 | 3-Cl-5-CF₃Pyridin-2-yl | 3-trifluoromethyl-1,2,4-oxadiazol-5-yl | CF₃ | |
| 1293 | 3-Cl-5-CF₃Pyridin-2-yl | 1,2,4-oxadiazol-3-yl | H | |
| 1294 | 3-Cl-5-CF₃Pyridin-2-yl | 1,2,4-oxadiazol-3-yl | methyl | |
| 1295 | 3-Cl-5-CF₃Pyridin-2-yl | 1,2,4-oxadiazol-3-yl | i-propyl | |
| 1296 | 3-Cl-5-CF₃Pyridin-2-yl | 1,2,4-oxadiazol-3-yl | cyclopropyl | |
| 1297 | 3-Cl-5-CF₃Pyridin-2-yl | 1,2,4-oxadiazol-3-yl | CF₃ | |
| 1298 | 3-Cl-5-CF₃Pyridin-2-yl | 5-methyl-1,2,4-oxadiazol-3-yl | H | |
| 1299 | 3-Cl-5-CF₃Pyridin-2-yl | 5-methyl-1,2,4-oxadiazol-3-yl | methyl | |
| 1300 | 3-Cl-5-CF₃Pyridin-2-yl | 5-methyl-1,2,4-oxadiazol-3-yl | i-propyl | |
| 1301 | 3-Cl-5-CF₃Pyridin-2-yl | 5-methyl-1,2,4-oxadiazol-3-yl | cyclopropyl | |
| 1302 | 3-Cl-5-CF₃Pyridin-2-yl | 5-methyl-1,2,4-oxadiazol-3-yl | CF₃ | |
| 1303 | 3-Cl-5-CF₃Pyridin-2-yl | 5-trifluoromethyl-1,2,4-oxadiazol-3-yl | H | |
| 1304 | 3-Cl-5-CF₃Pyridin-2-yl | 5-trifluoromethyl-1,2,4-oxadiazol-3-yl | methyl | |
| 1305 | 3-Cl-5-CF₃Pyridin-2-yl | 5-trifluoromethyl-1,2,4-oxadiazol-3-yl | i-propyl | |
| 1306 | 3-Cl-5-CF₃Pyridin-2-yl | 5-trifluoromehyl-1,2,4-oxadiazol-3-yl | cyclopropyl | |
| 1307 | 3-Cl-5-CF₃Pyridin-2-yl | 5-trifluoromethyl-1,2,4-oxadiazol-3-yl | CF₃ | |
| 1308 | 3-Cl-5-CF₃Pyridin-2-yl | 5-chloro-1,2,4-oxadiazol-3-yl | H | |
| 1309 | 3-Cl-5-CF₃Pyridin-2-yl | 5-chloro-1,2,4-oxadiazol-3-yl | methyl | |
| 1310 | 3-Cl-5-CF₃Pyridin-2-yl | 5-chloro-1,2,4-oxadiazol-3-yl | i-propyl | |
| 1311 | 3-Cl-5-CF₃Pyridin-2-yl | 5-chloro-1,2,4-oxadiazol-3-yl | cyclopropyl | |
| 1312 | 3-Cl-5-CF₃Pyridin-2-yl | 5-chloro-1,2,4-oxadiazol-3-yl | CF₃ | |
| 1313 | 3-Cl-5-CF₃Pyridin-2-yl | 1,3,4-oxadiazol-2-yl | H | |
| 1314 | 3-Cl-5-CF₃Pyridin-2-yl | 1,3,4-oxadiazol-2-yl | methyl | |
| 1315 | 3-Cl-5-CF₃Pyridin-2-yl | 1,3,4-oxadiazol-2-yl | i-propyl | |
| 1316 | 3-Cl-5-CF₃Pyridin-2-yl | 1,3,4-oxadiazol-2-yl | cyclopropyl | |
| 1317 | 3-Cl-5-CF₃Pyridin-2-yl | 1,3,4-oxadiazol-2-yl | CF₃ | |
| 1318 | 3-Cl-5-CF₃Pyridin-2-yl | 5-methylsulfonyl-1,3,4-oxadiazol-2-yl | H | |
| 1319 | 3-Cl-5-CF₃Pyridin-2-yl | 5-methylsulfonyl-1,3,4-oxadiazol-2-yl | methyl | |
| 1320 | 3-Cl-5-CF₃Pyridin-2-yl | 5-methylsulfonyl-1,3,4-oxadiazol-2-yl | i-propyl | |
| 1321 | 3-Cl-5-CF₃Pyridin-2-yl | 5-methylsulfonyl-1,3,4-oxadiazol-2-yl | cyclopropyl | |
| 1322 | 3-Cl-5-CF₃Pyridin-2-yl | 5-methylsulfonyl-1,3,4-oxadiazol-2-yl | CF₃ | |
| 1323 | 3-Cl-5-CF₃Pyridin-2-yl | 5-methyl-1,3,4-oxadiazol-2-yl | H | |
| 1324 | 3-Cl-5-CF₃Pyridin-2-yl | 5-methyl-1,3,4-oxadiazol-2-yl | methyl | |
| 1325 | 3-Cl-5-CF₃Pyridin-2-yl | 5-methyl-1,3,4-oxadiazol-2-yl | i-propyl | |
| 1326 | 3-Cl-5-CF₃Pyridin-2-yl | 5-methyl-1,3,4-oxadiazol-2-yl | cyclopropyl | |
| 1327 | 3-Cl-5-CF₃Pyridin-2-yl | 5-methyl-1,3,4-oxadiazol-2-yl | CF₃ | |
| 1328 | 3-Cl-5-CF₃Pyridin-2-yl | 5-trifluoromethyl-1,3,4-oxadiazol-2-yl | H | |
| 1329 | 3-Cl-5-CF₃Pyridin-2-yl | 5-trifluoromethyl-1,3,4-oxsdiazol-2-yl | methyl | |
| 1330 | 3-Cl-5-CF₃Piridin-2-yl | 5-ffifluoromethyl-1,3,4-oxadiazol-2-yl | i-propyl | |
| 1331 | 3-Cl-5-CF₃Pyridin-2-yl | 5-trifluorome+yl-1,3,4-oxadiazol-2-yl | cyclopropyl | |
| 1332 | 3-Cl-5-CF₃Piridin-2-yl | 5-trifluoromethyl-1,3,4-oxadiazol-2-yl | CF₃ | |
| 1333 | 3-Cl-5-CF₃Pyridin-2-yl | 1,2,3-triazol-4-yl | H | |
| 1334 | 3-Cl-5-CF₃Pyridin-2-yl | 1,2,3-triazol-4-yl | methyl | |
| 1335 | 3-Cl-5-CF₃Pyridin-2-yl | 1,2,3-triazol-4-yl | i-propyl | |
| 1336 | 3-Cl-5-CF₃Pyridin-2-yl | 1,2,3-triazol-4-yl | cyclopropyl | |
| 1337 | 3-Cl-5-CF₃Pyridin-2-yl | 1,2,3-triazol-4-yl | CF₃ | |
| 1338 | 3-Cl-5-CF₃Pyridin-2-yl | 1-methyl-1,2,3-triazol-4-yl | H | |
| 1339 | 3-Cl-5-CF₃Pyridin-2-yl | 1-methyl-1,2,3-t6azol-4-yl | methyl | |
| 1340 | 3-Cl-5-CF₃Pyridin-2-yl | 1-methyl-1,2,3-triazol-4-yl | i-propyl | |
| 1341 | 3-Cl-5-CF₃Pyridin-2-yl | 1-methyl-1,2,3-triazol-4-yl | cyclopropyl | |
| 1342 | 3-Cl-5-CF₃Pyridin-2-yl | 1-methyl-1,2,3-triazol-4-yl | CF₃ | |
| 1343 | 3-Cl-5-CF₃Pyridin-2-yl | 2-methyl-1,2,3-triazol-4-yl | H | |
| 1344 | 3-Cl-5-CF₃Pyridin-2-yl | 2-methyl-1,2,3-triazol-4-yl | methyl | |
| 1345 | 3-Cl-5-CF₃Pyridin-2-yl | 2-methyl-1,2,3-triazol-4-yl | i-propyl | |
| 1346 | 3-Cl-5-CF₃Pyridin-2-yl | 2-methyl-1,2,3-triazol-4-yl | cyclopropyl | |
| 1347 | 3-Cl-5-CF₃Pyridin-2-yl | 2-methyl-1,2,3-triazol-4-yl | CF₃ | |
| 1348 | 3-Cl-5-CF₃Pyridin-2-yl | 1,2,3-triazol-1-yl | H | |
| 1349 | 3-Cl-5-CF₃Pyridin-2-yl | 1,2,3-triazol-1-yl | methyl | |
| 1350 | 3-Cl-5-CF₃Pyridin-2-yl | 1,2,3-triazol-1-yl | i-propyl | |
| 1351 | 3-Cl-5-CF₃Pyridin-2-yl | 1,2,3-triazol-1-yl | cyclopropyl | |
| 1352 | 3-Cl-5-CF₃Pyridin-2-yl | 1,2,3-triazol-1-yl | CF₃ | |
| 1353 | 3-Cl-5-CF₃Pyridin-2-yl | 1,2,3-triazol-2-yl | H | |
| 1354 | 3-Cl-5-CF₃Pyridin-2-yl | 1,2,3-triazol-2-yl | methyl | |
| 1355 | 3-Cl-5-CF₃Pyridin-2-yl | 1,2,3-triazol-2-yl | i-propyl | |
| 1356 | 3-Cl-5-CF₃Pyridin-2-yl | 1,2,3-triazol-2-yl | cyclopropyl | |
| 1357 | 3-Cl-5-CF₃Pyridin-2-yl | 1,2,3-triazol-2-yl | CF₃ | |
| 1358 | 3-Cl-5-CF₃Pyridin-2-yl | 1,2,4-triazol-1-yl | H | |
| 1359 | 3-Cl-5-CF₃Pyridin-2-yl | 1,2,4-triazol-1-yl | methyl | |
| 1360 | 3-Cl-5-CF₃Pyridin-2-yl | 1,2,4-triazol-1-yl | i-propyl | |
| 1361 | 3-Cl-5-CF₃Pyridin-2-yl | 1,2,4-triazol-1-yl | cyclopropyl | |
| 1362 | 3-Cl-5-CF₃Pyridin-2-yl | 1,2,4-triazol-1-yl | CF₃ | |
| 1363 | 3-Cl-5-CF₃Pyridin-2-yl | imidazol-2-yl | H | |
| 1364 | 3-Cl-5-CF₃Pyridin-2-yl | imidazol-2-yl | methyl | |
| 1365 | 3-Cl-5-CF₃Pyridin-2-yl | imidazol-2-yl | i-propyl | |
| 1366 | 3-Cl-5-CF₃Pyridin-2-yl | imidazol-2-yl | cyclopropyl | |
| 1367 | 3-Cl-5-CF₃Pyridin-2-yl | imidazol-2-yl | CF₃ | |
| 1368 | 3-Cl-5-CF₃Pyridin-2-yl | imidazol-1-yl | H | |
| 1369 | 3-Cl-5-CF₃Pyridin-2-yl | imidazol-1-yl | methyl | |
| 1370 | 3-Cl-5-CF₃Pyridin-2-yl | imidazol-1-yl | i-propyl | |
| 1371 | 3-Cl-5-CF₃Pyridin-2-yl | imidazol-1-yl | cyclopropyl | |
| 1372 | 3-Cl-5-CF₃Pyridin-2-yl | imidazol-1-yl | CF₃ | |
| 1373 | 3-Cl-5-CF₃Pyridin-2-yl | imidazol-4-yl | H | |
| 1374 | 3-Cl-5-CF₃Pyridin-2-yl | imidazol-4-yl | methyl | |
| 1375 | 3-Cl-5-CF₃Pyridin-2-yl | imidazol-4-yl | i-propyl | |
| 1376 | 3-Cl-5-CF₃Pyridin-2-yl | imidazol-4-yl | cyclopropyl | |
| 1377 | 3-Cl-5-CF₃Pyridin-2-yl | imidazol-4-yl | CF₃ | |
| 1378 | 3-Cl-5-CF₃Pyridin-2-yl | thiazol-2-yl | H | |
| 1379 | 3-Cl-5-CF₃Pyridin-2-yl | thiazol-2-yl | methyl | |
| 1380 | 3-Cl-5-CF₃Pyridin-2-yl | thiazol-2-yl | i-propyl | |
| 1381 | 3-Cl-5-CF₃Pyridin-2-yl | thiazol-2-yl | cyclopropyl | |
| 1382 | 3-Cl-5-CF₃Pyridin-2-yl | thiazol-2-yl | CF₃ | |
| 1383 | 3-Cl-5-CF₃Pyridin-2-yl | 4-methylthiazol-2-yl | H | |
| 1384 | 3-Cl-5-CF₃Pyridin-2-yl | 4-methylthiazol-2-yl | methyl | |
| 1385 | 3-Cl-5-CF₃Pyridin-2-yl | 4-methylthiazol-2-yl | i-propyl | |
| 1386 | 3-Cl-5-CF₃Pyridin-2-yl | 4-methylthiazol-2-yl | cyclopropyl | |
| 1387 | 3-Cl-5-CF₃Pyridin-2-yl | 4-methylthiazol-2-yl | CF₃ | |
| 1388 | 3-Cl-5-CF₃Pyridin-2-yl | oxazol-2-yl | H | |
| 1389 | 3-Cl-5-CF₃Pyridin-2-yl | oxazol-2-yl | methyl | |
| 1390 | 3-Cl-5-CF₃Pyridin-2-yl | oxazol-2-yl | i-propyl | |
| 1391 | 3-Cl-5-CF₃Pyridin-2-yl | oxazol-2-yl | cyclopropyl | |
| 1392 | 3-Cl-5-CF₃Pyridin-2-yl | oxazol-2-yl | CF₃ | |
| 1393 | 3-Cl-5-CF₃Pyridin-2-yl | 4,5-dimethyloxazol-2-yl | H | |
| 1394 | 3-Cl-5-CF₃Pyridin-2-yl | 4,5-dimethyloxazol-2-yl | methyl | |
| 1395 | 3-Cl-5-CF₃Pyridin-2-yl | 4,5-dimethyloxazol-2-yl | i-propyl | |
| 1396 | 3-Cl-5-CF₃Pyridin-2-yl | 4,5-dimethyloxazol-2-yl | cyclopropyl | |
| 1397 | 3-Cl-5-CF₃Pyridin-2-yl | 4,5-dimethyloxazol-2-yl | CF₃ | |
| 1398 | 3-Cl-5-CF₃Pyridin-2-yl | 2-oxazolin-2-yl | H | |
| 1399 | 3-Cl-5-CF₃Pyridin-2-yl | 2-oxazolin-2-yl | methyl | |
| 1400 | 3-Cl-5-CF₃Pyridin-2-yl | 2-oxazolin-2-yl | i-propyl | |
| 1401 | 3-Cl-5-CF₃Pyridin-2-yl | 2-oxazolin-2-yl | cyclopropyl | |
| 1402 | 3-Cl-5-CF₃Pyridin-2-yl | 2-oxazolin-2-yl | CF₃ | |
| 1403 | 3-Cl-5-CF₃Pyridin-2-yl | 4,4-dimethyl-2-oxazolin-2-yl | H | |
| 1404 | 3-Cl-5-CF₃Pyridin-2-yl | 4,4-dimethyl-2-oxazolin-2-yl | methyl | |
| 1405 | 3-Cl-5-CF₃Pyridin-2-yl | 4,4-dimethyl-2-oxazolin-2-yl | i-propyl | |
| 1406 | 3-Cl-5-CF₃Pyridin-2-yl | 4,4-dimethyl-2-oxazolin 2-yl | cyclopropyl | |
| 1407 | 3-Cl-5-CF₃Pyridin-2-yl | 4,4-dimethyl-2-oxazolin-2-yl | CF₃ | |
| 1408 | 3-Cl-5-CF₃Pyridin-2-yl | 1,2,4-thiadiazol-5-yl | H | |
| 1409 | 3-Cl-5-CF₃Pyridin-2-yl | 1,2,4-thiadiazol-5-yl | methyl | |
| 1410 | 3-Cl-5-CF₃Pyridin-2-yl | 1,2,4-thiadiazol-5-yl | i-propyl | |
| 1411 | 3-Cl-5-CF₃Pyridin-2-yl | 1,2,4-thiadiazol-5-yl | cyclopropyl | |
| 1412 | 3-Cl-5-CF₃Pyridin-2-yl | 1,2,4-thiadiazol-5-yl | CF₃ | |
| 1413 | 3-Cl-5-CF₃Pyridin-2-yl | 3-methyl-1,2,4-thiadiazol-5-yl | H | |
| 1414 | 3-Cl-5-CF₃Pyridin-2-yl | 3-methyl-1,2,4-thiadiazol-5-yl | methyl | |
| 1415 | 3-Cl-5-CF₃Pyridin-2-yl | 3-methyl-1,2,4-thiadiazol-5-yl | i-propyl | |
| 1416 | 3-Cl-5-CF₃Pyridin-2-yl | 3-methyl-1,2,4-thiadiazol-5-yl | cyclopropyl | |
| 1417 | 3-Cl-5-CF₃Pyridin-2-yl | 3-methyl-1,2,4-thiadiazol-5-yl | CF₃ | |
| 1418 | 3-Cl-5-CF₃Pyridin-2-yl | 3-trifluoromethyl-1,2,4-thiadiazol-5-yl | H | |
| 1419 | 3-Cl-5-CF₃Pyridin-2-yl | 3-trifluoromethyl-1,2,4-thiadiazol-5-yl | methyl | |
| 1420 | 3-Cl-5-CF₃Pyridin-2-yl | 3-trifluoromethyl-1,2,4-thiadiazol-5-yl | i-propyl | |
| 1421 | 3-Cl-5-CF₃Pyridin-2-yl | 3-trifluoromethyl-1,2,4-thiadiazol-5-yl | cyclopropyl | |
| 1422 | 3-Cl-5-CF₃Pyridin-2-yl | 3-trifluoromethyl-1,2,4-thiadiazol-5-yl | CF₃ | |
| 1423 | 3-Cl-5-CF₃Pyridin-2-yl | 1,2,4-thiadiazol-3-yl | H | |
| 1424 | 3-Cl-5-CF₃Pyridin-2-yl | 1,2,4-thiadiazol-3-yl | methyl | |
| 1425 | 3-Cl-5-CF₃Pyridin-2-yl | 1,2,4-thiadiazol-3-yl | i-propyl | |
| 1426 | 3-Cl-5-CF₃Pyridin-2-yl | 1,2,4-thiadiazol-3-yl | cyclopropyl | |
| 1427 | 3-Cl-5-CF₃Pyridin-2-yl | 1,2,4-thiadiazol-3-yl | CF₃ | |
| 1428 | 3-Cl-5-CF₃Pyridin-2-yl | 5-methyl-1,2,4-thiadiazol-3-yl | H | |
| 1429 | 3-Cl-5-CF₃Pyridin-2-yl | 5-methyl-1,2,4-thiadiazol-3-yl | methyl | |
| 1430 | 3-Cl-5-CF₃Pyridin-2-yl | 5-methyl-1,2,4-thiadiazol-3-yl | i-propyl | |
| 1431 | 3-Cl-5-CF₃Pyridin-2-yl | 5-methyl-1,2,4-thiadiazol-3-yl | cyclopropyl | |
| 1432 | 3-Cl-5-CF₃Pyridin-2-yl | 5-methyl-1,2,4-thiadiazol-3-yl | CF₃ | |
| 1433 | 3-Cl-5-CF₃Pyridin-2-yl | 5-trifluoromethyl-1,2,4-thiadiazol-3-yl | H | |
| 1434 | 3-Cl-5-CF₃Pyridin-2-yl | 5-trifluoromethyl-1,2,4-thiadiazol-3-yl | methyl | |
| 1435 | 3-Cl-5-CF₃Pyridin-2-yl | 5-trifluoromethyl-1,2,4-thiadiazol-3-yl | i-propyl | |
| 1436 | 3-Cl-5-CF₃Pyridin-2-yl | 5-trifluoromethyl-1,2,4-thiadiazol-3-yl | cyclopropyl | |
| 1437 | 3-Cl-5-CF₃Pyridin-2-yl | 5-trifluoromethyl-1,2,4-thiadiazol-3-yl | CF₃ | |
| 1438 | 3-Cl-5-CF₃Pyridin-2-yl | 1,3,4-thiadiazol-2-yl | H | |
| 1439 | 3-Cl-5-CF₃Pyridin-2-yl | 1,3,4-thiadiazol-2-yl | methyl | |
| 1440 | 3-Cl-5-CF₃Pyridin-2-yl | 1,3,4-thiadiazol-2-yl | i-propyl | |
| 1441 | 3-Cl-5-CF₃Pyridin-2-yl | 1,3,4-thiadiazol-2-yl | cyclopropyl | |
| 1442 | 3-Cl-5-CF₃Pyridin-2-yl | 1,3,4-thiadiazol-2-yl | CF₃ | |
| 1443 | 3-Cl-5-CF₃Pyridin-2-yl | 5-methylsulfonyl-1,3,4-thiadiazol-2-yl | H | |
| 1444 | 3-Cl-5-CF₃Pyridin-2-yl | 5-methylsulfonyl-1,3,4-thiadiazol-2-yl | methyl | |
| 1445 | 3-Cl-5-CF₃Pyridin-2-yl | 5-methylsulfonyl-1,3,4-thiadiazol-2-yl | i-propyl | |
| 1446 | 3-Cl-5-CF₃Pyridin-2-yl | 5-methylsulfonyl-1,3,4-thiadiazol-2-yl | cyclopropyl | |
| 1447 | 3-Cl-5-CF₃Pyridin-2-yl | 5-methylsulfonyl-1,3,4-thiadiazol-2-yl | CF₃ | |
| 1448 | 3-Cl-5-CF₃Pyridin-2-yl | 5-methyl-1,3,4-thiadiazol-2-yl | H | |
| 1449 | 3-Cl-5-CF₃Pyridin-2-yl | 5-methyl-1,3,4-thiadiazol-2-yl | methyl | |
| 1450 | 3-Cl-5-CF₃Pyridin-2-yl | 5-methyl-1,3,4-thiadiazol-2-yl | i-propyl | |
| 1451 | 3-Cl-5-CF₃Pyridin-2-yl | 5-methyl-1,3,4-thiadiazol-2-yl | cyclopropyl | |
| 1452 | 3-Cl-5-CF₃Pyridin-2-yl | 5-methyl-1,3,4-thiadiazol-2-yl | CF₃ | |
| 1453 | 3-Cl-5-CF₃Pyridin-2-yl | benzoxazol-2-yl | H | |
| 1454 | 3-Cl-5-CF₃Pyridin-2-yl | benzoxazol-2-yl | methyl | |
| 1455 | 3-Cl-5-CF₃Pyridin-2-yl | benzoxazol-2-yl | i-propyl | |
| 1456 | 3-Cl-5-CF₃Pyridin-2-yl | benzoxazol-2-yl | cyclopropyl | |
| 1457 | 3-Cl-5-CF₃Pyridin-2-yl | benzoxazol-2-yl | CF₃ | |
| 1458 | 3-Cl-5-CF₃Pyridin-2-yl | 6-methylbenzoxazol-2-yl | H | |
| 1459 | 3-Cl-5-CF₃Pyridin-2-yl | 6-methylbenzoxazol-2-yl | methyl | |
| 1460 | 3-Cl-5-CF₃Pyridin-2-yl | 6-methylbenzoxazol-2-yl | i-propyl | |
| 1461 | 3-Cl-5-CF₃Pyridin-2-yl | 6-methylbenzoxazol-2-yl | cyclopropyl | |
| 1462 | 3-Cl-5-CF₃Pyridin-2-yl | 6-methylbenzoxazol-2-yl | CF₃ | |
| 1463 | 3-Cl-5-CF₃Pyridin-2-yl | benzothiazol-2-yl | H | |
| 1464 | 3-Cl-5-CF₃Pyridin-2-yl | benzothiazol-2-yl | methyl | |
| 1465 | 3-Cl-5-CF₃Pyridin-2-yl | benzothiazol-2-yl | i-propyl | |
| 1466 | 3-Cl-5-CF₃Pyridin-2-yl | benzothiazol-2-yl | cyclopropyl | |
| 1467 | 3-Cl-5-CF₃Pyridin-2-yl | benzothiazol-2-yl | CF₃ | |
| 1468 | 3-Cl-5-CF₃Pyridin-2-yl | pyrazol-1-yl | H | |
| 1469 | 3-Cl-5-CF₃Pyridin-2-yl | pyrazol-1-yl | methyl | |
| 1470 | 3-Cl-5-CF₃Pyridin-2-yl | pyrazol-1-yl | i-propyl | |
| 1471 | 3-Cl-5-CF₃Pyridin-2-yl | pyrazol-1-yl | cyclopropyl | |
| 1472 | 3-Cl-5-CF₃Pyridin-2-yl | pyrazol-1-yl | CF₃ | |
| 1473 | 3-Cl-5-CF₃Pyridin-2-yl | pyrazol-3-yl | H | |
| 1474 | 3-Cl-5-CF₃Pyridin-2-yl | pyrazol-3-yl | methyl | |
| 1475 | 3-Cl-5-CF₃Pyridin-2-yl | pyrazol-3-yl | i-propyl | |
| 1476 | 3-Cl-5-CF₃Pyridin-2-yl | pyrazol-3-yl | cyclopropyl | |
| 1477 | 3-Cl-5-CF₃Pyridin-2-yl | pyrazol-3-yl | CF₃ | |
| 1478 | 3-Cl-5-CF₃Pyridin-2-yl | 1-methylpyrazol-3-yl | H | |
| 1479 | 3-Cl-5-CF₃Pyridin-2-yl | 1-methylpyrazol-3-yl | methyl | |
| 1480 | 3-Cl-5-CF₃Pyridin-2-yl | 1-methylpyrazol-3-yl | i-propyl | |
| 1481 | 3-Cl-5-CF₃Pyridin-2-yl | 1-methylpyrazol-3-yl | cyclopropyl | |
| 1482 | 3-Cl-5-CF₃Pyridin-2-yl | 1-methylpyrazol-3-yl | CF₃ | |
| 1483 | 3-Cl-5-CF₃Pyridin-2-yl | tetrazol-1-yl | H | |
| 1484 | 3-Cl-5-CF₃Pyridin-2-yl | tetrazol-1-yl | methyl | |
| 1485 | 3-Cl-5-CF₃Pyridin-2-yl | tetrazol-1-yl | i-propyl | |
| 1486 | 3-Cl-5-CF₃Pyridin-2-yl | tetrazol-1-yl | cyclopropyl | |
| 1487 | 3-Cl-5-CF₃Pyridin-2-yl | tetrazol-1-yl | CF₃ | |
| 1488 | 3-Cl-5-CF₃Pyridnr2-yl | 5-methyltetrazol-1-yl | H | |
| 1489 | 3-Cl-5-CF₃Pyridin-2-yl | 5-methyltetrazol-1-yl | methyl | |
| 1490 | 3-Cl-5-CF₃Pyridin-2-yl | 5-methyltetrazol-1-yl | i-propyl | |
| 1491 | 3-Cl-5-CF₃Pyridin-2-yl | 5-methyltetrazol-1-yl | cyclopropyl | |
| 1492 | 3-Cl-5-CF₃Pyridin-2-yl | 5-methyltetrazol-1-yl | CF₃ | |
| 1493 | 3-Cl-5-CF₃Pyridin-2-yl | tetrazol-2-yl | H | |
| 1494 | 3-Cl-5-CF₃Pyridin-2-yl | tetrazol-2-yl | methyl | |
| 1495 | 3-Cl-5-CF₃Pyridin-2-yl | tetrazol-2-yl | i-propyl | |
| 1496 | 3-Cl-5-CF₃Pyridin-2-yl | tetrazol-2-yl | cyclopropyl | |
| 1497 | 3-Cl-5-CF₃Pyridin-2-yl | tetrazol-2-yl | CF₃ | |
| 1498 | 3-Cl-5-CF₃Pyridin-2-yl | 5-methyltetrazol-5-yl | H | |
| 1499 | 3-Cl-5-CF₃Pyridin-2-yl | 5-methyltetrazol-2-yl | methyl | |
| 1500 | 3-Cl-5-CF₃Pyridin-2-yl | 5-methyltetrazol-2-yl | i-propyl | |
| 1501 | 3-Cl-5-CF₃Pyridin-2-yl | 5-methyltetrazol-2-yl | cyclopropyl | |
| 1502 | 3-Cl-5-CF₃Pyridin-2-yl | 5-methyltetrazol-2-yl | CF₃ | |
| 1503 | 3-Cl-5-CF₃Pyridin-2-yl | 1-methyltetrazol-5-yl | H | |
| 1504 | 3-Cl-5-CF₃Pyridin-2-yl | 1-methyltetrazol-5-yl | methyl | |
| 1505 | 3-Cl-5-CF₃Pyridin-2-yl | 1-methyltetrazol-5-yl | i-propyl | |
| 1506 | 3-Cl-5-CF₃Pyridin-2-yl | 1-methyltetrazol-5-yl | cyclopropyl | |
| 1507 | 3-Cl-5-CF₃Pyndin-2-yl | 1-methyltetrazol-5-yl | CF₃ | |
| 1508 | 3-Cl-5-CF₃Pyridin-2-yl | 2-methyltetrazol-5-yl | H | |
| 1509 | 3-Cl-5-CF₃Pyridin-2-yl | 2-methyltetrazol-5-yl | methyl | |
| 1510 | 3-Cl-5-CF₃Pyridin-2-yl | 2-methyltetrazol-5-yl | i-propyl | |
| 1511 | 3-Cl-5-CF₃Pyridin-2-yl | 2-methyltetrazol-5-yl | cyclopropyl | |
| 1512 | 3-Cl-5-CF₃Pyridin-2-yl | 2-methyltetrazol-5-yl | CF₃ | |
| 1513 | 3-Cl-5-CF₃Pyridin-2-yl | pyridin-2-yl | H | |
| 1514 | 3-Cl-5-CF₃Pyridin-2-yl | pyridin-2-yl | methyl | |
| 1515 | 3-Cl-5-CF₃Pyridin-2-yl | pyridin-2-yl | i-propyl | |
| 1516 | 3-Cl-5-CF₃Pyridin-2-yl | pyridin-2-yl | cyclopropyl | |
| 1517 | 3-Cl-5-CF₃Pyridin-2-yl | pyridin-2-yl | CF₃ | |
| 1518 | 3-Cl-5-CF₃Pyridin-2-yl | pyridin-4-yl | H | |
| 1519 | 3-Cl-5-CF₃Pyridin-2-yl | pyridin-4-yl | methyl | |
| 1520 | 3-Cl-5-CF₃Pyridin-2-yl | pyridin-4-yl | i-propyl | |
| 1521 | 3-Cl-5-CF₃Pyridin-2-yl | pyridin-4-yl | cyclopropyl | |
| 1522 | 3-Cl-5-CF₃Pyridin-2-yl | pyridin-4-yl | CF₃ | |
| 1523 | 3-Cl-5-CF₃Pyridin-2-yl | pyridin-3-yl | H | |
| 1524 | 3-Cl-5-CF₃Pyridin-2-yl | pyridin-3-yl | methyl | |
| 1525 | 3-Cl-5-CF₃Pyridin-2-yl | pyridin-3-yl | i-propyl | |
| 1526 | 3-Cl-5-CF₃Pyridin-2-yl | pyridin-3-yl | cyclopropyl | |
| 1527 | 3-Cl-5-CF₃Pyridin-2-yl | pyridin-3-yl | CF₃ | |
| 1528 | 3-Cl-5-CF₃Pyridin-2-yl | 3-nitropyridin-4-yl | H | |
| 1529 | 3-Cl-5-CF₃Pyridin-2-yl | 3-nitropyridin-4-yl | methyl | |
| 1530 | 3-Cl-5-CF₃Pyridin-2-yl | 3-nitropyridin-4-yl | i-propyl | |
| 1531 | 3-Cl-5-CF₃Pyridin-2-yl | 3-nitropyridin-4-yl | cyclopropyl | |
| 1532 | 3-Cl-5-CF₃Pyridin-2-yl | 3-nitropyridin-4-yl | CF₃ | |
| 1533 | 3-Cl-5-CF₃Pyridin-2-yl | 5-cyanopyridin-2-yl | H | |
| 1534 | 3-Cl-5-CF₃Pyridin-2-yl | 5-cyanopyridin-2-yl | methyl | |
| 1535 | 3-Cl-5-CF₃Pyridin-2-yl | 5-cyanopyridin-2-yl | i-propyl | |
| 1536 | 3-Cl-5-CF₃Pyridin-2-yl | 5-cyanopyridin-2-yl | cyclopropyl | |
| 1537 | 3-Cl-5-CF₃Pyridin-2-yl | 5-cyanopyridin-2-yl | CF₃ | |
| 1538 | 3-Cl-5-CF₃Pyridin-2-yl | 5-trifluoromethylpyridin-2-yl | H | |
| 1539 | 3-Cl-5-CF₃Pyridin-2-yl | 5-tritluoromethylpyridin-2-yl | methyl | |
| 1540 | 3-Cl-5-CF₃Pyridin-2-yl | 5-trifluoromethylpyridin-2-yl | i-propyl | |
| 1541 | 3-Cl-5-CF₃Pyridin-2-yl | 5-trifluoromethylpyridin-2-yl | cyclopropyl | |
| 1542 | 3-Cl-5-CF₃Pyridin-2-yl | 5-trifluoromethylpyridin-2-yl | CF₃ | |
| 1543 | 3-Cl-5-CF₃Pyridin-2-yl | pyrimidin-2-yl | H | |
| 1544 | 3-Cl-5-CF₃Pyridin-2-yl | pyrimidin-2-yl | methyl | |
| 1545 | 3-Cl-5-CF₃Pyridin-2-yl | pyrimidin-2-yl | i-propyl | |
| 1546 | 3-Cl-5-CF₃Pyridin-2-yl | pyrimidin-2-yl | cyclopropyl | |
| 1547 | 3-Cl-5-CF₃Pyridin-2-yl | pyrimidin-2-yl | CF₃ | |
| 1548 | 3-Cl-5-CF₃Pyridin-2-yl | pyrimidin-4-yl | H | |
| 1549 | 3-Cl-5-CF₃Pyridin-2-yl | pyrimidin-4-yl | methyl | |
| 1550 | 3-Cl-5-CF₃Pyridin-2-yl | Pyrimidin-4-yl | i-propyl | |
| 1551 | 3-Cl-5-CF₃Pyridin-2-yl | pyrimidin-4-yl | cyclopropyl | |
| 1552 | 3-Cl-5-CF₃Pyridin-2-yl | pyrimidin-4-yl | CF₃ | |
| 1553 | 3-Cl-5-CF₃Pyridin-2-yl | 6-chloropyrimidin-4-yl | methyl | |
| 1554 | 3-Cl-5-CF₃Pyridin-2-yl | 6-chloropyrimidin-4-yl | i-propyl | |
| 1555 | 3-Cl-5-CF₃Pyridin-2-yl | 6-chloropyrimidin-4-yl | cyclopropyl | |
| 1556 | 3-Cl-5-CF₃Pyridin-2-yl | 6-chloropyrimidin-4-yl | CF₃ | |
| 1557 | 3-Cl-5-CF₃Pyridin-2-yl | pyridazin-3-yl | H | |
| 1558 | 3-Cl-5-CF₃Pyridin-2-yl | pyridazin-3-yl | methyl | |
| 1559 | 3-Cl-5-CF₃Pyridin-2-yl | pyridazin-3-yl | i-propyl | |
| 1560 | 3-Cl-5-CF₃Pyridin-2-yl | pyridazin-3-yl | cyclopropyl | |
| 1561 | 3-Cl-5-CF₃Pyridin-2-yl | pyridazin-3-yl | CF₃ | |
| 1562 | 3-Cl-5-CF₃Pyridin-2-yl | 6-chloropyridazin-3-yl | methyl | |
| 1563 | 3-Cl-5-CF₃Pyridin-2-yl | 6-chloropyridazin-3-yl | i-propyl | |
| 1564 | 3-Cl-5-CF₃Pyridin-2-yl | 6-chloropyridazin-3-yl | cyclopropyl | |
| 1565 | 3-Cl-5-CF₃Pyridin-2-yl | 6-chloropyridazin-3-yl | CF₃ | |
| 1566 | 3-Cl-5-CF₃Pyridin-2-yl | pyrazin-2-yl | methyl | |
| 1567 | 3-Cl-5-CF₃Pyridin-2-yl | pyrazin-2-yl | i-propyl | |
| 1568 | 3-Cl-5-CF₃Pyridin-2-yl | pyrazin-2-yl | cyclopropyl | |
| 1569 | 3-Cl-5-CF₃Pyridin-2-yl | pyrazin-2-yl | CF₃ | |
| 1570 | 3-Cl-5-CF₃Pyridin-2-yl | triazin-2-yl | methyl | |
| 1571 | 3-Cl-5-CF₃Pyridin-2-yl | triazin-2-yl | i-propyl | |
| 1572 | 3-Cl-5-CF₃Pyridin-2-yl | triazin-2-yl | cyclopropyl | |
| 1573 | 3-Cl-5-CF₃Pyridin-2-yl | triazin-2-yl | CF₃ | |
| 1574 | 3-Cl-5-CF₃Pyridin-2-yl | quinolin-2-yl | methyl | |
| 1575 | 3-Cl-5-CF₃Pyridin-2-yl | quinolin-2-yl | i-propyl | |
| 1576 | 3-Cl-5-CF₃Pyridin-2-yl | quinolin-2-yl | cyclopropyl | |
| 1577 | 3-Cl-5-CF₃Pyridin-2-yl | quinolin-2-yl | CF₃ | |
| 1578 | 3-Cl-5-CF₃Pyridin-2-yl | 4,4,6-trimethyl-5,6-dihydro-1,3(4H)-oxazin-2-yl | H | |
| 1579 | 3-Cl-5-CF₃Pyridin-2-yl | 4,4,6-trimethyl-5,6-dihydro-1,3(4H)-oxazin-2-yl | methyl | |
| 1580 | 3-Cl-5-CF₃Pyridin-2-yl | 4,4,6-trimethyl-5,6-dihydro-1,3(4H)oxazin-2-yl | i-propyl | |
| 1581 | 3-Cl-5-CF₃Pyridin-2-yl | 4,4,6-trimethyl-5,6-dihydro1,3(4H)-oxazin-2-yl | cyclopropyl | |
| 1582 | 3-Cl-5-CF₃Pyridin-2-yl | 4,4,6-trimethyl-5,6-dihydro-1,3(4H)-oxazin-2-yl | CF₃ | |
| 1583 | 3-Cl-5-CF₃Pyridin-2-yl | 2-oxazolidiaon-3-yl | H | |
| 1584 | 3-Cl-5-CF₃Pyridin-2-yl | 2-oxazolidinon-3-yl | methyl | |
| 1585 | 3-Cl-5-CF₃Pyridin-2-yl | 2-oxazolidinon-3-yl | i-propyl | |
| 1586 | 3-Cl-5-CF₃Pyridin-2-yl | 2-oxazolidinon-3-yl | cyclopropyl | |
| 1587 | 3-Cl-5-CF₃Pyridin-2-yl | 2-oxazolidinon-3-yl | CF₃ | |
| 1588 | 3-Cl-5-CF₃Pyridin-2-yl | 2-pyrrolidinon-1-yl | methyl | |
| 1589 | 3-Cl-5-CF₃Pyridin-2-yl | 2-pyrrolidinon-1-yl | i-propyl | |
| 1590 | 3-Cl-5-CF₃Pyridin-2-yl | 2-pyrrolidinon-1-yl | cyclopropyl | |
| 1591 | 3-Cl-5-CF₃Pyridin-2-yl | 2-pyrrolidinon-1-yl | CF₃ | |
| 1592 | 3-Cl-5-CF₃Pyridin-2-yl | 3-methylisoxazol-5-yl | methyl | |
| 1593 | 3-Cl-5-CF₃Pyridin-2-yl | 3-methylisoxazol-5-yl | i-propyl | |
| 1594 | 3-Cl-5-CF₃Pyridin-2-yl | 3-methylisoxazol-5-yl | cyclopropyl | |
| 1595 | 3-Cl-5-CF₃Pyridin-2-yl | 3-methylisoxazol-5-yl | CF₃ | |
| 1596 | 2,4-(Me)₂Thiazol-5-yl | 1,2,4-oxadiazol-5-yl | H | |
| 1597 | 2,4-(Me)₂Thiazol-5-yl | 1,2,4-oxadiazol-5-yl | methyl | |
| 1598 | 2,4-(Me)₂Thiazol-5-yl | 1,2,4-oxadiazol-5-yl | i-propyl | |
| 1599 | 2,4-(Me)₂Thiazol-5-yl | 1,2,4-oxadiazol-5-yl | cyclopropyl | |
| 1600 | 2,4-(Me)₂Thiazol-5-yl | 1,2,4-oxadiazol-5-yl | CF₃ | |
| 1601 | 2,4-(Me)₂Thiazol-5-yl | 3-methyl-1,2,4-oxadiazol-5-yl | H | |
| 1602 | 2,4-(Me)₂Thiazol-5-yl | 3-methyl-1,2,4-oxadiazol-5-yl | methyl | |
| 1603 | 2,4-(Me)₂Thiazol-5-yl | 3-methyl-1,2,4-oxadiazol-5-yl | i-propyl | |
| 1604 | 2,4-(Me)₂Thiazol-5-yl | 3-methyl-1,2,4-oxadiazol-5-yl | cyclopropyl | |
| 1605 | 2,4-(Me)₂Thiazol-5-yl | 3-methyl-1,2,4-oxadiazol-5-yl | CF₃ | |
| 1606 | 2,4-(Me)₂Thiazol-5-yl | 3-trifluoromethyl-1,2,4-oxadiazol-5-yl | H | |
| 1607 | 2,4-(Me)₂Thiazol-5-yl | 3-trifluoromethyl-1,2,4-oxadiazol-5-yl | methyl | |
| 1608 | 2,4-(Me)₂Thiazol-5-yl | 3-trifluorometyl-1,2,4-oxadiazol-5-yl | i-propyl | |
| 1609 | 2,4-(Me)₂Thiazol-5-yl | 3-trifluoromethyl-1,2,4-oxadiazol-5-yl | cyclopropyl | |
| 1610 | 2,4-(Me)₂Thiazol-5-yl | 3-trifloromethyl-1,2,4-oxadiazol-5-yl | CF₃ | |
| 1611 | 2,4-(Me)₂Thiazol-5-yl | 1,2,4-oxadiazol-3-yl | H | |
| 1612 | 2,4-(Me)₂Thiazol-5-yl | 1,2,4-oxadiazol-3-yl | methyl | |
| 1613 | 2,4-(Me)₂Thiazol-5-yl | 1,2,4-oxadiazol-3-yl | i-propyl | |
| 1614 | 2,4-(Me)₂Thiazol-5-yl | 1,2,4-oxadiazol-3-yl | cyclopropyl | |
| 1615 | 2,4-(Me)₂Thiazol-5-yl | 1,2,4-oxadiazol-3-yl | CF₃ | |
| 1616 | 2,4-(Me)₂Thiazol-5-yl | 5-methyl-1,2,4-oxadiazol-3-yl | H | |
| 1617 | 2,4-(Me)₂Thiazol-5-yl | 5-methyl-1,2,4-oxadiazol-3-yl | methyl | |
| 1618 | 2,4-(Me)₂Thiazol-5-yl | 5-methyl-1,2,4-oxadiazol-3-yl | i-propyl | |
| 1619 | 2,4-(Me)₂Thiazol-5-yl | 5-methyl-1,2,4-oxadiazol-3-yl | cyclopropyl | |
| 1620 | 2,4-(Me)₂Thiazol-5-yl | 5-methyl-1,2,4-oxadiazol-3-yl | CF₃ | |
| 1621 | 2,4-(Me)₂Thiazol-5-yl | 5-trifluoromethyl-1,2,4-oxadiazol-3-yl | H | |
| 1622 | 2,4-(Me)₂Thiazol-5-yl | 5-trifluoromethyl-1,2,4-oxadiazol-3-yl | methyl | |
| 1623 | 2,4-(Me)₂Thiazol-5-yl | 5-trifluoromethyl-1,2,4-oxadiazol-3-yl | i-propyl | |
| 1624 | 2,4-(Me)₂Thiazol-5-yl | 5-trifluoromethyl-1,2,4-oxadiazol-3-yl | cyclopropyl | |
| 1625 | 2,4-(Me)₂Thiazol-5-yl | 5-trifluoromethyl-1,2,4-oxadiazol-3-yl | CF₃ | |
| 1626 | 2,4-(Me)₂Thiazol-5-yl | 5-chloro-1,2,4-oxadiazol-3-yl | H | |
| 1627 | 2,4-(Me)₂Thiazol-5-yl | 5-chloro-1,2,4-oxadiazol-3-yl | methyl | |
| 1628 | 2,4-(Me)₂Thiazol-5-yl | 5-chloro-1,2,4-oxadiazol-3-yl | i-propyl | |
| 1629 | 2,4-(Me)₂Thiazol-5-yl | 5-chloro-1,2,4-oxadiazol-3-yl | cyclopropyl | |
| 1630 | 2,4-(Me)₂Thiazol-5-yl | 5-chloro-1,2,4-oxadiazol-3-yl | CF₃ | |
| 1631 | 2,4-(Me)₂Thiazol-5-yl | 1,3,4-oxadiazol-2-yl | H | |
| 1632 | 2,4-(Me)₂Thiazol-5-yl | 1,3,4-oxadiazol-2-yl | methyl | |
| 1633 | 2,4-(Me)₂Thiazol-5-yl | 1,3,4-oxadiazol-2-yl | i-propyl | |
| 1634 | 2,4-(Me)₂Thiazol-5-yl | 1,3,4-oxadiazol-2-yl | cyclopropyl | |
| 1635 | 2,4-(Me)₂Thiazol-5-yl | 1,3,4-oxadiazol-2-yl | CF₃ | |
| 1636 | 2,4-(Me)₂Thiazol-5-yl | 5-methylsulfonyl-1,3,4-oxadiazol-2-yl | H | |
| 1637 | 2,4-(Me)₂Thiazol-5-yl | 5-methylsulfonyl-1,3,4-oxadiazol-2-yl | methyl | |
| 1638 | 2,4-(Me)₂Thiazol-5-yl | 5-methylsulfonyl-1,3,4-oxadiazol-2-yl | i-propyl | |
| 1639 | 2,4-(Me)₂Thiazol-5-yl | 5-methylsulfonyl-1,3,4-oxadiazol-2-yl | cyclopropyl | |
| 1640 | 2,4-(Me)₂Thiazol-5-yl | 5-methylsulfonyl-1,3,4-oxadiazol-2-yl | CF₃ | |
| 1641 | 2,4-(Me)₂Thiazol-5-yl | 5-methyl-1,3,4-oxadiazol-2-yl | H | |
| 1642 | 2,4-(Me)₂Thiazol-5-yl | 5-methyl-1,3,4-oxadiazol-2-yl | methyl | |
| 1643 | 2,4-(Me)₂Thiazol-5-yl | 5-methyl-1,3,4-oxadiazol-2-yl | i-propyl | |
| 1644 | 2,4-(Me)₂Thiazol-5-yl | 5-methyl-1,3,4-oxadiazol-2-yl | cyclopropyl | |
| 1645 | 2,4-(Me)₂Thiazol-5-yl | 5-methyl-1,3,4-oxadiazol-2-yl | CF₃ | |
| 1646 | 2,4-(Me)₂Thiazol-5-yl | 5-trifluoromethyl-1,3,4-oxadiazol-2-yl | H | |
| 1647 | 2,4-(Me)₂Thiazol-5-yl | 5-trifluoromethyl-1,3,4-oxadiazol-2-yl | methyl | |
| 1648 | 2,4-(Me)₂Thiazol-5-yl | 5-trifluoromethyl-1,3,4-oxadiazol-2-yl | i-propyl | |
| 1649 | 2,4-(Me)₂Thiazol-5-yl | 5-trifluoromethyl-1,3,4-oxadiazol-2-yl | cyclopropyl | |
| 1650 | 2,4-(Me)₂Thiazol-5-yl | 5-trifluoromethyl-1,3,4-oxadiazol-2-yl | CF₃ | |
| 1651 | 2,4-(Me)₂Thiazol-5-yl | 1,2,3-triazol-4-yl | H | |
| 1652 | 2,4-(Me)₂Thiazol-5-yl | 1,2,3-triazol-4-yl | methyl | |
| 1653 | 2,4-(Me)₂Thiazol-5-yl | 1,2,3-triazol-4-yl | i-propyl | |
| 1654 | 2,4-(Me)₂Thiazol-5-yl | 1,2,3-triazol-4-yl | cyclopropyl | |
| 1655 | 2,4-(Me)₂Thiazol-5-yl | 1,2,3-triazol-4-yl | CF₃ | |
| 1656 | 2,4-(Me)₂Thiazol-5-yl | 1-methyl-1,2,3-triazol-4-yl | H | |
| 1657 | 2,4-(Me)₂Thiazol-5-yl | 1-methyl-1,2,3-triazol-4-yl | methyl | |
| 1658 | 2,4-(Me)₂Thiazol-5-yl | 1-methyl-1,2,3-triazol-4-yl | i-propyl | |
| 1659 | 2,4-(Me)₂Thiazol-5-yl | 1-methyl-1,2,3-triazol-4-yl | cyclopropyl | |
| 1660 | 2,4-(Me)₂Thiazol-5-yl | 1-methyl-1,2,3-triazol-4-yl | CF₃ | |
| 1661 | 2,4-(Me)₂Tbiazol-5-yl | 2-methyl-1,2,3-triazol-4-yl | H | |
| 1662 | 2,4-(Me)₂Thiazol-5-yl | 2-methyl-1,2,3-triazol-4-yl | methyl | |
| 1663 | 2,4-(Me)₂Thiazol-5-yl | 2-methyl-1,2,3-triazol-4-yl | i-propyl | |
| 1664 | 2,4-(Me)₂Thiazol-5-yl | 2-methyl-1,2,3-triazol-4-yl | cyclopropyl | |
| 1665 | 2,4-(Me)₂Thiazol-5-yl | 2-methyl-1,2,3-triazol-4-yl | CF₃ | |
| 1666 | 2,4-(Me)₂Thiazol-5-yl | 1,2,3-triazol-1-yl | H | |
| 1667 | 2,4-(Me)₂Thiazol-5-yl | 1,2,3-triazol-1-yl | methyl | |
| 1668 | 2,4-(Me)₂Thiazol-5-yl | 1,2,3-triazol-1-yl | i-propyl | |
| 1669 | 2,4-(Me)₂Thiazol-5-yl | 1,2,3-triazol-1-yl | cyclopropyl | |
| 1670 | 2,4-(Me)₂Thiazol-5-yl | 1,2,3-triazol-1-yl | CF₃ | |
| 1671 | 2,4-(Me)₂Thiazol-5-yl | 1,2,3-triazol-2-yl | H | |
| 1672 | 2,4-(Me)₂Thiazol-5-yl | 1,2,3-triazol-2-yl | methyl | |
| 1673 | 2,4-(Me)₂Thiazol-5-yl | 1,2,3-triazol-2-yl | i-propyl | |
| 1674 | 2,4-(Me)₂Thiazol-5-yl | 1,2,3-triazol-2-yl | cyclopropyl | |
| 1675 | 2,4-(Me)₂Thiazol-5-yl | 1,2,3-triazol-2-yl | CF₃ | |
| 1676 | 2,4-(Me)₂Thiazol-5-yl | 1,2,4-triazol-1-yl | H | |
| 1677 | 2,4-(Me)₂Thiazol-5-yl | 1,2,4-triazol-1-yl | methyl | |
| 1678 | 2,4-(Me)₂Thiazol-5-yl | 1,2,4-triazol-1-yl | i-propyl | |
| 1679 | 2,4-(Me)₂Thiazol-5-yl | 1,2,4-triazol-1-yl | cyclopropyl | |
| 1680 | 2,4-(Me)₂Thiazol-5-yl | 1,2,4-triazol-1-yl | CF₃ | |
| 1681 | 2,4-(Me)₂Thiazol-5-yl | imidazol-2-yl | H | |
| 1682 | 2,4-(Me)₂Thiazol-5-yl | imidazol-2-yl | methyl | |
| 1683 | 2,4-(Me)₂Thiazol-5-yl | imidazol-2-yl | i-propyl | |
| 1684 | 2,4-(Me)₂Thiazol-5-yl | imidazol-2-yl | cyclopropyl | |
| 1685 | 2,4-(Me)₂Thiazol-5-yl | imidazol-2-yl | CF₃ | |
| 1686 | 2,4-(Me)₂Thiazol-5-yl | imidazol-1-yl | H | |
| 1687 | 2,4-(Me)₂Thiazol-5-yl | imidazol-1-yl | methyl | |
| 1688 | 2,4-(Me)₂Thiazol-5-yl | imidazol-1-yl | i-propyl | |
| 1689 | 2,4-(Me)₂Thiazol-5-yl | imidazol-1-yl | cyclopropyl | |
| 1690 | 2,4-(Me)₂Thiazol-5-yl | imidazol-1-yl | CF₃ | |
| 1691 | 2,4-(Me)₂Thiazol-5-yl | imidazol-4-yl | H | |
| 1692 | 2,4-(Me)₂Thiazol-5-yl | imidazol-4-yl | methyl | |
| 1693 | 2,4-(Me)₂Thiazol-5-yl | imidazol-4-yl | i-propyl | |
| 1694 | 2,4-(Me)₂Thiazol-5-yl | imidazol-4-yl | cyclopropyl | |
| 1695 | 2,4-(Me)₂Thiazol-5-yl | imidazol-4-yl | CF₃ | |
| 1696 | 2,4-(Me)₂Thiazol-5-yl | thiazol-2-yl | H | |
| 1697 | 2,4-(Me)₂Thiazol-5-yl | thiazol-2-yl | methyl | |
| 1698 | 2,4-(Me)₂Thiazol-5-yl | thiazol-2-yl | i-propyl | |
| 1699 | 2,4-(Me)₂Thiazol-5-yl | thiazol-2-yl | cyclopropyl | |
| 1700 | 2,4-(Me)₂Thiazol-5-yl | thiazol-2-yl | CF₃ | |
| 1701 | 2,4-(Me)₂Thiazol-5-yl | 4-methylthiazol-2-yl | H | |
| 1702 | 2,4-(Me)₂Thiazol-5-yl | 4-methylthiazol-2-yl | methyl | |
| 1703 | 2,4-(Me)₂Thiazol-5-yl | 4-methylthiazol-2-yl | i-propyl | |
| 1704 | 2,4-(Me)₂Thiazol-5-yl | 4-methylthiazol-2-yl | cyclopropyl | |
| 1705 | 2,4-(Me)₂Thiazol-5-yl | 4-methylthiazol-2-yl | CF₃ | |
| 1706 | 2,4-(Me)₂Thiazol-5-yl | oxazol-2-yl | H | |
| 1707 | 2,4-(Me)₂Thiazol-5-yl | oxazol-2-yl | methyl | |
| 1708 | 2,4-(Me)₂Thiazol-5-yl | oxazol-2-yl | i-propyl | |
| 1709 | 2,4-(Me)₂Thiazol-5-yl | oxazol-2-yl | cyclopropyl | |
| 1710 | 2,4-(Me)₂Thiazol-5-yl | oxazol-2-yl | CF₃ | |
| 1700 | 2,4-(Me)₂Thiazol-5-yl | 4,5-dimethyloxazol-2-yl | H | |
| 1712 | 2,4-(Me)₂Thiazol-5-yl | 4,5-dimethyloxazol-2-yl | methyl | |
| 1713 | 2,4-(Me)₂Thiazol-5-yl | 4,5-dimethyloxazol-2-yl | i-propyl | |
| 1714 | 2,4-(Me)₂Thiazol-5-yl | 4,5-dimethyloxazol-2-yl | cyclopropyl | |
| 1715 | 2,4-(Me)₂Thiazol-5-yl | 4,5-dimethyloxazol-2-yl | CF₃ | |
| 1716 | 2,4-(Me)₂Thiazol-5-yl | 2-oxazolin-2-yl | H | |
| 1717 | 2,4-(Me)₂Thiazol-5-yl | 2-oxazolin-2-yl | methyl | |
| 1718 | 2,4-(Me)₂Thiazol-5-yl | 2-oxazolin-2-yl | i-propyl | |
| 1719 | 2,4-(Me)₂Thiazol-5-yl | 2-oxazolin-2-yl | cyclopropyl | |
| 1720 | 2,4-(Me)₂Thiazol-5-yl | 2-oxazolin-2-yl | CF₃ | |
| 1721 | 2,4-(Me)₂Thiazol-5-yl | 4,4-dimethyl-2-oxazolin-2-yl | H | |
| 1722 | 2,4-(Me)₂Thiazol-5-yl | 4,4-dimethyl-2-oxazolin-2-yl | methyl | |
| 1723 | 2,4-(Me)₂Thiazol-5-yl | 4,4-dimethyl-2-oxazolin-2-yl | i-propyl | |
| 1724 | 2,4-(Me)₂Thiazol-5-yl | 4,4-dimethyl-2-oxazolin-2-yl | cyclopropyl | |
| 1725 | 2,4-(Me)₂Thiazol-5-yl | 4,4-dimethyl-2-oxazolin-2-yl | CF₃ | |
| 1726 | 2,4-(Me)₂Thiazol-5-yl | 1,2,4-thiadiazol-5-yl | H | |
| 1727 | 2,4-(Me)₂Thiazol-5-yl | 1,2,4-thiadiazol-5-yl | methyl | |
| 1728 | 2,4-(Me)₂Thiazol-5-yl | 1,2,4-thiadiazol-5-yl | i-propyl | |
| 1729 | 2,4-(Me)₂Thiazol-5-yl | 1,2,4-thiadiazol-5-yl | cyclopropyl | |
| 1730 | 2,4-(Me)₂Thiazol-5-yl | 1,2,4-thiadiazol-5-yl | CF₃ | |
| 1731 | 2,4-(Me)₂Thiazol-5-yl | 3-methyl-1,2,4-thiadiazol-5-yl | H | |
| 1732 | 2,4-(Me)₂Thiazol-5-yl | 3-methyl-1,2,4-thiadiazol-5-yl | methyl | |
| 1733 | 2,4-(Me)₂Thiazol-5-yl | 3-methyl-1,2,4-thiadiazol-5-yl | i-propyl | |
| 1734 | 2,4-(Me)₂Thiazol-5-yl | 3-methyl-1,2,4-thiadiazol-5-yl | cyclopropyl | |
| 1735 | 2,4-(Me)₂Thiazol-5-yl | 3-methyl-1,2,4-thiadiazol-5-yl | CF₃ | |
| 1736 | 2,4-(Me)₂Thiazol-5-yl | 3-trifluoromethyl-1,2,4-thiadiazol-5-yl | H | |
| 1737 | 2,4-(Me)₂Thiazol-5-yl | 3-trifluoromethyl-1,2,4-thiadiazol-5-yl | methyl | |
| 1738 | 2,4-(Me)₂Thiazol-5-yl | 3 trifluoromethyl-1,2,4-thiadiazol-5-yl | i-propyl | |
| 1739 | 2,4-(Me)₂Thiazol-5-yl | 3-trifluoromethyl-1,2,4-thiadiazol-5-yl | cyclopropyl | |
| 1740 | 2,4-(Me)₂Thiazol-5-yl | 3-trifluoromethyl-1,2,4-thiadiazol-5-yl | CF₃ | |
| 1741 | 2,4-(Me)₂Thiazol-5-yl | 1,2,4-thiadiazol-3-yl | H | |
| 1742 | 2,4-(Me)₂Thiazol-5-yl | 1,2,4-thiadiazol-3-yl | methyl | |
| 1743 | 2,4-(Me)₂Thiazol-5-yl | 1,2,4-thiadiazol-3-yl | i-propyl | |
| 1744 | 2,4-(Me)₂Thiazol-5-yl | 1,2,4-thiadiazol-3-yl | cyclopropyl | |
| 1745 | 2,4-(Me)₂Thiazol-5-yl | 1,2,4-thiadiazol-3-yl | CF₃ | |
| 1746 | 2,4-(Me)₂Thiazol-5-yl | 5-methyl-1,2,4-thiadiazol-3-yl | H | |
| 1747 | 2,4-(Me)₂Thiazol-5-yl | 5-methyl-1,2,4-thiadiazol-3-yl | methyl | |
| 1748 | 2,4-(Me)₂Thiazol-5-yl | 5-methyl-1,2,4-thiadiazol-3-yl | i-propyl | |
| 1749 | 2,4-(Me)₂Thiazol-5-yl | 5-methyl-1,2,4-thiadiazol-3-yl | cyclopropyl | |
| 1750 | 2,4-(Me)₂Thiazol-5-yl | 5-methyl-1,2,4-thiadiazol-3-yl | CF₃ | |
| 1751 | 2,4-(Me)₂Thiazol-5-yl | 5-trifluoromethyl-1,2,4-thiadiazol-3-yl | H | |
| 1752 | 2,4-(Me)₂Thiazol-5-yl | 5-trifluoromethyl-1,2,4-thiadiazol-3-yl | methyl | |
| 1753 | 2,4-(Me)₂Thiazol-5-yl | 5-trifluoromethyl-1,2,4-thiadiazol-3-yl | i-propyl | |
| 1754 | 2,4-(Me)₂Thiazol-5-yl | 5-trifluoromethyl-1,2,4-thiadiazol-3-yl | cyclopropyl | |
| 1755 | 2,4-(Me)₂Thiazol-5-yl | 5-trifluoromethyl-1,2,4-thiadiazol-3-yl | CF₃ | |
| 1756 | 2,4-(Me)₂Thiazol-5-yl | 1,3,4-thiadiazol-2-yl | H | |
| 1757 | 2,4-(Me)₂Thiazol-5-yl | 1,3,4-thiadiazol-2-yl | methyl | |
| 1758 | 2,4-(Me)₂Thiazol-5-yl | 1,3,4-thiadiazol-2-yl | i-propyl | |
| 1759 | 2,4-(Me)₂Thiazol-5-yl | 1,3,4-thiadiazol-2-yl | cyclopropyl | |
| 1760 | 2,4-(Me)₂Thiazol-5-yl | 1,3,4-thiadiazol-2-yl | CF₃ | |
| 1761 | 2,4-(Me)₂Thiazol-5-yl | 5-methylsulfonyl-1,3,4-thiadiazol-2-yl | H | |
| 1762 | 2,4-(Me)₂Thiazol-5-yl | 5-methylsulfonyl-1,3,4-thiadiazol-2-yl | methyl | |
| 1763 | 2,4-(Me)₂Thiazol-5-yl | 5-methylsulfonyl-1,3,4-thiadiazol-2-yl | i-propyl | |
| 1764 | 2,4-(Me)₂Thiazol-5-yl | 5-methylsulfonyl-1,3,4-thiadiazol-2-yl | cyclopropyl | |
| 1765 | 2,4-(Me)₂Thiazol-5-yl | 5-methylsulfonyl-1,3,4-thiadiazol-2-yl | CF₃ | |
| 1766 | 2,4-(Me)₂Thiazol-5-yl | 5-methyl-1,3,4-thiadiazol-2-yl | H | |
| 1767 | 2,4-(Me)₂Thiazol-5-yl | 5-methyl-1,3,4-thiadiazol-2-yl | methyl | |
| 1768 | 2,4-(Me)₂Thiazol-5-yl | 5-methyl-1,3,4-thiadiazol-2-yl | i-propyl | |
| 1769 | 2,4-(Me)₂Thiazol-5-yl | 5-methyl-1,3,4-thiadiazol-2-yl | cyclopropyl | |
| 1770 | 2,4-(Me)₂Thiazol-5-yl | 5-methyl-1,3,4-thiadiazol-2-yl | CF₃ | |
| 1771 | 2,4-(Me)₂Thiazol-5-yl | benzoxazol-2-yl | H | |
| 1772 | 2,4-(Me)₂Thiazol-5-yl | benzoxazol-2-yl | methyl | |
| 1773 | 2,4-(Me)₂Thiazol-5-yl | benzoxazol-2-yl | i-propyl | |
| 1774 | 2,4-(Me)₂Thiazol-5-yl | benzoxazol-2-yl | cyclopropyl | |
| 1775 | 2,4-(Me)₂Thiazol-5-yl | benzoxazol-2-yl | CF₃ | |
| 1776 | 2,4-(Me)₂Thiazol-5-yl | 6-methylbenzoxazol-2-yl | H | |
| 1777 | 2,4-(Me)₂Thiazol-5-yl | 6-methylbenzoxazol-2-yl | methyl | |
| 1778 | 2,4-(Me)₂Thiazol-5-yl | 6-methylbenzoxazol-2-yl | i-propyl | |
| 1779 | 2,4-(Me)₂Thiazol-5-yl | 6-methylbenzoxazol-2-yl | cyclopropyl | |
| 1780 | 2,4-(Me)₂Thiazol-5-yl | 6-methylbenzoxazol-2-yl | CF₃ | |
| 1781 | 2,4-(Me)₂Thiazol-5-yl | benzothiazol-2-yl | H | |
| 1782 | 2,4-(Me)₂Thiazol-5-yl | benzothiazol-2-yl | methyl | |
| 1783 | 2,4-(Me)₂Thiazol-5-yl | benzothiazol-2-yl | i-propyl | |
| 1784 | 2,4-(Me)₂Thiazol-5-yl | benzothiazol-2-yl | cyclopropyl | |
| 1785 | 2,4-(Me)₂Thiazol-5-yl | benzothiazol-2-yl | CF₃ | |
| 1786 | 2,4-(Me)₂Thiazol-5-yl | pyrazol-1-yl | H | |
| 1787 | 2,4-(Me)₂Thiazol-5-yl | pyrazol-1-yl | methyl | |
| 1788 | 2,4-(Me)₂Thiazol-5-yl | pyrazol-1-yl | i-propyl | |
| 1789 | 2,4-(Me)₂Thiazol-5-yl | pyrazol-1-yl | cyclopropyl | |
| 1790 | 2,4-(Me)₂Thiazol-5-yl | pyrazol-1-yl | CF₃ | |
| 1791 | 2,4-(Me)₂Thiazol-5-yl | pyrazol-3-yl | H | |
| 1792 | 2,4-(Me)₂Thiazol-5-yl | pyrazol-3-yl | methyl | |
| 1793 | 2,4-(Me)₂Thiazol-5-yl | pyrazol-3-yl | i-propyl | |
| 1794 | 2,4-(Me)₂Thiazol-5-yl | pyrazol-3-yl | cyclopropyl | |
| 1795 | 2,4-(Me)₂Thiazol-5-yl | pyrazol-3-yl | CF₃ | |
| 1796 | 2,4-(Me)₂Thiazol-5-yl | 1-methylpyrazol-3-yl | H | |
| 1797 | 2,4-(Me)₂Thiazol-5-yl | 1-methylpyrazol-3-yl | methyl | |
| 1798 | 2,4-(Me)₂Thiazol-5-yl | 1-methylpyrazol-3-yl | i-propyl | |
| 1799 | 2,4-(Me)₂Thiazol-5-yl | 1-methylpyrazol-3-yl | cyclopropyl | |
| 1800 | 2,4-(Me)₂Thiazol-5-yl | 1-methylpyrazol-3-yl | CF₃ | |
| 1801 | 2,4-(Me)₂Thiazol-5-yl | tetrazol-1-yl | H | |
| 1802 | 2,4-(Me)₂Thiazol-5-yl | tetrazol-1-yl | methyl | |
| 1803 | 2,4-(Me)₂Thiazol-5-yl | tetrazol-1-yl | i-propyl | |
| 1804 | 2,4-(Me)₂Thiazol-5-yl | tetrazol-1-yl | cyclopropyl | |
| 1805 | 2,4-(Me)₂Thiazol-5-yl | tetrazol-1-yl | CF₃ | |
| 1806 | 2,4-(Me)₂Thiazol-5-yl | 5-methyltetrazol-1-yl | H | |
| 1807 | 2,4-(Me)₂Thiazol-5-yl | 5-methyltetrazol-1-yl | methyl | |
| 1808 | 2,4-(Me)₂Thiazol-5-yl | 5-methyltetrazol-1-yl | i-propyl | |
| 1809 | 2,4-(Me)₂Thiazol-5-yl | 5-methyltetrazol-1-yl | cyclopropyl | |
| 1810 | 2,4-(Me)₂Thiazol-5-yl | 5-methyltetrazol-1-yl | CF₃ | |
| 1811 | 2,4-(Me)₂Thiazol-5-yl | tetrazol-2-yl | H | |
| 1812 | 2,4-(Me)₂Thiazol-5-yl | tetrazol-2-yl | methyl | |
| 1813 | 2,4-(Me)₂Thiazol-5-yl | tetrazol-2-yl | i-propyl | |
| 1814 | 2,4-(Me)₂Thiazol-5-yl | tetrazol-2-yl | cyclopropyl | |
| 1815 | 2,4-(Me)₂Thiazol-5-yl | tetrazol-2-yl | CF₃ | |
| 1816 | 2,4-(Me)₂Thiazol-5-yl | 5-methyltetrazol-2-yl | H | |
| 1817 | 2,4-(Me)₂Thiazol-5-yl | 5-methyltetrazol-2-yl | methyl | |
| 1818 | 2,4-(Me)₂Thiazol-5-yl | 5-methyltetrazol-2-yl | i-propyl | |
| 1819 | 2,4-(Me)₂Thiazol-5-yl | 5-methyltetrazol-2-yl | cyclopropyl | |
| 1820 | 2,4-(Me)₂Thiazol-5-yl | 5-methyltetrazol-2-yl | CF₃ | |
| 1821 | 2,4-(Me)₂Thiazol-5-yl | 1-methyltetrazol-5-yl | H | |
| 1822 | 2,4-(Me)₂Thiazol-5-yl | 1-methyltetrazol-5-yl | methyl | |
| 1823 | 2,4-(Me)₂Thiazol-5-yl | 1-methyltetrazol-5-yl | i-propyl | |
| 1824 | 2,4-(Me)₂Thiazol-5-yl | 1-methyltetrazol-5-yl | cyclopropyl | |
| 1825 | 2,4-(Me)₂Thiazol-5-yl | 1-methyltetrazol-5-yl | CF₃ | |
| 1826 | 2,4-(Me)₂Thiazol-5-yl | 2-methyltetrazol-5-yl | H | |
| 1827 | 2,4-(Me)₂Thiazol-5-yl | 2-methyltetrazol-5-yl | methyl | |
| 1828 | 2,4-(Me)₂Thiazol-5-yl | 2-methyltetrazol-5-yl | i-propyl | |
| 1829 | 2,4-(Me)₂Thiazol-5-yl | 2-methyltetrazol-5-yl | cyclopropyl | |
| 1830 | 2,4-(Me)₂Thiazol-5-yl | 2-methyltetrazol-5-yl | CF₃ | |
| 1831 | 2,4-(Me)₂Thiazol-5-yl | pyridin-2-yl | H | |
| 1832 | 2,4-(Me)₂Thiazol-5-yl | pyridin-2-yl | methyl | |
| 1833 | 2,4-(Me)₂Thiazol-5-yl | pyridin-2-yl | i-propyl | |
| 1834 | 2,4-(Me)₂Thiazol-5-yl | pyridin-2-yl | cyclopropyl | |
| 1835 | 2,4-(Me)₂Thiazol-5-yl | pyridin-2-yl | CF₃ | |
| 1836 | 2,4-(Me)₂Thiazol-5-yl | pyridin-4-yl | H | |
| 1837 | 2,4-(Me)₂Thiazol-5-yl | pyridin-4-yl | methyl | |
| 1838 | 2,4-(Me)₂Thiazol-5-yl | pyridin-4-yl | i-propyl | |
| 1839 | 2,4-(Me)₂Thiazol-5-yl | pyridin-4-yl | cyclopropyl | |
| 1840 | 2,4-(Me)₂Thiazol-5-yl | pyridin-4-yl | CF₃ | |
| 1841 | 2,4-(Me)₂Thiazol-5-yl | pyridin-3-yl | H | |
| 1842 | 2,4-(Me)₂Thiazol-5-yl | pyridin-3-yl | methyl | |
| 1843 | 2,4-(Me)₂Thiazol-5-yl | pyridin-3-yl | i-propyl | |
| 1844 | 2,4-(Me)₂Thiazol-5-yl | pyridin-3-yl | cyclopropyl | |
| 1845 | 2,4-(Me)₂Thiazol-5-yl | pyridin-3-yl | CF₃ | |
| 1846 | 2,4-(Me)₂Thiazol-5-yl | 3-nitropyridin-4-yl | H | |
| 1847 | 2,4-(Me)₂Thiazol-5-yl | 3-nitropyridin-4-yl | methyl | |
| 1848 | 2,4-(Me)₂Thiazol-5-yl | 3-nitropyridin-4-yl | i-propyl | |
| 1849 | 2,4-(Me)₂Thiazol-5-yl | 3-nitropyridin-4-yl | cyclopropyl | |
| 1850 | 2,4-(Me)₂Thiazol-5-yl | 3-nitropyridin-4-yl | CF₃ | |
| 1851 | 2,4-(Me)₂Thiazol-5-yl | 5-cyanopyridin-2-yl | H | |
| 1852 | 2,4-(Me)₂Thiazol-5-yl | 5-cyanopyridin-2-yl | methyl | |
| 1853 | 2,4-(Me)₂Thiazol-5-yl | 5-cyanopyridin-2-yl | i-propyl | |
| 1854 | 2,4-(Me)₂Thiazol-5-yl | 5-cyanopyridin-2-yl | cyclopropyl | |
| 1855 | 2,4-(Me)₂Thiazol-5-yl | 5-cyanopyridin-2-yl | CF₃ | |
| 1856 | 2,4-(Me)₂Thiazol-5-yl | 5-trifluoromethylpyridin-2-yl | H | |
| 1857 | 2,4-(Me)₂Thiazol-5-yl | 5-trifluoromethylpyridin-2-yl | methyl | |
| 1858 | 2,4-(Me)₂Thiazol-5-yl | 5-trifluoromethylpyridin-2-yl | i-propyl | |
| 1859 | 2,4-(Me)₂Thiazol-5-yl | 5-trifluoromethylpyridin-2-yl | cyclopropyl | |
| 1860 | 2,4-(Me)₂Thiazol-5-yl | 5-trifluoromethylpyridin-2-yl | CF₃ | |
| 1861 | 2,4-(Me)₂Thiazol-5-yl | pyrimidin-2-yl | H | |
| 1862 | 2,4-(Me)₂Thiazol-5-yl | pyrimidin-2-yl | methyl | |
| 1863 | 2,4-(Me)₂Thiazol-5-yl | pyrimidin-2-yl | i-propyl | |
| 1864 | 2,4-(Me)₂Thiazol-5-yl | pyrimidin-2-yl | cyclopropyl | |
| 1865 | 2,4-(Me)₂Thiazol-5-yl | pyrimidin-2-yl | CF₃ | |
| 1866 | 2,4-(Me)₂Thiazol-5-yl | pyrimidin-4-yl | H | |
| 1867 | 2,4-(Me)₂Thiazol-5-yl | pyrimidin-4-yl | methyl | |
| 1868 | 2,4-(Me)₂Thiazol-5-yl | pyrimidin-4-yl | i-propyl | |
| 1869 | 2,4-(Me)₂Thiazol-5-yl | pyrimidin-4-yl | cyclopropyl | |
| 1870 | 2,4-(Me)₂Thiazol-5-yl | pyrimidin-4-yl | CF₃ | |
| 1871 | 2,4-(Me)₂Thiazol-5-yl | 6-chloropyrimidin-4-yl | methyl | |
| 1872 | 2,4-(Me)₂Thiazol-5-yl | 6-chloropyrimidin-4-yl | i-propyl | |
| 1873 | 2,4-(Me)₂Thiazol-5-yl | 6-chloropyrimidin-4-yl | cyclopropyl | |
| 1874 | 2,4-(Me)₂Thiazol-5-yl | 6-chloropyrimidin-4-yl | CF₃ | |
| 1875 | 2,4-(Me)₂Thiazol-5-yl | pyridazin-3-yl | H | |
| 1876 | 2,4-(Me)₂Thiazol-5-yl | pyridazin-3-yl | methyl | |
| 1877 | 2,4-(Me)₂Thiazol-5-yl | pyridazin-3-yl | i-propyl | |
| 1878 | 2,4-(Me)₂Thiazol-5-yl | pyridazin-3-yl | cyclopropyl | |
| 1879 | 2,4-(Me)₂Thiazol-5-yl | pyridazin-3-yl | CF₃ | |
| 1880 | 2,4-(Me)₂Thiazol-5-yl | 6-chloropyridazin-3-yl | methyl | |
| 1881 | 2,4-(Me)₂Thiazol-5-yl | 6-chloropyridazin-3-yl | i-propyl | |
| 1882 | 2,4-(Me)₂Thiazol-5-yl | 6-chloropyridazin-3-yl | cyclopropyl | |
| 1883 | 2,4-(Me)₂Thiazol-5-yl | 6-chloropyridazin-3-yl | CF₃ | |
| 1884 | 2,4-(Me)₂Thiazol-5-yl | pyrazin-2-yl | methyl | |
| 1885 | 2,4-(Me)₂Thiazol-5-yl | pyrazin-2-yl | i-propyl | |
| 1886 | 2,4-(Me)₂Thiazol-5-yl | pyrazin-2-yl | cyclopropyl | |
| 1887 | 2,4-(Me)₂Thiazol-5-yl | pyrazin-2-yl | CF₃ | |
| 1888 | 2,4-(Me)₂Thiazol-5-yl | triazin-2-yl | methyl | |
| 1889 | 2,4-(Me)₂Thiazol-5-yl | triazin-2-yl | i-propyl | |
| 1890 | 2,4-(Me)₂Thiazol-5-yl | triazin-2-yl | cyclopropyl | |
| 1891 | 2,4-(Me)₂Thiazol-5-yl | triazin-2-yl | CF₃ | |
| 1892 | 2,4-(Me)₂Thiazol-5-yl | quinolin-2-yl | methyl | |
| 1893 | 2,4-(Me)₂Thiazol-5-yl | quinolin-2-yl | i-propyl | |
| 1894 | 2,4-(Me)₂Thiazol-5-yl | quinolin-2-yl | cyclopropyl | |
| 1895 | 2,4-(Me)₂Thiazol-5-yl | quinolin-2-yl | CF₃ | |
| 1896 | 2,4-(Me)₂Thiazol-5-yl | 4,4,6-trimethyl-5,6-dihydro-1,3(4H)oxazin-2-yl | H | |
| 1897 | 2,4-(Me)₂Thiazol-5-yl | 4,4,6-trimethyl-5,6-dihydro-1,3(4H)oxazin-2-yl | methyl | |
| 1898 | 2,4-(Me)₂Thiazol-5-yl | 4,4,6-trimethyl-5,6-dihydro-1,3(4H)oxazin-2-yl | i-propyl | |
| 1899 | 2,4-(Me)₂Thiazol-5-yl | 4,4,6-trimethyl-5,6-dihydro-1,3(4H)oxazin-2-yl | cyclopropyl | |
| 1900 | 2,4-(Me)₂Thiazol-5-yl | 4,4,6-trimethyl-5,6-dihydro-1,3(4H)oxazin-2-yl | CF₃ | |
| 1901 | 2,4-(Me)₂Thiazol-5-yl | 2-oxazolidinon-3-yl | H | |
| 1902 | 2,4-(Me)₂Thiazol-5-yl | 2-oxazolidinon-3-yl | methyl | |
| 1903 | 2,4-(Me)₂Thiazol-5-yl | 2-oxazolidinon-3-yl | i-propyl | |
| 1904 | 2,4-(Me)₂Thiazol-5-yl | 2-oxazolidinon-3-yl | cyclopropyl | |
| 1905 | 2,4-(Me)₂Thiazol-5-yl | 2-oxazolidinon-3-yl | CF₃ | |
| 1906 | 2,4-(Me)₂Thiazol-5-yl | 2-pyrrolidinon-1-yl | methyl | |
| 1907 | 2,4-(Me)₂Thiazol-5-yl | 2-pyrrolidinon-1-yl | i-propyl | |
| 1908 | 2,4-(Me)₂Thiazol-5-yl | 2-pyrrolidinon-1-yl | cyclopropyl | |
| 1909 | 2,4-(Me)₂Thiazol-5-yl | 2-pyrrolidinon-1-yl | CF₃ | |
| 1910 | 2,4-(Me)₂Thiazol-5-yl | 3-methylisoxazol-5-yl | methyl | |
| 1911 | 2,4-(Me)₂Thiazol-5-yl | 3-methylisoxazol-5-yl | i-propyl | |
| 1912 | 2,4-(Me)₂Thiazol-5-yl | 3-methylisoxazol-5-yl | cyclopropyl | |
| 1913 | 2,4-(Me)₂Thiazol-5-yl | 3-methylisoxazol-5-yl | CF₃ | |
| 1914 | 2-Me-4-SO₂Me-3-(4,5-dihydroisoxazol-3-yl)Ph | 1,2,4-oxadiazol-5-yl | H | |
| 1915 | 2-Me-4-SO₂Me-3-(4,5-dihydroisoxazol-3-yl)Ph | 1,2,4-oxadiazol-5-yl | methyl | |
| 1916 | 2-Me-4-SO₂Me-3-(4,5-dihydroisoxazol-3-yl)Ph | 1,2,4-oxadiazol-5-yl | i-propyl | |
| 1917 | 2-Me-4-SO₂Me-3-(4,5-dihydroisoxazol-3-yl)Ph | 1,2,4-oxadiazol-5-yl | cyclopropyl | |
| 1918 | 2-Me-4-SO₂Me-3-(4,5-dihydroisoxazol-3-yl)Ph | 1,2,4-oxadiazol-5-yl | CF₃ | |
| 1919 | 2-Me-4-SO₂Me-3-(4,5-dihydroisoxazol-3-yl)Ph | 3-methyl-1,2,4-oxadiazol-5-yl | H | |
| 1920 | 2-Me-4-SO₂Me-3-(4,5-dihydroisoxazol-3-yl)Ph | 3-methyl-1,2,4-oxadiazol-5-yl | methyl | |
| 1921 | 2-Me-4-SO₂Me-3-(4,5-dihydroisoxazol-3-yl)Ph | 3-methyl-1,2,4-oxadiazol-5-yl | i-propyl | |
| 1922 | 2-Me-4-SO₂Me-3-(4,5-dihydroisoxazol-3-yl)Ph | 3-methyl-1,2,4-oxadiszol-5-yl | cyclopropyl | |
| 1923 | 2-Me-4-SO₂Me-3-(4,5-dihydroisoxazol-3-yl)Ph | 3-methyl-1,2,4-oxadiazol-5-yl | CF₃ | |
| 1924 | 2-Me-4-SO₂Me-3-(4,5-dihydroisoxazol-3-yl)Ph | 3-trifluoromethyl-1,2,4-oxadiazol-5-yl | H | |
| 1925 | 2-Me-4-SO₂Me-3-(4,5-dihydroisoxazol-3-yl)Ph | 3-trifluoromethyl-1,2,4-oxadiazol-5-yl | methyl | |
| 1926 | 2-Me-4-SO₂Me-3-(4,5-dihydroisoxazol-3-yl)Ph | 3-trifluoromethyl-1,2,4-oxadiazol-5-yl | i-propyl | |
| 1927 | 2-Me-4-SO₂Me-3-(4,5-dihydroisoxazol-3-yl)Ph | 3-trifluoromethyl-1,2,4-oxadiazol-5-yl | cyclopropyl | |
| 1928 | 2-Me-4-SO₂Me-3-(4,5-dihydroisoxazol-3-yl)Ph | 3-trifluoromethyl-1,2,4-oxadiazol-5-yl | CF₃ | |
| 1929 | 2-Me-4-SO₂Me-3-(4,5-dihydroisoxazol-3-yl)Ph | 1,2,4-oxadiazol-3-yl | H | |
| 1930 | 2-Me-4-SO₂Me-3-(4,5-dihydroisoxazol-3-yl)Ph | 1,2,4-oxadiazol-3-yl | methyl | |
| 1931 | 2-Me-4-SO₂Me-3-(4,5-dihydroisoxazol-3-yl)Ph | 1,2,4-oxadiazol-3-yl | i-propyl | |
| 1932 | 2-Me-4-SO₂Me-3-(4,5-dihydroisoxazol-3-yl)Ph | 1,2,4-oxadiazol-3-yl | cyclopropyl | |
| 1933 | 2-Me-4-SO₂Me-3-(4,5-dihydroisoxazol-3-yl)Ph | 1,2,4-oxadiazol-3-yl | CF₃ | |
| 1934 | 2-Me-4-SO₂Me-3-(4,5-dihydroisoxazol-3-yl)Ph | 5-methyl-1,2,4-oxadiazol-3-yl | H | |
| 1935 | 2-Me-4-SO₂Me-3-(4,5-dihydroisoxazol-3-yl)Ph | 5-methyl-1,2,4-oxadiazol-3-yl | methyl | |
| 1936 | 2-Me-4-SO₂Me-3-(4,5-dihydroisoxazol-3-yl)Ph | 5-methyl-1,2,4-oxadiazol-3-yl | i-propyl | |
| 1937 | 2-Me-4-SO₂Me-3-(4,5-dihydroisoxazol-3-yl)Ph | 5-methyl-1,2,4-oxadiazol-3-yl | cyclopropyl | |
| 1938 | 2-Me-4-SO₂Me-3-(4,5-dihydroisoxazol-3-yl)Ph | 5-methyl-1,2,4-oxadiazol-3-yl | CF₃ | |
| 1939 | 2-Me-4-SO₂Me-3-(4,5-dihydroisoxazol-3-yl)Ph | 5-trifluoromethyl-1,2,4-oxadiazol-3-yl | H | |
| 1940 | 2-Me-4-SO₂Me-3-(4,5-dihydroisoxazol-3-yl)Ph | 5-trifluoromethyl-1,2,4-oxadiazol-3-yl | methyl | |
| 1941 | 2-Me-4-SO₂Me-3-(4,5-dihydroisoxazol-3-yl)Ph | 5-trifluoromethyl-1,2,4-oxadiazol-3-yl | i-propyl | |
| 1942 | 2-Me-4-SO₂Me-3-(4,5-dihydroisoxazol-3-yl)Ph | 5-trifluoromethyl-1,2,4-oxadiazol-3-yl | cyclopropyl | |
| 1943 | 2-Me-4-SO₂Me-3-(4,5-dihydroisoxazol-3-yl)Ph | 5-trifluoromethyl-1,2,4-oxadiazol-3-yl | CF₃ | |
| 1944 | 2-Me-4-SO₂Me-3-(4,5-dihydroisoxazol-3-yl)Ph | 5-chloro-1,2,4-oxadiazol-3-yl | H | |
| 1945 | 2-Me-4-SO₂Me-3-(4,5-dihydroisoxazol-3-yl)Ph | 5-chloro-1,2,4-oxadiazol-3-yl | methyl | |
| 1946 | 2-Me-4-SO₂Me-3-(4,5-dihydroisoxazol-3-yl)Ph | 5-chloro-1,2,4-oxadiazol-3-yl | i-propyl | |
| 1947 | 2-Me-4-SO₂Me-3-(4,5-dihydroisoxazol-3-yl)Ph | 5-chloro-1,2,4-oxadiazol-3-yl | cyclopropyl | |
| 1948 | 2-Me-4-SO₂Me-3-(4,5-dihydroisoxazol-3-yl)Ph | 5-chloro-1,2,4-oxadiazol-3-yl | CF₃ | |
| 1949 | 2-Me-4-SO₂Me-3-(4,5-dihydroisoxazol-3-yl)Ph | 1,3,4-oxadiazol-2-yl | H | |
| 1950 | 2-Me-4-SO₂Me-3-(4,5-dihydroisoxazol-3-yl)Ph | 1,3,4-oxadiazol-2-yl | methyl | |
| 1951 | 2-Me-4-SO₂Me-3-(4,5-dihydroisoxazol-3-yl)Ph | 1,3,4-oxadiazol-2-yl | i-propyl | |
| 1952 | 2-Me-4-SO₂Me-3-(4,5-dihydroisoxazol-3-yl)Ph | 1,3,4-oxadiazol-2-yl | cyclopropyl | |
| 1953 | 2-Me-4-SO₂Me-3-(4,5-dihydroisoxazol-3-yl)Ph | 1,3,4-oxadiazol-2-yl | CF₃ | |
| 1954 | 2-Me-4-SO₂Me-3-(4,5-dihydroisoxazol-3-yl)Ph | 5-methylsulfonyl-1,3,4-oxadiazol-2-yl | H | - |
| 1955 | 2-Me-4-SO₂Me-3-(4,5-dihydroisoxazol-3-yl)Ph | 5-methylsulfonyl-1,3,4-oxadiazol-2-yl | methyl | |
| 1956 | 2-Me-4-SO₂Me-3-(4,5-dihydroisoxazol-3-yl)Ph | 5-methylsulfonyl-1,3,4-oxadiazol-2-yl | i-propyl | |
| 1957 | 2-Me-4-SO₂Me-3-(4,5-dihydroisoxazol-3-yl)Ph | 5-methylsulfonyl-1,3,4-oxadiazol-2-yl | cyclopropyl | |
| 1958 | 2-Me-4-SO₂Me-3-(4,5-dihydroisoxazol-3-yl)Ph | 5-methylsulfonyl-1,3,4-oxadiazol-2-yl | CF₃ | |
| 1959 | 2-Me-4-SO₂Me-3-(4,5-dihydroisoxazol-3-yl)Ph | 5-methyl-1,3,4-oxadiazol-2-yl | H | |
| 1960 | 2-Me-4-SO₂Me-3-(4,5-dihydroisoxazol-3-yl)Ph | 5-methyl-1,3,4-oxadiazol-2-yl | methyl | |
| 1961 | 2-Me-4-SO₂Me-3-(4,5-dihydroisoxazol-3-yl)Ph | 5-methyl-1,3,4-oxadiazol-2-yl | i-propyl | |
| 1962 | 2-Me-4-SO₂Me-3-(4,5-dihydroisoxazol-3-yl)Ph | 5-methyl-1,3,4-oxadiazol-2-yl | cyclopropyl | |
| 1963 | 2-Me-4-SO₂Me-3-(4,5-dihydroisoxazol-3-yl)Ph | 5-methyl-1,3,4-oxadiazol-2-yl | CF₃ | |
| 1964 | 2-Me-4-SO₂Me-3-(4,5-dihydroisoxazol-3-yl)Ph | 5-trifluoromethyl-1,3,4-oxadiazol-2-yl | H | |
| 1965 | 2-Me-4-SO₂Me-3-(4,5-dihydroisoxazol-3-yl)Ph | 5-trifluoromethyl-1,3,4-oxadiazol-2-yl | methyl | |
| 1966 | 2-Me-4-SO₂Me-3-(4,5-dihydroisoxazol-3-yl)Ph | 5-trifluoromethyl-1,3,4-oxadiazol-2-yl | i-propyl | |
| 1967 | 2-Me-4-SO₂Me-3-(4,5-dihydroisoxazol-3-yl)Ph | 5-trifluoromethyl-1,3,4-oxadiazol-2-yl | cyclopropyl | |
| 1968 | 2-Me-4-SO₂Me-3-(4,5-dihydroisoxazol-3-yl)Ph | 5-trifluoromethyl-1,3,4-oxadiazol-2-yl | CF₃ | |
| 1969 | 2-Me-4-SO₂Me-3-(4,5-dihydroisoxazol-3-yl)Ph | 1,2,3-triazol-4-yl | H | |
| 1970 | 2-Me-4-SO₂Me-3-(4,5-dihydroisoxazol-3-yl)Ph | 1,2,3-triazol-4-yl | methyl | |
| 1971 | 2-Me-4-SO₂Me-3-(4,5-dihydroisoxazol-3-yl)Ph | 1,2,3-triazol-4-yl | i-propyl | |
| 1972 | 2-Me-4-SO₂Me-3-(4,5-dihydroisoxazol-3-yl)Ph | 1,2,3-triazol-4-yl | cyclopropyl | |
| 1973 | 2-Me-4-SO₂Me-3-(4,5-dihydroisoxazol-3-yl)Ph | 1,2,3-triazol-4-yl | CF₃ | |
| 1974 | 2-Me-4-SO₂Me-3-(4,5-dihydroisoxazol-3-yl)Ph | 1-methyl-1,2,3-triazol-4-yl | H | |
| 1975 | 2-Me-4-SO₂Me-3-(4,5-dihydroisoxazol-3-yl)Ph | 1-methyl-1,2,3-triazol-4-yl | methyl | |
| 1976 | 2-Me-4-SO₂Me-3-(4,5-dihydroisoxazol-3-yl)Ph | 1-methyl-1,2,3-triazol-4-yl | i-propyl | |
| 1977 | 2-Me-4-SO₂Me-3-(4,5-dihydroisoxazol-3-yl)Ph | 1-methyl-1,2,3-triazol-4-yl | cyclopropyl | |
| 1978 | 2-Me-4-SO₂Me-3-(4,5-dihydroisoxazol-3-yl)Ph | 1-methyl-1,2,3-triazol-4-yl | CF₃ | |
| 1979 | 2-Me-4-SO₂Me-3-(4,5-dihydroisoxazol-3-yl)Ph | 2-methyl-1,2,3-triazol-4-yl | H | |
| 1980 | 2-Me-4-SO₂Me-3-(4,5-dihydroisoxazol-3-yl)Ph | 2-methyl-1,2,3-triazol-4-yl | methyl | |
| 1981 | 2-Me-4-SO₂Me-3-(4,5-dihydroisoxazol-3-yl)Ph | 2-methyl-1,2,3-triazol-4-yl | i-propyl | |
| 1982 | 2-Me-4-SO₂Me-3-(4,5-dihydroisoxazol-3-yl)Ph | 2-methyl-1,2,3-triazol-4-yl | cyclopropyl | |
| 1983 | 2-Me-4-SO₂Me-3-(4,5-dihydroisoxazol-3-yl)Ph | 2-methyl-1,2,3-triazol-4-yl | CF₃ | |
| 1984 | 2-Me-4-SO₂Me-3-(4,5-dihydroisoxazol-3-yl)Ph | 1,2,3-triazol-1-yl | H | |
| 1985 | 2-Me-4-SO₂Me-3-(4,5-dihydroisoxazol-3-yl)Ph | 1,2,3-triazol-1-yl | methyl | |
| 1986 | 2-Me-4-SO₂Me-3-(4,5-dihydroisoxazol-3-yl)Ph | 1,2,3-triazol-1-yl | i-propyl | |
| 1987 | 2-Me-4-SO₂Me-3-(4,5-dihydroisoxazol-3-yl)Ph | 1,2,3-triazol-1-yl | cyclopropyl | |
| 1988 | 2-Me-4-SO₂Me-3-(4,5-dihydroisoxazol-3-yl)Ph | 1,2,3-triazol-1-yl | CF₃ | |
| 1989 | 2-Me-4-SO₂Me-3-(4,5-dihydroisoxazol-3-yl)Ph | 1,2,3-triazol-2-yl | H | |
| 1990 | 2-Me-4-SO₂Me-3-(4,5-dihydroisoxazol-3-yl)Ph | 1,2,3-triazol-2-yl | methyl | |
| 1991 | 2-Me-4-SO₂Me-3-(4,5-dihydroisoxazol-3-yl)Ph | 1,2,3-triazol-2-yl | i-propyl | |
| 1992 | 2-Me-4-SO₂Me-3-(4,5-dihydroisoxazol-3-yl)Ph | 1,2,3-triazol-2-yl | cyclopropyl | |
| 1993 | 2-Me-4-SO₂Me-3-(4,5-dihydroisoxazol-3-yl)Ph | 1,2,3-triazol-2-yl | CF₃ | |
| 1994 | 2-Me-4-SO₂Me-3-(4,5-dihydroisoxazol-3-yl)Ph | 1,2,4-triazol-1-yl | H | |
| 1995 | 2-Me-4-SO₂Me-3-(4,5-dihydroisoxazol-3-yl)Ph | 1,2,4-triazol-1-yl | methyl | |
| 1996 | 2-Me-4-SO₂Me-3-(4,5-dihydroisoxazol-3-yl)Ph | 1,2,4-triazol-1-yl | i-propyl | |
| 1997 | 2-Me-4-SO₂Me-3-(4,5-dihydroisoxazol-3-yl)Ph | 1,2,4-triazol-1-yl | cyclopropyl | |
| 1998 | 2-Me-4-SO₂Me-3-(4,5-dihydroisoxazol-3-yl)Ph | 1,2,4-triazol-1-yl | CF₃ | |
| 1999 | 2-Me-4-SO₂Me-3-(4,5-dihydroisoxazol-3-yl)Ph | imidazol-2-yl | H | |
| 2000 | 2-Me-4-SO₂Me-3-(4,5-dihydroisoxazol-3-yl)Ph | imidazol-2-yl | methyl | |
| 2001 | 2-Me-4-SO₂Me-3-(4,5-dihydroisoxazol-3-yl)Ph | imidazol-2-yl | i-propyl | |
| 2002 | 2-Me-4-SO₂Me-3-(4,5-dihydroisoxazol-3-yl)Ph | imidazol-2-yl | cyclopropyl | |
| 2003 | 2-Me-4-SO₂Me-3-(4,5-dihydroisoxazol-3-yl)Ph | imidazol-2-yl | CF₃ | |
| 2004 | 2-Me-4-SO₂Me-3-(4,5-dihydroisoxazol-3-yl)Ph | imidazol-1-yl | H | |
| 2005 | 2-Me-4-SO₂Me-3-(4,5-dihydroisoxazol-3-yl)Ph | imidazol-1-yl | methyl | |
| 2006 | 2-Me-4-SO₂Me-3-(4,5-dihydroisoxazol-3-yl)Ph | imidazol-1-yl | i-propyl | |
| 2007 | 2-Me-4-SO₂Me-3-(4,5-dihydroisoxazol-3-yl)Ph | imidazol-1-yl | cyclopropyl | |
| 2008 | 2-Me-4-SO₂Me-3-(4,5-dihydroisoxazol-3-yl)Ph | imidazol-1-yl | CF₃ | |
| 2009 | 2-Me-4-SO₂Me-3-(4,5-dihydroisoxazol-3-yl)Ph | imidazol-4-yl | H | |
| 2010 | 2-Me-4-SO₂Me-3-(4,5-dihydroisoxazol-3-yl)Ph | imidazol-4-yl | methyl | |
| 2011 | 2-Me-4-SO₂Me-3-(4,5-dihydroisoxazol-3-yl)Ph | imidazol-4-yl | i-propyl | |
| 2012 | 2-Me-4-SO₂Me-3-(4,5-dihydroisoxazol-3-yl)Ph | imidazol-4-yl | cyclopropyl | |
| 2013 | 2-Me-4-SO₂Me-3-(4,5-dihydroisoxazol-3-yl)Ph | imidazol-4-yl | CF₃ | |
| 2014 | 2-Me-4-SO₂Me-3-(4,5-dihydroisoxazol-3-yl)Ph | thiazol-2-yl | H | |
| 2015 | 2-Me-4-SO₂Me-3-(4,5-dihydroisoxazol-3-yl)Ph | thiazol-2-yl | methyl | |
| 2016 | 2-Me-4-SO₂Me-3-(4,5-dihydroisoxazol-3-yl)Ph | thiazol-2-yl | i-propyl | |
| 2017 | 2-Me-4-SO₂Me-3-(4,5-dihydroisoxazol-3-yl)Ph | thiazol-2-yl | cyclopropyl | |
| 2018 | 2-Me-4-SO₂Me-3-(4,5-dihydroisoxazol-3-yl)Ph | thiazol-2-yl | CF₃ | |
| 2019 | 2-Me-4-SO₂Me-3-(4,5-dihydroisoxazol-3-yl)Ph | 4-methylthiazol-2-yl | H | |
| 2020 | 2-Me-4-SO₂Me-3-(4,5-dihydroisoxazol-3-yl)Ph | 4-methylthiazol-2-yl | methyl | |
| 2021 | 2-Me-4-SO₂Me-3-(4,5-dihydroisoxazol-3-yl)Ph | 4-methylthiazol-2-yl | i-propyl | |
| 2022 | 2-Me-4-SO₂Me-3-(4,5-dihydroisoxazol-3-yl)Ph | 4-methylthiazol-2-yl | cyclopropyl | |
| 2023 | 2-Me-4-SO₂Me-3-(4,5-dihydroisoxazol-3-yl)Ph | 4-methylthiazol-2-yl | CF₃ | |
| 2024 | 2-Me-4-SO₂Me-3-(4,5-dihydroisoxazol-3-yl)Ph | oxazol-2-yl | H | |
| 2025 | 2-Me-4-SO₂Me-3-(4,5-dihydroisoxazol-3-yl)Ph | oxazol-2-yl | methyl | |
| 2026 | 2-Me-4-SO₂Me-3-(4,5-dihydroisoxazol-3-yl)Ph | oxazol-2-yl | i-propyl | |
| 2027 | 2-Me-4-SO₂Me-3-(4,5-dihydroisoxazol-3-yl)Ph | oxazol-2-yl | cyclopropyl | |
| 2028 | 2-Me-4-SO₂Me-3-(4,5-dihydroisoxazol-3-yl)Ph | oxazol-2-yl | CF₃ | |
| 2029 | 2-Me-4-SO₂Me-3-(4,5-dihydroisoxazol-3-yl)Ph | 4,5-dimethyloxazol-2-yl | H | |
| 2030 | 2-Me-4-SO₂Me-3-(4,5-dihydroisoxazol-3-yl)Ph | 4,5-dimethyloxazol-2-yl | methyl | |
| 2031 | 2-Me-4-SO₂Me-3-(4,5-dihydroisoxazol-3-yl)Ph | 4,5-dimethyloxazol-2-yl | i-propyl | |
| 2032 | 2-Me-4-SO₂Me-3-(4,5-dihydroisoxazol-3-yl)Ph | 4,5-dimethyloxazol-2-yl | cyclopropyl | |
| 2033 | 2-Me-4-SO₂Me-3-(4,5-dihydroisoxazol-3-yl)Ph | 4,5-dimethyloxazol-2-yl | CF₃ | |
| 2034 | 2-Me-4-SO₂Me-3-(4,5-dihydroisoxazol-3-yl)Ph | 2-oxazolin-2-yl | H | |
| 2035 | 2-Me-4-SO₂Me-3-(4,5-dihydroisoxazol-3-yl)Ph | 2-oxazolin-2-yl | methyl | |
| 2036 | 2-Me-4-SO₂Me-3-(4,5-dihydroisoxazol-3-yl)Ph | 2-oxazolin-2-yl | i-propyl | |
| 2037 | 2-Me-4-SO₂Me-3-(4,5-dihydroisoxazol-3-yl)Ph | 2-oxazolin-2-yl | cyclopropyl | |
| 2038 | 2-Me-4-SO₂Me-3-(4,5-dihydroisoxazol-3-yl)Ph | 2-oxazolin-2-yl | CF₃ | |
| 2039 | 2-Me-4-SO₂Me-3-(4,5-dihydroisoxazol-3-yl)Ph | 4,4-dimethyl-2-oxazolin-2-yl | H | |
| 2040 | 2-Me-4-SO₂Me-3-(4,5-dihydroisoxazol-3-yl)Ph | 4,4-dimethyl-2-oxazolin-2-yl | methyl | |
| 2041 | 2-Me-4-SO₂Me-3-(4,5-dihydroisoxazol-3-yl)Ph | 4,4-dimethyl-2-oxazolin-2-yl | i-propyl | |
| 2042 | 2-Me-4-SO₂Me-3-(4,5-dihydroisoxazol-3-yl)Ph | 4,4-dimethyl-2-oxazolin-2-yl | cyclopropyl | |
| 2043 | 2-Me-4-SO₂Me-3-(4,5-dihydroisoxazol-3-yl)Ph | 4,4-dimethyl-2-oxazolin-2-yl | CF₃ | |
| 2044 | 2-Me-4-SO₂Me-3-(4,5-dihydroisoxazol-3-yl)Ph | 1,2,4-thiadiazol-5-yl | H | |
| 2045 | 2-Me-4-SO₂Me-3-(4,5-dihydroisoxazol-3-yl)Ph | 1,2,4-thiadiazol-5-yl | methyl | |
| 2046 | 2-Me-4-SO₂Me-3-(4,5-dihydroisoxazol-3-yl)Ph | 1,2,4-thiadiazol-5-yl | i-propyl | |
| 2047 | 2-Me-4-SO₂Me-3-(4,5-dihydroisoxazol-3-yl)Ph | 1,2,4-thiadiazol-5-yl | cyclopropyl | |
| 2048 | 2-Me-4-SO₂Me-3-(4,5-dihydroisoxazol-3-yl)Ph | 1,2,4-thiadiazol-5-yl | CF₃ | |
| 2049 | 2-Me-4-SO₂Me-3-(4,5-dihydroisoxazol-3-yl)Ph | 3-methyl-1,2,4-thiadiazol-5-yl | H | |
| 2050 | 2-Me-4-SO₂Me-3-(4,5-dihydroisoxazol-3-yl)Ph | 3-methyl-1,2,4-thiadiazol-5-yl | methyl | |
| 2051 | 2-Me-4-SO₂Me-3-(4,5-dihydroisoxazol-3-yl)Ph | 3-methyl-1,2,4-thiadiazol-5-yl | i-propyl | |
| 2052 | 2-Me-4-SO₂Me-3-(4,5-dihydroisoxazol-3-yl)Ph | 3-methyl-1,2,4-thiadiazol-5-yl | cyclopropyl | |
| 2053 | 2-Me-4-SO₂Me-3-(4,5-dihydroisoxazol-3-yl)Ph | 3-methyl-1,2,4-thiadiazol-5-yl | CF₃ | |
| 2054 | 2-Me-4-SO₂Me-3-(4,5-dihydroisoxazol-3-yl)Ph | 3-trifluoromethyl-1,2,4-thiadiazol-5-yl | H | |
| 2055 | 2-Me-4-SO₂Me-3-(4,5-dihydroisoxazol-3-yl)Ph | 3-trifluoromethyl-1,2,4-thiadiazol-5-yl | methyl | |
| 2056 | 2-Me-4-SO₂Me-3-(4,5-dihydroisoxazol-3-yl)Ph | 3-trifluoromethyl-1,2,4-thiadiazol-5-yl | i-propyl | |
| 2057 | 2-Me-4-SO₂Me-3-(4,5-dihydroisoxazol-3-yl)Ph | 3-trifluoromethyl-1,2,4-thiadiazol-5-yl | cyclopropyl | |
| 2058 | 2-Me-4-SO₂Me-3-(4,5-dihydroisoxazol-3-yl)Ph | 3-trifluoromethyl-1,2,4-thiadiazol-5-yl | CF₃ | |
| 2059 | 2-Me-4-SO₂Me-3-(4,5-dihydroisoxazol-3-yl)Ph | 1,2,4-thiadiazol-3-yl | H | |
| 2060 | 2-Me-4-SO₂Me-3-(4,5-dihydroisoxazol-3-yl)Ph | 1,2,4-thiadiazol-3-yl | methyl | |
| 2061 | 2-Me-4-SO₂Me-3-(4,5-dihydroisoxazol-3-yl)Ph | 1,2,4-thiadiazol-3-yl | i-propyl | |
| 2062 | 2-Me-4-SO₂Me-3-(4,5-dihydroisoxazol-3-yl)Ph | 1,2,4-thiadiazol-3-yl | cyclopropyl | |
| 2063 | 2-Me-4-SO₂Me-3-(4,5-dihydroisoxazol-3-yl)Ph | 1,2,4-thiadiazol-3-yl | CF₃ | |
| 2064 | 2-Me-4-SO₂Me-3-(4,5-dihydroisoxazol-3-yl)Ph | 5-methyl-1,2,4-thiadiazol-3-yl | H | |
| 2065 | 2-Me-4-SO₂Me-3-(4,5-dihydroisoxazol-3-yl)Ph | 5-methyl-1,2,4-thiadiazol-3-yl | methyl | |
| 2066 | 2-Me-4-SO₂Me-3-(4,5-dihydroisoxazol-3-yl)Ph | 5-methyl-1,2,4-thiadiazol-3-yl | i-propyl | |
| 2067 | 2-Me-4-SO₂Me-3-(4,5-dihydroisoxazol-3-yl)Ph | 5-methyl-1,2,4-thiadiazol-3-yl | cyclopropyl | |
| 2068 | 2-Me-4-SO₂Me-3-(4,5-dihydroisoxazol-3-yl)Ph | 5-methyl-1,2,4-thiadiazol-3-yl | CF₃ | |
| 2069 | 2-Me-4-SO₂Me-3-(4,5-dihydroisoxazol-3-yl)Ph | 5-trifluoromethyl-1,2,4-thiadiazol-3-yl | H | |
| 2070 | 2-Me-4-SO₂Me-3-(4,5-dihydroisoxazol-3-yl)Ph | 5-trifluoromethyl-1,2,4-thiadiazol-3-yl | methyl | |
| 2071 | 2-Me-4-SO₂Me-3-(4,5-dihydroisoxazol-3-yl)Ph | 5-trifluoromethyl-1,2,4-thiadiazol-3-yl | i-propyl | |
| 2072 | 2-Me-4-SO₂Me-3-(4,5-dihydroisoxazol-3-yl)Ph | 5-trifluoromethyl-1,2,4-thiadiazol-3-yl | cyclopropyl | |
| 2073 | 2-Me-4-SO₂Me-3-(4,5-dihydroisoxazol-3-yl)Ph | 5-trifluoromethyl-1,2,4-thiadiazol-3-yl | CF₃ | |
| 2074 | 2-Me-4-SO₂Me-3-(4,5-dihydroisoxazol-3-yl)Ph | 1,3,4-thiadiazol-2-yl | H | |
| 2075 | 2-Me-4-SO₂Me-3-(4,5-dihydroisoxazol-3-yl)Ph | 1,3,4-thiadiazol-2-yl | methyl | |
| 2076 | 2-Me-4-SO₂Me-3-(4,5-dihydroisoxazol-3-yl)Ph | 1,3,4-thiadiazol-2-yl | i-propyl | |
| 2077 | 2-Me-4-SO₂Me-3-(4,5-dihydroisoxazol-3-yl)Ph | 1,3,4-thiadiazol-2-yl | cyclopropyl | |
| 2078 | 2-Me-4-SO₂Me-3-(4,5-dihydroisoxazol-3-yl)Ph | 1,3,4-thiadiazol-2-yl | CF₃ | |
| 2079 | 2-Me-4-SO₂Me-3-(4,5-dihydroisoxazol-3-yl)Ph | 5-methylsulfonyl-1,3,4-thiadiazol-2-yl | H | |
| 2080 | 2-Me-4-SO₂Me-3-(4,5-dihydroisoxazol-3-yl)Ph | 5-methylsulfonyl-1,3,4-thiadiazol-2-yl | methyl | |
| 2081 | 2-Me-4-SO₂Me-3-(4,5-dihydroisoxazol-3-yl)Ph | 5-methylsulfonyl-1,3,4-thiadiazol-2-yl | i-propyl | |
| 2082 | 2-Me-4-SO₂Me-3-(4,5-dihydroisoxazol-3-yl)Ph | 5-methylsulfonyl-1,3,4-thiadiazol-2-yl | cyclopropyl | |
| 2083 | 2-Me-4-SO₂Me-3-(4,5-dihydroisoxazol-3-yl)Ph | 5-methylsulfonyl-1,3,4-thiadiazol-2-yl | CF₃ | |
| 2084 | 2-Me-4-SO₂Me-3-(4,5-dihydroisoxazol-3-yl)Ph | 5-methyl-1,3,4-thiadiazol-2-yl | H | |
| 2085 | 2-Me-4-SO₂Me-3-(4,5-dihydroisoxazol-3-yl)Ph | 5-methyl-1,3,4-thiadiazol-2-yl | methyl | |
| 2086 | 2-Me-4-SO₂Me-3-(4,5-dihydroisoxazol-3-yl)Ph | 5-methyl-1,3,4-thiadiazol-2-yl | i-propyl | |
| 2087 | 2-Me-4-SO₂Me-3-(4,5-dihydroisoxazol-3-yl)Ph | 5-methyl-1,3,4-thiadiazol-2-yl | cyclopropyl | |
| 2088 | 2-Me-4-SO₂Me-3-(4,5-dihydroisoxazol-3-yl)Ph | 5-methyl-1,3,4-thiadiazol-2-yl | CF₃ | |
| 2089 | 2-Me-4-SO₂Me-3-(4,5-dihydroisoxazol-3-yl)Ph | benzoxazol-2-yl | H | |
| 2090 | 2-Me-4-SO₂Me-3-(4,5-dihydroisoxazol-3-yl)Ph | benzoxazol-2-yl | methyl | |
| 2091 | 2-Me-4-SO₂Me-3-(4,5-dihydroisoxazol-3-yl)Ph | benzoxazol-2-yl | i-propyl | |
| 2092 | 2-Me-4-SO₂Me-3-(4,5-dihydroisoxazol-3-yl)Ph | benzoxazol-2-yl | cyclopropyl | |
| 2093 | 2-Me-4-SO₂Me-3-(4,5-dihydroisoxazol-3-yl)Ph | benzoxazol-2-yl | CF₃ | |
| 2094 | 2-Me-4-SO₂Me-3-(4,5-dihydroisoxazol-3-yl)Ph | 6-methylbenzoxazol-2-yl | H | |
| 2095 | 2-Me-4-SO₂Me-3-(4,5-dihydroisoxazol-3-yl)Ph | 6-methylbenzoxazol-2-yl | methyl | |
| 2096 | 2-Me-4-SO₂Me-3-(4,5-dihydroisoxazol-3-yl)Ph | 6-methylbenzoxazol-2-yl | i-propyl | |
| 2097 | 2-Me-4-SO₂Me-3-(4,5-dihydroisoxazol-3-yl)Ph | 6-methylbenzoxazol-2-yl | cyclopropyl | |
| 2098 | 2-Me-4-SO₂Me-3-(4,5-dihydroisoxazol-3-yl)Ph | 6-methylbenzoxazol-2-yl | CF₃ | |
| 2099 | 2-Me-4-SO₂Me-3-(4,5-dihydroisoxazol-3-yl)Ph | benzothiazol-2-yl | H | |
| 2100 | 2-Me-4-SO₂Me-3-(4,5-dihydroisoxazol-3-yl)Ph | benzothiazol-2-yl | methyl | |
| 2101 | 2-Me-4-SO₂Me-3-(4,5-dihydroisoxazol-3-yl)Ph | benzothiazol-2-yl | i-propyl | |
| 2102 | 2-Me-4-SO₂Me-3-(4,5-dihydroisoxazol-3-yl)Ph | benzothiazol-2-yl | cyclopropyl | |
| 2103 | 2-Me-4-SO₂Me-3-(4,5-dihydroisoxazol-3-yl)Ph | benzothiazol-2-yl | CF₃ | |
| 2104 | 2-Me-4-SO₂Me-3-(4,5-dihydroisoxazol-3-yl)Ph | pyrazol-1-yl | H | |
| 2105 | 2-Me-4-SO₂Me-3-(4,5-dihydroisoxazol-3-yl)Ph | pyrazol-1-yl | methyl | |
| 2106 | 2-Me-4-SO₂Me-3-(4,5-dihydroisoxazol-3-yl)Ph | pyrazol-1-yl | i-propyl | |
| 2107 | 2-Me-4-SO₂Me-3-(4,5-dihydroisoxazol-3-yl)Ph | pyrazol-1-yl | cyclopropyl | |
| 2108 | 2-Me-4-SO₂Me-3-(4,5-dihydroisoxazol-3-yl)Ph | pyrazol-1-yl | CF₃ | |
| 2109 | 2-Me-4-SO₂Me-3-(4,5-dihydroisoxazol-3-yl)Ph | pyrazol-3-yl | H | |
| 2110 | 2-Me-4-SO₂Me-3-(4,5-dihydroisoxazol-3-yl)Ph | pyrazol-3-yl | methyl | |
| 2111 | 2-Me-4-SO₂Me-3-(4,5-dihydroisoxazol-3-yl)Ph | pyrazol-3-yl | i-propyl | |
| 2112 | 2-Me-4-SO₂Me-3-(4,5-dihydroisoxazol-3-yl)Ph | pyrazol-3-yl | cyclopropyl | |
| 2113 | 2-Me-4-SO₂Me-3-(4,5-dihydroisoxazol-3-yl)Ph | pyrazol-3-yl | CF₃ | |
| 2114 | 2-Me-4-SO₂Me-3-(4,5-dihydroisoxazol-3-yl)Ph | 1-methylpyrazol-3-yl | H | |
| 2115 | 2-Me-4-SO₂Me-3-(4,5-dihydroisoxazol-3-yl)Ph | 1-methylpyrazol-3-yl | methyl | |
| 2116 | 2-Me-4-SO₂Me-3-(4,5-dihydroisoxazol-3-yl)Ph | 1-methylpyrazol-3-yl | i-propyl | |
| 2117 | 2-Me-4-SO₂Me-3-(4,5-dihydroisoxazol-3-yl)Ph | 1-methylpyrazol-3-yl | cyclopropyl | |
| 2118 | 2-Me-4-SO₂Me-3-(4,5-dihydroisoxazol-3-yl)Ph | 1-methylpyrazol-3-yl | CF₃ | |
| 2119 | 2-Me-4-SO₂Me-3-(4,5-dihydroisoxazol-3-yl)Ph | tetrazol-1-yl | H | |
| 2120 | 2-Me-4-SO₂Me-3-(4,5-dihydroisoxazol-3-yl)Ph | tetrazol-1-yl | methyl | |
| 2121 | 2-Me-4-SO₂Me-3-(4,5-dihydroisoxazol-3-yl)Ph | tetrazol-1-yl | i-propyl | |
| 2122 | 2-Me-4-SO₂Me-3-(4,5-dihydroisoxazol-3-yl)Ph | tetrazol-1-yl | cyclopropyl | |
| 2123 | 2-Me-4-SO₂Me-3-(4,5-dihydroisoxazol-3-yl)Ph | tetrazol-1-yl | CF₃ | |
| 2124 | 2-Me-4-SO₂Me-3-(4,5-dihydroisoxazol-3-yl)Ph | 5-methyltetrazol-1-yl | H | |
| 2125 | 2-Me-4-SO₂Me-3-(4,5-dihydroisoxazol-3-yl)Ph | 5-methyltetrazol-1-yl | methyl | |
| 2126 | 2-Me-4-SO₂Me-3-(4,5-dihydroisoxazol-3-yl)Ph | 5-methyltetrazol-1-yl | i-propyl | |
| 2127 | 2-Me-4-SO₂Me-3-(4,5-dihydroisoxazol-3-yl)Ph | 5-methyltetrazol-1-yl | cyclopropyl | |
| 2128 | 2-Me-4-SO₂Me-3-(4,5-dihydroisoxazol-3-yl)Ph | 5-methyltetrazol-1-yl | CF₃ | |
| 2129 | 2-Me-4-SO₂Me-3-(4,5-dihydroisoxazol-3-yl)Ph | tetrazol-2-yl | H | |
| 2130 | 2-Me-4-SO₂Me-3-(4,5-dihydroisoxazol-3-yl)Ph | tetrazol-2-yl | methyl | |
| 2131 | 2-Me-4-SO₂Me-3-(4,5-dihydroisoxazol-3-yl)Ph | tetrazol-2-yl | i-propyl | |
| 2132 | 2-Me-4-SO₂Me-3-(4,5-dihydroisoxazol-3-yl)Ph | tetrazol-2-yl | cyclopropyl | |
| 2133 | 2-Me-4-SO₂Me-3-(4,5-dihydroisoxazol-3-yl)Ph | tetrazol-2-yl | CF₃ | |
| 2134 | 2-Me-4-SO₂Me-3-(4,5-dihydroisoxazol-3-yl)Ph | 5-methyltetrazol-2-yl | H | |
| 2135 | 2-Me-4-SO₂Me-3-(4,5-dihydroisoxazol-3-yl)Ph | 5-methyltetrazol-2-yl | methyl | |
| 2136 | 2-Me-4-SO₂Me-3-(4,5-dihydroisoxazol-3-yl)Ph | 5-methyltetrazol-2-yl | i-propyl | |
| 2137 | 2-Me-4-SO₂Me-3-(4,5-dihydroisoxazol-3-yl)Ph | 5-methyltetrazol-2-yl | cyclopropyl | |
| 2138 | 2-Me-4-SO₂Me-3-(4,5-dihydroisoxazol-3-yl)Ph | 5-methyltetrazol-2-yl | CF₃ | |
| 2139 | 2-Me-4-SO₂Me-3-(4,5-dihydroisoxazol-3-yl)Ph | 1-methyltetrazol-5-yl | H | |
| 2140 | 2-Me-4-SO₂Me-3-(4,5-dihydroisoxazol-3-yl)Ph | 1-methyltetrazol-5-yl | methyl | |
| 2141 | 2-Me-4-SO₂Me-3-(4,5-dihydroisoxazol-3-yl)Ph | 1-methyltetrazol-5-yl | i-propyl | |
| 2142 | 2-Me-4-SO₂Me-3-(4,5-dihydroisoxazol-3-yl)Ph | 1-methyltetrazol-5-yl | cyclopropyl | |
| 2143 | 2-Me-4-SO₂Me-3-(4,5-dihydroisoxazol-3-yl)Ph | 1-methyltetrazol-5-yl | CF₃ | |
| 2144 | 2-Me-4-SO₂Me-3-(4,5-dihydroisoxazol-3-yl)Ph | 2-methyltetrazol-5-yl | H | |
| 2145 | 2-Me-4-SO₂Me-3-(4,5-dihydroisoxazol-3-yl)Ph | 2-methyltetrazol-5-yl | methyl | |
| 2146 | 2-Me-4-SO₂Me-3-(4,5-dihydroisoxazol-3-yl)Ph | 2-methyltetrazol-5-yl | i-propyl | |
| 2147 | 2-Me-4-SO₂Me-3-(4,5-dihydroisoxazol-3-yl)Ph | 2-methyltetrazol-5-yl | cyclopropyl | |
| 2148 | 2-Me-4-SO₂Me-3-(4,5-dihydroisoxazol-3-yl)Ph | 2-methyltetrazol-5-yl | CF₃ | |
| 2149 | 2-Me-4-SO₂Me-3-(4,5-dihydroisoxazol-3-yl)Ph | pyridin-2-yl | H | |
| 2150 | 2-Me-4-SO₂Me-3-(4,5-dihydroisoxazol-3-yl)Ph | pyridin-2-yl | methyl | |
| 2151 | 2-Me-4-SO₂Me-3-(4,5-dihydroisoxazol-3-yl)Ph | pyridin-2-yl | i-propyl | |
| 2152 | 2-Me-4-SO₂Me-3-(4,5-dihydroisoxazol-3-yl)Ph | pyridin-2-yl | cyclopropyl | |
| 2153 | 2-Me-4-SO₂Me-3-(4,5-dihydroisoxazol-3-yl)Ph | pyridin-2-yl | CF₃ | |
| 2154 | 2-Me-4-SO₂Me-3-(4,5-dihydroisoxazol-3-yl)Ph | pyridin4-yl | H | |
| 2155 | 2-Me-4-SO₂Me-3-(4,5-dihydroisoxazol-3-yl)Ph | pyridin-4-yl | methyl | |
| 2156 | 2-Me-4-SO₂Me-3-(4,5-dihydroisoxazol-3-yl)Ph | pyridin-4-yl | i-propyl | |
| 2157 | 2-Me-4-SO₂Me-3-(4,5-dihydroisoxazol-3-yl)Ph | pyridin-4-yl | cyclopropyl | |
| 2158 | 2-Me-4-SO₂Me-3-(4,5-dihydroisoxazol-3-yl)Ph | pyridin-4-yl | CF₃ | |
| 2159 | 2-Me-4-SO₂Me-3-(4,5-dihydroisoxazol-3-yl)Ph | pyridin-3-yl | H | |
| 2160 | 2-Me-4-SO₂Me-3-(4,5-dihydroisoxazol-3-yl)Ph | pyridin-3-yl | methyl | |
| 2161 | 2-Me-4-SO₂Me-3-(4,5-dihydroisoxazol-3-yl)Ph | pyridin-3-yl | i-propyl | |
| 2162 | 2-Me-4-SO₂Me-3-(4,5-dihydroisoxazol-3-yl)Ph | pyridin-3-yl | cyclopropyl | |
| 2163 | 2-Me-4-SO₂Me-3-(4,5-dihydroisoxazol-3-yl)Ph | pyridin-3-yl | CF₃ | |
| 2164 | 2-Me-4-SO₂Me-3-(4,5-dihydroisoxazol-3-yl)Ph | 3-nitropyridin-4-yl | H | |
| 2165 | 2-Me-4-SO₂Me-3-(4,5-dihydroisoxazol-3-yl)Ph | 3-nitropyridin-4-yl | methyl | |
| 2166 | 2-Me-4-SO₂Me-3-(4,5-dihydroisoxazol-3-yl)Ph | 3-nitropyridin-4-yl | i-propyl | |
| 2167 | 2-Me-4-SO₂Me-3-(4,5-dihydroisoxazol-3-yl)Ph | 3-nitropyridin-4-yl | cyclopropyl | |
| 2168 | 2-Me-4-SO₂Me-3-(4,5-dihydroisoxazol-3-yl)Ph | 3-nitropyridin-4-yl | CF₃ | |
| 2169 | 2-Me-4-SO₂Me-3-(4,5-dihydroisoxazol-3-yl)Ph | 5-cyanopyridin-2-yl | H | |
| 2170 | 2-Me-4-SO₂Me-3-(4,5-dihydroisoxazol-3-yl)Ph | 5-cyanopyridin-2-yl | methyl | |
| 2171 | 2-Me-4-SO₂Me-3-(4,5-dihydroisoxazol-3-yl)Ph | 5-cyanopyridin-2-yl | i-propyl | |
| 2172 | 2-Me-4-SO₂Me-3-(4,5-dihydroisoxazol-3-yl)Ph | 5-cyanopyridin-2-yl | cyclopropyl | |
| 2173 | 2-Me-4-SO₂Me-3-(4,5-dihydroisoxazol-3-yl)Ph | 5-cyanopyridin-2-yl | CF₃ | |
| 2174 | 2-Me-4-SO₂Me-3-(4,5-dihydroisoxazol-3-yl)Ph | 5-trifluoromethylpyridin-2-yl | H | |
| 2175 | 2-Me-4-SO₂Me-3-(4,5-dihydroisoxazol-3-yl)Ph | 5-trifluoromethylpyridin-2-yl | methyl | |
| 2176 | 2-Me-4-SO₂Me-3-(4,5-dihydroisoxazol-3-yl)Ph | 5-trifluoromethylpyridin-2-yl | i-propyl | |
| 2177 | 2-Me-4-SO₂Me-3-(4,5-dihydroisoxazol-3-yl)Ph | 5-trifluoromethylpyridin-2-yl | cyclopropyl | |
| 2178 | 2-Me-4-SO₂Me-3-(4,5-dihydroisoxazol-3-yl)Ph | 5-trifluoromethylpyridin-2-yl | CF₃ | |
| 2179 | 2-Me-4-SO₂Me-3-(4,5-dihydroisoxazol-3-yl)Ph | pyrimidin-2-yl | H | |
| 2180 | 2-Me-4-SO₂Me-3-(4,5-dihydroisoxazol-3-yl)Ph | pyrimidin-2-yl | methyl | |
| 2181 | 2-Me-4-SO₂Me-3-(4,5-dihydroisoxazol-3-yl)Ph | pyrimidin-2-yl | i-propyl | |
| 2182 | 2-Me-4-SO₂Me-3-(4,5-dihydroisoxazol-3-yl)Ph | pyrimidin-2-yl | cyclopropyl | |
| 2183 | 2-Me-4-SO₂Me-3-(4,5-dihydroisoxazol-3-yl)Ph | pyrimidin-2-yl | CF₃ | |
| 2184 | 2-Me-4-SO₂Me-3-(4,5-dihydroisoxazol-3-yl)Ph | pyrimidin-4-yl | H | |
| 2185 | 2-Me-4-SO₂Me-3-(4,5-dihydroisoxazol-3-yl)Ph | pyrimidin-4-yl | methyl | |
| 2186 | 2-Me-4-SO₂Me-3-(4,5-dihydroisoxazol-3-yl)Ph | pyrimidin4-yl | i-propyl | |
| 2187 | 2-Me-4-SO₂Me-3-(4,5-dihydroisoxazol-3-yl)Ph | pyrimidin-4-yl | cyclopropyl | |
| 2188 | 2-Me-4-SO₂Me-3-(4,5-dihydroisoxazol-3-yl)Ph | pyrimidin-4-yl | CF₃ | |
| 2189 | 2-Me-4-SO₂Me-3-(4,5-dihydroisoxazol-3-yl)Ph | 6-chloropyrimidin4-yl | methyl | |
| 2190 | 2-Me-4-SO₂Me-3-(4,5-dihydroisoxazol-3-yl)Ph | 6-chloropyrimidin-4-yl | i-propyl | |
| 2191 | 2-Me-4-SO₂Me-3-(4,5-dihydroisoxazol-3-yl)Ph | 6-chloropyrimidin4-yl | cyclopropyl | |
| 2192 | 2-Me-4-SO₂Me-3-(4,5-dihydroisoxazol-3-yl)Ph | 6-chloropyrimidin-4-yl | CF₃ | |
| 2193 | 2-Me-4-SO₂Me-3-(4,5-dihydroisoxazol-3-yl)Ph | pyridazin-3-yl | H | |
| 2194 | 2-Me-4-SO₂Me-3-(4,5-dihydroisoxazol-3-yl)Ph | pyridazin-3-yl | methyl | |
| 2195 | 2-Me-4-SO₂Me-3-(4,5-dihydroisoxazol-3-yl)Ph | pyridazin-3-yl | i-propyl | |
| 2196 | 2-Me-4-SO₂Me-3-(4,5-dihydroisoxazol-3-yl)Ph | pyridazin-3-yl | cyclopropyl | |
| 2197 | 2-Me-4-SO₂Me-3-(4,5-dihydroisoxazol-3-yl)Ph | pyridazin-3-yl | CF₃ | |
| 2198 | 2-Me-4-SO₂Me-3-(4,5-dihydroisoxazol-3-yl)Ph | 6-chloropyridazin-3-yl | methyl | |
| 2199 | 2-Me-4-SO₂Me-3-(4,5-dihydroisoxazol-3-yl)Ph | 6-chloropyridazin-3-yl | i-propyl | |
| 2200 | 2-Me-4-SO₂Me-3-(4,5-dihydroisoxazol-3-yl)Ph | 6-chloropyridazin-3-yl | cyclopropyl | |
| 2201 | 2-Me-4-SO₂Me-3-(4,5-dihydroisoxazol-3-yl)Ph | 6-chloropyridazin-3-yl | CF₃ | |
| 2202 | 2-Me-4-SO₂Me-3-(4,5-dihydroisoxazol-3-yl)Ph | pyrazin-2-yl | methyl | |
| 2203 | 2-Me-4-SO₂Me-3-(4,5-dihydroisoxazol-3-yl)Ph | pyrazin-2-yl | i-propyl | |
| 2204 | 2-Me-4-SO₂Me-3-(4,5-dihydroisoxazol-3-yl)Ph | pyrazin-2-yl | cyclopropyl | |
| 2205 | 2-Me-4-SO₂Me-3-(4,5-dihydroisoxazol-3-yl)Ph | pyrazin-2-yl | CF₃ | |
| 2206 | 2-Me-4-SO₂Me-3-(4,5-dihydroisoxazol-3-yl)Ph | triazin-2-yl | methyl | |
| 2207 | 2-Me-4-SO₂Me-3-(4,5-dihydroisoxazol-3-yl)Ph | triazin-2-yl | i-propyl | |
| 2208 | 2-Me-4-SO₂Me-3-(4,5-dihydroisoxazol-3-yl)Ph | triazin-2-yl | cyclopropyl | |
| 2209 | 2-Me-4-SO₂Me-3-(4,5-dihydroisoxazol-3-yl)Ph | triazin-2-yl | CF₃ | |
| 2210 | 2-Me-4-SO₂Me-3-(4,5-dihydroisoxazol-3-yl)Ph | quinolin-2-yl | methyl | |
| 2211 | 2-Me-4-SO₂Me-3-(4,5-dihydroisoxazol-3-yl)Ph | quinolin-2-yl | i-propyl | |
| 2212 | 2-Me-4-SO₂Me-3-(4,5-dihydroisoxazol-3-yl)Ph | quinolin-2-yl | cyclopropyl | |
| 2213 | 2-Me-4-SO₂Me-3-(4,5-dihydroisoxazol-3-yl)Ph | quinolin-2-yl | CF₃ | |
| 2214 | 2-Me-4-SO₂Me-3-(4,5-dihydroisoxazol-3-yl)Ph | 4,4,6-trimethyl-5,6-dihydro-1,3(4H)-oxazin-2-yl | H | |
| 2215 | 2-Me-4-SO₂Me-3-(4,5-dihydroisoxazol-3-yl)Ph | 4,4,6-trimethyl-5,6-dihydro-1,3(4H)-oxazin-2-yl | methyl | |
| 2216 | 2-Me-4-SO₂Me-3-(4,5-dihydroisoxazol-3-yl)Ph | 4,4,6-trimethyl-5,6-dihydro-1,3(4H)-oxazin-2-yl | i-propyl | |
| 2217 | 2-Me-4-SO₂Me-3-(4,5-dihydroisoxazol-3-yl)Ph | 4,4,6-trimethyl-5,6-dihydro-1,3(4H)-oxazin-2-yl | cyclopropyl | |
| 2218 | 2-Me-4-SO₂Me-3-(4,5-dihydroisoxazol-3-yl)Ph | 4,4,6-trimethyl-5,6-dihydro-1,3(4H)-oxazin-2-yl | CF₃ | |
| 2219 | 2-Me-4-SO₂Me-3-(4,5-dihydroisoxazol-3-yl)Ph | 2-oxazolidinon-3-yl | H | |
| 2220 | 2-Me-4-SO₂Me-3-(4,5-dihydroisoxazol-3-yl)Ph | 2-oxazolidinon-3-yl | methyl | |
| 2221 | 2-Me-4-SO₂Me-3-(4,5-dihydroisoxazol-3-yl)Ph | 2-oxazolidinon-3-yl | i-propyl | |
| 2222 | 2-Me-4-SO₂Me-3-(4,5-dihydroisoxazol-3-yl)Ph | 2-oxazolidinon-3-yl | cyclopropyl | |
| 2223 | 2-Me-4-SO₂Me-3-(4,5-dihydroisoxazol-3-yl)Ph | 2-oxazolidinon-3-yl | CF₃ | |
| 2224 | 2-Me-4-SO₂Me-3-(4,5-dihydroisoxazol-3-yl)Ph | 2-pyrrolidinon-1-yl | methyl | |
| 2225 | 2-Me-4-SO₂Me-3-(4,5-dihydroisoxazol-3-yl)Ph | 2-pyrrolidinon-1-yl | i-propyl | |
| 2226 | 2-Me-4-SO₂Me-3-(4,5-dihydroisoxazol-3-yl)Ph | 2-pyrrolidinon-1-yl | cyclopropyl | |
| 2227 | 2-Me-4-SO₂Me-3-(4,5-dihydroisoxazol-3-yl)Ph | 2-pyrrolidinon-1-yl | CF₃ | |
| 2228 | 2-Me-4-SO₂Me-3-(4,5-dihydroisoxazol-3-yl)Ph | 3-methylisoxazol-5-yl | methyl | |
| 2229 | 2-Me-4-SO₂Me-3-(4,5-dihydroisoxazol-3-yl)Ph | 3-methylisoxazol-5-yl | i-propyl | |
| 2230 | 2-Me-4-SO₂Me-3-(4,5-dihydroisoxazol-3-yl)Ph | 3-methylisoxazol-5-yl | cyclopropyl | |
| 2231 | 2-Me-4-SO₂Me-3-(4,5-dihydroisoxazol-3-yl)Ph | 3-methylisoxazol-5-yl | CF₃ | |
| 2232 | 4,4-dioxide-8-Mo-2,3-dihydro-1,4-benzoxathiin-7-yl | 1,2,4-oxadiazol-5-yl | H | |
| 2233 | 4,4-dioxide-8-Mo-2,3-dihydro-1,4-benzoxathiin-7-yl | 1,2,4-oxadiazol-5-yl | methyl | |
| 2234 | 4,4-dioxide-8-Mo-2,3-dihydro-1,4-benzoxathiin-7-yl | 1,2,4-oxadiazol-5-yl | i-propyl | |
| 2235 | 4,4-dioxide-8-Mo-2,3-dihydro-1,4-benzoxathiin-7-yl | 1,2,4-oxadiazol-5-yl | cyclopropyl | |
| 2236 | 4,4-dioxide-8-Mo-2,3-dihydro-1,4-benzoxathiin-7-yl | 1,2,4-oxadiazol-5-yl | CF₃ | |
| 2237 | 4,4-dioxide-8-Mo-2,3-dihydro-1,4-benzoxathiin-7-yl | 3-methyl-1,2,4-oxadiazol-5-yl | H | |
| 2238 | 4,4-dioxide-8-Mo-2,3-dihydro-1,4-benzoxathiin-7-yl | 3-methyl-1,2,4-oxadiazol-5-yl | methyl | |
| 2239 | 4,4-dioxide-8-Mo-2,3-dihydro-1,4-benzoxathiin-7-yl | 3-methyl-1,2,4-oxadiazol-5-yl | i-propyl | |
| 2240 | 4,4-dioxde-8-Mo-2,3-dihydro-1,4-benzoxathiin-7-yl | 3-methyl-1,2,4-oxadiazol-5-yl | cyclopropyl | |
| 2241 | 4,4-dioxde-8-Mo-2,3-dihydro-1,4-benzoxathiin-7-yl | 3-methyl-1,2,4-oxadiazol-5-yl | CF₃ | |
| 2242 | 4,4-dioxde-8-Mo-2,3-dihydro-1,4-benzoxathiin-7-yl | 3-trifluoromethyl-1,2,4-oxadiazol-5-yl | H | |
| 2243 | 4,4-dioxde-8-Mo-2,3-dihydro-1,4-benzoxathiin-7-yl | 3-trifluoromethyl-1,2,4-oxadiazol-5-yl | methyl | |
| 2244 | 4,4-dioxide-8-Mo-2,3-dihydro-1,4-benzoxathiin-7-yl | 3-trifluoromethyl-1,2,4-oxadiazol-5-yl | i-propyl | |
| 2245 | 4,4-dioxide-8-Mo-2,3-dihydro-1,4-benzoxathiin-7-yl | 3-trifluoromethyl-1,2,4-oxadiazol-5-yl | cyclopropyl | |
| 2246 | 4,4-dioxide-8-Mo-2,3-dihydro-1,4-benzoxathiin-7-yl | 3-trifluoromethyl-1,2,4-oxadiazol-5-yl | CF | |
| 2247 | 4,4-dioxide-8-Mo-2,3-dihydro-1,4-benzoxathiin-7-yl | 1,2,4-oxadiazol-3-yl | H | |
| 2248 | 4,4-dioxide-8-Mo-2,3-dihydro-1,4-benzoxathiin-7-yl | 1,2,4-oxadiazol-3-yl | methyl | |
| 2249 | 4,4-dioxide-8-Mo-2,3-dihydro-1,4-benzoxathiin-7-yl | 1,2,4-oxadiazol-3-yl | i-propyl | |
| 2250 | 4,4-dioxide-8-Mo-2,3-dihydro-1,4-benzoxathiin-7-yl | 1,2,4-oxadiazol-3-yl | cyclopropyl | |
| 2251 | 4,4-dioxide-8-Mo-2,3-dihydro-1,4-benzoxathiin-7-yl | 1,2,4-oxadiazol-3-yl | CF₃ | |
| 2252 | 4,4-dioxide-8-Mo-2,3-dihydro-1,4-benzoxathiin-7-yl | 5-methyl-1,2,4-oxadiazol-3-yl | H | |
| 2253 | 4,4-dioxide-8-Mo-2,3-dihydro-1,4-benzoxathiin-7-yl | 5-methyl-1,2,4-oxadiazol-3-yl | methyl | |
| 2254 | 4,4-dioxide-8-Mo-2,3-dihydro-1,4-benzoxathiin-7-yl | 5-methyl-1,2,4-oxadiazol-3-yl | i-propyl | |
| 2255 | 4,4-dioxide-8-Mo-2,3-dihydro-1,4-benzoxathiin-7-yl | 5-methyl-1,2,4-oxadiazol-3-yl | cyclopropyl | |
| 2256 | 4,4-dioxide-8-Mo-2,3-dihydro-1,4-benzoxathiin-7-yl | 5-methyl-1,2,4-oxadiazol-3-yl | CF₃ | |
| 2257 | 4,4-dioxide-8-Mo-2,3-dihydro-1,4-benzoxathiin-7-yl | 5-trifluoromethyl-1,2,4-oxadiazol-3-yl | H | |
| 2258 | 4,4-dioxide-8-Mo-2,3-dihydro-1,4-benzoxathiin-7-yl | 5-trifluoromethyl-1,2,4-oxadiazol-3-yl | methyl | |
| 2259 | 4,4-dioxide-8-Mo-2,3-dihydro-1,4-benzoxathiin-7-yl | 5-trifluoromethyl-1,2,4-oxadiazol-3-yl | i-propyl | |
| 2260 | 4,4-dioxide-8-Mo-2,3-dihydro-1,4-benzoxathiin-7-yl | 5-trifluoromethyl-1,2,4-oxadiazol-3-yl | cyclopropyl | |
| 2261 | 4,4-dioxide-8-Mo-2,3-dihydro-1,4-benzoxathiin-7-yl | 5-trifluoromethyl-1,2,4-oxadiazol-3-yl | CF₃ | |
| 2262 | 4,4-dioxide-8-Mo-2,3-dihydro-1,4-benzoxathiin-7-yl | 5-chloro-1,2,4-oxadiazol-3-yl | H | |
| 2263 | 4,4-dioxide-8-Mo-2,3-dihydro-1,4-benzoxathiin-7-yl | 5-chloro-1,2,4-oxadiazol-3-yl | methyl | |
| 2264 | 4,4-dioxide-8-Mo-2,3-dihydro-1,4-benzoxathiin-7-yl | 5-chloro-1,2,4-oxadiazol-3-yl | i-propyl | |
| 2265 | 4,4-dioxide-8-Mo-2,3-dihydro-1,4-benzoxathiin-7-yl | 5-chloro-1,2,4-oxadiazol-3-yl | cyclopropyl | |
| 2266 | 4,4-dioxide-8-Mo-2,3-dihydro-1,4-benzoxathiin-7-yl | 5-chloro-1,2,4-oxadiazol-3-yl | CF₃ | |
| 2267 | 4,4-dioxide-8-Mo-2,3-dihydro-1,4-benzoxathiin-7-yl | 1,3,4-oxadiazol-2-yl | H | |
| 2268 | 4,4-dioxide-8-Mo-2,3-dihydro-1,4-benzoxathiin-7-yl | 1,3,4-oxadiazol-2-yl | methyl | |
| 2269 | 4,4-dioxide-8-Mo-2,3-dihydro-1,4-benzoxathiin-7-yl | 1,3,4-oxadiazol-2-yl | i-propyl | |
| 2270 | 4,4-dioxide-8-Mo-2,3-dihydro-1,4-benzoxathiin-7-yl | 1,3,4-oxadiazol-2-yl | cyclopropyl | |
| 2271 | 4,4-dioxide-8-Mo-2,3-dihydro-1,4-benzoxathiin-7-yl | 1,3,4-oxadiazol-2-yl | CF₃ | |
| 2272 | 4,4-dioxide-8-Mo-2,3-dihydro-1,4-benzoxathiin-7-yl | 5-methylsulfonyl-1,3,4-oxadiazol-2-yl | H | |
| 2273 | 4,4-dioxide-8-Mo-2,3-dihydro-1,4-benzoxathiin-7-yl | 5-methylsulfonyl-1,3,4-oxadiazol-2-yl | methyl | |
| 2274 | 4,4-dioxide-8-Mo-2,3-dihydro-1,4-benzoxathiin-7-yl | 5-methylsulfonyl-1,3,4-oxadiazol-2-yl | i-propyl | |
| 2275 | 4,4-dioxide-8-Mo-2,3-dihydro-1,4-benzoxathiin-7-yl | 5-methylsulfonyl-1,3,4-oxadiazol-2-yl | cyclopropyl | |
| 2276 | 4,4-dioxide-8-Mo-2,3-dihydro-1,4-benzoxathiin-7-yl | 5-methylsulfonyl-1,3,4-oxadiazol-2-yl | CF₃ | |
| 2277 | 4,4-dioxide-8-Mo-2,3-dihydro-1,4-benzoxathiin-7-yl | 5-methyl-1,3,4-oxadiazol-2-yl | H | |
| 2278 | 4,4-dioxide-8-Mo-2,3-dihydro-1,4-benzoxathiin-7-yl | 5-methyl-1,3,4-oxadiazol-2-yl | methyl | |
| 2279 | 4,4-dioxide-8-Mo-2,3-dihydro-1,4-benzoxathiin-7-yl | 5-methyl-1,3,4-oxadiazol-2-yl | i-propyl | |
| 2280 | 4,4-dioxide-8-Mo-2,3-dihydro-1,4-benzoxathiin-7-yl | 5-methyl-1,3,4-oxadiazol-2-yl | cyclopropyl | |
| 2281 | 4,4-dioxide-8-Mo-2,3-dihydro-1,4-benzoxathiin-7-yl | 5-methyl-1,3,4-oxadiazol-2-yl | CF₃ | |
| 2282 | 4,4-dioxide-8-Mo-2,3-dihydro-1,4-benzoxathiin-7-yl | 5 trifluoromethyl-1,3,4-oxadiazol-2-yl | H | |
| 2283 | 4,4-dioxide-8-Mo-2,3-dihydro-1,4-benzoxathiin-7-yl | 5 trifluoromethyl-1,3,4-oxadiazol-2-yl | methyl | |
| 2284 | 4,4-dioxide-8-Mo-2,3-dihydro-1,4-benzoxathiin-7-yl | 5 trifluoromethyl-1,3,4-oxadiazol-2-yl | i-propyl | |
| 2285 | 4,4-dioxide-8-Mo-2,3-dihydro-1,4-benzoxathiin-7-yl | 5 trifluoromethyl-1,3,4-oxadiazol-2-yl | cyclopropyl | |
| 2286 | 4,4-dioxide-8-Mo-2,3-dihydro-1,4-benzoxathiin-7-yl | 5 trifluoromethyl-1,3,4-oxadiazol-2-yl | CF₃ | |
| 2287 | 4,4-dioxide-8-Mo-2,3-dihydro-1,4-benzoxathiin-7-yl | 1,2,3-triazol-4-yl | H | |
| 2288 | 4,4-dioxide-8-Mo-2,3-dihydro-1,4-benzoxathiin-7-yl | 1,2,3-triazol-4-yl | methyl | |
| 2289 | 4,4-dioxide-8-Mo-2,3-dihydro-1,4-benzoxathiin-7-yl | 1,2,3-triazol-4-yl | i-propyl | |
| 2290 | 4,4-dioxide-8-Mo-2,3-dihydro-1,4-benzoxathiin-7-yl | 1,2,3-triazol-4-yl | cyclopropyl | |
| 2291 | 4,4-dioxide-8-Mo-2,3-dihydro-1,4-benzoxathiin-7-yl | 1,2,3-triazol-4-yl | CF₃ | |
| 2292 | 4,4-dioxide-8-Mo-2,3-dihydro-1,4-benzoxathiin-7-yl | 1-methyl-1,2,3-triazol-4-yl | H | |
| 2293 | 4,4-dioxide-8-Mo-2,3-dihydro-1,4-benzoxathiin-7-yl | 1-methyl-1,2,3-triazol-4-yl | methyl | |
| 2294 | 4,4-dioxide-8-Mo-2,3-dihydro-1,4-benzoxathiin-7-yl | 1-methyl-1,2,3-triazol-4-yl | i-propyl | |
| 2295 | 4,4-dioxide-8-Mo-2,3-dihydro-1,4-benzoxathiin-7-yl | 1-methyl-1,2,3-triazol-4-yl | cyclopropyl | |
| 2296 | 4,4-dioxide-8-Mo-2,3-dihydro-1,4-benzoxathiin-7-yl | 1-methyl-1,2,3-triazol-4-yl | CF₃ | |
| 2297 | 4,4-dioxide-8-Mo-2,3-dihydro-1,4-benzoxathiin-7-yl | 2-methyl-1,2,3-triazol-4-yl | H | |
| 2298 | 4,4-dioxide-8-Mo-2,3-dihydro-1,4-benzoxathiin-7-yl | 2-methyl-1,2,3-triazol-4-yl | methyl | |
| 2299 | 4,4-dioxide-8-Mo-2,3-dihydro-1,4-benzoxathiin-7-yl | 2-methyl-1,2,3-triazol-4-yl | i-propyl | |
| 2300 | 4,4-dioxide-8-Mo-2,3-dihydro-1,4-benzoxathiin-7-yl | 2-methyl-1,2,3-triazol-4-yl | cyclopropyl | |
| 2301 | 4,4-dioxide-8-Mo-2,3-dihydro-1,4-benzoxathiin-7-yl | 2-methyl-1,2,3-triazol-4-yl | CF₃ | |
| 2302 | 4,4-dioxide-8-Mo-2,3-dihydro-1,4-benzoxathiin-7-yl | 1,2,3-triazol-1-yl | H | |
| 2303 | 4,4-dioxide-8-Mo-2,3-dihydro-1,4-benzoxathiin-7-yl | 1,2,3-triazol-1-yl | methyl | |
| 2304 | 4,4-dioxide-8-Mo-2,3-dihydro-1,4-benzoxathiin-7-yl | 1,2,3-triazol-1-yl | i-propyl | |
| 2305 | 4,4-dioxide-8-Mo-2,3-dihydro-1,4-benzoxathiin-7-yl | 1,2,3-triazol-1-yl | cyclopropyl | |
| 2306 | 4,4-dioxide-8-Mo-2,3-dihydro-1,4-benzoxathiin-7-yl | 1,2,3-triazol-1-yl | CF₃ | |
| 2307 | 4,4-dioxide-8-Mo-2,3-dihydro-1,4-benzoxathiin-7-yl | 1,2,3-triazol-1-yl | H | |
| 2308 | 4,4-dioxide-8-Mo-2,3-dihydro-1,4-benzoxathiin-7-yl | 1,2,3-triazol-1-yl | methyl | |
| 2309 | 4,4-dioxide-8-Mo-2,3-dihydro-1,4-benzoxathiin-7-yl | 1,2,3-triazol-1-yl | i-propyl | |
| 2310 | 4,4-dioxide-8-Mo-2,3-dihydro-1,4-benzoxathiin-7-yl | 1,2,3-triazol-1-yl | cyclopropyl | |
| 2311 | 4,4-dioxide-8-Mo-2,3-dihydro-1,4-benzoxathiin-7-yl | 1,2,3-triazol-1-yl | CF₃ | |
| 2312 | 4,4-dioxide-8-Mo-2,3-dihydro-1,4-benzoxathiin-7-yl | 1,2,3-triazol-1-yl | H | |
| 2313 | 4,4-dioxide-8-Mo-2,3-dihydro-1,4-benzoxathiin-7-yl | 1,2,3-triazol-1-yl | methyl | |
| 2314 | 4,4-dioxide-8-Mo-2,3-dihydro-1,4-benzoxathiin-7-yl | 1,2,3-triazol-1-yl | i-propyl | |
| 2315 | 4,4-dioxide-8-Mo-2,3-dihydro-1,4-benzoxathiin-7-yl | 1,2,3-triazol-1-yl | cyclopropyl | |
| 2316 | 4,4-dioxide-8-Mo-2,3-dihydro-1,4-benzoxathiin-7-yl | 1,2,4-triazol-1-yl | CF₃ | |
| 2317 | 4,4-dioxide-8-Mo-2,3-dihydro-1,4-benzoxathiin-7-yl | imidazol-2-yl | H | |
| 2318 | 4,4-dioxide-8-Mo-2,3-dihydro-1,4-benzoxathiin-7-yl | imidazol-2-yl | methyl | |
| 2319 | 4,4-dioxide-8-Mo-23-dihydro-1,4-benzoxathiin-7-yl | imidazol-2-yl | i-propyl | |
| 2320 | 4,4-dioxide-8-Mo-23-dihydro-1,4-benzoxathiin-7-yl | imidazol-2-yl | cyclopropyl | |
| 2321 | 4,4-dioxide-8-Mo-23-dihydro-1,4-benzoxathiin-7-yl | imidazol-2-yl | CF₃ | |
| 2322 | 4,4-dioxide-8-Mo-23-dihydro-1,4-benzoxathiin-7-yl | imidazol-1-yl | H | |
| 2323 | 4,4-dioxide-8-Mo-23-dihydro-1,4-benzoxathiin-7-yl | imidazol-1-yl | methyl | |
| 2324 | 4,4-dioxide-8-Mo-23-dihydro-1,4-benzoxathiin-7-yl | imidazol-1-yl | i-propyl | |
| 2325 | 4,4-dioxide-8-Mo-23-dihydro-1,4-benzoxathiin-7-yl | imidazol-1-yl | cyclopropyl | |
| 2326 | 4,4-dioxide-8-Mo-23-dihydro-1,4-benzoxathiin-7-yl | imidazol-1-yl | CF₃ | |
| 2327 | 4,4-dioxide-8-Mo-23-dihydro-1,4-benzoxathiin-7-yl | imidazol-4-yl | H | |
| 2328 | 4,4-dioxide-8-Mo-23-dihydro-1,4-benzoxathiin-7-yl | imidazol-4-yl | methyl | |
| 2329 | 4,4-dioxide-8-Mo-23-dihydro-1,4-benzoxathiin-7-yl | imidazol-4-yl | i-propyl | |
| 2330 | 4,4-dioxide-8-Mo-23-dihydro-1,4-benzoxathiin-7-yl | imidazol-4-yl | cyclopropyl | |
| 2331 | 4,4-dioxide-8-Mo-23-dihydro-1,4-benzoxathiin-7-yl | imidazol-4-yl | CF₃ | |
| 2332 | 4,4-dioxide-8-Mo-23-dihydro-1,4-benzoxathiin-7-yl | thiazol-2-yl | H | |
| 2333 | 4,4-dioxide-8-Mo-23-dihydro-1,4-benzoxathiin-7-yl | thiazol-2-yl | methyl | |
| 2334 | 4,4-dioxide-8-Mo-23-dihydro-1,4-benzoxathiin-7-yl | thiazol-2-yl | i-propyl | |
| 2335 | 4,4-dioxide-8-Mo-23-dihydro-1,4-benzoxathiin-7-yl | thiazol-2-yl | cyclopropyl | |
| 2336 | 4,4-dioxide-8-Mo-23-dihydro-1,4-benzoxathiin-7-yl | thiazol-2-yl | CF₃ | |
| 2337 | 4,4-dioxide-8-Mo-23-dihydro-1,4-benzoxathiin-7-yl | 4-memyhylazol-2-yl | H | |
| 2338 | 4,4-dioxide-8-Mo-23-dihydro-1,4-benzoxathiin-7-yl | 4-methylthiazol-2-yl | methyl | |
| 2339 | 4,4-dioxide-8-Mo-23-dihydro-1,4-benzoxathiin-7-yl | 4 methylthiazol-2-yl | i-propyl | |
| 2340 | 4,4-dioxide-8-Mo-23-dihydro-1,4-benzoxathiin-7-yl | 4-methylthiazol-2-y1 | cyclopropyl | |
| 2341 | 4,4-dioxide-8-Mo-23-dihydro-1,4-benzoxathiin-7-yl | 4-methylthiazol-2-yl | CF₃ | |
| 2342 | 4,4-dioxide-8-Mo-23-dihydro-1,4-benzoxathiin-7-yl | oxazol-2-yl | H | |
| 2343 | 4,4-dioxide-8-Mo-23-dihydro-1,4-benzoxathiin-7-yl | oxazol-2-yl | methyl | |
| 2344 | 4,4-dioxide-8-Mo-23-dihydro-1,4-benzoxathiin-7-yl | oxazol-2-yl | i-propyl | |
| 2345 | 4,4-dioxide-8-Mo-23-dihydro-1,4-benzoxathiin-7-yl | oxazol-2-yl | cyclopropyl | |
| 2346 | 4,4-dioxide-8-Mo-23-dihydro-1,4-benzoxathiin-7-yl | oxazol-2-yl | CF₃ | |
| 2347 | 4,4-dioxide-8-Mo-23-dihydro-1,4-benzoxathiin-7-yl | 4,5-dimethyloxazol-2-yl | H | |
| 2348 | 4,4-dioxide-8-Mo-23-dihydro-1,4-benzoxathiin-7-yl | 4,5-dimethyloxazol-2-yl | methyl | |
| 2349 | 4,4-dioxide-8-Mo-23-dihydro-1,4-benzoxathiin-7-yl | 4,5-dimethyloxazol-2-yl | i-propyl | |
| 2350 | 4,4-dioxide-8-Mo-23-dihydro-1,4-benzoxathiin-7-yl | 4,5-dimethyloxazol-2-yl | cyclopropyl | |
| 2351 | 4,4-dioxide-8-Mo-23-dihydro-1,4-benzoxathiin-7-yl | 4,5-dimethyloxazol-2-yl | CF₃ | |
| 2352 | 4,4-dioxide-8-Mo-23-dihydro-1,4-benzoxathiin-7-yl | 2-oxazolin-2-yl | H | |
| 2353 | 4,4-dioxide-8-Mo-23-dihydro-1,4-benzoxathiin-7-yl | 2-oxazolin-2-yl | methyl | |
| 2354 | 4,4-dioxide-8-Mo-23-dihydro-1,4-benzoxathiin-7-yl | 2-oxazolin-2-yl | i-propyl | |
| 2355 | 4,4-dioxide-8-Mo-23-dihydro-1,4-benzoxathiin-7-yl | 2-oxazolin-2-yl | cyclopropyl | |
| 2356 | 4,4-dioxide-8-Mo-23-dihydro-1,4-benzoxathiin-7-yl | 2-oxazolin-2-yl | CF₃ | |
| 2357 | 4,4-dioxide-8-Mo-23-dihydro-1,4-benzoxathiin-7-yl | 4,4-dimethyl-2-oxazolin-2-yl | H | |
| 2358 | 4,4-dioxide-8-Mo-23-dihydro-1,4-benzoxathiin-7-yl | 4,4-dimethyl-2-oxazolin-2-yl | methyl | |
| 2359 | 4,4-dioxide-8-Mo-23-dihydro-1,4-benzoxathiin-7-yl | 4,4-dimethyl-2-oxazolin-2-yl | i-propyl | |
| 2360 | 4,4-dioxide-8-Mo-23-dihydro-1,4-benzoxathiin-7-yl | 4,4-dimethyl-2-oxazolin-2-yl | cyclopropyl | |
| 2361 | 4,4-dioxide-8-Mo-23-dihydro-1,4-benzoxathiin-7-yl | 4,4-dimethyl-2-oxazolin-2-yl | CF₃ | |
| 2362 | 4,4-dioxide-8-Mo-23-dihydro-1,4-benzoxathiin-7-yl | 1,2,4-thiadiazol-5-yl | H | |
| 2363 | 4,4-dioxide-8-Mo-23-dihydro-1,4-benzoxathiin-7-yl | 1,2,4-thiadiazol-5-yl | methyl | |
| 2364 | 4,4-dioxide-8-Mo-23-dihydro-1,4-benzoxathiin-7-yl | 1,2,4-thiadiazol-5-yl | i-propyl | |
| 2365 | 4,4-dioxide-8-Mo-2,3-dihydro-1,4-benzoxathiin-7-yl | 1,2,4-thiadiazol-5-yl | cyclopropyl | |
| 2366 | 4,4-dioxide-8-Mo-2,3-dihydro-1,4-benzoxathiin-7-yl | 1.2,4-thiadiazol-5-yl | CF₃ | |
| 2367 | 4,4-dioxide-8-Mo-2,3-dihydro-1,4-benzoxathiin-7-yl | 3-methyl-1,2,4-thiadiazol-5-yl | H | |
| 2368 | 4,4-dioxide-8-Mo-2,3-dihydro-1,4-benzoxathiin-7-yl | 3-methyl-1,2,4-thiadiazol-5-yl | methyl | |
| 2369 | 4,4-dioxide-8-Mo-2,3-dihydro-1,4-benzoxathiin-7-yl | 3-methyl-1,2,4-thiadiazol-5-yl | i-propyl | |
| 2370 | 4,4-dioxide-8-Mo-2,3-dihydro-1,4-benzoxathiin-7-yl | 3-methyl-1,2,4-thiadiazol-5-yl | cyclopropyl | |
| 2371 | 4,4-dioxide-8-Mo-2,3-dihydro-1,4-benzoxathiin-7-yl | 3-methyl-1,2,4-thiadiazol-5-yl | CF₃ | |
| 2372 | 4,4-dioxide-8-Mo-2,3-dihydro-1,4-benzoxathiin-7-yl | 3-trifluoromethyl-1,2,4-thiadiazol-5-yl | H | |
| 2373 | 4,4-dioxide-8-Mo-2,3-dihydro-1,4-benzoxathiin-7-yl | 3-trifluoromethyl-1,2,4-thiadiazol-5-yl | methyl | |
| 2374 | 4,4-dioxide-8-Mo-2,3-dihydro-1,4-benzoxathiin-7-yl | 3-trifluoromethyl-1,2,4-thiadiazol-5-yl | i-propyl | |
| 2375 | 4,4-dioxide-8-Mo-2,3-dihydro-1,4-benzoxathiin-7-yl | 3-trifluoromethyl-1,2,4-thiadiazol-5-yl | cyclopropyl | |
| 2376 | 4,4-dioxide-8-Mo-2,3-dihydro-1,4-benzoxathiin-7-yl | 3-trifluoromethyl-1,2,4-thiadiazol-5-yl | CF₃ | |
| 2377 | 4,4-dioxide-8-Mo-2,3-dihydro-1,4-benzoxathiin-7-yl | 1,2,4-thiadiazol-3-yl | H | |
| 2378 | 4,4-dioxide-8-Mo-2,3-dihydro-1,4-benzoxathiin-7-yl | 1,2,4-thiadiazol-3-yl | methyl | |
| 2379 | 4,4-dioxide-8-Mo-2,3-dihydro-1,4-benzoxathiin-7-yl | 1,2,4-thiadiazol-3-yl | i-propyl | |
| 2380 | 4,4-dioxide-8-Mo-2,3-dihydro-1,4-benzoxathiin-7-yl | 1,2,4-thiadiazol-3-yl | cyclopropyl | |
| 2381 | 4,4-dioxide-8-Mo-2,3-dihydro-1,4-benzoxathiin-7-yl | 1,2,4-thiadiazol-3-yl | CF₃ | |
| 2382 | 4,4-dioxide-8-Mo-2,3-dihydro-1,4-benzoxathiin-7-yl | 5-methyl-1,2,4-thiadiazol-3-yl | H | |
| 2383 | 4,4-dioxide-8-Mo-2,3-dihydro-1,4-benzoxathiin-7-yl | 5-methyl-1,2,4-thiadiazol-3-yl | methyl | |
| 2384 | 4,4-dioxide-8-Mo-2,3-dihydro-1,4-benzoxathiin-7-yl | 5-methyl-1,2,4-thiadiazol-3-yl | i-propyl | |
| 2385 | 4,4-dioxide-8-Mo-2,3-dihydro-1,4-benzoxathiin-7-yl | 5-methyl-1,2,4-thiadiazol-3-yl | cyclopropyl | |
| 2386 | 4,4-dioxide-8-Mo-2,3-dihydro-1,4-benzoxathiin-7-yl | 5-methyl-1,2,4-thiadiazol-3-yl | CF₃ | |
| 2387 | 4,4-dioxide-8-Mo-2,3-dihydro-1,4-benzoxathiin-7-yl | 5-trifluoromethyl-1,2,4-thiadiazo-3-yl | H | |
| 2388 | 4,4-dioxide-8-Mo-2,3-dihydro-1,4-benzoxathiin-7-yl | 5-trifluoromethyl-1,2,4-thiadiazo-3-yl | methyl | |
| 2389 | 4,4-dioxide-8-Mo-2,3-dihydro-1,4-benzoxathiin-7-yl | 5-trifluoromethyl-1,2,4-thiadiazo-3-yl | i-propyl | |
| 2390 | 4,4-dioxide-8-Mo-2,3-dihydro-1,4-benzoxathiin-7-yl | 5-trifluoromethyl-1,2,4-thiadiazo-3-yl | cyclopropyl | |
| 2391 | 4,4-dioxide-8-Mo-2,3-dihydro-1,4-benzoxathiin-7-yl | 5-trifluoromethyl-1,2,4-thiadiazo-3-yl | CF₃ | |
| 2392 | 4,4-dioxide-8-Mo-2,3-dihydro-1,4-benzoxathiin-7-yl | 1,3,4-thiadiazol-2-yl | H | |
| 2393 | 4,4-dioxide-8-Mo-2,3-dihydro-1,4-benzoxathiin-7-yl | 1,3,4-thiadiazol-2-yl | methyl | |
| 2394 | 4,4-dioxide-8-Mo-2,3-dihydro-1,4-benzoxathiin-7-yl | 1,3,4-thiadiazol-2-yl | i-pmpyl | |
| 2395 | 4,4-dioxide-8-Mo-2,3-dihydro-1,4-benzoxathiin-7-yl | 1,3,4-thiadiazol-2-yl | cyclopropyl | |
| 2396 | 4,4-dioxide-8-Mo-2,3-dihydro-1,4-benzoxathiin-7-yl | 1,3,4-thiadiazol-2-yl | CF₃ | |
| 2397 | 4,4-dioxide-8-Mo-2,3-dihydro-1,4-benzoxathiin-7-yl | 5-methylsufonyl-1,3,4-thiadiazol-2-yl | H | |
| 2398 | 4,4-dioxide-8-Mo-2,3-dihydro-1,4-benzoxathiin-7-yl | 5-methylsufonyl-1,3,4-thiadiazol-2-yl | methyl | |
| 2399 | 4,4-dioxide-8-Mo-2,3-dihydro-1,4-benzoxathiin-7-yl | 5-methylsufonyl-1,3,4-thiadiazol-2-yl | i-propyl | |
| 2400 | 4,4-dioxide-8-Mo-2,3-dihydro-1,4-benzoxathiin-7-yl | 5-methylsufonyl-1,3,4-thiadiazol-2-yl | cyclopropyl | |
| 2401 | 4,4-dioxide-8-Mo-2,3-dihydro-1,4-benzoxathiin-7-yl | 5-methylsufonyl-1,3,4-thiadiazol-2-yl | CF₃ | |
| 2402 | 4,4-dioxide-8-Mo-2,3-dihydro-1,4-benzoxathiin-7-yl | 5-methyl-1,3,4-thiadiazol-2-yl | H | |
| 2403 | 4,4-dioxide-8-Mo-2,3-dihydro-1,4-benzoxathiin-7-yl | 5-methyl-1,3,4-thiadiazol-2-yl | methyl | |
| 2404 | 4,4-dioxide-8-Mo-2,3-dihydro-1,4-benzoxathiin-7-yl | 5-methyl-1,3,4-thiadiazol-2-yl | i-propyl | |
| 2405 | 4,4-dioxide-8-Mo-2,3-dihydro-1,4-benzoxathiin-7-yl | 5-methyl-1,3,4-thiadiazol-2-yl | cyclopropyl | |
| 2406 | 4,4-dioxide-8-Mo-2,3-dihydro-1,4-benzoxathiin-7-yl | 5-methyl-1,3,4-thiadiazol-2-yl | CF₃ | |
| 2407 | 4,4-dioxide-8-Mo-2,3-dihydro-1,4-benzoxathiin-7-yl | benzoxazol-2-yl | H | |
| 2408 | 4,4-dioxide-8-Mo-2,3-dihydro-1,4-benzoxathiin-7-yl | benzoxazol-2-yl | methyl | |
| 2409 | 4,4-dioxide-8-Mo-2,3-dihydro-1,4-benzoxathiin-7-yl | benzoxazol-2-yl | i-propyl | |
| 2410 | 4,4-dioxide-8-Mo-2,3-dihydro-1,4-benzoxathiin-7-yl | benzoxazol-2-yl | cyclopropyl | |
| 2411 | 4,4-dioxide-8-Mo-2,3-dihydro-1,4-benzoxathiin-7-yl | benzoxazol-2-yl | CF₃ | |
| 2412 | 4,4-dioxide-8-Mo-2,3-dihydro-1,4-benzoxathiin-7-yl | 6-methylbenzoxazol-2-yl | H | |
| 2413 | 4,4-dioxide-8-Mo-2,3-dihydro-1,4-benzoxathiin-7-yl | 6-methylbenzoxazol-2-yl | methyl | |
| 2414 | 4,4-dioxide-8-Mo-2,3-dihydro-1,4-benzoxathiin-7-yl | 6-methylbenzoxazol-2-yi | i-propyl | |
| 2415 | 4,4-dioxide-8-Mo-2,3-dihydro-1,4-benzoxathiin-7-yl | 6-methylbeazoxaaol-2-yl | cyclopropyl | |
| 2416 | 4,4-dioxide-8-Mo-2,3-dihydro-1,4-benzoxathiin-7-yl | 6-methylbenzoxazol-2-yl | CF₃ | |
| 2417 | 4,4-dioxide-8-Mo-2,3-dihydro-1,4-benzoxathiin-7-yl | benzothiazol-2-yl | H | |
| 2418 | 4,4-dioxide-8-Mo-2,3-dihydro-1,4-benzoxathiin-7-yl | benzothiazol-2-yl | methyl | |
| 2419 | 4,4-dioxide-8-Mo-2,3-dihydro-1,4-benzoxathiin-7-yl | benzothiazol-2-yl | i-propyl | |
| 2420 | 4,4-dioxide-8-Mo-2,3-dihydro-1,4-benzoxathiin-7-yl | benzothiazol-2-yl | cyclopropyl | |
| 2421 | 4,4-dioxide-8-Mo-2,3-dihydro-1,4-benzoxathiin-7-yl | benzothiazol-2-yl | CF₃ | |
| 2422 | 4,4-dioxide-8-Mo-2,3-dihydro-1,4-benzoxathiin-7-yl | pyrazol-1-yl | H | |
| 2423 | 4,4-dioxide-8-Mo-2,3-dihydro-1,4-benzoxathiin-7-yl | pyrazol-1-yl | methyl | |
| 2424 | 4,4-dioxide-8-Mo-2,3-dihydro-1,4-benzoxathiin-7-yl | pyrazol-1-yl | i-propyl | |
| 2425 | 4,4-dioxide-8-Mo-2,3-dihydro-1,4-benzoxathiin-7-yl | pyrazol-1-yl | cyclopropyl | |
| 2426 | 4,4-dioxide-8-Mo-2,3-dihydro-1,4-benzoxathiin-7-yl | pyrazol-1-yl | CF₃ | |
| 2427 | 4,4-dioxide-8-Mo-2,3-dihydro-1,4-benzoxathiin-7-yl | pyrazol-3-yl | H | |
| 2428 | 4,4-dioxide-8-Mo-2,3-dihydro-1,4-benzoxathiin-7-yl | pyrazol-3-yl | methyl | |
| 2429 | 4,4-dioxide-8-Mo-2,3-dihydro-1,4-benzoxathiin-7-yl | pyrazol-3-yl | i-propyl | |
| 2430 | 4,4-dioxide-8-Mo-2,3-dihydro-1,4-benzoxathiin-7-yl | pyrazol-3-yl | cyclopropyl | |
| 2431 | 4,4-dioxide-8-Mo-2,3-dihydro-1,4-benzoxathiin-7-yl | pyrazol-3-yl | CF₃ | |
| 2432 | 4,4-dioxide-8-Mo-2,3-dihydro-1,4-benzoxathiin-7-yl | 1-methylpyrazol-3-yl | H | |
| 2433 | 4,4-dioxide-8-Mo-2,3-dihydro-1,4-benzoxathiin-7-yl | 1-methylpyrazol-3-yl | methyl | |
| 24324 | 4,4-dioxide-8-Mo-2,3-dihydro-1,4-benzoxathiin-7-yl | 1-methylpyrazol-3-yl | i-propyl | |
| 2435 | 4,4-dioxide-8-Mo-2,3-dihydro-1,4-benzoxathiin-7-yl | 1-methylpyrazol-3-yl | cyclopropyl | |
| 2436 | 4,4-dioxide-8-Mo-2,3-dihydro-1,4-benzoxathiin-7-yl | 1-methylpyrazol-3-yl | CF₃ | |
| 2437 | 4,4-dioxide-8-Mo-2,3-dihydro-1,4-benzoxathiin-7-yl | tetrazol-1-yl | H | |
| 2438 | 4,4-dioxide-8-Mo-2,3-dihydro-1,4-benzoxathiin-7-yl | tetrazol-1-yl | methyl | |
| 2439 | 4,4-dioxide-8-Mo-2,3-dihydro-1,4-benzoxathiin-7-yl | tetrazol-1-yl | i-propyl | |
| 2440 | 4,4-dioxide-8-Mo-2,3-dihydro-1,4-benzoxathiin-7-yl | tetrazol-1-yl | cyclopropyl | |
| 2441 | 4,4-dioxide-8-Mo-2,3-dihydro-1,4-benzoxathiin-7-yl | tetrazol-1-yl | CF₃ | |
| 24342 | 4,4-dioxide-8-Mo-2,3-dihydro-1,4-benzoxathiin-7-yl | 5-methyltetrazol-1-yl | H | |
| 2443 | 4,4-dioxide-8-Mo-2,3-dihydro-1,4-benzoxathiin-7-yl | 5-methyltetrazol-1-yl | methyl | |
| 2444 | 4,4-dioxide-8-Mo-2,3-dihydro-1,4-benzoxathiin-7-yl | 5-methyltetrazol-1-yl | i-propyl | |
| 2445 | 4,4-dioxide-8-Mo-2,3-dihydro-1,4-benzoxathiin-7-yl | 5-methyltetrazol-1-yl | cyclopropyl | |
| 2446 | 4,4-dioxide-8-Mo-2,3-dihydro-1,4-benzoxathiin-7-yl | 5-methyltetrazol-1-yl | CF₃ | |
| 2447 | 4,4-dioxide-8-Mo-2,3-dihydro-1,4-benzoxathiin-7-yl | tetrazol-2-yl | H | |
| 2448 | 4,4-dioxide-8-Mo-2,3-dihydro-1,4-benzoxathiin-7-yl | tetrazol-2-yl | methyl | |
| 2449 | 4,4-dioxide-8-Mo-2,3-dihydro-1,4-benzoxathiin-7-yl | tetrazol-2-yl | 1-propyl | |
| 2450 | 4,4-dioxide-8-Mo-2,3-dihydro-1,4-benzoxathiin-7-yl | tetrazol-2-yl | cyclopropyl | |
| 2451 | 4,4-dioxide-8-Mo-2,3-dihydro-1,4-benzoxathiin-7-yl | tetrazol-2-yl | CF₃ | |
| 2452 | 4,4-dioxide-8-Mo-2,3-dihydro-1,4-benzoxathiin-7-yl | 5-methyltetrazol-2-yl | H | |
| 2453 | 4,4-dioxide-8-Mo-2,3-dihydro-1,4-benzoxathiin-7-yl | 5-methyltetrazol-2-yl | methyl | |
| 2454 | 4,4-dioxide-8-Mo-2,3-dihydro-1,4-benzoxathiin-7-yl | 5-methyltetrazol-2-yl | i-propyl | |
| 2455 | 4,4-dioxide-8-Mo-2,3-dihydro-1,4-benzoxathiin-7-yl | 5-methyltetrazol-2-yl | cyclopropyl | |
| 2456 | 4,4-dioxide-8-Mo-2,3-dihydro-1,4-benzoxathiin-7-yl | 5-methyltetrazol-2-yl | CF₃ | |
| 2457 | 4,4-dioxide-8-Mo-2,3-dihydro-1,4-benzoxathiin-7-yl | 1-methyltetrazol-5-yl | H | |
| 2458 | 4,4-dioxide-8-Mo-2,3-dihydro-1,4-benzoxathiin-7-yl | 1-methyltetrazol-5-yl | methyl | |
| 2459 | 4,4-dioxide-8-Mo-2,3-dihydro-1,4-benzoxathiin-7-yl | 1-methyltetrazol-5-yl | i-propyl | |
| 2460 | 4,4-dioxide-8-Mo-2,3-dihydro-1,4-benzoxathiin-7-yl | 1-methyltetrazol-5-yl | cyclopropyl | |
| 2461 | 4,4-dioxide-8-Mo-2,3-dihydro-1,4-benzoxathiin-7-yl | 1-methyltetrazol-5-yl | CF₃ | |
| 2462 | 4,4-dioxide-8-Mo-2,3-dihydro-1,4-benzoxathiin-7-yl | 2-methyltetrazol-5-yl | H | |
| 2463 | 4,4-dioxide-8-Mo-2,3-dihydro-1,4-benzoxathiin-7-yl | 2-methyltetrazol-5-yl | methyl | |
| 2464 | 4,4-dioxide-8-Mo-2,3-dihydro-1,4-benzoxathiin-7-yl | 2-methyltetrazol-5-yl | i-propyl | |
| 2465 | 4,4-dioxide-8-Mo-2,3-dihydro-1,4-benzoxathiin-7-yl | 2-methyltetrazol-5-yl | cyclopropyl | |
| 2466 | 4,4-dioxide-8-Mo-2,3-dihydro-1,4-benzoxathiin-7-yl | 2-methyltetrazol-5-yl | CF₃ | |
| 2467 | 4,4-dioxide-8-Mo-2,3-dihydro-1,4-benzoxathiin-7-yl | pyridin-2-yl | H | |
| 2468 | 4,4-dioxide-8-Mo-2,3-dihydro-1,4-benzoxathiin-7-yl | pyridin-2-yl | methyl | |
| 2469 | 4,4-dioxide-8-Mo-2,3-dihydro-1,4-benzoxathiin-7-yl | pyridin-2-yl | i-propyl | |
| 2470 | 4,4-dioxide-8-Mo-2,3-dihydro-1,4-benzoxathiin-7-yl | pyridin-2-yl | cyclopropyl | |
| 2471 | 4,4-dioxide-8-Mo-2,3-dihydro-1,4-benzoxathiin-7-yl | pyridin-2-yl | CF₃ | |
| 2472 | 4,4-dioxide-8-Mo-2,3-dihydro-1,4-benzoxathiin-7-yl | pyridin-4-yl | H | |
| 2473 | 4,4-dioxide-8-Mo-2,3-dihydro-1,4-benzoxathiin-7-yl | pyridin-4-yl | methyl | |
| 2474 | 4,4-dioxide-8-Mo-2,3-dihydro-1,4-benzoxathiin-7-yl | pyridin-4-yl | i-propyl | |
| 2475 | 4,4-dioxide-8-Mo-2,3-dihydro-1,4-benzoxathiin-7-yl | pyridin-4-yl | cyclopropyl | |
| 2476 | 4,4-dioxide-8-Mo-2,3-dihydro-1,4-benzoxathiin-7-yl | pyridin-4-yl | CF₃ | |
| 2477 | 4,4-dioxide-8-Mo-2,3-dihydro-1,4-benzoxathiin-7-yl | pyridin-3-yl | H | |
| 2478 | 4,4-dioxide-8-Mo-2,3-dihydro-1,4-benzoxathiin-7-yl | pyridin-3-yl | methyl | |
| 2479 | 4,4-dioxide-8-Mo-2,3-dihydro-1,4-benzoxathiin-7-yl | pyridin-3-yl | i-propyl | |
| 2480 | 4,4-dioxide-8-Mo-2,3-dihydro-1,4-benzoxathiin-7-yl | pyridin-3-yl | cyclopropyl | |
| 2481 | 4,4-dioxide-8-Mo-2,3-dihydro-1,4-benzoxathiin-7-yl | pyridin-3-yl | CF₃ | |
| 2482 | 4,4-dioxide-8-Mo-2,3-dihydro-1,4-benzoxathiin-7-yl | 3-nitropyridin-4-yl | H | |
| 2483 | 4,4-dioxide-8-Mo-2,3-dihydro-1,4-benzoxathiin-7-yl | 3-nitropyridin-4-yl | methyl | |
| 2484 | 4,4-dioxide-8-Mo-2,3-dihydro-1,4-benzoxathiin-7-yl | 3-nitropyndin-4-yl | i-propyl | |
| 2485 | 4,4-dioxide-8-Mo-2,3-dihydro-1,4-benzoxathiin-7-yl | 3-nitropyridin-4-yl | cyclopropyl | |
| 2486 | 4,4-dioxide-8-Mo-2,3-dihydro-1,4-benzoxathiin-7-yl | 3-nitropyridin-4-yl | CF₃ | |
| 2487 | 4,4-dioxide-8-Mo-2,3-dihydro-1,4-benzoxathiin-7-yl | 5-nitropyridin-2-yl | H | |
| 2488 | 4,4-dioxide-8-Mo-2,3-dihydro-1,4-benzoxathiin-7-yl | 5-nitropyridin-2-yl | methyl | |
| 2489 | 4,4-dioxide-8-Mo-2,3-dihydro-1,4-benzoxathiin-7-yl | 5-nitropyridin-2-yl | i-propyl | |
| 2490 | 4,4-dioxide-8-Mo-2,3-dihydro-1,4-benzoxathiin-7-yl | 5-nitropyridin-2-yl | cyclopropyl | |
| 2491 | 4,4-dioxide-8-Mo-2,3-dihydro-1,4-benzoxathiin-7-yl | 5-cyanopyridin-2-yl | CF₃ | |
| 2492 | 4,4-dioxide-8-Mo-2,3-dihydro-1,4-benzoxathiin-7-yl | 5-trifluoromethylpyridin-2-yl | H | |
| 2493 | 4,4-dioxide-8-Mo-2,3-dihydro-1,4-benzoxathiin-7-yl | 5-trifluoromethylpyridin-2-yl | methyl | |
| 2494 | 4,4-dioxide-8-Mo-2,3-dihydro-1,4-benzoxathiin-7-yl | 5-trifluoromethylpyridin-2-yl | i-propyl | |
| 2495 | 4,4-dioxide-8-Mo-2,3-dihydro-1,4-benzoxathiin-7-yl | 5-trifluoromethylpyridin-2-yl | cyclopropyl | |
| 2496 | 4,4-dioxide-8-Mo-2,3-dihydro-1,4-benzoxathiin-7-yl | 5-trifluoromethylpyridin-2-yl | CF₃ | |
| 2497 | 4,4-dioxide-8-Mo-2,3-dihydro-1,4-benzoxathiin-7-yl | pyrimidin-2-yl | H | |
| 2498 | 4,4-dioxide-8-Mo-2,3-dihydro-1,4-benzoxathiin-7-yl | pyrimidin-2-yl | methyl | |
| 2499 | 4,4-dioxide-8-Mo-2,3-dihydro-1,4-benzoxathiin-7-yl | pyrimidin-2-yl | i-propyl | |
| 2500 | 4,4-dioxide-8-Mo-2,3-dihydro-1,4-benzoxathiin-7-yl | pyrimidin-2-yl | cyclopropyl | |
| 2501 | 4,4-dioxide-8-Mo-2,3-dihydro-1,4-benzoxathiin-7-yl | pyrimidin-2-yl | CF₃ | |
| 2502 | 4,4-dioxide-8-Mo-2,3-dihydro-1,4-benzoxathiin-7-yl | pyrimidin-4-yl | H | |
| 2503 | 4,4-dioxide-8-Mo-2,3-dihydro-1,4-benzoxathiin-7-yl | pyrimidin-2-yl | methyl | |
| 2504 | 4,4-dioxide-8-Me-2,3-dihydro-1,4-benzoxathiin-7-yl | pyrimidin-4-yl | i-propyl | |
| 2505 | 4,4-dioxide-8-Me-2,3-dihydro-1,4-benzoxathiin-7-yl | pyrimidin-4-yl | cyclopropyl | |
| 2506 | 4,4-dioxide-8-Me-2,3-dihydro-1,4-benzoxathiin-7-yl | pyrimidin-4-yl | CF₃ | |
| 2507 | 4,4-dioxide-8-Me-2,3-dihydro-1,4-benzoxathiin-7-yl | 6-chloropyrimidin-4-yl | methyl | |
| 2508 | 4,4-dioxide-8-Me-2,3-dihydro-1,4-benzoxathiin-7-yl | 6-chloropyrimidin-4-yl | i-propyl | |
| 2509 | 4,4-dioxide-8-Me-2,3-dihydro-1,4-benzoxathiin-7-yl | 6-chloropyrimidin-4-yl | cyclopropyl | |
| 2510 | 4,4-dioxide-8-Me-2,3-dihydro-1,4-benzoxathiin-7-yl | 6-chloropyrimidin-4-yl | CF₃ | |
| 2511 | 4,4-dioxide-8-Me-2,3-dihydro-1,4-benzoxathiin-7-yl | pyridazin-3-yl | H | |
| 2512 | 4,4-dioxide-8-Me-2,3-dihydro-1,4-benzoxathiin-7-yl | pyridazin-3-yl | methyl | |
| 2513 | 4,4-dioxide-8-Me-2,3-dihydro-1,4-benzoxathiin-7-yl | pyridazin-3-yl | i-propyl | |
| 2514 | 4,4-dioxide-8-Me-2,3-dihydro-1,4-benzoxathiin-7-yl | pyridazin-3-yl | cyclopropyl | |
| 2515 | 4,4-dioxide-8-Me-2,3-dihydro-1,4-benzoxathiin-7-yl | pyridazin-3-yl | CF₃ | |
| 2516 | 4,4-dioxide-8-Me-2,3-dihydro-1,4-benzoxathiin-7-yl | 6-chloropyridazin-3-yl | methyl | |
| 2517 | 4,4-dioxide-8-Me-2,3-dihydro-1,4-benzoxathiin-7-yl | 6-chloropyridazin-3-yl | i-propyl | |
| 2518 | 4,4-dioxide-8-Me-2,3-dihydro-1,4-benzoxathiin-7-yl | 6-chloropyridazin-3-yl | cyclopropyl | |
| 2519 | 4,4-dioxide-8-Me-2,3-dihydro-1,4-benzoxathiin-7-yl | 6-chloropyridazin-3-yl | CF₃ | |
| 2520 | 4,4-dioxide-8-Me-2,3-dihydro-1,4-benzoxathiin-7-yl | pyrazin-2-yl | methyl | |
| 2521 | 4,4-dioxide-8-Me-2,3-dihydro-1,4-benzoxathiin-7-yl | pyrazin-2-yl | i-propyl | |
| 2522 | 4,4-dioxide-8-Me-2,3-dihydro-1,4-benzoxathiin-7-yl | pyrazin-2-yl | cyclopropyl | |
| 2523 | 4,4-dioxide-8-Me-2,3-dihydro-1,4-benzoxathiin-7-yl | pyrazin-2-yl | CF₃ | |
| 2524 | 4,4-dioxide-8-Me-2,3-dihydro-1,4-benzoxathiin-7-yl | triazin-2-yl | methyl | |
| 2525 | 4,4-dioxide-8-Me-2,3-dihydro-1,4-benzoxathiin-7-yl | triazin-2-yl | i-propyl | |
| 2526 | 4,4-dioxide-8-Me-2,3-dihydro-1,4-benzoxathiin-7-yl | triazin-2-yl | cyclopropyl | |
| 2527 | 4,4-dioxide-8-Me-2,3-dihydro-1,4-benzoxathiin-7-yl | triazin-2-yl | CF₃ | |
| 2528 | 4,4-dioxide-8-Me-2,3-dihydro-1,4-benzoxathiin-7-yl | triazin-2-yl | methyl | |
| 2529 | 4,4-dioxide-8-Me-2,3-dihydro-1,4-benzoxathiin-7-yl | triazin-2-yl | i-propyl | |
| 2530 | 4,4-dioxide-8-Me-2,3-dihydro-1,4-benzoxathiin-7-yl | quinolin-2-yl | cyclopropyl | |
| 2531 | 4,4-dioxide-8-Me-2,3-dihydro-1,4-benzoxathiin-7-yl | quinolin-2-yl | CF₃ | |
| 2532 | 4,4-dioxide-8-Me-2,3-dihydro-1,4-benzoxathiin-7-yl | 4,4,6-trimethyl-5,6-dihydro-1,3(4H)-oxazin-2-yl | H | |
| 2533 | 4,4-dioxide-8-Me-2,3-dihydro-1,4-benzoxathiin-7-yl | 4,4,6-trimethyl-5,6-dihydro-1,3(4H)-oxazin-2-yl | methyl | |
| 2534 | 4,4-dioxide-8-Me-2,3-dihydro-1,4-benzoxathiin-7-yl | 4,4,6-trimethyl-5,6-dihydro-1,3(4H)-oxazin-2-yl | i-propyl | |
| 2535 | 4,4-dioxide-8-Me-2,3-dihydro-1,4-benzoxathiin-7-yl | 4,4,6-trimethyl-5,6-dihydro-1,3(4H)-oxazin-2-yl | cyclopropyl | |
| 2536 | 4,4-dioxide-8-Me-2,3-dihydro-1,4-benzoxathiin-7-yl | 4,4,6-trimethyl-5,6-dihydro-1,3(4H)-oxazin-2-yl | CF₃ | |
| 2537 | 4,4-dioxide-8-Me-2,3-dihydro-1,4-benzoxathiin-7-yl | 2-oxazolidinon-3-yl | H | |
| 2538 | 4,4-dioxide-8-Me-2,3-dihydro-1,4-benzoxathiin-7-yl | 2-oxazolidinon-3-yl | methyl | |
| 2539 | 4,4-dioxide-8-Me-2,3-dihydro-1,4-benzoxathiin-7-yl | 2-oxazolidinon-3-yl | i-propyl | |
| 2540 | 4,4-dioxide-8-Me-2,3-dihydro-1,4-benzoxathiin-7-yl | 2-oxazolidinon-3-yl | cyclopropyl | |
| 2541 | 4,4-dioxide-8-Me-2,3-dihydro-1,4-benzoxathiin-7-yl | 2-oxazolidinon-3-yl | CF₃ | |
| 2542 | 4,4-dioxide-8-Me-2,3-dihydro-1,4-benzoxathiin-7-yl | 2-pyrrolidinon-1-yl | methyl | |
| 2543 | 4,4-dioxide-8-Me-2,3-dihydro-1,4-benzoxathiin-7-yl | 2-pyrrolidinon-1-yl | i-propyl | |
| 2544 | 4,4-dioxide-8-Me-2,3-dihydro-1,4-benzoxathiin-7-yl | 2-pyrrolidinon-1-yl | cyclopropyl | |
| 2545 | 4,4-dioxide-8-Me-2,3-dihydro-1,4-benzoxathiin-7-yl | 2-pyrrolidinon-1-yl | CF₃ | |
| 2546 | 4,4-dioxide-8-Me-2,3-dihydro-1,4-benzoxathiin-7-yl | 3-methylisoxazol-5-yl | methyl | |
| 2547 | 4,4-dioxide-8-Me-2,3-dihydro-1,4-benzoxathiin-7-yl | 3-methylisoxazol-5-yl | i-propyl | |
| 2548 | 4,4-dioxide-8-Me-2,3-dihydro-1,4-benzoxathiin-7-yl | 3-methylisoxazol-5-yl | cyclopropyl | |
| 2549 | 4,4-dioxide-8-Me-2,3-dihydro-1,4-benzoxathiin-7-yl | 3-methylisoxazol-5-yl | CF₃ | |
| 2550 | 2-Cl-4-SO₂MePh | 2-trifluoromethyl-1,3,4-thiadiazol-5-yl | cyclopropyl | 185 |
| 2551 | 2-Cl-SO₂MePh | 1,1-dioxido-3-oxo-1,2-benzisothiazol-2(3*H*)-yl | cyclopropyl | |
| 2552 | 4-Cl-Ph | 2-*t*-butyl-1,3,4-oxadiazol-5-yl | CF3 | 166 |
| 2553 | 2-Me-CF₃pyridin-3-yl | 2-methyltetrazol-5-yl | cyclopropyl | |
| 2554 | 2-[(2-methoxyethoxy)methyl]-6-CF₃ pyridin-3-yl | 2-methyltetrazol-5-yl | cyclopropyl | oil |
| 2555 | 2-Cl-4-SO₂MePh | 2,5-dioxopyrrolidin-1-yl | cyclopropyl | |
| 2556 | 2-Cl-4-SO₂MePh | 2-oxopyridin-1(2*H*)-yl | cyclopropyl | |
| 2557 | 2-Cl-4-SO₂MePh | 2-oxoquinolin-1(2*H*)-yl | cyclopropyl | |
| 2558 | 2-Cl-4-SO₂MePh | 1,2-benzisoxazol-3-yl | cyclopropyl | |
| 2559 | 2-Cl-4-SO₂MePh | 2-oxo-1,3-benoxazol-3(2*H*)-yl | cyclopropyl | |
| 2560 | 2-Cl-4-SO₂MePh | 3-oxo-2,3-dihydrozol-4*H*-1,4-benzoxazin-4-yl | cyclopropyl | |
| 2561 | 2-Cl-4-SO₂MePh | 2-oxopyrimidin-1(2*H*)-yl | cyclopropyl | |
| 2562 | 2-Cl-4-SO₂MePh | 1*H*-1,2,3-benzotriazol-1-yl | cyclopropyl | |
| 2563 | 2-NO₂-4-SO₂MePh | 2,5-dioxopyrrolidin-1-yl | cyclopropyl | |
| 2564 | 2-NO₂-4-SO₂MePh | 2-oxopyridin-1(2*H*)-yl | cyclopropyl | |
| 2565 | 2-NO₂-4-SO₂MePh | 2-oxoquinolin-1(2*H*)-yl | cyclopropyl | |
| 2566 | 2-NO₂₋4-SO₂MePh | 1,2-benzisoxazol-3-yl | cyclopropyl | |
| 2567 | 2-NO₂-4-SO₂MePh | 2-oxo-1,3-benzoxazol-3(2*H*)-yl | cyclopropyl | |
| 2568 | 2-NO₂-4-SO₂MePh | 3-oxo-2,3-dihydro-4*H*-1,4-benzoxazin-4-yl | cyclopropyl | |
| 2569 | 2-NO₂-4-SO₂MePh | 2-oxopyrimidin-1(2*H*)-yl | cyclopropyl | |
| 2570 | 2-NO₂4-SO₂MePh | 1*H*-1,2,3-benzotriazol-1-yl | cyclopropyl | |

### EXAMPLE 30

### Determination of the herbicidal activity and phytotoxicity in pre-emergence.

The herbicidal activity of the compounds of the invention in pre-emergence was evaluated according to the following operative procedures.

The plant species of interest (weeds or crops) were sown in pots with an upper diameter of 10 cm, a height of 10 cm and containing sandy soil. 10 pots were used for each plant species.

Water was added to each pot in such a quantity as to germinate the seeds. The pots were divided into two groups, each containing 5 pots for each weed or crop.

After one day from the sowing, the first set of pots was treated with a hydro-acetonic dispersion containing acetone at 10% in volume, the product under evaluation at the desired concentration and Tween 20 at 0.5%.

The second set was treated with a hydro-acetonic solution only, containing acetone at 10% in volume and Tween 20 at 0.5%, and was used as comparison (blank).

All pots were kept under observation in a conditioned environment under the following conditions:
- temperature: 24°C;
- relative humidity: 60%;
- photoperiod: 16 hours;
- light intensity: 10000 lux.

The pots were uniformly watered in order to ensure a sufficient humidity degree for a good development of the plants.

Fifteen days after the treatment, the herbicidal activity was evaluated on the basis of the following values, which refer to the damage percentage tested on the treated plants, with respect to the non-treated plants (blank):
- 0 = 0 - 10 % damage;
- 1 = 11 - 30 % damage;
- 2 = 31 - 50 % damage;
- 3 = 51 - 70 % damage;
- 4 = 71 - 90 % damage;
- 5 = 91 % damage - death of the plant.

Table 3 shows the results obtained by treating the plant species listed below with compounds 6, 7 and 11 with a dosage of 500 g/ha:
Abutilon theofrasti (AT); Amaranthus retroflexus (AR); Chenopodium album (CA); Galium aparine (GA); Ipomea purpurea (IP); Portulaca oleracea (PO); Solanum nigrum (SN); Stellaria media (SM).

**Table 3: Pre-emergence herbicidal activity at rate of 500 g/ha**

| Plant species: | AT | AR | CA | GA | IP | PO | SN | SM |
|---|---|---|---|---|---|---|---|---|
| | | | | | | | | |
| Compound N° 6: | 5 | 5 | 5 | 5 | 5 | 5 | 5 | 5 |
| Compound N° 7: | 5 | 5 | 5 | - | - | 5 | 5 | 5 |
| Compound N° 11: | 5 | - | 5 | - | 5 | 5 | - | - |
| | | | | | | | | |

### EXAMPLE 31

### Determination of the herbicidal activity and phytotoxicity in post-emergence.

The herbicidal activity of the compounds of the invention in post-emergence was evaluated according to the following operative procedures.

The plant species of interest (weeds or crops) were sown in pots with an upper diameter of 10 cm, a height of 10 cm and containing sandy soil. 10 pots were used for each plant species.

Water was added to each pot in such a quantity as to germinate the seeds. The pots were divided into two groups, each containing 5 pots for each weed or crop.

Fifteen days after sowing (ten, in the case of wheat), when the weeds and crops, according to the species, were 10-15 cm high, the first set of pots was treated with a hydro-acetonic dispersion containing acetone at 10% in volume, the product under evaluation at the desired concentration and Tween 20 at 0.5%.

The second set was treated with a hydro-acetonic solution only, containing acetone at 10% in volume and Tween 20 at 0.5%, and was used as comparison (blank).

All pots were kept under observation in a conditioned environment under the following conditions:
- temperature: 24°C;
- relative humidity: 60%;
- photo-period: 16 hours;
- light intensity: 10000 lux.

The pots were uniformly watered every other day so as to ensure a humidity degree sufficient for a good development of the plants.

The herbicidal activity was evaluated fifteen days after the treatment, on the basis of the following values which refer to the percentage of damage tested on the treated plants with respect to the non-treated plants (blank) :
- 0 = 0 - 10 % damage;
- 1 = 11 - 30 % damage;
- 2 = 31 - 50 % damage;
- 3 = 51 - 70 % damage;
- 4 = 71 - 90 % damage;
- 5 = 91 % damage - death of the plant.
Table 4 shows the results obtained by treating the plant species listed below with compounds 6 and 11 with a dosage of 500 g/ha:
Abutilon theofrasti (AT); Chenopodium album (CA); Galium aparine (GA); Portulaca oleracea (PO); Solanum nigrum (SN); Stellaria media (SM).

**Table 4: Post-emergence herbicidal activity at rate of 500 g/ha**

| Plant species: | AT | CA | GA | PO | SN | SM |
|---|---|---|---|---|---|---|
| | | | | | | |
| Compound N° 6: | 5 | 5 | 5 | 5 | 5 | 5 |
| Compound N° 11: | 5 | 5 | - | - | 5 | - |
| | | | | | | |

## Claims

1. Derivatives of 1,3-diones having general formula (I): 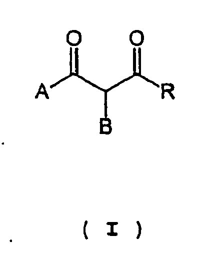 wherein:
- A represents:
an aryl group optionally substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁, -Q, -ZQ_{1,}
or it represents a heterocyclic group selected from pyridyl, pyrimidyl, quinolinyl, pyrazolyl, thiazolyl, oxazolyl, thienyl, furyl,
4,4-dioxide-2,3-dihydro-1,4-benzoxathiin-7-yl,
with said groups optionally substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁,
- B represents a D-(Rₓ)ₙ group;
- R represents a linear or branched C₁-C₆ haloalkyl group; a C₃-C₆ cycloalkyl or C₄-C₁₂ cycloalkylalkyl group optionally substituted with halogen atoms or C₁-C₆ alkyl;
- R₁ represents a C₁-C₆ alkyl group or a C₁-C₆ haloalkyl group,
- m is equal to 0, 1 or 2;
- t is equal to 1 or 2;
- R₂, R₃, R₆, R₇, R₈, R₉, the same or different, represent a hydrogen atom, a linear or branched C₁-C₆ alkyl group;
- R₄, R₅ represent a hydrogen atom, a linear or branched C₁-C₆ alkyl group in turn optionally substituted with halogen atoms;
- Q, Q₁, represent an aryl group, a C₃-C₆ cycloalkyl group, a heterocyclic group selected from pyrazolyl, tetrazolyl, tetrazolonyl, isoxazolyl, oxazolyl, thiazolyl, oxadiazolyl, thiadiazolyl, isothiazolyl, isoxazolinyl, 1,3-dioxolanyl, tetrahydropyranyl, oxethanyl, oxyranyl, thiazolidinyl, oxazolidinyl,
said groups optionally substituted by one or more substituents selected from halogen, NO₂, OH, CN, CHO, linear or branched C₁-C₆ alkyl, linear or branched C₁-C₆ haloalkyl, linear or branched C₁-C₆ alkoxyl, linear or branched C₁-C₆ haloalkoxyl;
- Z = O, S(O)ᵣ;
- r is equal to 0, 1 or 2;
- D represents:
a monocyclic heterocyclic group which can be connected to the rest of the structure either through one of its carbon atoms or, when possible, through one of its nitrogen atoms;
- Rₓ represents a substituent selected from hydrogen, halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ allcoxyalkoxyalkyl, -S (O)ₘR₁, -OS (O)ₜR₁, -SO₂NR₂R₃, -CO₂R₄, -COR₅, -CONR₆R₇, -CSNR₈R₉; -Q,
if more than one Rₓ groups are present, they can be the same or different;
- n = 1-9.

2. The derivatives according to claim 1, **characterized in that** the compound having formula (I) are present as tautomeric forms, pure or as blends of tautomeric forms, in any proportion whatsoever.

3. Use of derivatives of 1,3-dines having general formula (I') 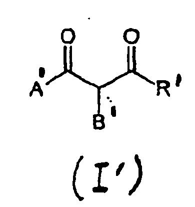 wherein:
- A¹ represents:
an aryl group optionally substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl C₂-C₆ alkylthioalkoxyl, C₂-C₆ haloalkylthioalkoxyl, C₃-C₁₂ dialkoxyalkyl, C₃-C₁₂ dialkylthioakyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl -S(O)ₘR₁, -OS(O)ₜR₁, ₋SO₂NR₂R₃, -CO₂R₄, -COR₅, -CONR₆R₇, -CSNR₈R₉, -Q, -ZQ_{1,}
or represents a heterocyclic group selected from pyridyl, pyrimidyl, quinolinyl, pyrazolyl, thiazolyl, oxazolyl, thienyl, furyl, 4,4-dioxide-2,3-dihydro-1,4-benzoxathiin-7-yl,
with all these groups possibly substituted by one or more substituents selected from halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁,
- B' represents a D'-(Rₓ)ₙ group;
- R' represents a hydrogen atom; a linear or branched C₁-C₆ alkyl group; a linear or branched C₁-C₆ haloalkyl group; a C₃-C₆ cycloalkyl group or a C₄-C₁₂ cycloalkylalkyl group possibly substituted with halogen atoms or C₁-C₆ alkyl;
- R₁ represents a C₁-C₆ alkyl or C₁-C₆ haloalkyl group;
- m is equal to 0, 1 or 2;
- t is equal to 1 or 2;
- R₂, R₃, R₆, R₇, R₈, R₉, the same or different, represent a hydrogen atom, a linear or branched C₁-C₆ alkyl group;
- R₄, R₅ represent a hydrogen atom, a linear or branched C₁-C₆ alkyl group in turn possibly substituted with halogen atoms;
- Q, Q₁, represent an aryl group, a C₃-C₆ cycloalkyl group, a heterocyclic group selected from pyrazolyl, tetrazolyl, tetrazolonyl, isoxazolyl, oxazolyl, thiazolyl, oxadiazolyl, thiadiazolyl; isothiazolyl, isoxazolinyl, 1,3-dioxolanyl, tetrahydropyranyl, oxethanyl, oxyranyl, thiazolidinyl, oxazolidinyl,
said groups optionally substituted by one or more substituents selected from halogen, NO₂, CN, CHO, linear or branched C₁-C₆ alkyl, linear or branched C₁-C₆ haloalkyl, linear or branched C₁-C₆ alkoxyl, linear or branched C₁-C₆ haloalkoxyl;
- Z = O, S(O)ᵣ;
- r is equal to 0, 1 or 2;
- D' represents:
a heterocyclic group which can be mono or polycyclic and can be connected to the rest of the structure either through one of its carbon atoms or, when possible, through one of its nitrogen atoms;
- Rₓ represents a substituent selected from hydrogen, halogen, NO₂, CN, CHO, OH, linear or branched C₁-C₆ alkyl, linear or branched C₁-C₆ haloalkyl, linear or branched C₁-C₆ alkoxyl, linear or branched C₁-C₆ haloalkoxyl, C₁-C₆ cyanoalkyl, C₂-C₆ alkoxyalkyl, C₂-C₆ alkylthioalkyl, C₂-C₆ alkylsulfinylalkyl, C₂-C₆ alkylsulfonylalkyl, C₂-C₆ haloalkoxyalkyl, C₂-C₆ haloalkylthioalkyl, C₂-C₆ haloalkylsulfinylalkyl, C₂-C₆ haloalkylsulfonylalkyl, C₂-C₆ alkoxyalkoxyl or C₂-C₆ haloalkoxyalkoxyl, C₂-C₆ alkylthioalkoxyl, C₂-C₆ haloalkylthioalkoxyl, C₃-C₁₂ dialkoxyalkyl, C₃-C₁₂ dialkylthioalkyl, C₃-C₁₂ dialkylthioalkoxyl, C₃-C₁₂ dialkoxyalkoxyl, C₂-C₆ haloalkoxyhaloalkoxyl, C₃-C₁₀ alkoxyalkoxyalkyl, -S(O)ₘR₁, -OS(O)ₜR₁, -SO₂NR₂R₃, -CO₂R₄, -COR₅, -CONR₆R₇, -CSNR₈R₉, Q;
if more than one Rₓ groups are present, these can be the same or different;
- n = 1-9;
and of their agronomically compatible salts, as herbicides.

4. Use according to claim 3, for the control under pre-emergence and post-emergence of monocotyledon and dicotyledon weeds.

5. A method for the control of weeds in agricultural crops, by the application of compounds having general formula (I'): 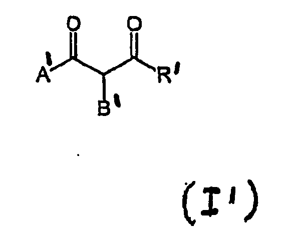 wherein:
- A', B' and R' have the meanings according to claim 3.

6. The method according to claim 5, **characterized in that** the quantity of compound having formula (I') to be applied ranges from 1 g to 4,000 g per hectare.

7. Herbicidal compositions containing, as active principle, one or more compounds having general formula (I): 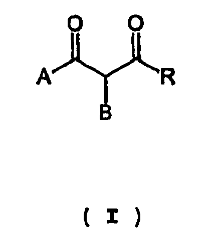 wherein:
- A, B and R have the meanings according to claim 1 , possibly also as a blend of tautomers.

8. Herbicidal compositions according to claim 7, including one or more compounds having general formula (I') as defined in claim 3 and one or more further compounds selected from a herbicide, fungicide, insecticide, acaricide, fertilizer.

9. The herbicidal compositions according to claim 8, **characterized in that** the additional herbicides are selected from:
acetochlor, acifluorfen, aclonifen, AKH-7088, alachlor, alloxydim, ametryn, amicarbazone, amidosulfuron, amitrole, anilofos, asulam, atrazine, azafenidin, azimsulfuron, aziprotryne, BAS 670 H, BAY MKH 6561, beflubutamid, benazolin, benfluralin, benfuresate, bensulfuron, bensulide, bentazone, benzfendizone, benzobicyclon, benzofenap, benzthiazuron, bifenox, bilanafos, bispyribac-sodium, bromacil, bromobutide, bromofenoxim, bromoxynil, butachlor, butafenacil, butamifos, butenachlor, butralin, butroxydim, butylate, cafenstrole, carbetamide, carfentrazone-ethyl, chlomethoxyfen, chloramben, chlorbromuron, chlorbufam, chlorflurenol, chloridazon, chlorimuron, chlornitrofen, chlorotoluron, chloroxuron, chlorpropham, chlorsulfuron, chlorthal, chlorthiamid, cinidon ethyl, cinmethylin, cinosulfuron, clethodim, clodinafop, clomazone, clomeprop, clopyralid, cloransulam-methyl, cumyluron (JC-940), cyanazine, cycloate, cyclosulfamuron, cycloxydim, cyhalofop-butyl, 2,4-D, 2,4-DB, daimuron, dalapon, desmedipham, desmetryn, dicamba, dichlobenil, dichlorprop, dichlorprop-P, diclofop, diclosulam, diethatyl, difenoxuron, difenzoquat, diflufenican, diflufenzopyr, dimefuron, dimepiperate, dimethachlor, dimethametryn, dimethenamid, dinitramine, dinosseb, dinoseb acetate, dinoterb, diphenamid, dipropetryn, diquat, dithiopyr, 1-diuron, eglinazine, endothal, EPTC, espropcarb, ethalfluralin, ethametsulfuron-methyl, ethidimuron, ethiozin (SMY 1500), ethofumesate, ethoxyfen-ethyl (HC-252), ethoxysulfuron, etobenzanid (HW 52), fenoxaprop, fenoxaprop-P, fentrazamide, fenuron, flamprop, flamprop-M, flazasulfuron, florasulam, fluazifop, fluazifop-P, fluazolate (JV 485), flucarbazone-sodium, fluchloralin, flufenacet, flufenpyr ethyl, flumetsulam, flumiclorac-pentyl, flumioxazin, flumipropin, fluometuron, fluoroglycofen, fluoronitrofen, flupoxam, fluproanate, flupyrsulfuron, flurenol, fluridone, flurochloridone, fluroxypyr, flurtamone, fluthiacet-methyl, fomesafen, foramsulfuron, fosamine, furyloxyfen, glufosinate, glyphosate, halosulfuron-methyl, haloxyfop, haloxyfop-P-methyl, hexazinone, imazamethabenz, imazamox, imazapic, imazapyr, imazaquin, imazethapyr, imazosulfuron, indanofan, iodosulfuron, ioxynil, isopropalin, isoproturon, isouron, isoxaben, isoxachlortole, isoxaflutole, isoxapyrifop, KPP-421, lactofen, lenacil, linuron, LS830556, MCPA, MCPA-thioethyl, MCPB, mecoprop, mecoprop-P, mefenacet, mesosulfuron, mesotrione, metamitron, metazachlor, methabenzthiazuron, methazole, methoprotryne, methyldymron, metobenzuron, metobromuron, metolachlor, S-metolachlor, metosulam, metoxuron, metribuzin, metsulfuron, molinate, monalide, monolinuron, naproanilide, napropamide, naptalam, NC-330, neburon, nicosulfuron, nipyraclofen, norflurazon, orbencarb, oryzalin, oxadiargyl, oxadiazon, oxasulfuron, oxaziclomefone, oxyfluorfen, paraquat, pebulate, pendimethalin, penoxsulam, pentanochlor, pentoxazone, pethoxamid, phenmedipham, picloram, picolinafen, piperophos, pretilachlor, primisulfuron, prodiamine, profluazol, proglinazine, prometon, prometryne, propachlor, propanil, propaquizafop, propazine, propham, propisochlor, propyzamide, prosulfocarb, prosulfuron, pyraclonil, pyraflufen-ethyl, pyrazogyl (HAS-961), pyrazolynate, pyrazosulfuron, pyrazoxyfen, pyribenzoxim, pyributicarb, pyridafol, pyridate, pyriftalid, pyriminobac-methyl, pyrithiobac-sodium, quinclorac, quinmerac, quizalofop, quizalofop-P, rimsulfuron, sethoxydim, siduron, simazine, simetryn, sulcotrione, sulfentrazone, sulfometuron-methyl, sulfosulfuron, 2,3,6-TBA, TCA-sodium, tebutam, tebuthiuron, tepraloxydim, terbacil, terbumeton, terbuthyl-azine, terbutryn, thenylchlor, thiazafluron, thiazopyr, thidiazimin, thifensulfuron-methyl, thiobencarb, tiocarbazil, tioclorim, tralkoxydim, tri-allate, triasulfuron, triaziflam, tribenuron, triclopyr, trietazine, trifloxysulfuron, trifluralin, triflusulfuron-methyl, tritosulfuron, UBI-C4874, vernolate.

10. The compositions according to any of the claims 7-8, **characterized in that** the concentration of active substance ranges from 1 to 90%.

## Patentansprüche

1. Derivate von 1,3-Dionen der allgemeinen Formel (I) : 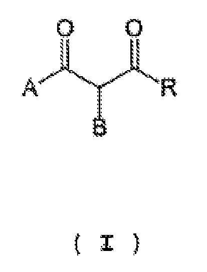 worin :
- A:
eine Arylgruppe bedeutet, die gegebenfalls durch eine oder mehrere Sustituententen substituiert ist, die aus Halogen, NO₂, CN, CHO, OH, geradkettiges oder verzweigtes C₁-C₆-Alkyl, geradkettiges oder verzweigtes C₁-C₆-Halogenalkyl, geradkettiges oder verzweigtes C₁-C₆₋Alkoxy, geradkettiges oder verzweigtes C₁-C₆-Halogenalkoxy, C₁-C₆₋Cyanoalkyl, C₂-C₆-Alkoxyalkyl, C₂-C₆-Alkylthioalkyl, C₂-C₆-Alkylsulfinylalkyl, C₂-C₆-Alkylsulfonylalkyl, C₂-C₆-Halogenalkoxyalkyl, C₂-C₆-Halogenalkylthioalkyl, C₂-C₆ Halogenalkylsulfinylalkyl, C₂-C₆ Halogenalkylsulfonylalkyl, C₂-C₆ Alkoxyalkoxy oder C₂-C₆ Halogenalcoxyalkoxy, C₂-C₆ Alkylthioalkoxy, C₂-C₆ Halogenalkylthioalkoxy, C₃-C₁₂ Dialkoxyalkyl, C₃-C₁₂ Dialkylthioalkyl, C₃-C₁₂ Dialkylthioalkoxy, C₃-C₁₂ Dialcoxyalkoxy, C₂-C₆ Halgenoalcoxyhalogenalkoxy, C₃-C₁₀ Alkoxyalkoxyalkyl, -5(O)ₘR₁, -Q, -ZQ₁,
oder eine heterozyklische Gruppe, die aus Pyridyl, Pyrimidyl, Quinolinyl, Pyrazolyl, Thiazolyl, Oxazolyl, Thienyl, Furyl, 4,4-dioxo-2,3-dihydro-1,4-benzoxathin-7-yl,
wobei diese Gruppe gegebenfalls durch eine oder mehrere Substituenten substituiert ist, die aus Halogen, NO₂, CN, CHO, OH, geradkettigem oder verzweigtem C₁-C₆-Alkyl, geradkettigem oder verzweigtem C₁-C₆-Halogenalkyl, geradkettigem oder verzweigtem C₁-C₆-Alkoxy, geradkettigem oder verzweigtem C₁-C₆-Haloalkoxy, C₁-C₆₋Cyanoalkyl, C₂-C₆-Alkoxyalkyl, C₂-C₆-Alkylthioalkyl , C₂-C₆-Alkylsulfinylalkyl, C₂-C₆-Alkylsulfonylalkyl, C₂-C₆₋Halogenalkoxyalkyl, C₂-C₆-Halogenalkylthioalkyl, C₂-C₆-Halogenalkylsulfinylalkyl, C₂-C₆-Halogenalkylsulfonylalkyl, C₂-C₆-Alkoxyalkoxy oder C₂-C₆-Halogenalkoxyalkoxy, C₂-C₆-Alkylthioalkoxy, C₂-C₆-Halogenalkylthioalkoxy, C₃-C₁₂-Dialkoxyalkyl, C₃-C₁₂-Dialkylthioalkyl, C₃-C₁₂-Dialkylthioalkoxy, C₃-C₁₂-Dialkoxyalkoxy, C₂-C₆-Halogenalkoxyhaloalkoxy , C₃-C₁₀-Alkoxyalkoxyalkyl, -S(O)ₘR₁, ausgewählt ist;
- B eine D-(R_{X})ₙ Gruppe bedeutet;
- R eine geradkettige oder verzweigte C₁-C₆-Halogenalkylgruppe; eine C₃-C₆-Cykloalkylgruppe oder eine C₄-C₁₂-Cycloalkylalkylgruppe, gegebenenfalls mit Halogenatomen oder C₁-C₆ Alkyl substituiert, bedeutet;
- R₁ eine C₁-C₆-Alkylgruppe oder eine C₁-C₆-Halogenalkylgruppe bedeutet;
- m 0, 1 oder 2 ist;
- t 1 oder 2 ist;
- R₂, R₃, R₆, R₇, R₈, R₉, gleich oder verschieden ein Wasserstoffatom, eine geradkettige oder verzweigte C₁-C₆-Alkylgruppe bedeuten;
- R₄, R₅ ein Wasserstoffatom, eine geradkettige oder verzweigte C₁-C₆-Alkylgruppe bedeuten, die wiederum durch Wasserstoffatome gegebenenfalls substituiert ist;
- Q, Q₁ eine Arylgruppe, eine C₃-C₆-Cycloalkylgruppe, eine heterocyklische Gruppe bedeuten, die aus Pyrazolyl, Tetrazolyl, Tetrazolonyl, Isoxazolyl, Oxazolyl, Thiazolyl, Oxadiazolyl, Thiadazolyl, Isothiazolyl, Isoxazolinyl, 1,3-Dioxolanyl, Tetrahydropyranyl, Oxetanyl, Oxyranyl, Thiazolidinyl, Oxazolidinyl ausgewählt sind;
wobei diese Gruppe gegebenfalls durch eine oder mehrere Substituenten substitutiert sind, die aus Halogen, NO₂, OH, CN, CHO, geradkettigem oder verzweigtem C₁-C₆-Alkyl, geradkettigem oder verzweigtem C₁-C₆-Halogenalkyl, geradkettigem oder verzweigtem C₁-C₆-Alkoxy, geradkettigem oder verzweigtem C₁-C₆-Halogenalkoxy ausgewählt sind,
- Z = O, S(O)ᵣ;
- r 0, 1 oder 2 ist;
- D eine heterocyklische, monocyklische Gruppe bedeutet, die mit dem übrigen Teil der Struktur durch eine ihrer Kohlensäurenatomen oder möglicherweise durch eine ihrer Stickstoffatomen verbunden werden kann;
- Rₓ einen Substituenten bedeutet, der aus Wasserstoff, Halogen, NO₂, CN, CHO, OH, geradkettigem oder verzweigtem C₁-C₆-Alkyl, geradkettigem oder verzweigtem C₁-C₆ Halogenalkyl, geradkettigem oder verzweigtem C₁-C₆-Alkoxy, geradkettigem oder verzweigtem C₁-C₆-Haloalkoxy, C₁-C₆ Cyanoalkyl, C₂-C₆-Alkoxyalkyl, C₂-C₆-Alkylthioalkyl, C₂-C₆-Alkylsulfinylalkyl, C₂-C₆-Alkylsulfonylalkyl, C₂-C₆Halogenalkoxyalkyl, C₂-C₆-Halogenalkylthioalkyl, C₂-C₆ Halogenalkylsulfinylalkyl, C₂-C₆-Halogenalkylsulfonylalkyl, C₂-C₆-Alkoxyalkoxy oder C₂-C₆ Halogenalkoxyalkoxy, C₂-C₆-Halogenalkylthioalkoxy, C₃-C₁₂-Dialkoxyalkyl, C₃-C₁₂ Dialkylthioalkyl, C₃-C₁₂-Dialkylthioalkoxy, C₃-C_{12.}Dialkoxyalkoxy, C₂-C₆ Halogenalkoxyhalogenalkoxy, C₃-C₁₀-Alkoxyalkoxyalkyl, -S(O)ₘR₁, -OS(O)ₜR₁, -SO₂NR₂R₃, -CO₂R₄, -COR₅, -CONR₆R₇, - CSNR₈R₉ ; -Q ausgewählt ist;
wenn mehr als eine Rₓ-Gruppe vorliegt, diese können gleich oder verschieden sein;
- n=1-9.

2. Derivate nach Anspruch 1, **dadurch gekennzeichnet, dass** die Verbindungen der Formel (I) in tautomerischer Form, in reiner Form oder als Mischungen tautomerischer Formen in jedem beliebigen Verhältnis vorliegen.

3. Verwendung der Derivate von 1,3-dionen der allgemeinen Formel (I') : 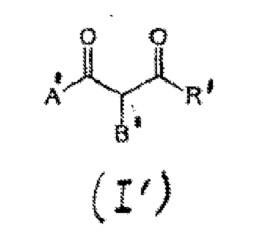 worin :
- A' :
eine Arylgruppe bedeutet, die optional durch eine oder mehrere Substituenten substituiert ist, die aus Halogen, NO₂, CN, CHO, OH, geradkettigem oder verzweigtem C₁-C₆-Alkyl, geradkettigem oder verzweigtem C₁-C₆-Halogenalkyl, geradkettigem oder verzweigtem C₁-C₆-Alkoxy, geradkettigem oder verzweigtem C₁-C₆-Halogenalkoxy, C₁-C₆-Cyanoalkyl, C₂-C₆ Alkoxyalkyl, C₂-C₆-Alkylthioalkyl, C₂-C₆ Alkylsulfinylalkyl, C₂-C₆-Alkylsulfonylalkyl, C₂-C₆ Halogenalkoxyalkyl, C₂-C₆-Halogenalkylthioalkyl, C₂-C₆-Halogenalkylsulfinylalkyl, C₂-C₆-Halogenalkylsulfonylalkyl, C₂-C₆-Alkoxyalkoxy oder C₂-C₆-Halogenalkoxyalkoxy, C₂-C₆-Alkylthioalkoxy, C₂-C₆-Halogenalkylthioalkoxy, C₃-C₁₂-Dialkoxyalkyl, C₃-C₁₂ Dialkylthioalkyl, C₃-C₁₂-Dialkylthioalkoxy, C₃-C₁₂-Dialkoxyalkoxy, C₂-C₆-Halgenoalkoxyhalogenalkoxy, C₃-C₁₀-Alkoxyalkoxyalkyl, -S(O)ₘR₁, -OS(O)ₜR₁, - SO₂NR₂R₃, -CO₂R₄, -COR₅, -CONR₆R₇, -CSNR₈R₉, -Q, -ZQ₁ ausgewählt ist,
bzw. A' eine heterocyklische Gruppe bedeutet, die aus Pyridyl, Pyrimidyl, Quinolinyl, Pyrazolyl, Thiazolyl, Oxazolyl, Thienyl, Furyl, 4,4-dioxo-2,3-dihydro-1,4-benzoxathiin-7-yl, ausgewählt ist;
wobei alle diese Gruppen gegebenfalls durch eine oder mehrere Substituenten substituiert werden, die aus Halogen, NO₂, CN, CHO, OH, geradkettigem oder verzweigtem C₁-C₆-Alkyl, geradkettigem oder verzweigtem C₁-C₆-Halogenalkyl, geradkettigem oder verzweigtem C₁-C₆-Alkoxy, geradkettigem oder verzweigtem C₁-C₆-Halogenalkoxy, C₁-C₆-Cyanoalkyl, C₂-C₆-Alkoxyalkyl, C₂-C₆-Alkylthioalkyl, C₂-C₆-Alkylsulfinylalkyl, C₂-C₆-Alkylsulfonylalkyl, C₂-C₆-Halogenalkoxyalkyl, C₂-C₆-Halogenalkylthioalkyl, C₂-C₆-Halogenalkylsulfinylalkyl, C₂-C₆-Halogenalkylsulfonylalkyl, C₂-C₆-Alkoxyalkoxy oder C₂-C₆-Halogenalkoxyalkoxy, C₂-C₆-Alkylthioalkoxy, C₂-C₆-Halogenalkylthioalkoxy, C₃-C₁₂-Dialkoxyalkyl, C₃-C₁₂-Dialkylthioalkyl, C₃-C₁₂-Dialkylthioalkoxy, C₃-C₁₂-Dialkoxyalkoxy, C₂-C₆-Halogenalkoxyhalogenalkoxy, C₃-C₁₀-Alkoxyalkoxyalkyl, -S(O)ₘR₁, ausgewählt sind,
- B' eine D'(Rₓ)ₙ Gruppe bedeutet;
- R' ein Wasserstoffatom, eine geradkettiges oder verzweigtes C₁-C₆-Alkylgruppe; eine geradkettiges oder verzweigtes C₁-C₆-Halogenalkylgruppe; eine C₃-C₆-Cycloalkylgruppe oder eine C₄-C₁₂-Cycloalkylalkylgruppe bedeutet, die gegebenenfalls von Halogenatomen oder C₁-C₆-Alkyl substituiert ist ;
- R¹ eine C₁-C₆-Alkylgruppe oder eine C₁-C₆-Halogenalkylgruppe ist;
- m 0, 1 oder 2 ist;
- t 1 oder 2 ist;
- R₂, R₃, R₆, R₇, R₈, R₉, gleich oder verschieden, ein Wasserstoffatom, eine geradkettige oder verzweigte C₁-C₆-Alkylgruppe sind;
- R₄, R₅ ein Wasserstoffatom, eine geradkettige oder verzweigte C₁-C₆₋Alkylgruppe bedeuten, die wiederum gegebenenfalls durch Halogenatome substituiert sind;
- Q, Q₁, eine Arylgruppe, eine C₃-C₆-Cycloalkylgruppe, eine heterocyklische Gruppe bedeuten, die aus Pyrazolyl, Tetrazolyl, Tetrazolonyl, Isoxazolyl, Oxazolyl, Thiazolyl, Oxadiazolyl, Thiadazolyl, Isothiazolyl, Isoxazolinyl, 1,3-Dioxolanyl, Tetrahydropyranyl, Oxetanyl, Oxyranyl, Thiazolidinyl, Oxazolidinyl ausgewählt sind;
wobei diese Gruppen gegebenfalls durch eine oder mehrere Substituenten substituiert sind, die aus Halogen, NO₂, CN, CHO, geradkettiges oder verzweigtes C₁-C₆-Alkyl, geradkettiges oder verzweigtes C₁-C₆-Halogenalkyl, geradkettiges oder verzweigtes C₁-C₆-Alkoxy, geradkettiges oder verzweigtes C₁-C₆-Halogenalkoxy ausgewählt sind,
- Z = O, S(O)ᵣ;
- r 0, 1 oder 2 bedeutet;
- D' eine heterocyklische Gruppe bedeutet, die mono- oder polyzyklisch sein kann und die mit dem übrigen Teil der Struktur durch eine ihrer Carbonsäurenatomen oder, womöglich, durch eine ihrer Stickstoffatome verbunden werden kann;
- Rₓ einen Substituenten bedeutet, der aus Wasserstoff, Halogen, NO₂, CN, CHO, OH, geradkettigem oder verzweigtem C₁-C₆-Alkyl, geradkettigem oder verzweigtem C₁-C₆-Halogenalkyl, geradkettigem oder verzweigtem C₁-C₆-Alkoxy, geradkettigem oder verzweigtem C₁-C₆-Halogenalkoxy, C₁-C₆-Cyanoalkyl, C₂-C₆-Alkoxyalkyl, C₂-C₆-Alkylthioalkyl, C₂-C₆ Alkylsulfinylalkyl, C₂-C₆-Alkylsulfonylalkyl, C₂-C₆-Halogenalkoxyalkyl, C₂-C₆ -Halogenalkylthioalkyl, C₂-C₆-Halogenalkylsulfinylalkyl, C₂-C₆-Halogenalkylsulfonylalkyl, C₂-C₆-Alkoxyalkoxy oder C₂-C₆-Halogenalkoxyalkoxy, C₂-C₆-Alkylthioalkoxy, C₂-C₆ Halogenalkylthioalkoxy, C₃-C₁₂-Dialkoxyalkyl, C₃-C₁₂-Dialkylthioalkyl, C₃-C₁₂-Dialkylthioalkoxy, C₃-C₁₂-Dialkoxyalkoxy, C₂-C₆-Halogenalkoxyhalogenalkoxy, C₃-C₁₀-Alkoxyalkoxyalkyl, -S(O)ₘR₁, -OS(O)ₜR₁, - SO₂NR₂R₃, -CO₂R₄, -COR₅, -CONR₆R₇, -CSNR₈R₉, Q ausgewählt ist;
wenn mehr als eine Rₓ-Gruppe vorliegt, können diese gleich oder verschieden sein;
- n=1-9;
und ihre agrarwissenschaftlich kompatiblen Salze wie Herbizide.

4. Verwendung nach Anspruch 3, für die Vor- und Nachwachstumsüberwachung von monokotylen und dikotylen Unkräutern.

5. Verfahren zur Unkrautüberwachung in Anbauflächen durch die Anwendung der Verbindungen der allgemeinen Formel (I'): 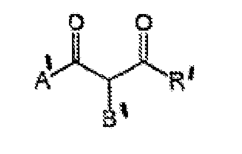 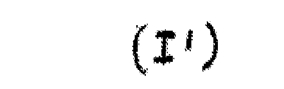 worin :
- A', B' und R' die jeweilige Bedeutung nach Anspruch 3 haben.

6. Verfahren nach Anspruch 5, **dadurch gekennzeichnet, dass** die Menge der zu benutzenden Verbindung der Formel (I') zwischen 1 g und 4 000 g pro Hektar liegt.

7. Herbizide Zusammensetzungen, die als Wirkstoff eine oder mehrere Verbindungen der allgemeinen Formel (I) umfassen: 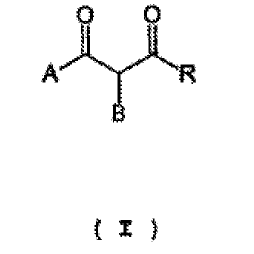 worin :
- A, B und R die jeweilige Bedeutung nach Anspruch 1 haben, gegebenfalls auch als Mischung von Tautomeren.

8. Herbizide Zusammensetzungen nach Anspruch 7, die eine oder mehrere Verbindungen der allgemeinen Formel (I') wie im Anspruch 3 definiert einschliessen und eine oder mehrere weitere Verbindungen, die aus Herbiziden, Fungiziden, Insektiziden, Akariziden, Düngemitteln ausgewählt sind.

9. Herbizide Zusammensetzungen nach Anspruch 8, **dadurch gekennzeichnet, dass** die weitere Herbizide aus: Acetochlor, Acifluorfen, Aclonifen, AKH-7088, Alachlor, Alloxydim, Ametryn, Amicarbazon, Amidosulfuron, ami-Trole, Anilofos, Asulam, Atrazin, Azafenidin, Azimsulfuron, Aziprotryne, BAS 670 H, BAY MKH 6561, Beflubutamid, Benazolin, Benfluralin, Benfuresat, Bensulfuron, Bensulid, Bentazon, Benzfendizon, Benzobicyclon, Benzofenap, Benzthia-zuron, Bifenox, Bilanafos, Bispyribac-Natrium, Bromacil, Bromobutid, Bromofenoxim, Bromoxynil, Butachlor, Buta-fenacil, Butamifos, Butenachlor, Butralin, Butroxydim, Butylat, Cafenstrole, Carbetamid, Carfentrazone-ethyl, Chlomethoxyfen, Chloramben, Chlorbromuron, Chlorbufam, Chlorflurenol, Chloridazon, Chlorimuron, Chlornitrofen, Chlortoluron, Chloroxuron, Chlorpropham, Chlorsulfuron, Chlorthal, Chlorthiamid, Cinidon-ethyl, Cinmethylin, Cinosulfuron, Clethodim, Clodinafop, Clomazon, Clomeprop, Clopyralid, Cloransulam-methyl, Cumyluron (JC-940), Cyanazin, Cycloat, Cyclosulfamuron, Cycloxydim, Cyhalo-fop-Butyl-, 2,4-D, 2,4-DB, Daimuron , Dalapon, Desmedipham, Desmetryn, Dicamba, Dichlobenil, Dichlorprop, Dichlorprop-P, Diclofop, Diclosulam, Diethatyl, Difenoxuron, Difenzoquat, Diflufenican, Diflufenzopyr, Dimefuron, Dimepiperat, Dimethachlor, Dimethametryn, Dimethenamid, Dinitramin, Dinoseb, Dinoseb Acetat, Dinoterb, Diphen-amid, Dipropetryn, Diquat, Dithiopyr, 1-Diuron, Eglinazin, Endothal, EPTC, Esprocarb, Ethalfluralin, Ethametsulfuron-methyl, Ethidimuron, Ethiozin (SMY 1500), Ethofumesat, Ethoxyfen-Ethyl (HC- 252), Ethoxysulfuron, Etobenzanid (HW 52), Fenoxaprop, Fenoxaprop-P, Fentrazamide, Fenuron, Flamprop, Flamprop-M, Flazasulfuron, Florasulam, Fluazifop, Fluazifop-P, Fluazolate (JV 485), Flucarbazone-Natrium, Fluchloralin, Flufenacet, Flufenpyr Ethyl, Flumetsulam, Flumiclorac-Pentyl, Flumioxazin, Flumipropin, Fluometuron, Fluoroglycofen, Fluoronitrofen, Flupoxam, Flupropanat, Flupyrsulfuron, Flurenol, Fluridon, Flurochloridon, Fluroxypyr, Flurtamon, Fluthi-Acet-methyl, Fomesafen, Foramsulfuron, Fosamin, Furiloxyfen, Glufosinat, Glyphosat, Halosulfuron-methyl, Haloxyfop, Haloxyfop-P-Methyl, Hexazinon, Imazamethabenz, Imazamox, Imazapic, Imazapyr, Imazaquin, Imazethapyr, Imazosulfuron, Indanofan, Iodosulfuron, Ioxynil, Isopropalin, Isoproturon, Isouron, Isoxaben, Isoxachlortol, Isoxaflutol, Isoxapyrifop, KPP-421, Lactofen, Lenacil, Linuron, LS830556, MCPA, MCPA-Thioethyl, MCPB, Mecoprop, Mecoprop-P, Mefenacet, Mesosulfuron, Mesotrione, Metamitron, Metazachlor, Methabenzthiazuron, Methazol, Methoprotryne, Methyldymron, Metobenzuron, Metobromuron, Metolachlor, S-Metolachlor, Metosulam, Metoxuron, Metribuzin, Metsulfuron, Molinat, Monalid, Monolinuron, Naproanilid, Napropamid, Naptalam, NC-330, Neburon, Ni-cosulfuron, Nipyraclofen, Norflurazon, Orbencarb, Oryzalin, Oxadiargyl, Oxadiazon, Oxasulfuron, Oxaziclomefon, Oxyfluorfen, Paraquat, Pebulat, Pendimethalin, Penoxsulam, Pentanochlor, Pentoxazon, Pethoxamid, Phenmedipham, Picloram, Picolinafen, Piperophos, Pretilachlor, Primisulfuron, Prodiamin, Profluazol, Proglinazin, Prometon, Prometryn, Propachlor, Propanyl, Propaquizafop, Propazin, Propham, Propisochlor, Propyzamid, Prosulfocarb, Prosulfuron, Pyraclonil, Pyraflufen-Ethyl, Pyrazogyl (HSA-961), Pyrazolynat, Pyrazosulfuron, Pyrazoxyfen, Pyribenzoxim, Pyributicarb, Pyridafol, Pyridat, Pyriftalid, Pyriminobac-methyl, Pyrithiobac-Natrium, Quinclorac, Quinmerac, Quizalofop, Quizalofop-P, Rimsulfuron, Sethoxydim, Siduron, Simazin, Simetryn, Sulco-trion, Sulfentrazon, Sulfometuron-methyl, sulfosulfuron, 2,3,6-TBA, TCA-Natrium, Tebutam, Tebuthiuron, Tepra-loxydim, Terbacil, Terbumeton, Terbuthyl-azin, Terbu-tryn, Thenylchlor, Thiazafluron, Thiazopyr, Thidiazimin, Thifensulfuron-methyl, Thiobencarb, Tiocarbazil, Tio-clorim, Tralkoxydim, Tri-allat, Triasulfuron, Triaziflam, Tribenuron, Triclopyr, Trietazine, Trifloxysulfuron, Trifluralin, Triflusulfuron-methyl, Tritosulfuron, UBI-C4874 und Vernolat ausgewählt sind.

10. Die Zusammensetzungen nach irgendeinem der Ansprüche 7-9, **dadurch gekennzeichnet, dass** die Konzentration des Wirkstoffes zwischen 1 und 90% liegt.

## Revendications

1. Dérivés de 1,3-diones ayant la formule générale (I) : 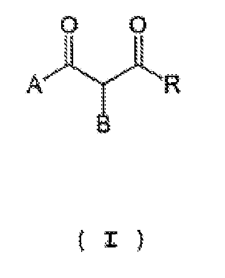 où :
- A représente :
un groupe aryle éventuellement substitué par un ou plusieurs substituants choisis parmi halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, alkylthioalcoxyle en C₂-C₆, haloalkylthioalcoxyle en C₂-C₆, dialcoxyalkyle en C₃-C₁₂, dialkylthioalkyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -5(O)ₘR₁, -Q, -ZQ₁,
ou il représente un groupe hétérocyclique choisi parmi pyridyle, pyrimidyle, quinolinyle, pyrazolyle, thiazolyle, oxazolyle, thiényle, furyle, 4,4-dioxyde-2,3-dihydro-1,4-benzoxathiin-7-yle,
lesdits groupes éventuellement substitués par un ou plusieurs substituants choisis parmi halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, alkylthioalcoxyle en C₂-C₆, haloalkylthioalcoxyle en C₂-C₆, dialcoxyalkyle en C₃-C₁₂, dialkylthioalkyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -S(O)ₘR₁,
- B représente un groupe D-(Rₓ)ₙ ;
- R représente un groupe haloalkyle en C₁-C₆ linéaire ou ramifié ; un groupe cycloalkyle en C₃-C₆ ou cycloalkylalkyle en C₄-C₁₂ éventuellement substitué par des atomes d'halogène ou alkyle en C₁-C₆ ;
- R₁ représente un groupe alkyle en C₁-C₆ ou un groupe haloalkyle en C₁-C₆,
- m est égal à 0, 1 ou 2 ;
- t est égal à 1 ou 2 ;
- R₂, R₃, R₆, R₇, R₈, R₉, identiques ou différents, représentent un atome d'hydrogène, un groupe alkyle en C₁-C₆ linéaire ou ramifié ;
- R₄, R₅ représentent un atome d'hydrogène, un groupe alkyle en C₁-C₆ linéaire ou ramifié à son tour éventuellement substitué par des atomes d'halogène ;
- Q, Q₁, représentent un groupe aryle, un groupe cycloalkyle en C₃-C₆, un groupe hétérocyclique choisi parmi pyrazolyle, tétrazolyle, tétrazolonyle, isoxazolyle, oxazolyle, thiazolyle, oxadiazolyle, thiadiazolyle, isothiazolyle, isoxazolinyle, 1,3-dioxolanyle, tétrahydropyranyle, oxétanyle, oxyranyle, thiazolidinyle, oxazolidinyle,
lesdits groupes éventuellement substitués par un ou plusieurs substituants choisis parmi halogène, NO₂, OH, CN, CHO, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié ;
- Z = O, S(O)ᵣ ;
- r est égal à 0, 1 ou 2 ;
- D représente :
un groupe hétérocyclique monocyclique qui peut être relié au reste de la structure par un de ses atomes de carbone ou, si possible, par un de ses atomes d'azote ;
- Rₓ représente un substituant choisi parmi hydrogène, halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, haloalkylthioalcoxyle en C₂-C₆, dialcoxyalkyle en C₃-C₁₂, dialkylthioalkyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -S(O)ₘR₁, -OS(O)ₜR₁, -SO₂NR₂R₃, -CO₂R₄, -COR₅, - CONR₆R₇, -CSNR₈R₉ ; -Q ;
si plus d'un groupe Rₓ est présent, ils peuvent être identiques ou différents ;
- n = 1-9.

2. Les dérivés suivant la revendication 1, **caractérisés en ce que** les composés ayant la formule (I) sont présents sous forme tautomère, purs ou sous forme de mélanges de formes tautomères, dans n'importe quelle proportion.

3. Utilisation des dérivés de 1,3-diones ayant la formule générale (I') : 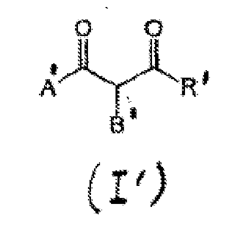 où :
- A' représente :
un groupe aryle éventuellement substitué par un ou plusieurs substituants choisis parmi halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, alkylthioalcoxyle en C₂-C₆, haloalkylthioalcoxyle en C₂-C₆, dialcoxyalkyle en C₃-C₁₂, dialkylthioakyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -S(O)ₘR₁, -OS(O)ₜR₁, -SO₂NR₂R₃, -CO₂R₄, -COR₅, - CONR₆R₇, -CSNR₈R₉, -Q, -ZQ₁ ;
ou représente un groupe hétérocyclique choisi parmi pyridyle, pyrimidyle, quinolinyle, pyrazolyle, thiazolyle, oxazolyle, thiényle, furyle, 4,4-dioxyde-2,3-dihydro-1,4-benzoxathiin-7-yle,
tous ces groupes éventuellement substitués par un ou plusieurs substituants choisis parmi halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, alkylthioalcoxyle en C₂-C₆, haloalkylthioalcoxyle en C₂-C₆, dialcoxyalkyle en C₃-C₁₂, dialkylthioalkyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -S(O)ₘR₁,
- B' représente un groupe D'-(Rₓ)ₙ ;
- R' représente un atome d'hydrogène ; un groupe alkyle en C₁-C₆ linéaire ou ramifié ; un groupe haloalkyle en C₁-C₆ linéaire ou ramifié ; un groupe cycloalkyle en C₃-C₆ ou un groupe cycloalkylalkyle en C₄-C₁₂ éventuellement substitué par des atomes d'halogène ou alkyle en C₁-C₆ ;
- R¹ représente un groupe alkyle en C₁-C₆ ou un groupe haloalkyle en C₁-C₆ ;
- m est égal à 0, 1 ou 2 ;
- t est égal à 1 ou 2 ;
- R₂, R₃, R₆, R₇, R₈, R₉, identiques ou différents, représentent un atome d'hydrogène, un groupe alkyle en C₁-C₆ linéaire ou ramifié ;
- R₄, R₅ représentent un atome d'hydrogène, un groupe alkyle en C₁-C₆ linéaire ou ramifié à son tour éventuellement substitué par des atomes d'halogène ;
- Q, Q₁, représentent un groupe aryle, un groupe cycloalkyle en C₃-C₆, un groupe hétérocyclique choisi parmi pyrazolyle, tétrazolyle, tétrazolonyle, isoxazolyle, oxazolyle, thiazolyle, oxadiazolyle, thiadiazolyle, isothiazolyle, isoxazolinyle, 1,3-dioxolanyle, tétrahydropyranyle, oxétanyle, oxyranyle, thiazolidinyle, oxazolidinyle,
lesdits groupes éventuellement substitués par un ou plusieurs substituants choisis parmi halogène, NO₂, CN, CHO, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié ;
- Z = O, S(O)ᵣ ;
- r est égal à 0, 1 ou 2 ;
- D' représente :
un groupe hétérocyclique qui peut être mono ou polycyclique et peut être relié au reste de la structure par un de ses atomes de carbone ou, si possible, par un de ses atomes d'azote ;
- Rₓ représente un substituant choisi parmi hydrogène, halogène, NO₂, CN, CHO, OH, alkyle en C₁-C₆ linéaire ou ramifié, haloalkyle en C₁-C₆ linéaire ou ramifié, alcoxyle en C₁-C₆ linéaire ou ramifié, haloalcoxyle en C₁-C₆ linéaire ou ramifié, cyanoalkyle en C₁-C₆, alcoxyalkyle en C₂-C₆, alkylthioalkyle en C₂-C₆, alkylsulfinylalkyle en C₂-C₆, alkylsulfonylalkyle en C₂-C₆, haloalcoxyalkyle en C₂-C₆, haloalkylthioalkyle en C₂-C₆, haloalkylsulfinylalkyle en C₂-C₆, haloalkylsulfonylalkyle en C₂-C₆, alcoxyalcoxyle en C₂-C₆ ou haloalcoxyalcoxyle en C₂-C₆, alkylthioalcoxyle en C₂-C₆, haloalkylthioalcoxyle en C₂-C₆, dialcoxyalkyle en C₃-C₁₂, dialkylthioalkyle en C₃-C₁₂, dialkylthioalcoxyle en C₃-C₁₂, dialcoxyalcoxyle en C₃-C₁₂, haloalcoxyhaloalcoxyle en C₂-C₆, alcoxyalcoxyalkyle en C₃-C₁₀, -S(O)ₘR₁, -OS(O)ₜR₁, - SO₂NR₂R₃, -CO₂R₄, -COR₅, -CONR₆R₇, -CSNR₈R₉, Q ;
si plus d'un groupe Rₓ est présent, ceux-ci peuvent être identiques ou différents ;
- n = 1-9 ;
et de leurs sels agronomiquement compatibles, comme herbicides.

4. Utilisation suivant la revendication 3, pour le contrôle de prélevée e de post-levée des mauvaises herbes monocotylédones ou dicotylédones.

5. Un procédé pour le contrôle des mauvaises herbes dans les cultures agricoles, par l'application des composés ayant la formule générale (I') : 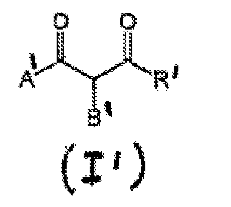 où :
- A', B' et R' ont les significations suivant la revendication 3.

6. Le procédé suivant la revendication 5, **caractérisé en ce que** la quantité de composé ayant la formule (I') à appliquer va de 1 g à 4 000 g par hectare.

7. Compositions herbicides contenant, comme principe actif, un ou plusieurs composés ayant la formule générale (I) : 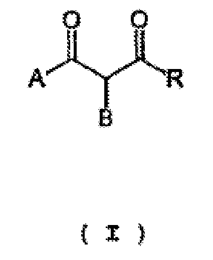 où :
- A, B et R ont les significations suivant la revendication 1, éventuellement aussi sous forme d'un mélange de tautomères.

8. Les compositions herbicides suivant la revendication 7, comprenant un ou plusieurs composés ayant la formule générale (I') tels que définis dans la revendication 3 et un ou plusieurs composés supplémentaires choisis parmi un herbicide, fongicide, insecticide, acaricide, engrais.

9. Les compositions herbicides suivant la revendication 8, **caractérisé en ce que** les herbicides supplémentaires sont choisies parmi :
acétochlore, acifluorfène, aclonifen, AKH-7088, alachlore, alloxydime, amétryne, amicarbazone, amidosulfuron, amitrole, anilofos, asulame, atrazine, azafenidine, azimsulfuron, aziprotryne, BAS 670 H, BAY MKH 6561, beflubutamide, benazoline, benfluraline, benfuresate, bensulfuron, bensulide, bentazone, benzfendizone, benzobicyclon, benzofenap, benzthiazuron, bifenox, bilanafos, bispyribac sodium, bromacil, bromobutide, bromophenoxyme, bromoxynil, butachlore, butafenacil, butamifos, butenachlor, butraline, butroxydim, butilate, cafenstrole, carbetamide, carfentrazone-éthyle, chlométhoxyfene, chlorambene, chlorbromuron, chlorbufame, chlorflurenol, chloridazone, chlorimuron, chlornitrofene, chlorotoluron, chloroxuron, chlorprophame, chlorsulfuron, chlorthal, chlorthiamide, cinidon-éthyl, cimmethyline, cinosulfuron, clethodim, clodinafop, clomazone, clomeprop, clopyralid, cloransulam-méthyl, cumyluron (JC-940), cyanazine, cycloate, cyclosulfamuron, cycloxydime, cyhalo-fop-butyle, 2,4-D, 2,4-DB, daimuron, dalapon, desmediphame, desmétryne, dicamba, dichlobenil, dichlorprop, dichlorprop-P, diclofop, diclosulam, diéthatyl, difenoxuron, difenzoquat, diflufenican, diflufenzopyr, dimefuron, dimepiperate, dimethachlore, dimethamétryne, dimethenamid, dinitramine, dinosèbe, acétate de dinosèbe, dinoterbe, difenamide, dipropetryn, diquat, dithiopyr, 1-diuron, eglinazine, endothal, EPTC, esprocarb, ethalfluraline, ethametsulfuron-méthyl, ethidimuron, ethiozin (SMY 1500), ethofumesate, ethoxyfen-éthyle (HC-252), ethoxysulfuron, etobenzanid (HW 52), fenoxaprop, fenoxaprop-P, fentrazamide, fenuron, flamprop, flamprop-M, flazasulfuron, florasulam, fluazifop, fluazifop-P, fluazolate (JV 485), flucarbazone-sodium, fluchloraline, flufenacet, flufenpyr-ethyl, flumetsulam, flumiclorac-pentyl, flumioxazine, flumipropin, fluometuron, fluoroglycophène, fluoronitrofene, flupoxam, flupropanate, flupyrsulfuron, flurenol, fluridone, flurochloridone, fluroxypyr, flurtamone, fluthiacet-methyl, fomesafen, foramsulfuron, fosamine, furyloxyfen, glufosinate, glyphosate, halosulfuron-methyl, haloxyfop, haloxyfop-P-méthyle, hexazinone, imazamethabenz, imazamox, imazapic, imazapyr, imazaquine, imazethapyr, imazosulfuron, indanofan, iodosulfuron, ioxynil, isopropaline, isoproturon, isouron, isoxaben, isoxachlortole, isoxaflutole, isoxapyrifop, KPP-421, lactofen, lenacile, linuron, LS830556, MCPA, MCPA-thioethyl, MCPB, mecoprop, mecoprop-P, mefenacet, mesosulfuron, mesotrione, metamitron, metazachlor, methabenzthiazuron, methazole, methoprotryne, methyldymron, metobenzuron, metobromuron, metolachlore, S-metolachlore, metosulam, metoxuron, metribuzine, metsulfuron, molinate, monalide, monolinuron, naproanilide, napropamide, naptalame, NC-330, neburon, nicosulfuron, nipyraclofen, norflurazon, orbencarb, oryzalin, oxadiargyl, oxadiazone, oxasulfuron, oxaziclomefone, oxyfluorfene, paraquat, pebulate, pendimethaline, phenoxsulam, pentanochlore, pentoxazone, pethoxamid, phenmediphame, piclorame, picolinafen, piperophos, pretilachlore, primisulfuron, prodiamine, profluazol, proglinazine, prometone, prometryne, propachlore, propanil, propaquizafop, propazine, prophame, propisochlore, propyzamide, prosulfocarbe, prosulfuron, pyraclonil, pyraflufen-éthyl, pyrazogyl (HAS-961), pyrazolynate, pyrazosulfuron, pyrazoxyfène, pyribenzoxim, pyributicarb, pyridafol, pyridate, pyriftalid, pyriminobac-methyl, pyrithiobac-sodium, quinclorac, quinmerac, quizalofop, quizalofop-P, rimsulfuron, séthoxydime, siduron, simazine, simetryne, sulcotrione, sulfentrazone, sulfometuron-methyl, sulfosulfuron, 2,3,6-TBA, TCA-sodium, tebutame, tebuthiuron, tepraloxydim, terbacile, terbuméton, terbuthylazine, terbutryne, thenylchlor, thiazafluron, thiazopyr, thidiazimin, thifensulfuron-méthyle, thiobencarb, tiocarbazil, tioclorim, tralcoxydime, triallate, triasulfuron, triaziflam, tribenuron, triclopyr, triétazine, trifloxysulfuron, trifluralin, triflusulfuron-méthyle, tritosulfuron, UBI-C4874, vernolate.

10. Les compositions suivant l'une quelconque des revendications 7-9, **caractérisées en ce que** la concentration de la substance active va de 1 à 90 %.
